# EUROPEAN PATENT SPECIFICATION

(11) **EP 1 636 224 B1**
(45) Date of publication and mention of the grant of the patent: **14.07.2010**
(21) Application number: 04753127.2
(22) Date of filing: 20.05.2004
(51) Int. Cl.: C07D 471/00, C07D 519/00, A61K 31/437, A61P 9/10

(54) **PYRROLOPYRIDINE-2-CARBOXYLIC ACID AMIDE INHIBITORS OF GLYCOGEN PHOSHORYLASE**
PYRROLOPYRIDIN-2-CARBONSÄUREN ALS HEMMER DER GLYCOGENPHOSPHORYLASE
AMIDES D'ACIDE PYRROLOPYRIDINE-2-CARBOXYLIQUE EN TANT QU'INHIBITEURS DE LA GLYCOGENE PHOSPHORYLASE

(30) Priority: 21.05.2003 US 472375 P; 08.03.2004 US 551256 P
(43) Date of publication of application: 22.03.2006
(73) Proprietor: Prosidion Limited, Oxford, OX4 6LT (GB)
(72) Inventor: BRADLEY, Stuart, Edward, Oxford OX4 6LT (GB); KRULLE, Thomas, Martin, Oxford OX4 6LT (GB); MURRAY, Peter, John, c/o Patents Department, Oxford OX4 6LT (GB); PROCTER, Martin, James, Oxford OX4 6LT (GB); ROWLEY, Robert, John, Oxford OX4 6LT (GB); SAMBROOK SMITH, Colin, Peter, Oxford OX4 6LT (GB); THOMAS, Gerard, Hugh, Oxford OX4 6LT (GB); SCHOFIELD, Karen, Lesley, Oxford, OX4 6LT (GB)
(74) Representative: Blakey, Alison Jane
(86) International application number: PCT/US2004/016243
(87) International publication number: WO 2004/104001

(56) References cited:
- EP-A- 0 321 192
- EP-A- 0 712 844
- EP-A- 0 846 464
- WO-A-00/44753
- WO-A-02/20530
- WO-A-03/064423
- WO-A-2004/039807
- US-A- 5 952 322
- US-A- 6 107 329
- US-B1- 6 399 601
- WERMUTH ET AL: "The Practise of Medicinal Chemistry" PRACTICE OF MEDICINAL CHEMISTRY, XX, XX, 1996, pages 203-237, XP002190259
- FRESNEDA P M ET AL: "Synthetic studies towards the 2-aminopyrimidine alkaloids variolins and meridianins from marine origin" TETRAHEDRON LETTERS, ELSEVIER SCIENCE PUBLISHERS, AMSTERDAM, NL, vol. 41, no. 24, June 2000 (2000-06), pages 4777-4780, XP004205617 ISSN: 0040-4039
- YAKHONTOV L N ET AL: "ABOUT REACTIVITY OF ISOMERIC AZAINDOLES" TETRAHEDRON LETTERS, ELSEVIER SCIENCE PUBLISHERS, AMSTERDAM, NL, no. 24, 1 May 1969 (1969-05-01), pages 1909-1912, XP000575941 ISSN: 0040-4039
- FRYDMAN B ET AL: "PYRROLES FROM AZAINDOLES. A SYNTHESIS OF PORPHOBILINOGEN AND RELATED PYRROLES" JOURNAL OF THE AMERICAN CHEMICAL SOCIETY, AMERICAN CHEMICAL SOCIETY, WASHINGTON, DC, US, vol. 91, no. 9, 23 April 1969 (1969-04-23), pages 2338-2342, XP002928165 ISSN: 0002-7863
- MONNET, MARIE ODILE ET AL: "Synthesis of chiral NADH model compounds in the pyrrolo[3,2-b]pyridine series: models with a chiral group on the pyrrole nitrogen or on the carboxamide side chain" TETRAHEDRON , 49(26), 5831-44 CODEN: TETRAB; ISSN: 0040-4020, 1993, XP002303902

## Description

### BACKGROUND OF THE INVENTION

The present invention is directed to pyrrolopyridine-2-carboxylic acid amides. In particular, the present invention is directed to pyrrolopyridine-2-carboxylic acid amides that are inhibitors of glycogen phosphorylase.

Insulin dependent Type I diabetes and non-insulin dependent Type II diabetes continue to present treatment difficulties even though clinically accepted regimens that include diet, exercise, hypoglycemic agents, and insulin are available. Treatment is patient dependent - therefore there is a continuing need for novel hypoglycemic agents, particularly ones that may be better tolerated with fewer adverse effects.

The liver and certain other organs produce glucose - thereby raising the blood sugar level - by breaking down glycogen or by synthesizing glucose from small molecule precursors. The breakdown of glycogen is catalyzed by glycogen phosphorylase enzyme. Accordingly, inhibiting glycogen phosphorylase ("GP") may lower the elevated blood sugar level in diabetic patients.

Similarly, hypertension and its associated pathologies such as, for example, atherosclerosis, lipidemia, hyperlipidemia and hypercholesterolemia have been associated with elevated insulin levels (hyperinsulinemia), which can lead to abnormal blood sugar levels. Furthermore, myocardial ischemia can result. Such maladies may be treated with hypoglycemic agents, including compounds that inhibit glycogen phosphorylase. The cardioprotective effects of glycogen phosphorylase inhibitors, for example following reperfusion injury, has also been described (see, for example, Ross et al., American Journal of Physiology. Heart and Circulatory Physiology, Mar 2004, 286(3), H1177-84). Accordingly, it is accepted that compounds that inhibit glycogen phosphorylase (see, for example, U.S. Patent No. 6,297,269) are useful in the treatment of diabetes, hyperglycemia, hypercholesterolemia, hyperinsulinemia, hyperlipidemia, atherosclerosis or myocardial ischemia. Nevertheless, it would be desirable to obtain other novel compounds that inhibit glycogen phosphorylase.

R. Kurukulasuriya, J.T. Link, et al., Current Medicinal Chem., 10:99-121(2003) describes "Prospects for Pharmacologic Inhibition of Hepatic Glucose Production." R. Kurukulasuriya, J.T. Link, et al., Current Medicinal Chem., 10:123-153(2003) describes "Potential Drug Targets and Progress Towards Pharmacologic Inhibition of Hepatic Glucose Production."

U.S. Patent No. 6,297,269 and European Patent Application No. EP 0832066 describe substituted *N*-(indole-2-carbonyl)amides and derivatives as glycogen phosphorylase inhibitors. U.S. Patent Nos. 6,107,329 and 6,277,877 describe substituted *N*-(indole-2-carbonyl)glycinamides and derivatives as glycogen phosphorylase inhibitors. U.S. Patent No. 6,399,601 describes bicyclic pyrrolyl amides as glycogen phosphorylase inhibitors. European Patent Application Nos. EP 0978276 and EP 1136071 describe inhibitors of human glycogen phosphorylase and their use. International Patent Publication No. WO 01/68055 describes glycogen phosphorylase inhibitors. U.S. Patent No. 5,952,322 describes a method of reducing non-cardiac ischemial tissue damage using glycogen phosphorylase inhibitors.

International Patent Publication No. WO 01/55146 describes arylamidines. International Patent Publication No. WO 01/62775 describes antiarrhythmic peptides. International Patent Publication No. WO 01/96346 describes tricyclic compounds. International Patent Publication No. WO 02/16314 describes substituted polyamine compounds. International Patent Publication No. WO 02/20475 describes serine protease activity inhibitors. International Patent Publication No. WO 02/40469 describes bombesin receptor antagonists. International Patent Publication No. WO 02/46159 describes guanidine and amidine derivatives. International Patent Publication No. WO 00/69815 describes ureido-substituted cyclic amine derivatives.

International Patent Publication No. WO 00/43384 describes aromatic heterocyclic compounds. International Patent Publication Nos. WO 02/26697 and WO 00/76970 describe aromatic derivatives. International Patent Publication No. WO 01/32622 describes indoles. European Patent Application No. EP 1101759 describes phenylazole compounds. European Patent Application No. EP 1179341 describes cyclic amino compounds. U.S. Patent No. 6,037,325 describes substituted heterocyclic compounds. U.S. Patent No. 5,672,582 describes 4-substituted cyclohexylamine derivatives. European Patent Application No. EP 1201239 describes cyclic amine CCR3 antagonists. International Patent Publication No. WO 98/25617 describes substituted aryl piperazines. U.S. Patent No. 5,756,810 describes preparing 3-nitrobenzoate compounds.

U.S. Patent No. 5,710,153 describes tetrazole compounds. U.S. Patent Nos. 6,174,887 and 6,420,561 describe amide compounds. S.P. Hiremath et al., Acta Ciencia Indica, XVIII:397(1992) describes the synthesis and biological activities of indolylthiosemicarbazides and semicarbazides. International Patent Publication No. WO 96/36595 describes 3,4-disubstituted phenylsulfonamides. U.S. Patent No. 5,618,825 describes combinatorial sulfonamide libraries. European Patent Application No. EP 0810221 describes oxygen-containing heterocyclic derivatives. European Patent Application No. EP 0345990 describes polypeptide compounds. European Patent Application No. EP 0254545 describes diamine compounds.

International Patent Publication No. WO 97/31016 describes inhibitors of SH2-mediated processes. U.S. Patent No. 6,034,067 describes serine protease inhibitors. International Patent Publication No. WO 97/17985 and U.S. Patent No. 6,107,309 describe hemoregulatory compounds. U.S. Patent No. 6,432,921 describes thrombin inhibitors. U.K. Patent Application No. GB 2292149 describes peptide inhibitors of pro-interleukin-1β converting enzyme. U.S. Patent No. 5,821,241 describes fibrinogen receptor antagonists.

International Patent Publication No. WO 01/02424 describes peptide boronic acid compounds. U.S. Patent Nos. 6,001,811, 5,869,455 and 5,618,792 describe oxadiazole, thiadiazole and triazole peptoids. U.S. Patent Nos. 5,885,967, 6,090,787 and 6,124,277 describe thrombin inhibiting peptide derivatives. U.S. Patent No. 6,455,529 describes adhesion receptor antagonists. U.S. Patent No. 6,410,684 describes serine protease inhibitors.

International Patent Publication No. WO 01/94310 describes bis-heterocyclic alkaloids. U.S. Patent Publication No. 20030004162A1, European Patent Application No. EP 0846464, and International Publication No. WO 96/39384 describe glycogen phosphorylase inhibitors. International Patent Publication No. WO 97/28798 describes pyrrolidine derivatives. U.S. Patent No. 5,346,907 describes amino acid analogs.

### SUMMARY OF THE INVENTION

Compounds represented by Formula (I): 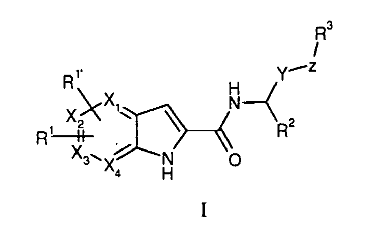 or stereoisomers or pharmaceutically acceptable salts thereof, are inhibitors of glycogen phosphorylase and are useful in the prophylactic or therapeutic treatment of diabetes, hyperglycemia, hypercholesterolemia, hyperinsulinemia, hyperlipidemia, hypertension, atherosclerosis or tissue ischemia e.g. myocardial ischemia, and as cardioprotectants.

### DETAILED DESCRIPTION OF THE INVENTION

The present invention is directed to a compound of Formula (I): 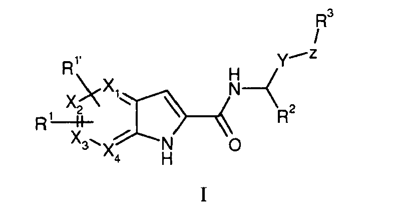 or a stereoisomer, or a pharmaceutically acceptable salt thereof, wherein:
one of X₁, X₂, X₃ and X₄ must be N and the others must be C;
R¹ and R^{1'} are each independently, hydrogen, halogen, hydroxy, cyano, C₁-₄alkyl, C₁₋₄alkoxy, fluoromethyl, difluoromethyl, trifluoromethyl, ethenyl, or ethynyl;
R² is hydrogen, C₁₋₄alkyl, COOR⁶, COR⁶, C₁₋₄alkoxyC₁₋₄alkyl-, hydroxyC₁-₄alkyl-, cycloalkyl, cycloalkylC₁₋₄alkyl-, aryl, arylC₁₋₄alkyl-, hetaryl, or hetarylC₁-₄alkyl-, wherein any of the aryl or hetaryl rings are optionally substituted with 1-2 independent halogen, cyano, C₁₋₄alkyl, C₁₋₄alkoxy, -NR¹³R¹⁴, -SO₂C₁₋₄alkyl, -SO₂NR¹³R¹⁴, hydroxy, fluoromethyl, difluoromethyl, or trifluoromethyl substituents;
Y is a direct bond, C₁₋₂alkyl, or -CH(OH)-;
Z is CH₂, -C(O)-, -O-, >NH, >N(C₁₋₄alkyl), >N(C₃₋₆Cycloalkyl), or absent; but when Y is -CH(OH)-, Z or R³ must be bonded to Y through a carbon-carbon bond;
R³ is hydrogen, -COOH, -COOC₁₋₄alkyl, C₁₋₄alkoxy, C₁₋₄alkyl, arylC₁₋₄-alkylthio-, aryl, -C₁₋₄alkylaryl, hetaryl, -C₁₋₄alkylhetaryl, cycloalkyl, -C₁₋₄-alkylcycloalkyl, heterocyclyl or -C₁₋₄alkylheterocyclyl, wherein any of the rings is optionally substituted with 1-3 independent halogen, cyano, C₁₋₄alkyl, fluoromethyl, difluoromethyl, trifluoromethyl, -NHC(O)O(C₁₋₄alkyl), -C₁₋₄alkylNHC(O)O(C₁₋₄-alkyl), -NR⁷R⁸, -C₁₋₄alkylNR⁷R⁸, -C(O)R⁹, C₁₋₄alkoxy-, C₁₋₄alkoxyC₁₋₄alkyl-, COOH, -COOC₁₋₄alkyl, -NHC(O)R⁹, -C₁₋₄alkylNHC(O)R⁹, -C(O)N(R¹⁰)₂, -C₁₋₄-alkylC(O)N(R¹⁰)₂, -C₁₋₄alkoxyC₁₋₄alkoxy, hydroxy, hydroxyC₁₋₄alkyl-, -NHSO₂R¹⁰, -SO₂(C₁₋₄alkyl), -SO₂NR¹¹R¹², 5- to 6-membered heterocyclyl, phenyl, phenylC1_ ₂alkoxy, or phenylC₁₋₂alkyl substituents, wherein phenyl is optionally substituted with 1-2 independent halogen, cyano, C₁₋₄alkyl, C₁₋₄alkoxy, -NR¹³R¹⁴, -SO₂C₁₋₄alkyl, - SO₂NR¹³R¹⁴, hydroxy, fluoromethyl, difluoromethyl or trifluoromethyl substituents, or two bonds on a ring carbon of the heterocyclyl group optionally can form an oxo ( =O) substituent;
or R³ is -NR⁴R⁵ or -NR⁴(-C₁₋₄alkylR⁵);
R⁴ is hydrogen, C₁₋₃alkyl, -C₂₋₃alkyl-NR⁷R⁸, C₃₋₆Cycloalkyl optionally substituted by hydroxy or hydroxyC₁₋₄alkyl- further optionally substituted by hydroxy, C₁₋₂alkoxyC₂₋₄alkyr-, or C₁₋₂alkyl-S(O)ₙ-C₂₋₃alkyl-;
n is 0, 1, or 2;
R⁵ is hydrogen, hydroxyC₂₋₃alkyl-, C₁₋₂alkoxy, C₁₋₂alkoxyC₁₋₄alkyl-, or aryl, hetaryl, or heterocyclyl;
wherein a heterocyclic nitrogen-containing R⁵ ring optionally is mono-substituted on the ring nitrogen with C₁₋₄alkyl, benzyl, benzoyl, C₁₋₄alkyl-C(O)-, -SO₂C₁₋₄alkyl, -SO₂NR¹³R¹⁴, C₁₋₄alkoxycarbonyl, or aryl(C₁₋₄alkoxy)carbonyl; and wherein the R⁵ rings are optionally mono-substituted on a ring carbon with halogen, cyano, C₁₋₄alkyl-C(O)-, C₁₋₄alkyl-SO₂-, C₁₋₄alkyl, C₁₋₄alkoxy, hydroxy, -NR¹³R¹⁴, hydroxy, hydroxyC₁₋₄alkyl-, carbamoyl, or C₁₋₄alkylcarbamoyl-, provided that no quaternised nitrogen is included; or two bonds on a ring carbon of the heterocyclyl group optionally can form an oxo ( =O) substituent;
R⁶ is C₁₋₄alkyl, aryl, or hetaryl;
R⁷ and R⁸ are independently hydrogen, C₁₋₄alkyl, C₃₋₆cycloalkyl, or CO(C₁₋₄-alkyl);
R⁹ is C₁₋₄alkyl, or C₃₋₆cycloalkyl;
R¹⁰ is hydrogen, C₁₋₄alkyl, or C₃₋₆cycloalkyl;
R¹¹ and R¹² are independently hydrogen or C₁₋₄alkyl or together with the nitrogen to which they are attached may form a 4- to 6-membered heterocycle;
R¹³ and R¹⁴ are independently hydrogen or C₁₋₄alkyl; and
wherein there are no nitrogen-oxygen, nitrogen-nitrogen or nitrogen-halogen bonds in linking the three components -Y-Z-R³ to each other;
provided that the compound is not 2R,4S,5S-6-cyclohexyl-5-(6'-azaindol-2'-ylcarbonylamino)-2-(2'-methylpropyl)-gamma-hexanolactone.

The molecular weight of the compounds of Formula (I) is preferably less than 800, more preferably less than 600.

In the first aspect, the present invention is directed to a compound represented by Formula (I), or a stereoisomer, or a pharmaceutically acceptable salt thereof, wherein X₁ is N, and the other variables are as defined above for Formula (I).

In an embodiment of the first aspect, the present invention is directed to a compound represented by Formula (I), or a stereoisomer, or a pharmaceutically acceptable salt thereof, wherein X₁ is N, Y is a direct bond or C₁₋₂alkyl, and Z is - C(O)-, and the other variables are as defined above for Formula (I).

In another embodiment of the first aspect, the present invention is directed to a compound represented by Formula (I), or a stereoisomer, or a pharmaceutically acceptable salt thereof, wherein X₁ is N, Y is a direct bond or C₁₋₂alkyl, and Z is -O-, and the other variables are as defined above for Formula (I).

In yet another embodiment of the first aspect, the present invention is directed to a compound represented by Formula (I), or a stereoisomer, or a pharmaceutically acceptable salt thereof, wherein X₁ is N, Y is a direct bond or C₁₋₂alkyl, and Z is >NH or >N(C₁₋₄alkyl), and the other variables are as defined above for Formula (I).

In a second aspect, the present invention is directed to a compound represented by Formula (I), or a stereoisomer, or a pharmaceutically acceptable salt thereof, wherein X₂ is N, and the other variables are as defined above for Formula (I).

In an embodiment of the second aspect, the present invention is directed to a compound represented by Formula (I), or a stereoisomer, or a pharmaceutically acceptable salt thereof, wherein X₂ is N, Y is a direct bond or C₁₋₂alkyl, and Z is - C(O)-, and the other variables are as defined above for Formula (I).

In a third aspect, the present invention is directed to a compound represented by Formula (I), or a stereoisomer, or a pharmaceutically acceptable salt thereof, wherein X₃ is N, and the other variables are as defined above for Formula (I).

In an embodiment of the third aspect, the present invention is directed to a compound represented by Formula (I), or a stereoisomer, or a pharmaceutically acceptable salt thereof, wherein X₃ is N, Y is a direct bond or C₁₋₂alkyl, and Z is - C(O)-, and the other variables are as defined above for Formula (I).

In another embodiment of the third aspect, the present invention is directed to a compound represented by Formula (I), or a stereoisomer, or a pharmaceutically acceptable salt thereof, wherein X₃ is N, Y is -CH(OH)-, and Z is -C(O)-, and the other variables are as defined above for Formula (I).

In yet another embodiment of the third aspect, the present invention is directed to a compound represented by Formula (I), or a stereoisomer, or a pharmaceutically acceptable salt thereof, wherein X₃ is N, Y is a direct bond or C₁₋₂alkyl, and Z is -O-, and the other variables are as defined above for Formula (I).

In still another embodiment of the third aspect, the present invention is directed to a compound represented by Formula (I), or a stereoisomer, or a pharmaceutically acceptable salt thereof, wherein X₃ is N, Y is a direct bond or C₁₋₂-alkyl, and Z is absent, and the other variables are as defined above for Formula (I).

In yet still another embodiment of the third aspect, the present invention is directed to a compound represented by Formula (I), or a stereoisomer, or a pharmaceutically acceptable salt thereof, wherein X₃ is N, Y is a direct bond or C₁₋₂-alkyl, and Z is >NH or >N(C₁₋₄alkyl), and the other variables are as defined above for Formula (I).

In a fourth aspect, the present invention is directed to a compound represented by Formula (I), or a stereoisomer, or a pharmaceutically acceptable salt thereof, wherein X₄ is N, and the other variables are as defined above for Formula (I).

In an embodiment of the fourth aspect, the present invention is directed to a compound represented by Formula (I), or a stereoisomer, or a pharmaceutically acceptable salt thereof, wherein X₄ is N, Y is -CH(OH)-, and Z is -C(O)-, and the other variables are as defined above for Formula (I).

When Y is a direct bond then Z is preferably other than -O-, >NH, >N(C₁₋₄-alkyl) or >N(C₃₋₆cycloalkyl).

Preferably X₃ is N.

Preferably R¹ and R¹ are each independently, halogen, cyano, hydrogen, methyl, methoxy, or ethynyl. More preferably R¹ and R¹ are each independently, halogen, cyano, or hydrogen.

Preferably at least one of R' and R¹ is hydrogen. More preferably one of R¹ and R¹ is hydrogen.

A preferred group of compounds are those where X₃ is N, one of R¹ and R¹ is hydrogen and the other is a 5-halo or 5-cyano group.

Preferably Y is a direct bond or C₁₋₂alkyl, more preferably Y is a direct bond.

Preferably Z is -C(O)-.

A preferred group of compounds are those wherein
X₃ is N;
Y is a direct bond or C₁₋₂alkyl; and
Z is -C(O)-.

Preferably R² is hydrogen, C₁₋₄alkyl, aryl or arylC₁₋₄alkyl-, wherein the aryl ring is optionally substituted with 1-2 independent halogen, cyano, C₁₋₄alkyl, C₁₋₄-alkoxy, -NR¹³R¹⁴, -SO₂C₁₋₄alkyl, -SO₂NR¹³R¹⁴, hydroxy, fluoromethyl, difluoromethyl, or trifluoromethyl substituents. More preferably R² is benzyl optionally substituted with 1-2 halogen substituents. A particular R² substituent which may be mentioned is -(*S*)-(4-fluorobenzyl).

Preferably R³ is heterocyclyl or -C₁₄alkylheterocyclyl optionally substituted with 1-3 independent halogen, cyano, C₁₋₄alkyl, fluoromethyl, difluoromethyl, trifluoromethyl, -NHC(O)O(C₁₋₄alkyl), -C₁₋₄alkylNHC(O)O(C₁₋₄alkyl), -NR⁷R⁸, -C₁₋₄-alkylNR⁷R⁸, -C(O)R⁹, C₁₋₄alkoxy, C₁₋₄alkoxyC₁₋₄alkyl-, -OOH, -COOC₁₋₄alkyl, - NHC(O)R⁹, -C₁₋₄alkylNHC(O)R⁹, -C(O)N(R¹⁰)₂, -C₁₋₄alkylC(O)N(R¹⁰)2, -C₁₋₄-alkoxyC₁₋₄alkoxy, hydroxy, hydroxyC₁₋₄alkyl-, -NHSO₂R¹⁰, -SO₂(C₁₋₄alkyl),-SO₂NR¹¹R¹², 5- to 6-membered heterocyclyl, phenyl, phenylC₁₋₂alkoxy, or phenylC₁₋₂-alkyl substituents, wherein phenyl is optionally substituted with 1-2 independent halogen, cyano, C₁₋₄alkyl , C₁₋₄alkoxy, -NR¹³R¹⁴, -SO₂C₁₋₄alkyl, -SO₂NR¹³R¹⁴, hydroxy, fluoromethyl, difluoromethyl, or trifluoromethyl substituents, or two bonds on a ring carbon of the heterocyclyl group optionally can form an oxo (=O) substituent; or R³ is -NR⁴R⁵ or -NR⁴-C₁₋₄alkylR⁵).

More preferably R³ is a nitrogen containing heterocyclyl group, especially a 4-8-membered nitrogen containing heterocyclyl group, linked to Z via a ring nitrogen atom, optionally substituted with 1-3 independent halogen, cyano, C₁₋₄alkyl, fluoromethyl, difluoromethyl, trifluoromethyl, -NHC(O)O(C₁₋₄alkyl), -C₁₋₄-alkylNHC(O)O(C₁₋₄alkyl), -NR⁷R⁸, -C₁₋₄alkylNR⁷R⁸, -C(O)R⁹, C₁₋₄alkoxy, C₁₋₄-alkoxyC₁₋₄alkyl-, -COOH, -COOC₁₋₄alkyl, -NHC(O)R⁹, -C₁₋₄alkylNHC(O)R⁹,-C(O)N(R¹⁰)₂, -C₁₋₄alkylC(O)N(R¹⁰)₂, -C₁₋₄alkoxyC₁₋₄alkoxy, hydroxy, hydroxyC₁₋₄-alkyl-, -NHSO₂R¹⁰, -SO₂(C₁₋₄alkyl), -SO₂NR¹¹R¹², 5- to 6-membered heterocyclyl, phenyl, phenylC₁₋₂alkoxy, or phenylC₁₋₂alkyl substituents, wherein phenyl is optionally substituted with 1-2 independent halogen, cyano, C₁₋₄alkyl, C₁₋₄alkoxy,-NR¹³R¹⁴, -SO₂C₁₋₄alkyl, -SO₂NR¹³R¹⁴, hydroxy, fluoromethyl, difluoromethyl, or trifluoromethyl substituents, or two bonds on a ring carbon of the heterocyclyl group optionally can form an oxo ( =O) substituent; or R³ is -NR⁴R⁵ or -NR⁴(-C₁₋₄alkylR⁵)

Examples of nitrogen containing heterocyclyl groups which R³ may represent include azetidin-1-yl, pyrrolidin-1-yl, piperidin-1-yl, 1,4-diazapan-1-yl, piperazin-1-yl, morpholin-4-yl, thiomorpholin-4-yl, 1,1-dioxo-thiomorpholin-4-yl, or thiazolidin-3-yl; which groups may be optionally substituted as described above

Preferred substituent groups for R³ include -C₁₋₄alkoxy, hydroxy and oxo.

Even more preferably R³ is pyrrolidin-1-yl or piperidin-1-yl optionally substituted with hydroxy, e.g. 4-hydroxypiperidin-1-yl and 3-(*S*)-hydroxypyrrolidin-1-yl.

Specific compounds of the invention which may be mentioned are those included in the examples, in particular 5-chloro-1*H*-pyrrolo[2,3-c]pyridine-2-carboxylic acid [1-(*S*)-(4-fluorobenzyl)-2-(4-hydroxypiperidin-1-yl)-2-oxoethyl]amide and 5-chloro-1*H*-pyrrolo [2,3-c] pyridine-2-carboxylic acid [1-(*S*)-(4-fluorobenzyl)-2-(3-(*S*)-hydroxypyrrolidin-1-yl)-2-oxoethyl]amide.

A particular group of compounds which may be mentioned are those represented by Formula (IA): 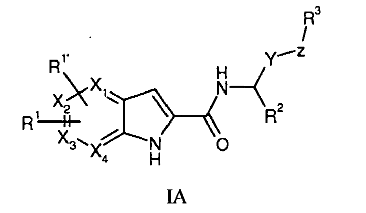 or a stereoisomer, or a pharmaceutically acceptable salt thereof, wherein:
one of X₁, X₂, X₃ and X₄ must be N and the others must be C;
R¹ and R¹ are each independently, hydrogen, halogen, hydroxy, cyano, C₁₋₄-alkyl, C₁₋₄alkoxy, fluoromethyl, difluoromethyl, trifluoromethyl, ethenyl, or ethynyl;
R² is hydrogen, C₁₋₄alkyl, aryl, arylC₁₋₄alkyl-, hetaryl, hetarylC₁₋₄alkyl-, wherein any of the aryl or hetaryl rings are optionally substituted with 1-2 independent halogen, cyano, C₁₋₄alkyl, C₁₋₄alkoxy, -NR¹³R¹⁴, -SO₂C₁₋₄alkyl, -SO₂N R¹¹R¹⁴, hydroxy, fluoromethyl, difluoromethyl, or trifluoromethyl substituents;
Y is a direct bond, C₁₋₂alkyl or -CH(OH)-;
Z is CH₂, -C(O)-, -O-, >NH, >N(C₁₋₄alkyl), >N(C₃₋₆cycloalkyl), or absent; but when Y is -CH(OH)-, Z or R³ must be bonded to Y through a carbon-carbon bond;
R³ is hydrogen, -COOH, -COOC₁₋₄alkyl, C₁₋₄alkoxy, arylC₁₋₄alkylthio-, aryl, -C₁₋₄alkylaryl, hetaryl, -C₁₋₄alkylhetaryl, heterocyclyl, or -C₁₋₄alkylheterocyclyl, wherein any of the rings is optionally substituted with 1-3 independent halogen, cyano, C₁₋₄alkyl, fluoromethyl, difluoromethyl, trifluoromethyl, -NR¹³R¹⁴, -C₁₋₄-alkylNR¹³R¹⁴, -C(O)H, -C(O)(C₁₋₄alkyl), C₁₋₄alkoxy, C₁₋₄alkoxyC₁₋₄alkyl-, -COOH, -COOC₁₋₄alkyl, carbamoyl, C₁₋₄alkylcarbamoyl-, -C₁₋₄alkoxymethoxy, hydroxy, hydroxyC₁₋₄alkyl-, -SO₂)(C₁₋₄alkyl), phenyl or phenylC₁₋₂alkyl substituents, or two bonds on a ring carbon of the heterocyclyl group optionally can form an oxo ( =O ) substituent;
or R³ is -NR⁴R⁵ or -NR⁴(-C₁₋₄alkylR⁵);
R⁴ is hydrogen, C₁₋₃alkyl, -C₂₋₃alkyl-N(C₁₋₃alkyl)(C₁₋₃alkyl), C₃₋₆cycloalkyl, hydroxyC₂₋₃alkyl-, C₁₋₂alkoxyC₂₋₄alkyl-, or C₁₋₂alkyl-S(O)ₙ-C₂₋₃alkyl-;
n is 0, 1, or 2;
R⁵ is hydrogen, hydroxyC₂₋₃alkyl-, C₁₋₂alkoxyC₂₋₄alkyl, or an aryl, hetaryl, or heterocyclyl;
wherein a heterocyclic nitrogen-containing R⁵ ring optionally is mono-substituted on the ring nitrogen with C₁₋₄alkyl, benzyl, benzoyl, C₁₋₄alkyl-C(O)-, -SO₂C₁₋₄alkyl, -SO₂NR¹³R¹⁴, or C₁₋₄alkoxycarbonyl aryl(C₁₋₄alkoxy)carbonyl; and wherein the R⁵ rings are optionally mono-substituted on a ring carbon with halogen, cyano, C₁₋₄alkyl-C(O)-, C₁₋₄alkyl-SO₂-, C₁₋₄alkyl, C₁₋₄alkoxy, -NR¹³R¹⁴, hydroxy, hydroxyC₁₋₄alkyl-, carbamoyl or C₁₋₄alkylcarbamoyl-, provided that no quatemised nitrogen is included; and
wherein there are no nitrogen-oxygen, nitrogen-nitrogen or nitrogen-halogen bonds in linking the three components -Y-Z-R³ to each other.

While the preferred groups for each variable have generally been listed above separately for each variable, preferred compounds of this invention include those in which several or each variable in Formula (I) is selected from the preferred, more preferred, most preferred, especially or particularly listed groups for each variable. Therefore, this invention is intended to include all combinations of preferred, more preferred, most preferred, especially and particularly listed groups.

As used herein, unless stated otherwise, "alkyl" as well as other groups having the prefix "alk" such as, for example, alkoxy, alkanyl, alkenyl, alkynyl, and the like, means carbon chains which may be linear or branched or combinations thereof. Examples of alkyl groups include methyl, ethyl, propyl, isopropyl, butyl, sec- and tert-butyl, pentyl, hexyl, heptyl and the like. "Alkenyl", "alkynyl" and other like terms include carbon chains having at least one unsaturated carbon-carbon bond.

As used herein, for example, "C₁₋₄alkyl" is used to mean an alkyl having 1-4 carbons - that is, 1, 2, 3, or 4 carbons in a straight or branched configuration.

The terms "cycloalkyl" and "carbocyclic ring" mean carbocycles containing no heteroatoms, and include mono-, bi-, and tricyclic saturated carbocycles, as well as fused and bridged systems. Such fused ring systems can include one ring that is partially or fully unsaturated, such as a benzene ring, to form fused ring systems, such as benzofused carbocycles. Cycloalkyl includes such fused ring systems as spirofused ring systems. Examples of cycloalkyl and carbocyclic rings include C₃₋₁₀cycloalkyl groups, particularly C₃₋₈cycloalkyl groups, such as cyclopropyl, cyclobutyl, cyclopentyl, cyclohexyl, and decahydronaphthalene, adamantane, indanyl, 1,2,3,4-tetrahydronaphthalene and the like.

The term "halogen" includes fluorine, chlorine, bromine, and iodine atoms.

The term "carbamoyl" unless specifically described otherwise means -C(O)-NH- or -NH-C(O)-.

The term "aryl" is well known to chemists. The preferred aryl groups are phenyl and naphthyl, more preferably phenyl.

The term "hetaryl" is well known to chemists. The term includes 5- or 6-membered heteroaryl rings containing 1-4 heteroatoms chosen from oxygen, sulfur, and nitrogen in which oxygen and sulfur are not next to each other. Examples of such heteroaryl rings are furyl, thienyl, pyrrolyl, pyrazolyl, imidazolyl, oxazolyl, isoxazolyl, thiazolyl, isothiazolyl, triazolyl, oxadiazolyl, thiadiazolyl, tetrazolyl, pyridyl, pyridazinyl, pyrimidinyl, pyrazinyl, and triazinyl. The term "hetaryl" includes hetaryl rings with fused carbocyclic ring systems that are partially or fully unsaturated, such as a benzene ring, to form a benzofused hetaryl. For example, benzimidazole, benzoxazole, benzothiazole, benzofuran, quinoline, isoquinoline, quinoxaline, and the like.

Unless otherwise stated, the terms "heterocyclic ring", "heterocyclyl" and "heterocycle" are equivalent, and include 4-10-membered, e.g. 4-8-membered, saturated or partially saturated rings containing one or two heteroatoms chosen from oxygen, sulfur, and nitrogen. The sulfur and oxygen heteroatoms are not directly attached to one another. Any nitrogen heteroatoms in the ring may optionally be substituted with C₁₋₄alkyl. Examples of heterocyclic rings include azetidine, oxetane, tetrahydrofuran, tetrahydropyran, oxepane, oxocane, thietane, thiazolidine, oxazolidine, oxazetidine, pyrazolidine, isoxazolidine, isothiazolidine, tetrahydrothiophene, tetrahydrothiopyran, thiepane, thiocane, azetidine, pyrrolidine, piperidine, *N*-methylpiperidine, azepane, 1,4-diazapane, azocane, [1,3]dioxane, oxazolidine, piperazine, homopiperazine, morpholine, thiomorpholine, 1,2,3,6-tetrahydropyridine and the like. Other examples of heterocyclic rings include the oxidized forms of the sulfur-containing rings. Thus, tetrahydrothiophene-1-oxide, tetrahydrothiophene-1, -dioxide, thiomorpholine-1-oxide, thiomorpholine-1,1-dioxide, tetrahydrothiopyran-1-oxide, tetrahydrothiopyran-1,1-dioxide, thiazolidine-1-oxide, and thiazolidine-1,1-dioxide are also considered to be heterocyclic rings. The term "heterocyclic also includes fused ring systems and can include a carbocyclic ring that is partially or fully unsaturated, such as a benzene ring, to form benzofused heterocycles. For example, 3,4-dihydro-1,4-benzodioxine, tetrahydroquinoline, tetrahydroisoquinoline and the like.

Compounds described herein may contain one or more asymmetric centers and may thus give rise to diastereomers and optical isomers. The present invention includes all such possible diastereomers as well as their racemic mixtures, their substantially pure resolved enantiomers, all possible geometric isomers, and pharmaceutically acceptable salts thereof. The above Formula (I) is shown without a definitive stereochemistry at certain positions. The present invention includes all stereoisomers of Formula (I) and pharmaceutically acceptable salts thereof. Further, mixtures of stereoisomers as well as isolated specific stereoisomers are also included. During the course of the synthetic procedures used to prepare such compounds, or in using racemization or epimerization procedures known to those skilled in the art, the products of such procedures can be a mixture of stereoisomers.

When a tautomer of the compound of Formula (I) exists, the present invention includes any possible tautomers and pharmaceutically acceptable salts thereof, and mixtures thereof, except where specifically drawn or stated otherwise.

When the compound of Formula (I) and pharmaceutically acceptable salts thereof exist in the form of solvates or polymorphic forms, the present invention includes any possible solvates and polymorphic forms. A type of a solvent that forms the solvate is not particularly limited so long as the solvent is pharmacologically acceptable. For example, water, ethanol, propanol, acetone or the like can be used.

The invention also encompasses a pharmaceutical composition that is comprised of a compound of Formula (I), or a pharmaceutically acceptable salt thereof, in combination with a pharmaceutically acceptable carrier.

Preferably the composition is comprised of a pharmaceutically acceptable carrier and a non-toxic therapeutically effective amount of a compound of Formula (I), or a pharmaceutically acceptable salt thereof.

Moreover, within this preferred embodiment, the invention encompasses a pharmaceutical composition for the treatment of disease by inhibiting glycogen phosphorylase, resulting in the prophylactic or therapeutic treatment of diabetes, hyperglycemia, hypercholesterolemia, hyperinsulinemia, hyperlipidemia, hypertension, atherosclerosis or tissue ischemia e.g. myocardial ischemia comprising a pharmaceutically acceptable carrier and a non-toxic therapeutically effective amount of compound of Formula (I), or a pharmaceutically acceptable salt thereof.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include arginine, betaine, caffeine, choline, *N'N*'-dibenzylethylenediamine, diethylamine, 2-diethylaminoethanol, 2-dimethylaminoethanol, ethanolamine, ethylenediamine, *N*-ethylmorpholine, *N-*ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Particularly preferred are citric, hydrobromic, hydrochloric, maleic, phosphoric, sulfuric and tartaric acids.

Since the compounds of Formula (I) are intended for pharmaceutical use they are preferably provided in substantially pure form, for example at least 60% pure, more suitably at least 75% pure especially at least 98% pure (% are on a weight for weight basis).

The pharmaceutical compositions of the present invention comprise a compound represented by Formula (I), or a pharmaceutically acceptable salt thereof, as an active ingredient, a pharmaceutically acceptable carrier and optionally other therapeutic ingredients or adjuvants. The compositions include those suitable for oral, rectal, topical, and parenteral (including subcutaneous, intramuscular, and intravenous) administration, although the most suitable route in any given case will depend on the particular host, and nature and severity of the conditions for which the active ingredient is being administered. The compositions are preferably suitable for oral administration. The pharmaceutical compositions may be conveniently presented in unit dosage form and prepared by any of the methods well known in the art of pharmacy.

In practice, the compounds of Formula (I), or pharmaceutically acceptable salts thereof, can be combined as the active ingredient in intimate admixture with a pharmaceutical carrier according to conventional pharmaceutical compounding techniques. The carrier may take a wide variety of forms depending on the form of preparation desired for administration, e.g. oral or parenteral (including intravenous). Thus, the pharmaceutical compositions of the present invention can be presented as discrete units suitable for oral administration such as capsules, sachets or tablets each containing a predetermined amount of the active ingredient. Further, the compositions can be presented as a powder, as granules, as a solution, as a suspension in an aqueous liquid, as a non-aqueous liquid, as an oil-in-water emulsion, or as a water-in-oil liquid emulsion. In addition to the common dosage forms set out above, the compounds of Formula (I), or pharmaceutically acceptable salts thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately admixing the active ingredient with liquid carriers or finely divided solid carriers or both. The product can then be conveniently shaped into the desired presentation.

Thus, the pharmaceutical compositions of this invention may include a pharmaceutically acceptable carrier and a compound of Formula (I) or a pharmaceutically acceptable salt thereof. The compounds of Formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each sachet or capsule preferably contains from about 0.05mg to about 5g of the active ingredient.

For example, a formulation intended for oral administration to humans may contain from about 0.5 mg to about 5g of active agent, compounded with an appropriate and convenient amount of carrier material, which may vary from about 5 to about 95% of the total composition. Unit dosage forms will generally contain from about 1mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g. glycerol, propylene glycol and liquid polyethylene glycol), vegetable oils, and suitable mixtures thereof.

Pharmaceutical compositions of the present invention can be in a form suitable for topical use such as, for example, an aerosol, cream, ointment, lotion, dusting powder, or the like. Further, the compositions can be in a form suitable for use in transdermal devices. These formulations may be prepared, utilizing a compound of Formula (I), or a pharmaceutically acceptable salt thereof, *via* conventional processing methods. As an example, a cream or ointment is prepared by admixing hydrophilic material and water, together with about 5wt% to about 10wt% of the compound, to produce a cream or ointment having a desired consistency.

Pharmaceutical compositions of this invention can be in a form suitable for rectal administration wherein the carrier is a solid. It is preferable that the mixture forms unit dose suppositories. Suitable carriers include cocoa butter and other materials commonly used in the art. The suppositories may be conveniently formed by first admixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of Formula (I), or a pharmaceutically acceptable salt thereof, may also be prepared in powder or liquid concentrate form.

Generally, dosage levels on the order of 0.01mg/kg to about 150mg/kg of body weight per day are useful in the treatment of the above-indicated conditions, or alternatively about 0.5mg to about 7g per patient per day. For example, diabetes and hyperglycemia may be effectively treated by the administration of from about 0.01 to 50mg of the compound per kilogram of body weight per day, or alternatively about 0.5mg to about 3.5g per patient per day. Similarly, hypercholesterolemia, hyperinsulinemia, hyperlipidemia, hypertension, atherosclerosis or tissue ischemia e.g. myocardial ischemia may be effectively treated by the administration of from about 0.01 to 50mg of the compound per kilogram of body weight per day, or alternatively about 0.5mg to about 3.5g per patient per day.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

The compounds of Formula (I) and pharmaceutically acceptable salts thereof, may be used in the treatment of diseases or conditions in which glycogen phosphorylase plays a role.

Thus the invention also provides a compound of Formula (I), or a pharmaceutically acceptable salt thereof, for use in the treatment of a disease or condition in which glycogen phosphorylase plays a role.

Diseases or conditions in which glycogen phosphorylase plays a role include diabetes (including Type I and Type II, impaired glucose tolerance, insulin resistance and diabetic complications such as neuropathy, nephropathy, retinopathy and cataracts), hyperglycemia, hypercholesterolemia, hyperinsulinemia, hyperlipidemia, hypertension, atherosclerosis, tissue ischemia e.g. myocardial ischemia.

The invention also provides a compound of Formula (I), or a pharmaceutically acceptable salt thereof for use in the treatment of hyperglycemia or diabetes.

The invention also provides a compound of Formula (I), or a pharmaceutically acceptable salt thereof, for use in the prevention of diabetes in a human demonstrating pre-diabetic hyperglycemia or impaired glucose tolerance.

The invention also provides a compound of Formula (I), or a pharmaceutically acceptable salt thereof, for use in the treatment of hypercholesterolemia, hyperinsulinemia, hyperlipidemia, hypertension, atherosclerosis or tissue ischemia.

The invention also provides a compound of Formula (I), or pharmaceutically acceptable salt thereof, for use in cardioprotection e.g. following reperfusion injury.

The invention also provides the use of a compound of Formula (I), or a pharmaceutically acceptable salt thereof, in the manufacture of a medicament for the treatment of a condition as defined above.

In the uses of the compounds of the invention the term "treatment" includes both therapeutic and prophylactic treatment.

The compounds of Formula (I), or pharmaceutically acceptable salts thereof, may be administered alone or in combination with one or more other therapeutically active compounds. The other therapeutically active compounds may be for the treatment of the same disease or condition as the compounds of Formula (I) or a different disease or condition. The therapeutically active compounds may be administered simultaneously, sequentially or separately.

The compounds of Formula (I) may be administered with other active compounds for the treatment of diabetes, for example insulin and insulin analogs, sulfonyl ureas and analogs, biguanides, α2 agonists, fatty acid oxidation inhibitors, α-glucosidase inhibitors, β-agonists, phosphodiesterase inhibitors, lipid lowering agents, antiobesity agents, amylin antagonists, lipoxygenase inhibitors, somostatin analogs, glucokinase activators, glucagon antagonists, insulin signalling agonists, PTP1B inhibitors, gluconeogenesis inhibitors, antilypolitic agents, GSK inhibitors, galanin receptor agonists, anorectic agents, CCK receptor agonists, leptin, CRF antagonists or CRF binding proteins.

The compounds of Formula (I) may also be administered in combination with thyromimetic compounds, aldose reductase inhibitors, glucocorticoid receptor antagonists, NHE-1 inhibitors or sorbitol dehydrogenase inhibitors.

The compounds of Formula (I) may exhibit advantageous properties compared to known glycogen phosphorylase inhibitors, for example, the compounds may exhibit improved solubility thus improving absorption properties and bioavailability. Furthermore the compounds of Formula (I) may exhibit further advantageous properties such as reduced inhibition of cytochrome P450 enzymes, meaning that they are less likely to cause adverse drug-drug interactions than known glycogen phosphorylase inhibitors.

In accordance with this invention, the compounds of Formula (I) can be prepared as outlined in **Scheme 1** below wherein R¹, R^{1'}, R², R³, X₁, X₂, X₃, X₄, Y and Z are as defined above for Formula (I):

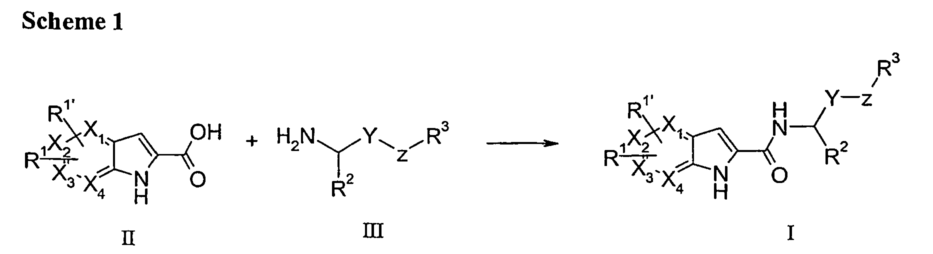

According to **Scheme 1**, the compounds of Formula (I) may be prepared by coupling the appropriate pyrrolopyridine-2-carboxylic acid of Formula (II), or a protected or activated derivative thereof, with the appropriate amine of Formula (III). Compounds of Formula (II) can be obtained by the syntheses described in **Schemes 3** and 5 below. Compounds of Formula (III) are generally commercially available or can be obtained by the syntheses described in **Schemes 8** and **9** below.

Typically, the compound of Formula (II), or a protected or activated derivative thereof, is combined with a compound of Formula (III) in the presence of a suitable coupling agent. Examples of suitable coupling reagents are 1-(3-dimethylaminopropyl)-3-ethylcarbodiimide hydrochloride/hydroxybenzotriazole (EDCI / HOBt), 1,1-carbonyldiimidazole (CDI), dicyclohexylcarbodiimide/hydroxybenzotriazole (DCC / HOBt), *O-*(1*H-*benzotriazol-1-yl)-*N,N,N',N*'-tetramethyluronium tetrafluoroborate (R. Knorr et al., Tetrahedron Lett., 1989, 30, 1927-1930) and polymer supported carbodiimide-1-hydroxybenzotriazole (for representative procedures, see for example, Argonaut Technical Note 501 available from Argonaut Technologies, Inc., Foster City, California). The couplings are performed in an inert solvent, preferably an aprotic solvent at a temperature of about 0°C to about 45°C for about 1 to 72h in the presence of a tertiary amine base such as diisopropylethylamine (DIPEA) or triethylamine. Exemplary solvents include acetonitrile, chloroform, dichloromethane, *N,N-*dimethylformamide (DMF) or mixtures thereof. Use of these coupling agents and appropriate selection of solvents and temperatures are known to those skilled in the art or can be readily determined from the literature. These and other exemplary conditions useful for coupling carboxylic acids are described in Houben-Weyl, Vol XV, part II, E. Wunsch, Ed., G. Thieme Verlag, 1974, Stuttgart, and M. Bodansky, Principles of Peptide Synthesis, Springer-Verlag, Berlin, 1984 and The Peptides, Analysis, Synthesis and Biology (Ed., E. Gross and J. Meienhofer), Vols 1-5, Academic Press NY 1979-1983.

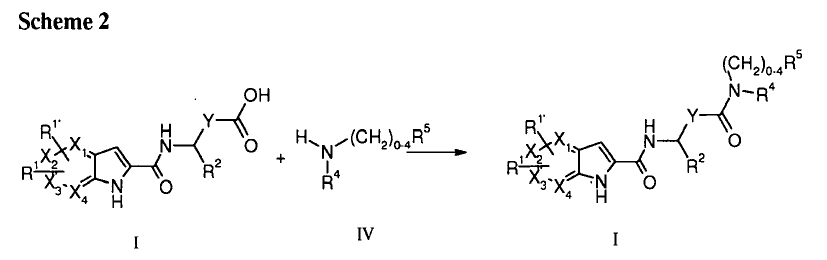

In a second process, the compounds of Formula (I) (wherein Z is C=O and R³ is -NR⁴R⁵ or -NR⁴-C₁₋₄alkylR⁵)) may be prepared according to **Scheme 2** by coupling the appropriate carboxylic acid of Formula (I), or a protected or activated derivative thereof, (wherein Z is absent and R³ is -CO₂H) with the appropriate amine of Formula (IV). Examples of suitable coupling agents and conditions are as described above. Compounds of Formula (IV) are commercially available or are readily prepared by known techniques.

Compounds of Formula (II) can be prepared as illustrated in **Scheme 3.**

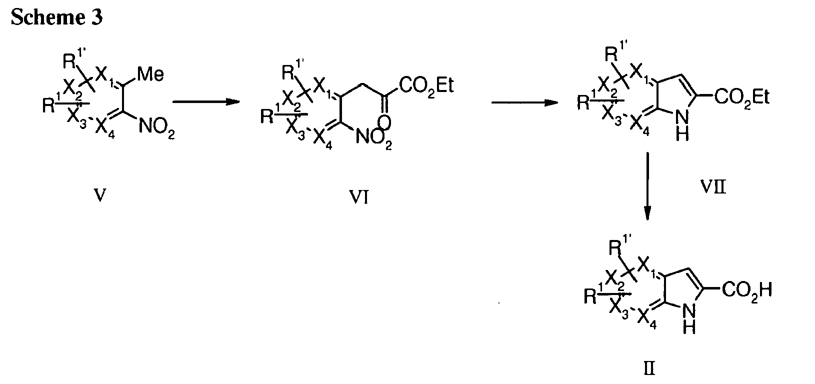

Compounds of Formula (VI) may be prepared by condensation of *ortho* methyl nitro compounds of Formula (V) with an oxalate ester in a solvent such as diethyl ether in the presence of a base such as potassium ethoxide or DBU. Compounds of Formula (VII) are prepared from compounds of Formula (VI) under reducing conditions, such as iron powder and ammonium chloride, or by hydrogenation in ethanol using palladium catalysis. Compounds of Formula (VII) undergo ester hydrolysis using aqueous alkali to give pyrrolopyridine-2-carboxylic acids of Formula (II). Further information on the conversion of compounds of Formula (V) to compounds of Formula (II) are described in the literature (Kermack, et al., J. Chem, Soc., 1921, 119, 1602; Cannon et al., J. Med. Chem., 1981, 24, 238; Julian et al., in Heterocyclic Compounds, Vol 3 (Wiley, New York, NY, 1962, R.C. Elderfield, Ed.) p 18.

Alternatively, the compound of Formula (VII) wherein X₂ is nitrogen can be prepared as illustrated in **Scheme 4.**

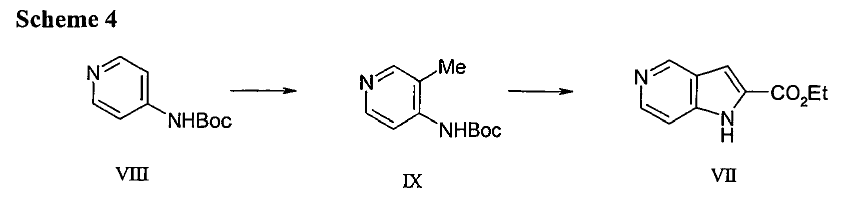

Deprotonation of compounds of Formula (VIII) with an organolithium such as *n*-butyllithium in a suitable solvent such as THF, followed by quenching with methyl iodide gives compounds of Formula (IX). Such compounds can undergo further deprotonation with *tert*-butyllithium, in a suitable solvent such as THF, followed by quenching with diethyl oxalate and subsequent heating of the intermediate under reflux in hydrochloric acid, to give compounds of Formula (VII).

Compounds of Formula (II) may also be prepared according to **Scheme 5** by Heck coupling of an *ortho*-iodo aminopyridine (XIV) followed by cyclisation at a temperature of between 100 to 150°C in the presence of catalyst such as palladium acetate and a base such as DABCO in a solvent such as DMF (See Chen et al, J. Org. Chem. 1997, 62, 2676). The *ortho*-iodo aminopyridines (XIV) can be made by direct iodination of the appropriate aminopyridine (XIII) using iodine in the presence of silver sulfate in a solvent such as ethanol at ambient temperature (see Sy, W., Synth. Commun., 1992, 22, 3215).

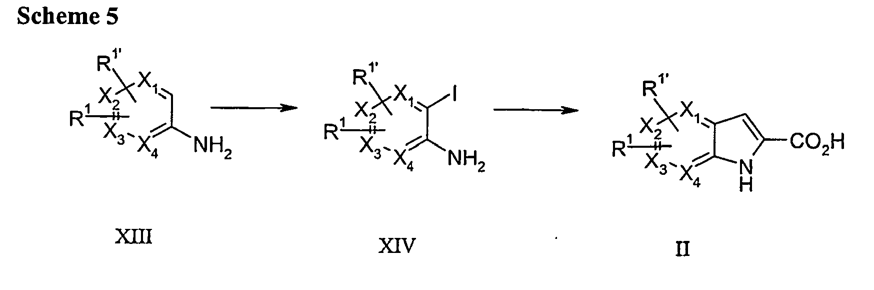

Alternatively compounds of Formula (XIV) may be prepared according to **Scheme** 6 by deprotection of N-pivaloyl compounds (XV) by heating under reflux using hydrochloric acid. The N-pivaloyl compounds (XV) are in turn made by deprotonation of compounds of Formula (XVI) with an organolithium such as *tert-*butyllithium in a suitable solvent such as THF, followed by quenching with iodine at a low temperature. Compounds of Formula (XVI) may be made by protection of commercially available aminopyridines (XIII) with trimethylacetyl chloride and a base such as triethylamine in a solvent such as dichloromethane.

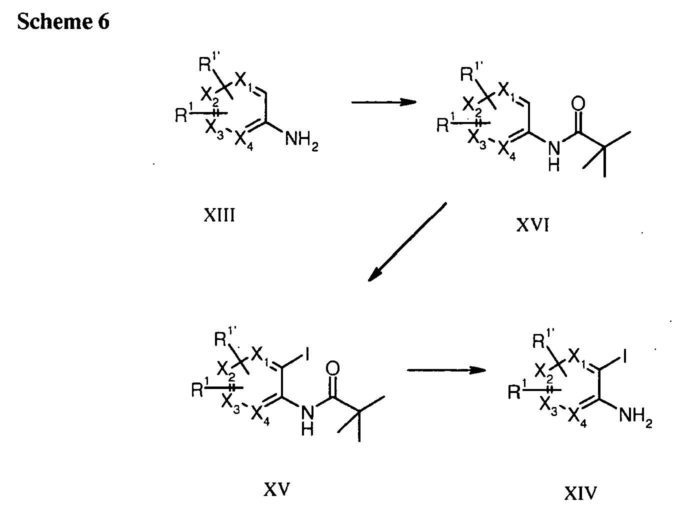

Alternatively compounds of Formula (XIV) may be prepared according to **Scheme 7** by deprotection of N-BOC protected compounds (XVII) using an acid such as trifluoroacetic acid in a solvent such as dichloromethane at ambient temperature. The N-BOC compounds (XVII) are in turn made by deprotonation of compounds of Formula (XVIII) with an organolithium such as *n*-butyllithium in the presence of N,N,N',N'-tetramethylethylenediamine (TMEDA) in a suitable solvent such as ether at temperatures around -70°C followed by the addition of iodine at temperatures around-10°C. The N-BOC aminopyridines (XVIII) are routinely made from the commercially available aminopyridines (XIII) using di-*tert*-butyldicarbonate by heating in a solvent such as 1,4-dioxane.

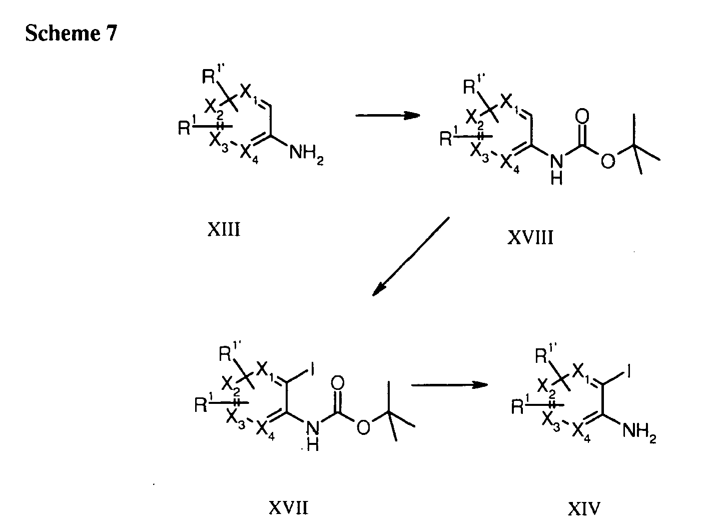

Protected or activated derivatives of the compounds of Formula (II) may be prepared by methods known to those skilled in the art.

Compounds of Formula (III) can be prepared as illustrated in **Scheme 8.**

Compounds of Formula (X) are generally commercially available or are readily prepared by known techniques. PG represents a protecting group such as, for example, *tert*-butyloxycarbonyl (Boc). Compounds of Formula (XI) are made from carboxylic acids of Formula (X) using standard coupling conditions as described above for **Scheme 1**.

Compounds of Formula (III) can be prepared as illustrated in **Scheme 8.**

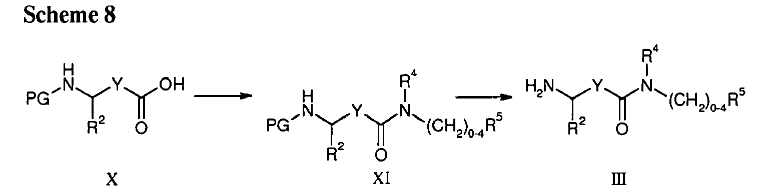

Compounds of Formula (III) may be prepared from compounds of Formula (XI) by removal of the protecting group, where PG = Boc, under acidic conditions using for example trifluoroacetic acid in dichloromethane at temperatures of around 25°C.

Compounds of Formula (III) wherein R² is H, Y is a direct bond, Z is -C(O)- and R³ is aryl or -hetaryl can be prepared according to **Scheme 9.**

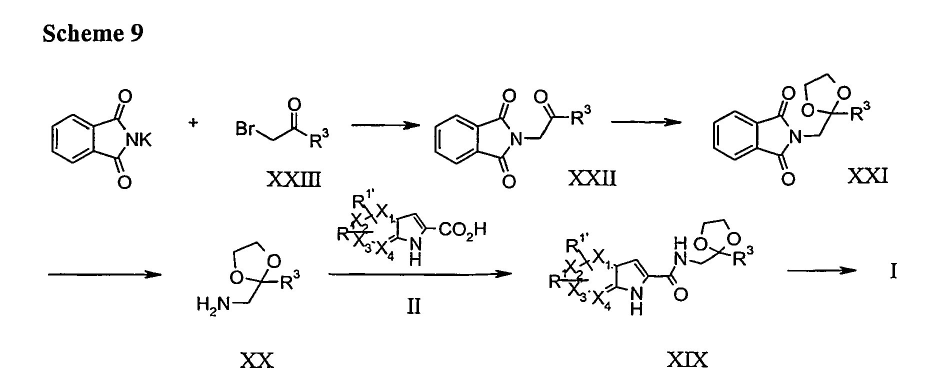

Compounds of Formula (XXIII) are reacted with potassium phthalimide in a solvent such as DMF to give compounds of Formula (XXII) which can then be reacted with ethylene glycol in the presence of a catalytic amount of acid such as p-toluene sulfonic acid in a solvent such as toluene whilst removing water to give compounds of Formula (XXI). The phthalimide protecting group can then be removed using hydrazine hydrate by heating as a neat solution or by heating in a solvent such as ethanol to give compounds of Formula (XX). These amines are then coupled with compounds of Formula (II) under standard coupling conditions as described in **Scheme 1,** and then the ketal group is removed in the presence of acid such as hydrochloric acid in a solvent such as acetone at reflux temperature to give the compounds of Formula (I).

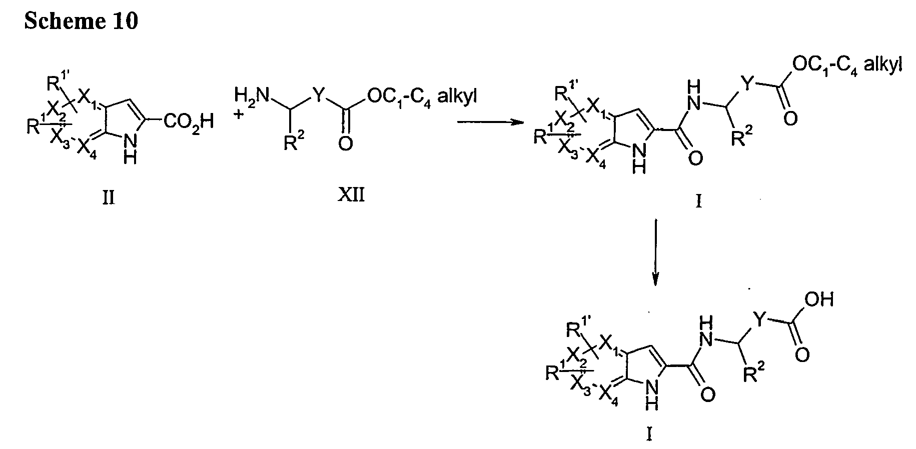

Compounds of Formula (I) (wherein Z is C=O and R³ is C₁₋₄alkoxy) may be prepared as illustrated in **Scheme 10** by combination of compounds of Formula (II) and compounds of Formula (XII) under standard coupling conditions as described for **Scheme 1**. Compounds of Formula (XII) are generally commercially available or are readily prepared by known techniques

Compounds of Formula (I) (wherein Z is absent and R³ is -CO₂H) may be prepared by ester hydrolysis of compounds of Formula (I) (where Z is C=O and R³ is a C₁₋₄alkoxy group) using aqueous alkali typically at a temperature of around 25"C for 30min to 20h.

The compounds of Formula (I) may be prepared singly or as compound libraries comprising at least 2, for example 5 to 1,000 compounds and more preferably 10 to 100 compounds of Formula (I). Compound libraries may be prepared by a combinatorial "split and mix" approach or by multiple parallel synthesis using either solution or solid phase chemistry, using procedures known to those skilled in the art.

During the synthesis of the compounds of Formula (I), labile functional groups in the intermediate compounds, e.g. hydroxy, carboxy and amino groups, may be protected. The compounds of Formula (II) may be protected in the 1-position e.g. with an arylmethyl, acyl, alkoxycarbonyl, sulfonyl or silyl group. The protecting groups may be removed at any stage in the synthesis of the compounds of Formula (I) or may be present on the final compound of Formula (I). A comprehensive discussion of the ways in which various labile functional groups may be protected and methods for cleaving the resulting protected derivatives is given in for example, Protective Groups in Organic Chemistry, T.W. Greene and P.G.M. Wuts, (1991) Wiley-Interscience, New York, 2nd edition.

Any novel intermediates as defined above are also included within the scope of the invention.

The invention also provides novel compounds of Formula (IIA). Specific compounds of Formula (IIA) include:
5-Chloro-1*H*-pyrrolo[3,2-b]pyridine-2-carboxylic acid;
5-Bromo-1*H*-pyrrolo[3,2-b]pyridine-2-carboxylic acid;
5-Cyano-1*H*-pyrrolo[3,2-b]pyridine-2-carboxylic acid;
5-methoxy-1*H*-pyrrolo[3,2-b]pyridine-2-carboxylic acid;
1*H*-Pyrrolo[3,2-c]pyridine-2-carboxylic acid;
6-Chloro-1*H*-pyrrolo[3,2-c]pyridine-2-carboxylic acid;
6-Cyano-1*H*-pyrrolo[3,2-c]pyridine-2-carboxylic acid;
5-Chloro-1*H*-pyrrolo[2,3-c]pyridine-2-carboxylic acid;
5-Bromo-1*H*-pyrrolo[2,3-c]pyridine-2-carboxylic acid;
5-Ethynyl-1*H*-pyrrolo[2,3-c]pyridine-2-carboxylic acid;
5-Cyano-1*H*-pyrrolo[2,3-c]pyridine-2-carboxylic acid;
5-Methyl-1*H*-pyrrolo[2,3-c]pyridine-2-carboxylic acid;
5-Chloro-1*H*-pyrrolo[2,3-b]pyridine-2-carboxylic acid;
6-Chloro-1*H*-pyrrolo[2,3-b]pyridine-2-carboxylic acid; and
6-Cyano-1*H*-pyrrolo[2,3-b]pyridine-2-carboxylic acid;
or a C₁₋₄alkyl ester of any one thereof.

The invention also provides a compound of Formula (XIX): 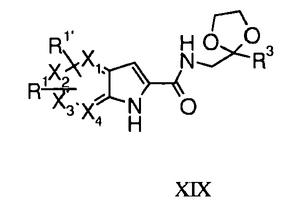 wherein R¹, R¹, X¹, X₂, X₃, and X₄ are as defined above for Formula (I) and R³ is -aryl or -hetaryl.

The invention also provides the novel compound 4(*S*)-(4-fluorobenzyl)oxazolidine-2,5-dione, which may be prepared as described in the Experimental section below.

### EXPERIMENTAL

### Materials & methods

Column chromatography was carried out on SiO₂ (40-63 mesh). LCMS data were obtained using a Waters Symmetry 3.5µ C₁₈ column (2.1 × 30.0mum, flow rate = 0.8mL/min) eluting with a (5% MeCN in H₂O)-MeCN solution containing 0.1% HCO₂H over 6min and UV detection at 220nm. Gradient information: 0.0-1.2min: 100% (5% MeCN in H₂O); 1.2-3.8min: ramp up to 10% (5% MeCN in H₂O)-90% MeCN; 3.8-4.4min: hold at 10% (5% MeCN in H₂O)-90% MeCN; 4.4-5.5min: ramp up to 100% MeCN; 5.5-6.0min: return to 100% (5% MeCN in H₂O). The mass spectra were obtained employing an electrospray ionisation source in the positive (ES⁺) ion mode. NMR spectra were acquired at 27°C on a Varian Mercury 400 spectrometer operating at 400 MHz or on a Bruker AMX2 500 spectrometer operating at 500MHz. Mass directed purification was performed on a Micromass Platform LC with cone voltage 30v, employing an electrospray ionisation source in the positive (ES⁺) ion mode, Waters 996 Photodiode Array Detector (210-390nm), Xterra Prep MS, C₁₈, 5µ. 19x50mm columns, and a mobile Phase of MeCN + 0.1% Formic Acid / H₂0+5%MeCN+0.1% Formic Acid.

Abbreviations and acronyms: BOC: *tert*-butyloxycarbonyl; DABCO: bicyclo(2,2,2)-1,4-diazaoctane; DBU:1,8-Diazabicyclo[5.4.0]undec-7-ene; DCM: Dichloromethane; DIPEA: *N,N*-Diisopropylethylamine; DMAP: 4-(N,N-dimethylamino)pyridine; DMF: *N,N*-Dimethylformamide; DMSO: Dimethylsulfoxide; DMTMM: 4-(4,6-dimethoxy[1.3.5]triazin-2-yl)-4-methylmorpholinium chloride hydrate; EDCI: 1-(3-Dimethylaminopropyl)-3-ethylcarbodiimide hydrochloride; GP: Glycogen Phosphorylase; HATU: O-(7-Azabenzotriazol-1-yl)-*N,N,N',N*'-tetramethyluronium hexafluorophosphate; HOBt: 1-Hydroxybenzotriazole; MDP: Mass directed purification; MgSO₄: Magnesium sulfate; PS: Polymer supported; rt: room temperature; RT: Retention time; THF: Tetrahydrofuran, TBTU: *O*-(benzotriazol-1-yl) *N, N, N',N*'-tetramethyluronium tetrafluoroborate.

### Preparation 1 : 6-Methyl-5-nitropyridin-2-ylamine

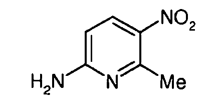

The title compound was prepared according to the method of Parker and Shive (J. Am. Chem . Soc., 1947, 69, 63) as a brown powder. δ_{H} (d₆ DMSO): 2.6 (3H, s), 6.37 (1H, d, 9.13Hz), 7.31 (2H, s), 8.08 (1H, d, 9.13Hz); m/z (ES⁺) = 154.06 [*M*+ H]⁺; RT = 0.57min.

### Preparation 2 : 6-Methyl-5-nitro-1H-pyridin-2-one

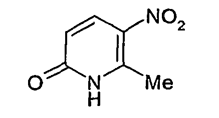

The title compound was prepared according to the method of Baumgarten and Su (J. Am. Chem. Soc, 1952, 74, 3828) as a brown powder. δ_{H} (d₆ DMSO): 2.62 (3H, s), 6.28 (1H, d, 9.94Hz), 8.10 (1H, d, 9.94Hz).

### Preparation 3 : 2-Chloro-6-methyl-5-nitropyridine

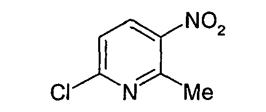

A suspension of 6-methyl-5-nitro-1*H*-pyridin-2-one (**Preparation 2,** 3.53g, 22.9mmol) in phosphorous oxychloride (20mL) was heated to 115°C (oil bath temperature) for 3h then allowed to cool to rt. The phosphorous oxychloride was removed *in vacuo* and the residue poured into iced water (100mL). The mixture was quenched by addition of saturated sodium bicarbonate solution, then the aqueous mixture was extracted with ethyl acetate (3 x 100mL). The combined organics were washed with brine, dried (MgSO₄), filtered and concentrated *in vacuo* to furnish the title compound as a brown solid. δ_{H} (CDCl₃): 2.86 (3H, s), 7.36 (1H, d, 8.59Hz), 8.27 (1H, d, 8.32Hz).

### Preparation 4 : 3-(2-Chloro-5-nitropyridin-6-yl)-2-oxopropionic acid ethyl ester

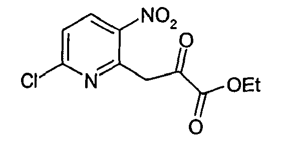

To a solution of potassium ethoxide (134mg, 1.59mmol) in diethyl ether (5mL) and ethanol (1mL) was added diethyl oxalate (218µL, 1.59mmol) in one portion and the resulting solution was stirred for 30min at rt. 2-Chloro-6-methyl-5-nitropyridine (**Preparation 3**, 250mg, 1.45mmol) was added as a suspension in diethyl ether (2mL, anhydrous) and stirring was continued for 17h at rt. The mixture was filtered on a sinter, washing with cold diethyl ether. The collected precipitate was dissolved in glacial acetic acid then evaporated to dryness *in vacuo* to give the title compound as a brown powder. δ_{H} (CDCl₃): 1.40 (3H, t, 7.27Hz), 4.38 (2H, q, 7.25Hz), 7.33 (1H, d, 8.59Hz), 7.37 (1H, s), 8.40 (1H, d, 8.86Hz).

### Preparation 5 : 5-Chloro-1H-pyrrolo[3,2-b]pyridine-2-carboxylic acid ethyl ester

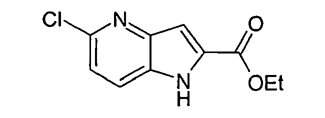

To a solution of 3-(2-chloro-5-nitropyridin-6-yl)-2-oxopropionic acid ethyl ester (**Preparation 4**,1.53g, 5.6mmol) in THF (65mL) and ethanol (30mL) was added saturated aqueous ammonium chloride solution (30mL) and the suspension was vigorously stirred at rt. Iron powder (1.95g, 34.8mmol) was added portionwise and the mixture was heated under reflux for 2h then allowed to cool prior to filtration through a celite plug, and washed through with warm THF. The mixture was concentrated under reduced pressure to give an aqueous suspension, which was filtered through a sinter, washing with water. The wet solid was washed with methanol and dried. The residue was adsorbed onto silica gel and purified *via* flash chromatography eluting with ethyl acetate/hexane (1:19) to give the title compound as a white solid. δ_{H} (CD₃OD): 1.42 (3H, t, 7.03Hz), 4.42 (2H, q, 7.32Hz), 7.15 (1H, s), 7.30 (1H, d, 8.79Hz), 7.89 (1H, d, 8.35Hz); m/z (ES⁺) = 225.03 [*M*+ H]⁺; RT = 3.32min.

### Preparation 6 : 5-Chloro-1H-pyrrolo[3,2-b]pyridine-2-carboxylic acid

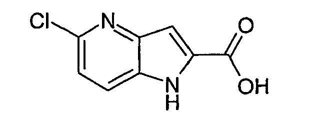

To a stirred solution of 5-chloro-1*H*-pyrrolo[3,2-b]pyridine-2-carboxylic acid ethyl ester (**P**reparation 5, 151mg, 0.67mmol) in ethanol (10mL) was added sodium hydroxide (0.35mL, 2M) and the stirred solution was heated at 70°C for 2h. The reaction mixture was then allowed to cool to rt and left to stand for 16h. The pH was adjusted to 4 by addition of glacial acetic acid, the solvents *removed in vacua* to give a white solid, which was suspended in dichloromethane and filtered through a sinter, washing with additional dichloromethane. The filter cake was washed with ethyl acetate (3 x 30mL) and dried to give the title compound as a white solid. δ_{H} (CD₃OD): 6.97 (1H, s), 7.17 (1H, d, 8.35Hz), 7.83 (1H, d, 8.35Hz); m/z (ES⁺) = 197 [*M* + H]⁺; RT = 2.82min.

### Preparation 7 : N-tert-Butyloxycarbonyl-(S)-phenylalanine dimethylamide

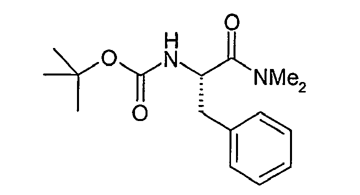

A solution of dimethylamine hydrochloride (1.45g, 17.8mmol) in DMF (46mL) was cooled to -10°C and triethylamine (2.7mL, 19.4mmol) was added. N-Boc-L-phenylalanine (4.59g, 17.3mmol, Aldrich) and HOBt (3.49g, 26mmol) were then added and the reaction stirred for 5min before addition of EDCI (3.33g, 17.4mmol). The reaction mixture was left to stir for 16h then diluted with ethyl acetate (400mL), washed sequentially with aqueous sodium hydroxide solution (2M, 2 x 100mL), hydrochloric acid (2N, 2 x 100mL), brine (250mL) and then dried (MgSO₄). Evaporation *in vacuo* gave the title compound as a pale yellow oil. δ_{H} (CDCl₃): 1.41 (9H, s), 2.61, 2.85 (6H, 2s), 2.91-2.99 (2H, m), 4.83 (1H, m), 5.40 (1H, br d), 7.18-7.29 (5H,m).

### Preparation 8 : 2-(S)-Amino-N,N-dimethyl-3-phenylpropionamide hydrochloride

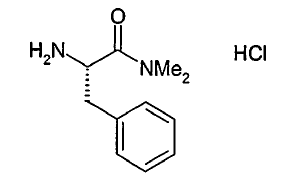

To a solution of *N*-*tert*-butyloxycarbonyl-(*S*)-phenylalanine dimethylamide (**Preparation 7,** 24.8g, 84mmol) in methanol (50mL) was added a solution of hydrochloric acid (4N, in dioxane, 40mL) and the mixture stirred at rt for 3h. The resulting solution was concentrated *in vacuo* then dissolved in water (300mL). The aqueous solution was washed with ethyl acetate (3 x 100mL) and concentrated again. Successive recrystallisations with methanol (200mL) and a mixture of methanol and toluene (1:1, 200mL) gave the title compound as colourless crystals. δ_{H} (D₂O) 2.75, 2.90 (6H, 2 x s), 3.18 (2H, m), 4.72 (1H, t), 7.28-7.45 (5H, m).

### Preparation 9 : Pyridin-4-ylcarbamic acid tert-butyl ester

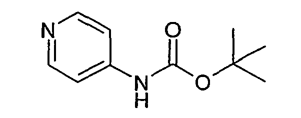

Pyridin-4-ylcarbamic acid *tert*-butyl ester was prepared according to the method of Spivey et al., (J. Org. Chem., 1999, 64, 9430) to give the title compound as a white crystalline solid. δ_{H} (CDCl₃): 1.52 (9H, s), 7.08 (1H, br s), 7.32 (2H, d), 8.43 (2H, d).

### Preparation 10 : (3-Methylpyridin-4-yl)carbamic acid tert-butyl ester

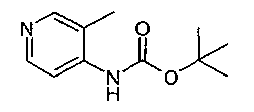

(3-Methylpyridin-4-yl)carbamic acid *tert*-butyl ester was prepared according to the method of Hands et al., (Synthesis, 1996, 7, 877) to give the title compound as a pale yellow solid. δ_{H} (CDCl₃): 1.55 (9H, s), 2.22 (3H, s), 6.52 (1H, s), 7.97 (1H, d), 8.27 (1H, s), 8.36 (1H, d).

### Preparation 11 : 1H-Pyrrolo[3,2-c]pyridine-2-carboxylic acid ethyl ester

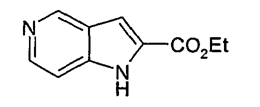

A solution of (3-methylpyridin-4-yl)carbamic acid *tert*-butyl ester (**Preparation 10**, 1.0g, 4.8mmol) in anhydrous THF (10mL) was cooled to -40°C and *tert*-butyl lithium (5.9mL, 10.1mmol) added dropwise. The temperature was maintained at -40°C for 1h and then a solution of diethyl oxalate (0.72mL, 5.3mmol) in THF (20mL) was added to the mixture. The reaction was warmed to 0°C, maintained at this temperature for 2h, warmed to rt and stirred for 16h. Hydrochloric acid (2N, 40mL) was added and the reaction heated under reflux for 90min, concentrated *in vacuo* and adjusted to pH 8 with saturated aqueous sodium hydrogen carbonate solution. The mixture was extracted with ethyl acetate (3 x 100mL) and the organic solution dried (MgSO₄) and concentrated *in vacuo* to give the title compound. δ_{H} (d₆ DMSO): 1.33 (3H, t), 4.35 (2H, q), 7.27 (1H, s), 7.38 (1H, d), 8.25 (1H, d), 8.95 (1H, s); m/z (ES⁺) =191 [*M*+ H]⁺.

### Preparation 12 : 1H-Pyrrolo[3,2-c]pyridine-2-carboxylic acid

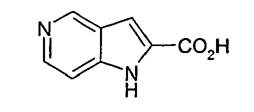

Aqueous sodium hydroxide solution (2.4mL, 2M, 4.8mmol) was added to a solution of 1*H*-pyrrolo[3,2-c]pyridine-2-carboxylic acid ethyl ester (**Preparation 11,** 0.76g, 4.0mmol) in ethanol (40mL) and the mixture heated under reflux for 2h before being cooled and concentrated *in vacuo.* The residue was dissolved in a minimum amount of water, glacial acetic acid (1mL) was added and the solution cooled in a refrigerator for 3 days. The resultant precipitate was collected by filtration washed with ether and dried *in vacuo* to give the title compound as a cream coloured solid. δ_{H} (D₂O): 7.05 (1H, s), 7.63 (1H, d), 8.08 (1H, d), 8.94 (1H, s).

### Preparation 13 : 3-(3-Nitropyridin-4-yl)-2-oxopropionic acid ethyl ester

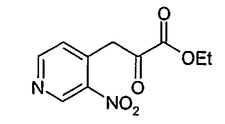

To a solution of potassium ethoxide (3.1g, 36.2mmol) in diethyl ether (70mL) and ethanol (10mL) under an argon atmosphere was added diethyl oxalate (4.9mL, 36.2mmol) and the reaction stirred at rt for 30min. A solution of 4-methyl-3-nitropyridine (5.0g, 36.2mmol) in diethyl ether (20mL) was added resulting in the immediate formation of a dark red precipitate. The reaction mixture was stirred at rt for 72h, then cooled to 0°C and filtered. The solid was dissolved in water (500mL) acidified to pH 4 with acetic acid and the precipitate collected and dried to give the title compound as a red solid. δ_{H} (d₆ DMSO): 1.27 (3H, t), 4.25 (2H, q), 6.74 (1H, s), 8.34 (1H, d), 8.43 (1H, d), 8.98 (1H, s); m/z (ES⁺) = 239 [*M*+H]⁺.

### Preparation 14 : 1H-Pyrrolo[2,3-c]pyridine-2-carboxylic acid ethyl ester

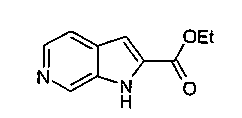

To a solution of 3-(3-nitropyridin-4-yl)-2-oxopropionic acid ethyl ester (**Preparation 13**, 500mg, 2.1mmol) in ethanol (20mL) and THF (10mL) was added saturated ammonium chloride solution (10mL) and iron powder (700mg, 12.6mmol). The reaction was heated under reflux for 1h, then filtered through celite and washed through with hot ethyl acetate (3 x 30mL). The combined organic fractions were washed with brine (20mL), dried (MgSO₄) and concentrated *in vacuo* to give the title compound as a brown solid. δ_{H} (CD₃OD): 1.44 (3H, t), 4.43 (2H, q), 7.21 (1H, s), 7.69 (1H, d), 8.12 (1H, d), 8.80 (1H, s).

### Preparation 15 : 1H-Pyrrolo[2,3-c]pyridine-2-carboxylic acid

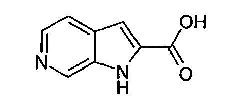

To a solution of 1*H*-pyrrolo[2,3-c]pyridine-2-carboxylic acid ethyl ester (**Preparation 14,** 310mg, 1.6mmol) in ethanol (20mL) was added 2N sodium hydroxide solution (1mL, 2.0mmol) and the reaction mixture heated under reflux for 1.5h then concentrated *in vacuo.* The residue was dissolved in water (10mL) and acidified with acetic acid giving an immediate brown precipitate. The solid was filtered and dried to give the title compound as a beige solid. δ_{H} (D₂O): 7.11 (1H, s), 7.99 (1H, d), 8.05 (1H, d), 8.85 (1H, s); m/z (ES⁺) = 163 [M + H]⁺.

### Preparation 16 : 3-(2-Chloro-5-nitropyridin-4-yl)-2-oxopropionic acid ethyl ester

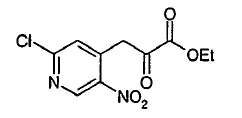

**Route A** : To a solution of potassium ethoxide (1.46g, 17.4mmol) in diethyl ether (80mL) and ethanol (10mL) under an argon atmosphere was added diethyl oxalate (2.4mL, 17.4mmol) and the mixture stirred at rt for 0.5h. A solution of 2-chloro-4-methyl-5-nitropyridine (3.0g, 17.4mmol) in diethyl ether (20mL) was added resulting in the formation of a dark green precipitate. The reaction was stirred at rt for 15h, cooled to 0°C, filtered and washed with cold diethyl ether to give a dark green solid. The solid was dissolved in water (200mL) and acidified to pH 4 with acetic acid to give an orange precipitate. The solid was collected by filtration and dried to give the title compound. m/z (ES⁺) = 273 [*M* + H]⁺.
**Route B :** To a solution of 2-chloro-4-methyl-5-nitropyridine (1.0g, 5.8mmol) in diethyl oxalate (4.23g, 29mmol) under an argon atmosphere was added 1,8-diazabicyclo[5.4.0]undec-7-ene (0.95mL, 6.4mol). The mixture was stirred at rt for 1.5h then diluted with t-butyl methyl ether (40mL), water (30mL) and acetic acid (1ml). The organic layer was separated, washed with water, dried (MgSO₄) and evaporated to dryness. The resultant damp red solid residue was finally dried under high vacuum at 40-50°C to give the title compound.

### Preparation 17 : 5-Chloro-1H-pyrrolo[2,3-c]pyridine-2-carboxylic acid ethyl ester

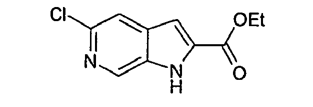

3-(2-Chloro-5-nitropyridin-4-yl)-2-oxopropionic acid ethyl ester (**Preparation 16**, 3.0g, 11.0mmol) was dissolved in ethanol (100mL) and THF (50mL). Iron powder (3.7g, 66.0mmol) and saturated ammonium chloride solution (50mL) were added and the mixture heated under reflux for 2h. The mixture was cooled, filtered through celite and washed several times with ethyl acetate. The organic layers were combined, washed with brine (100mL), dried (MgSO₄) and concentrated *in vacuo* to give the title compound as a brown solid. δ_{H} (CD₃OD): 1.42 (3H, t), 4.44 (2H, q), 7.15 (1H, s), 7.70 (1H, s), 8.59 (1H, s); m/z (ES⁺) = 225 [M+ H]⁺.

### Preparation 18 : 5-Chloro-1H-pyrrolo[2,3-c]pyridine-2-carboxylic acid

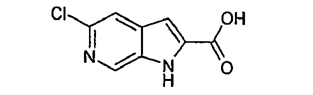

**Route A :** To a solution of 5-chloro-1*H*-pyrrolo[2,3-c]pyridine-2-carboxylic acid ethyl ester (**Preparation 17,** 1.78g, 7.9mmol) in ethanol (70mL) was added sodium hydroxide solution (5.2mL, 2M, 10.3mmol) and the mixture heated under reflux for 2h. The solvent was *removed in vacuo* and the solid dissolved in water (150mL) and acidified to pH 4 with acetic acid to give the title compound as a brown solid that was isolated by filtration. δ_{H} (CD₃OD): 7.13 (1H, s), 7.68 (1H, s), 8.58 (1H, s); m/z (ES⁺) = 197 [*M* + H]⁺.
**Route B :** A mixture of 6-chloro-4-iodopyridin-3-ylamine (**Preparation 106,** 0.33g, 1.30mmol), pyruvic acid (0.27mL, 3.89mmol), DABCO (0.44g, 3.89mmol) and palladium acetate (0.015g, 0.07mmol) in dry DMF was stirred vigorously and degassed with argon for 15min. The reaction mixture was heated to 107°C for 5h. The reaction mixture was allowed to cool to rt and stirred for 16h. The volatiles were removed under reduced pressure and the residue partitioned between ethyl acetate (100mL) and water (50mL). The layers were separated and the aqueous extracted with ethyl acetate (2x50mL). The combined organics were extracted with aqueous NaOH (2M, 3x70mL). The combined aqueous extracts were acidified to pH 4 by careful addition of glacial acetic acid, then extracted with ethyl acetate (3x60mL). The combined organics were washed with brine (50mL), dried (MgSO₄) filtered and concentrated *in vacuo* to give the title compound as a brown solid. RT=2.72min, m/z (ES⁺) =197 [*M* + H]⁺

### Preparation 19 : [1-(S)-(4-Fluorobenzyl)-2-(4-hydroxypiperidin-1-yl)-2-oxoethyl]carbamic acid tert-butyl ester

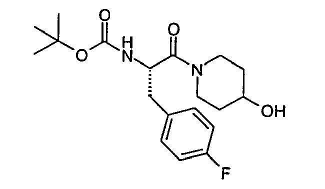

To a stirred solution of Boc-3-(4-fluorophenyl)-(*S*)-alanine (10.0g, 35.3mmol), 4-hydroxypiperidine hydrochloride (5.1g, 37.1mmol) and HOBt (7.2g, 52.9mmol) in DMF (100mL), was added DIPEA (12.3mL, 70.6mmol) and after 5min, EDCI (7.4g, 38.8mmol) and the reaction stirred at rt for 16h. The solvent was removed *in vacuo* and the residue partitioned between water (150mL) and ethyl acetate (2 x 150mL). The combined organic fractions were washed with sodium hydroxide solution (2M, 50mL), hydrochloric acid (2N, 50mL), dried (MgSO₄) and concentrated *in vacuo.* The product was chromatographed on silica gel eluting with ethyl acetate to give the title compound as a white solid. m/z (ES⁺) = 367 [*M* + H]⁺; RT = 3.28min.

### Preparation 20 : 2-(S)-Amino-3-(4-fluorophenyl)-1-(4-hydroxypiperidin-1-yl)propan-1-one hydrochloride

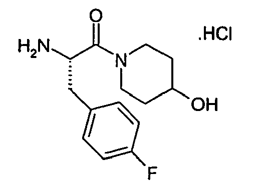

**Route A** : To a solution of [1(*S*)-(4-fluorobenzyl)-2-(4-hydroxypiperidin-1-yl)-2-oxoethyl]carbamic acid *tert*-butyl ester (**Preparation 19**, 11.6g, 31.7mmol) in methanol (40mL) was added hydrochloric acid in dioxane (24mL, 4N, 95.0mmol) and the reaction mixture stirred at rt for 6h. The solvent was removed *in vacuo* and the residue was dissolved in water (100mL) and extracted into ethyl acetate (2 x 50mL). The aqueous phase was evaporated to dryness to give the title compound as a white solid. δ_{H} (D₂O): 0.52-0.63 (0.5H, m), 1.12-1.23 (0.5H, m), 1.26-1.38 (1H, m), 1.42-1.50 (0.5H, m), 1.59-1.69 (0.5H, m), 1.72-1.82 (1H, m), 2.61-2.71 (0.5H, m), 2.91-3.15 (4H, m), 3.33-3.47 (1H, m), 3.69-3.78 (1H, m), 3.88-3.96 (1H, m), 4.60-4.72 (1H, m), 7.02-7.11 (2H, m), 7.14-7.26 (2H, m).
**Route B** : To a solution of 4-hydroxypiperidine (40mg, 0.4mmol) in anhydrous THF (3mL) under an argon atmosphere was added a solution of 4(*S*)-(4-fluorobenzyl)oxazolidine-2,5-dione (**Preparation 117**, 100mg, 0.48mmol) in THF (2mL) dropwise over 15min. The resulting mixture was stirred for 40h at rt before removal of the solvent *in vacuo.* The crude material was purified by column chromatography (SiO₂, 9:1 dichloromethane/methanol) to afford an oil. The free amine was dissolved in methanol (2mL) and a solution of 4M HCl in dioxane (0.3mL) was added and stirring was continued for 15min. The solvent was removed *in vacuo* and the material partitioned between ethyl acetate (5mL) and water (5mL). The aqueous layer was concentrated *in vacuo* to give the title compound. δ_{H} (CD₃OD): 7.36-7.29 (2H, m), 7.17-7.10 (2H, m), 4.70 (1H, t), 4.09-4.00 (0.5H, m), 3.91-3.74 (1.5H, m), 3.64-3.56 (0.5H, m), 3.43-3.31 (1H, m), 3.26-3.07 (3H, m), 2.89-2.80 (0.5H, m), 1.87-1.69 (1.5H, m), 1.56-1.36 (2H, m), 1.09-0.99 (0.5H, m).

### Preparation 21 : (3S,2R)-3-Amino-2-hydroxy-4-phenylbutyric acid methyl ester

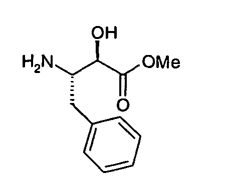

(3*S*,2*R*)-3-Amino-2-hydroxy-4-phenylbutyric acid methyl ester was synthesized according to the method of A. Fässler et al., (Tetrahedron Lett., 1998, 39, 4925) in three steps from commercially available *N*-(*tert*-butyloxycarbonyl)-L-phenylalaninal. R_{f} 0.29 (dichloromethane / methanol : 9 /1); δ_{H} (CDCl₃): 2.04 (3H, m), 2.73 (1H, dd), 2.92 (1H, dd), 3.36 (1H, ddd), 3.79 (3H, s), 4.08 (1H, d), 7.22-7.33 (5H, m).

### Preparation 21A : (3S,2S)-3-Amino-2-hydroxy-4-phenylbutyric acid methyl ester

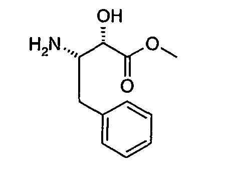

(3*S*,2*S*)-3-Amino-2-hydroxy-4-phenylbutyric acid methyl ester was obtained as a side product in **Preparation 21**. R_{f} 0.19 (dichloromethane / methanol: 9./ 1); δ_{H} (CDCl₃): 2.10 (2H, br s), 2.61 (1H, dd), 2.84 (1H, dd), 3.38 (1H, m), 3.78 (3H, s), 4.14(1H, br s).

### Preparation 22 : Cis-3,4-Dihydroxypyrrolidine-1-carboxylic acid benzyl ester

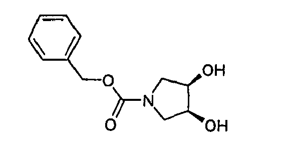

A solution of benzyl-2,5-dihydro-1*H* pyrrole-1-carboxylate (10.0g, 49.3mmol) in THF (200mL) was treated with osmium tetroxide solution (2.5% in *tert*-butanol, 5mL) and *N*-methylmorpholine (6.90g, 59.0mmol) and the reaction mixture stirred at rt under argon for 72h. Aqueous sodium thiosulfate solution (10%, 200mL) was added and the mixture stirred for a further 1h and then concentrated *in vacuo.* The resulting aqueous layer was extracted with ethyl acetate (3 x 200mL) and the combined organic layers washed with aqueous sodium thiosulfate solution (10%, 300mL) and hydrochloric acid (1N, 300mL). The organic fraction was dried (MgSO₄) and concentrated *in vacuo* to give the title compound as an off-white solid. δ_{H} (CDCl₃): 2.67 (2H, br s), 3.38-3.45 (2H, m), 3.63-3.67 (2H, m), 4.22-4.26 (2H, m), 5.12 (2H, s), 7.30-7.38 (5H, m); m/z (ES⁺) = 238 [*M* + H]⁺.

### Preparation 23 : Cis-3,4-Dihydroxypyrrolidine

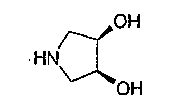

Palladium-on-carbon (144mg, 10 wt%) was added to a solution of *cis*-3,4-dihydroxypyrrolidine-1-carboxylic acid benzyl ester (**Preparation 22**, 403mg, 1.70mmol) in ethanol (20mL) and cyclohexene (2mL) and the mixture stirred and heated under reflux for 6h. After filtration through celite and repeated washing of the catalyst with methanol (CARE!), the filtrate and washings were combined and *concentrated in vacuo* to give the title compound as a colourless oil. δ_{H} (d₄ MeOH): 2.81 (2H, dd), 3.03 (2H, m), 4.07 (2H, m).

### Preparation 24 : 2-Bromo-4-methyl-5-nitropyridine

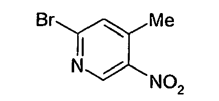

To a suspension of 2-hydroxy-4-methyl-5-nitropyridine (1g, 6.5mmol) in dichloroethane (10mL) was added a solution of phosphorus oxybromide (2.8g, 9.7mmol) in dichloroethane (10mL). The reaction mixture was heated under reflux for 4h, then cooled to rt and quenched with water (40mL). The layers were separated and the aqueous layer extracted into dichloromethane (2 x 30mL). The combined organics were dried (MgSO₄), concentrated *in vacuo* and chromatographed on silica gel eluting with dichloromethane to give the title compound as a pale yellow solid. δ_{H} (CDCl₃): 2.63 (3H, s), 7.52 (1H, s), 8.96 (1H, s).

### Preparation 25 : 3-(2-Bromo-5-nitropyridin-4-yl)-2-oxopropionic acid ethyl ester

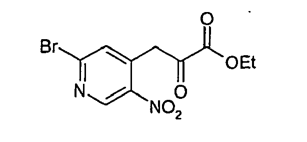

To a solution of potassium ethoxide (2.05g, 24.3mmol) in diethyl ether (70mL) and ethanol (10mL) under an argon atmosphere was added diethyl oxalate (3.3mL, 24.3mmol) and the reaction stirred at rt for 15min. A solution of 2-bromo-4-methyl-5-nitropyridine (**Preparation 24,** 4.8g, 22.1mmol) in diethyl ether (20mL) was added to the reaction mixture giving an immediate black precipitate. The reaction was allowed to stir at rt for 6h, then cooled to 0°C and filtered to give a black solid. The solid was dissolved in water (250mL) and acidified to pH 4 with acetic acid resulting in formation of a red precipitate. The solid was collected and dried to give the title compound as a red solid. δ_{H} (d₆ DMSO): 1.16 (3H, t), 4.01 (2H, q), 6.55 (1H, s), 7.92 (1H, s), 8.96 (1H, s).

### Preparation 26 : 5-Bromo-1H-pyrrolo[2,3-c]pyridine-2-carboxylic acid ethyl ester

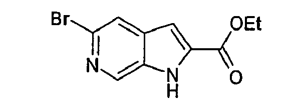

**Route A :** To a solution of 3-(2-bromo-5-nitropyridin-4-yl)-2-oxopropionic acid ethyl ester (**Preparation 2**5, 3.38g, 10.7mmol) in THF (50mL) and ethanol (100mL) was added saturated ammonium chloride solution (50mL) and iron powder (3.57g, 64.0mmol) and the reaction heated under reflux for 2h. The reaction mixture was filtered through celite and washed several times with ethyl acetate. The solvent was removed *in vacuo* and the remainder partitioned between saturated sodium hydrogen carbonate solution (100mL) and ethyl acetate (3 x 150mL). The combined organic fractions were dried (MgSO₄) and concentrated *in vacuo* to give the title compound as a brown solid. δ_{H} (CD₃OD): 1.42 (3H, t), 4.43 (2H, q), 7.14 (1H, s), 7.85 (1H, s), 8.58 (1H, s); m/z (ES⁺) = 269 [*M* + H]⁺.
**Route B** : To a solution of 2-bromo-4-methyl-5-nitropyridine (**Preparation 24,** 5.7g, 26.3mmol) in diethyl oxalate (17.9mL) under argon was added 1,8-diazabicyclo[5,4,0]undec-7-ene (4.5mL, 30.2mmol) to give a dark red precipitate. Reaction mixture was stirred at rt for 4.5h and concentrated *in vacuo.* Acetic acid (140mL) was added to the residue under argon and heated to 60°C. Iron (2.94g, 52.6mmol) was added in small portions over a period of 1h. he reaction mixture was heated at 80°C for 4h. The reaction mixture was cooled to rt and poured into water (300mL) which gave a beige precipitate. The precipitate was isolated and washed with water. The solid obtained was dissolved in ethyl acetate (700ml) and filtered. The filtrate was concentrated *in vacuo* to give the title compound. m/z (ES+) = 269 [*M* + H]⁺; RT = 3.39min.

### Preparation 27 : 5-Bromo-1H pyrrolo[2,3-c]pyridine-2-carboxylic acid

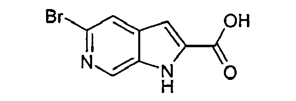

Sodium hydroxide solution (1.1mL, 2M, 2.23mmol) was added to a solution of 5-bromo-1*H*-pyrrolo[2,3-c]pyridine-2-carboxylic acid ethyl ester (**Preparation 26**, 500mg, 1.86mmol) in ethanol (20mL), and the reaction mixture heated under reflux for 1.5h and then concentrated *in vacuo.* The residue was dissolved in water (15mL) and acidified with acetic acid resulting in formation of a brown precipitate. The solid was collected by filtration and dried to give the title compound as a brown solid. δ_{H} (d₆ DMSO): 7.08 (1H, s), 7.97 (1H, s), 8.60 (1H, s); m/z (ES⁺) = 241 [*M* + H]⁺.

### Preparation 28 : 1H-Pyrrolo[2,3-b]pyridine-2-carboxylic acid

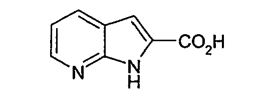

The title compound was prepared according to the method of Romero and Mitchell (WO 91/09849).

### Preparation 29 : 2-Methyl-3-nitropyridine

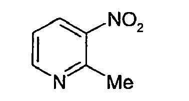

A mixture of 2-chloro-3-nitropyridine (1.00, 6.30mmol), potassium carbonate (2.62g, 18.90mmol), *tetrakis*(triphenylphosphine)palladium (0.73g, 0.63mmol) and trimethyl boroxine (0.88mL, 6.30mmol) in 1,4-dioxane (2mL) and water (8mL) was heated to 110°C (oil bath temperature) for 6h and then stirred for 16h at rt. The mixture was then filtered through a celite pad, washing through with THF. The filtrate was adsorbed onto silica gel *in vacuo* and purified via flash column chromatography eluting with ethyl acetate/hexanes (3:7) to give the title compound as a yellow solid. δ_{H} (CDCl₃): 2.86 (3H, s), 7.34 (1H, dd), 8.26 (1H, dd), 8.71 (1H, dd).

### Preparation 30 : 3-(3-Nitropyridin-2-yl)-2-oxopropionic acid ethyl ester

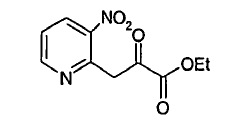

To a solution of potassium ethoxide (2.44g, 27.7mmol) in diethyl ether (90mL) and ethanol (8mL) was added diethyl oxalate (3.79mL, 27.7mmol) resulting in a yellow suspension. The reaction mixture was stirred for 5min prior to the addition of 2-methyl-3-nitropyridine (**Preparation 29**, 3.40g, 24.6mmol) in one portion. The resulting red suspension was stirred at rt, under argon, for 20h. The mixture was filtered, washed thoroughly with diethyl ether and dried. The red solid was dissolved in water and the mixture adjusted to pH 4 by addition of glacial acetic acid. The resulting precipitate was collected by filtration, dissolved in dichloromethane, washed with brine, dried (MgSO₄), and then filtered and concentrated *in vacuo* to give the title compound as an orange solid. δ_{H} (CDCl₃): 1.40 (3H, t), 4.39 (2H, q), 7.34 (1H, s), 7.36 (1H, dd), 8.43 (1H, dd), 8.66 (1H, dd).

### Preparation 31 : 1H-Pyrrolo[3,2-b]pyridine-2-carboxylic acid ethyl ester

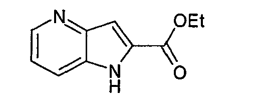

To a suspension of 3-(3-nitropyridin-2-yl)-2-oxopropionic acid ethyl ester (**Preparation 30**, 1.00g, 4.20mmol) in ethanol (30mL, absolute) was added palladium (10% on activated carbon, 447mg, 0.42mmol) and the reaction mixture placed under an atmosphere of hydrogen at a pressure of 20-30psi for 12h with vigorous stirring. The reaction mixture was filtered through celite, washing with ethyl acetate and the filtrate concentrated *in vacuo* to ca. 20mL. Water (150mL) was added and the mixture cooled to between 0°C and 5°C. The precipitate that formed was collected by filtration and dried to give the title compound as a beige solid. δ_{H} (CDCl₃): 1.44 (3H, t), 4.45 (2H, q), 7.25 (1H, dd), 7.39 (1H, s), 7.75 (1H, dd), 8.57 (1H, dd), 8.98 (1H, s).

### Preparation 32 : 1H-Pyrrolo[3,2-b]pyridine-2-carboxylic acid

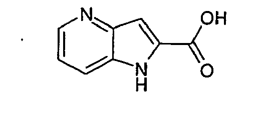

A suspension of 1*H*-pyrrolo[3,2-b]pyridine-2-carboxylic acid ethyl ester (**Preparation 31**, 0.34g, 1.77mmol) in aqueous sodium hydroxide solution (2M, 10mL) was heated under reflux for 3h and the resulting solution was allowed to cool to rt. The pH was adjusted to 4 by addition of glacial acetic acid. Excess acetic acid was removed *in vacuo* and the resulting suspension cooled to 0°C and then left standing at rt for 16h. The resulting beige precipitate was collected by filtration and dried to give the title compound as a beige solid. δ_{H} (d₆ DMSO): 7.12 (1H, s), 7.23 (1H, dd), 7.79 (1H, d), 8.42 (1H, dd).

### Preparation 33 : 6-Methoxy-2-methyl-3-nitropyridine

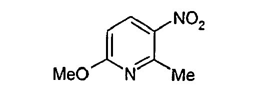

To a stirred suspension of 2-chloro-6-methoxy-3-nitropyridine (2.44g, 12.9mmol) in 10% v/v aqueous dioxane (25mL) was added *tetrakis*(triphenyl phosphine) palladium (1.50g, 1.3mmol) and the mixture stirred for 15min prior to the addition of trimethylboroxine (1.81mL, 12.9mmol) and potassium carbonate (5.36g, 38.8mmol). The reaction mixture was heated under reflux for 6h then allowed to cool to rt over 16h. Ethyl acetate (100mL) was added and the mixture stirred vigorously for 1h. The mixture was filtered through celite, washing through with ethyl acetate. The aqueous phase was separated and extracted with ethyl acetate (3 x 30mL) and the combined organics were washed with brine (50mL), dried (MgSO₄), filtered and adsorbed onto silica gel. Purification *via* flash column chromatography (SiO₂, ethyl acetate / isohexane, 1:20) gave the title compound as a pale yellow solid. δ_{H} (CDCl₃): 2.81 (3H, s), 4.01 (3H, s), 6.65 (1, d), 8.26 (1H, d).

### Preparation 34 : 3-(6-Methoxy-3-nitropyridin-2-yl)-2-oxopropionic acid ethyl ester

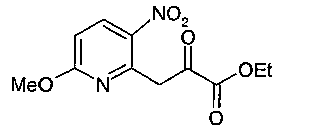

To a stirred suspension of potassium ethoxide (0.55g, 6.55mmol) in diethyl ether (20mL, anhydrous) and ethanol (4mL) was added diethyl oxalate (894µL, 6.55mmol) and the reaction mixture was stirred for 30min prior to the addition of 6-methoxy-2-methyl-3-nitropyridine (**Preparation 33**, 1.0g, 5.95mmol) in diethyl ether (8mL). The resulting red suspension was stirred at rt, under argon, for 20h. The mixture was filtered, the solid washed thoroughly with diethyl ether then dried. The red solid was then taken up in hot water and the solution cooled to 0°C. The precipitate was filtered, washed with cold water, and dried to give the title compound as a beige solid. δ_{H} (CDCl₃): 1.40 (3H, t), 4.07 (3H, s), 4.39 (2H, q), 6.74 (1H, d), 7.57 (1H, s), 8.40 (1H, d), 13.82 (1H, s).

### Preparation 35 : 5Methoxy-1H-pyrrolo[3,2-b]pyridine-2-carboxylic acid ethyl ester

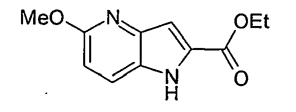

To a suspension of 3-(6-methoxy-3-nitropyridin-2-yl)-2-oxopropionic acid ethyl ester (**Preparation 34,** 276mg, 1.03mmol) in THF (12mL) and ethanol (5mL) was added saturated aqueous ammonium chloride solution (5mL) and iron powder (346mg, 6.18mmol) in one portion. The reaction mixture was heated under reflux for 1h then filtered whilst still hot through a celite plug, washing with hot ethyl acetate. The filtrate was cooled and washed with brine (20mL), dried (MgS0₄), filtered and adsorbed onto silica gel *in vacuo.* Purification *via* flash column chromatography (SiO₂, ethyl acetate/hexanes, 1:9) gave the title compound as a beige solid. δ_{H} (CDCl₃): 1.40 (3H, t), 3.99 (3H, s), 4.41 (2H, q), 6.74 (1H, d), 7.20 (1H, m), 7.62 (1H, d), 9.27 (1H, s).

### Preparation 36 : 5-Methoxy-1H-pyrrolo[3,2-b]pyridine-2-carboxylic acid

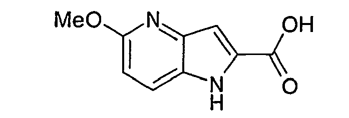

To a solution of 5-methoxy-1*H*-pyrrolo[3,2-b]pyridine-2-carboxylic acid ethyl ester (**Preparation 35**, 128mg, 0.58mmol) in ethanol (10mL, absolute) was added aqueous sodium hydroxide (0.35mL, 2M) and the mixture heated to 70°C for 3h then cooled to rt. The solution was adjusted to pH 4 by addition of glacial acetic acid and the solvents removed under reduced pressure. Ethyl acetate (20mL) was added to the resulting oil and the mixture sonicated until a fine suspension formed. The mother liquor was decanted and the remaining solid was washed with ethyl acetate and dried *in vacuo* to give the title compound as a pale orange powder. δ_{H} (CD₃OD): 3.92 (3H, s), 6.63 (1H, d), 6.94 (1H, s), 7.72 (1H, d).

### Preparation 37 : 2-[2-(4-Methoxyphenyl)-2-oxoethyl]isoindole-1,3-dione

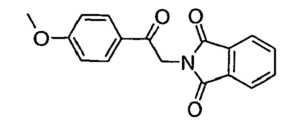

To a solution of 4-methoxyphenacyl bromide (5.78g, 25.23mmol) in DMF (20mL) was added potassium phthalimide (5.00g, 26.99mmol) and the reaction stirred at rt for 18h. The reaction mixture was partitioned between dichloromethane (200mL) and water (100mL). The layers were separated and the aqueous layer extracted with dichloromethane (3 x 50mL). The combined organics were washed with sodium hydroxide (2M, 50mL), water (50mL) and brine (50mL) and dried (MgSO₄). Filtration, then concentration *in vacuo* gave an off white solid. Trituration with diethyl ether followed by collection by filtration gave the title compound as a white solid. δ_{H} (CDCl₃): 3.86 (3H, s), 5.15 (2H, s), 7.09 (2H, d), 7.81-8.01 (4H, m), 8.05 (2H, d).

The following compounds were synthesised according to **Preparation 37** from potassium phthalimide and the appropriate α-bromoketone.

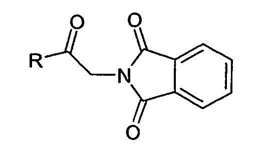

| **Preparation** | **R** | **NMR** |
|---|---|---|
| **38** | 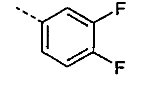 | δ_{H} (d₆ DMSO): 5.25 (2H, d), 7.60-7.75 (1H, m), 7.85-8.06 (5H, m), 8.10-8.24 (1H, m) |
| **39** | 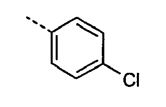 | δ_{H} (d₆ DMSO): 5.24 (2H, s), 7.66 (2H, d), 7.83-8.02 (4H, m), 8.10 (2H, d) |
| **40** | 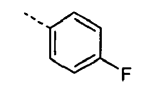 | 5_{H} (d₆ DMSO): 5.24 (2H, s), 7.36-7.52 (2H, m), 7.84-8.02 (4H, m), 8.11-8.25 (2H, m) |

### Preparation 41 : 2-[2-(4-Methoxyphenyl)-[1,3]dioxolan-2-ylmethyl]isoindole-1,3-dione

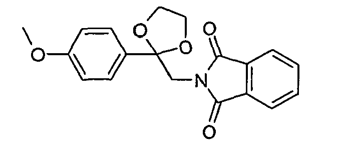

2-[2-(4-Methoxyphenyl)-2-oxoethyl]isoindole-1,3-dione (**Preparation 37,** 6.35g, 21.5mmol) was suspended in toluene (50mL) and ethylene glycol (12mL) added. *p*-Toluenesulfonic acid (300mg, 1.58mmol) was added and the resulting mixture heated under reflux for 40h, removing water with a Dean-Stark trap. The reaction mixture was allowed to cool to rt then partitioned between ethyl acetate (200mL) and saturated aqueous sodium bicarbonate solution (100mL). The organic layer was washed with brine (50mL), dried (MgSO₄) filtered and concentrated under reduced pressure, to give the title compound as an off-white solid. δ_{H} (d₆ DMSO): 3.66 (2H, t), 3.72 (3H, s), 3.87 (4H, m), 6.88 (2H, d), 7.32 (2H, d), 7.83 (4H, m).

The following compounds were synthesised according to **Preparation 41** from ethylene glycol and the appropriate ketone.

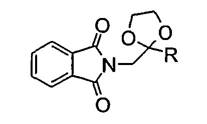

| **Preparation** | **R** | **NMR** |
|---|---|---|
| **42** | 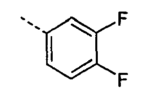 | δ_{H} (d₆ DMSO): 3.69-3.81 (2H, m), 3.85-4.04 (4H, m), 7.15-7.30 (1H, m), 7.32-7.49 (2H, m), 7.74-7.96 (4H, m) |
| **43** | 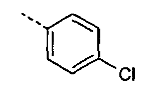 | δ_{H} (d₆ DMSO): 3.62-3.78 (2H, m), 3.84-3.99 (4H, m), 7.32-7.49 (4H, m), 7.74-7.94 (4H, m) |
| **44** | 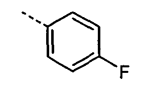 | δ_{H} (d₆ DMSO): 3.64-3.80 (2H, m), 3.85-4.03 (4H, m), 7.08-7.24 (2H, m), 7.35-7.52 (2H, m), 7.75-7.93 (4H, m) |

### Preparation 45 : [2-(4-Methoxyphenyl)-[1,3]dioxolan-2-yl]methylamine

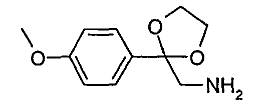

2-[2-(4-Methoxyphenyl)-[1,3]dioxolan-2-ylmethyl]isoindole-1,3-dione (**Preparation 41**, 2.0g, 5.90mmol) and hydrazine hydrate (5mL) were combined. The stirred reaction mixture was heated under reflux for 48h then allowed to cool to rt. Aqueous sodium hydroxide (2M, 10-15mL) and water (20mL) were added and the mixture stirred until a solution was formed. Diethyl ether (20mL) was added and the biphasic mixture stirred vigorously for 16h. The layers were separated and the aqueous layer was extracted with diethyl ether (3x20mL), then the combined organic extracts were washed with brine (20mL). The ethereal solution was passed through a filter paper then evaporated to dryness *in vacuo* to give the title compound as a yellow oil, which solidified on standing. δ_{H} (EDCl₃): 1.42 (2H, br s), 3.06 (2H, s), 3.97 (3H, s), 4.00 (2H, t), 4.21 (2H, t), 7.04 (2H, d), 7.53 (2H, d).

The following compounds were synthesised according to **Preparation 45** from hydrazine hydrate and the corresponding phthalimide.

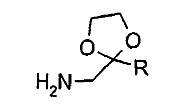

| **Preparation** | **R** | **NMR** |
|---|---|---|
| **46** | 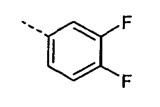 | δ_{H} (CDCl₃): 1.31 (2H, br s), 2.87 (2H, s), 3.79-3.87 (2H, m), 3.99-4.08 (2H, m), 7.00-7.20 (2H, m), 7.23-7.29 (1H, m) |
| **47** | 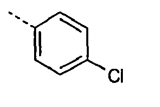 | 5_{H} (CDCl₃): 1.23 (2H, br s), 2.89 (2H, s), 3.75-3.87 (2H, m), 3.99-4.10 (2H, m), 7.29-7.33 (2H, m), 7.36-7.40 (2H, m) |
| **48** | 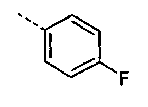 | δ_{H} (d₆ DMSO): 2.72 (2H, s), 3.25 (2H, br s), 3.67-3.78 (2H, m), 3.93-4.06 (2H, m), 7.11-7.18 (2H, m), 7.37-7.44 (2H, m) |

### Preparation 49 : 5-Chloro-1H-pyrrolo[2,3-c]pyridine-2-carboxylic acid [2-(4-methoxyphenyl)-[1,3]-dioxolan-2-ylmethyl]amide

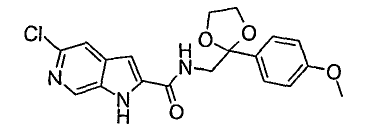

To a solution of [2-(4-methoxyphenyl)-[1,3]-dioxolan-2-yl]methylamine (**Preparation 45**, 0.117g, 0.56mmol) in dichloromethane (5mL) was added DIPEA (213µL, 1.22mmol), 5-chloro-1*H*-pyrrolo[2,3-c]pyridine-2-carboxylic acid (**Preparation 18,** 0.100g, 0.51mmol) and HOBT (0.076g, 0.56mmol). The resulting solution was stirred for 2min then EDCI (0.117g, 0.61mmol) was added and stirring was continued for 18h at rt. The reaction mixture was partitioned between dichloromethane (30mL) and water (20mL) and the layers separated. The aqueous phase was extracted with dichloromethane (3x20mL) then the combined organics were washed with brine (20mL), dried (MgSO₄), filtered and concentrated *in vacuo.* Recrystallisation from methanol/dichloromethane gave the title compound as an orange solid. δ_{H} (CDCl₃): 3.81 (3H, s), 3.84-3.91 (4H, m), 4.05 (2H, m), 6.56 (1H, t), 6.76 (1H, s), 6.89 (2H, d), 7.44 (2H, d), 7.57 (1H, s), 8.68 (1H, s), 9.94 (1H, br s).

The following compounds were synthesised according to **Preparation 49** from 5-chloro-1*H-*pyrrolo[2,3-c]pyridine-2-carboxylic acid (**Preparation 18**) and the appropriate amine.

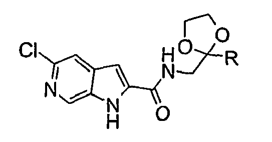

| **Preparation** | **R** | **m/z** |
|---|---|---|
| **50** | 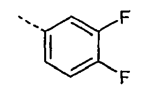 | m/z (ES⁺) = 394 [*M* + H]⁺; RT = 3.49min |
| **51** | 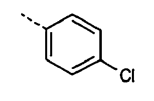 | m/z (ES⁺) = 392 [*M* + H]⁺; RT = 1.48min |
| **52** | 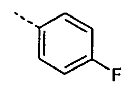 | m/z (ES⁺) = 376 [*M* + H]⁺; RT = 3.26min |

### Preparation 53 : (S)-2-Ainino-N,N-dimethyl-3-pyridin-3-ylpropionamide hydrochloride

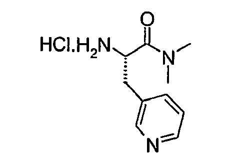

To a solution of (*S*)-2-*tert*-butoxycarbonylamino-3-pyridin-3-ylpropionic acid (500mg, 1.88mmol) in DMF (10mL) was added dimethylamine hydrochloride (153mg, 1.88mmol), DIPEA (1.2mL, 6.57mmol) and TBTU (602mg, 1.88mol). The reaction mixture was stirred at rt for 16h. The solvent was removed *in vacuo* and the residue dissolved in methanol (20mL). To this was added 4M hydrochloric acid in dioxane (20mL) and the reaction was stirred for 16h at rt. The solvent was removed *in vacuo* and the residue was partitioned between water (100mL) and ethyl acetate (2x100mL). The aqueous layer was evaporated to dryness and the residue recrystallised from ethanol/ethoxyethanol (9:1), to give the title compound as a white solid. δ_{H} (CD₃OD): 2.99 (3H, s), 3.06 (3H, s), 3.35-3.42 (1H, m), 3.45-3.53 (1H, m), 4.87-4.94 (1H, m), 8.04-8.10 (1H, m), 8.48-8.53 (1H, m), 8.84-8.90 (2H, m).

### Preparation 54 : (S)-2-Amino-3-pyridin-3-yl-1-pyrrolidin-1-ylpropan-1-one hydrochloride

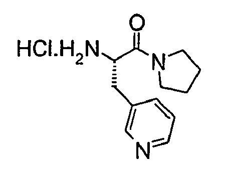

To a solution of pyrrolidine (157µL, 1.88mmol) in DMF (10mL) was added DIPEA (817µL, 4.69mmol), (*S*)-2-*tert*-butoxycarbonylamino-3-pyridin-3-ylpropionic acid (500mg, 1.88mmol) and TBTU (663mg, 2.06mmol). The reaction was stirred at rt for 72h. The solvent was removed *in vacuo* and the residue was dissolved in methanol (15mL). To this was added 4M hydrochloric acid in dioxane (20mL) and the reaction was stirred at rt for 16h. The solvent was removed *in vacuo* and the residue was partitioned between water (100mL) and ethyl acetate (2x100mL). The aqueous layer was evaporated to dryness, to give the title compound as a green oil. δ_{H} (CD₃PD): 1.86-2.05 (4H, m), 3.20-3.28 (2H, m), 3.39-3.58 (2H, m), 3.68-3.78 (2H, m), 4.63-4.71 (1H, m), 8.11-8.17 (1H, m), 8.56-8.64 (1H, m), 8.85-8.93 (2H, m).

### Preparation 55 : (S)-2-Amino-N,N-dimethylamino-3-pyridin-2-ylpropionamide hydrochloride

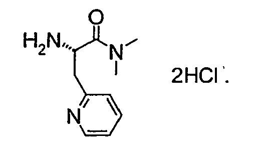

To a stirred solution of dimethylamine hydrochloride (0.153g, 1.87mmol) in DMF (8mL) was added DIPEA (2mL, 6.55mmol), (*S*)-2-*tert*-butoxycarbonylamino-3-pyridin-2-ylpropionic acid (0.50g, 1.87mmol, *Acros*) and TBTU (0.60g, 1.87mmol). The reaction mixture was stirred at rt for 16h. The solvent was *removed in vacuo* and the residue was dissolved in methanol (15mL). To this was added 4M hydrochloric acid in dioxane (20mL) and the reaction stirred at rt for 16h. The solvent was *removed in vacuo* and the residue partitioned between water (100mL) and ethyl acetate (2x100mL). The aqueous layer was evaporated to dryness to give a solid, which was recrystallised from ethanol to give the title compound as a pale brown solid. m/z (ES⁺) = 194.

### Preparation 56 : 5-Chloro-3-iodopyridin-2-ylamine

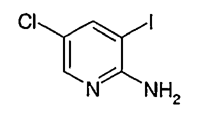

Silver sulfate (3.40g, 10.9mmol) and 2-amino-5-chloropyridine (1g, 7.8mmol) was added to a solution of iodine (2.76g, 10.9mmol) in ethanol (50ml) and the reaction mixture stirred at rt for 72h. The mixture was filtered, washed with methanol and the filtrate concentrated *in vacuo.* The residue was partitioned between saturated Na₂S₂O₃ solution (50ml) and DCM (2 x 50ml). The combined organics were dried (MgSO₄), concentrated *in vacuo* and purified by chromatography on silica gel eluting with DCM to give the title compound as a beige solid. δ_{H} (CDCl₃): 4.95 (2H, br s), 7.84 (1H, d), 7.98 (1H, d).

### Preparation 57 : 5-Chloro-1H-pyrrolo[2,3-b]pyridine-2-carboxylic acid

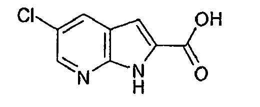

Pyruvic acid (0.43ml, 6.24mmol) was added to a solution of 5-chloro-3-iodopyridin-2-ylamine (**Preparation 56**, 500mg, 2.08mmol), palladium acetate (23mg, 0.10mmol) and DABCO (700mg, 6.24mmol) in anhydrous DMF (20ml). The reaction mixture was degassed with argon for 20min, then heated to 110°C for 16h. The solvent was removed *in vacuo* and the residue suspended in water (10ml) and acetic acid (5ml) and then filtered. The solid was dissolved in EtOAc (50ml), extracted into 2N NaOH solution (50ml) and the organic layer discarded. The aqueous solution was acidified with concentrated HCl and extracted into EtOAc (2 x 40ml). The combined organics were dried (MgSO₄) and concentrated *in vacuo* to give the title compound as a beige solid. 6H 7.14 (1H, s), 8.14 (1H, d), 8.35 (1H, d).

### Preparation 58 : 5-Trimethylsilylacetylene-1H-pyrrolo[2,3-c]pyridine-2-carboxylic acid ethyl ester

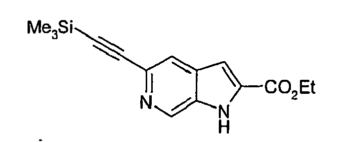

PdCl₂(PPh₃)₂ (0.026g, 0.037mmol) and Cu(l)I (0.007g, 0.037mmol) were added sequentially to 5-bromo-1*H*-pyrrolo[2,3-c]pyfdine-2-carboxylic acid ethyl ester (**Preparation 26**, 0.100g, 0.370mmol) under an argon atmosphere. 1,4-Dioxane (7mL, anhydrous) followed by diisopropylamine (0.063mL, 0.45mmol) were added and the stirred mixture was purged with argon for 5min. Trimethylsilylacetylene (0.064mL, 0.45mmol) was added dropwise and the resulting mixture stirred at rt for 24h. The reaction mixture was partitioned between water (50mL) and ethyl acetate (100mL) and the layers separated. The aqueous phase was extracted with ethyl acetate (3x30mL). The combined organics were washed with brine (50mL), dried (MgSO₄), filtered and concentrated *in vacuo.* The residue was dissolved in the minimum amount of dichloromethane and loaded onto a silica column. Purification *via* flash column chromatography (SiO₂, dichloromethane then 25% ethyl acetate / isohexane) gave a pale yellow solid. δ_{H} (CDCl₃): 0.28 (9H, s), 1.43 (3H, t), 4.45 (2H, q), 7.18 (1H, s), 7.83 (1H, s), 8.86 (1H, s), 9.21 (1H, br s).

### Preparation 59 : 5-Ethynyl-1H pyrrolo[2,3-c]pyridine-2-carboxylic acid ethyl ester

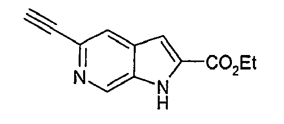

To a stirred solution of the ester (**Preparation 58**, 0.123g) was added tetra-*n-*butylammonium fluoride (1.0M in THF, 5%wt H₂O, 0.47mL, 0.47mmol) and the solution immediately turned dark pink in colour. After 5min the reaction was partitioned between ethyl acetate (60mL) and water (40mL). The layers were separated and the aqueous layer extracted with ethyl acetate (2x20mL). Glacial acetic acid was added to the combined organics until the colour changed from pink to yellow. The solution was washed with water (20mL), brine (20mL) then dried (MgSO₄), filtered and concentrated *in vacuo.* The residue was dissolved in methanol and adsorbed onto silica gel. Purification *via* flash column chromatography (SiO₂, ethyl acetate : isohexane, 1:1, v/v) gave the title compound as a pale yellow powder. δ_{H} (EDCl₃): 1.44 (3H, t), 3.08 (1H, s), 4.46 (2H, q), 7.20 (1H, s), 7.85 (1H, s), 8.89 (1H, s), 9.33 (1H, s).

### Preparation 60 : 5-Ethynyl-1H-pyrrolo[2,3-c]pyridine-2-carboxylic acid

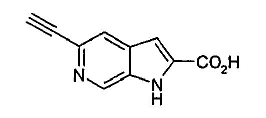

To a suspension of ester (**Preparation 59**, 0.065g, 0.30mmol) in ethanol (6mL) was added sodium hydroxide (2M aqueous, 1.5mL, 3.0mmol) and the reaction mixture stirred at 50°C for 3h. The mixture was allowed to cool to rt and glacial acetic acid added, causing precipitation of a white solid. This was collected by filtration, washed with water (20mL) and then diethyl ether (20mL). The solid was air dried to give the title compound as a white powder. m/z (ES⁺) = 187 [*M* + H]⁺; RT = 1.85min.

### Preparation 61 : 5-Cyano-1H-pyrrolo[2,3-c]pyridine-2-carboxylic acid ethyl ester

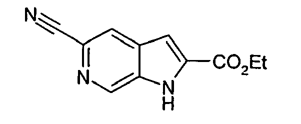

To a solution of 5-bromo-1*H*-pyrrolo[2,3-c]pyridine-2-carboxylic acid ethyl ester (**Preparation 26**, 0.160g, 0.590mmol) in DMF (anhydrous, 5mL) was added zinc (II) cyanide (0.041g, 0.35mmol) then *tetrakis*-triphenylphosphine palladium (0). The reaction mixture was degassed by bubbling argon through it for 10min. The reaction mixture was heated to reflux temperature for 4.5h then allowed to cool to rt. Water (30mL) was added and the mixture extracted with ethyl acetate (2x50mL). The combined organics were washed with brine (30mL), dried (MgSO₄), filtered and *concentrated in vacuo.* The residue was dissolved in ethyl acetate then adsorbed onto silica gel. Purification *via* flash column chromatography (SiO₂, ethyl acetate : isohexane, 1:3, v/v) gave the title compound as a white solid. δ_{H} (CDCl₃): 1.45 (3H, t), 4.49 (2H, quartet), 7.31 (1H, s), 8.09 (1H, s), 8.97 (1H, s), 9.60 (1H, br s); m/z (ES⁺) = 216 [*M* + H]⁺; RT = 3.03min.

### Preparation 62 : 5-Cyano-1H-pyrrolo[2,3-c]pyridine-2-carboxylic acid

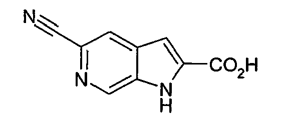

To a stirred suspension of ester (**Preparation 61**, 0.266g, 1.24mmol) in ethanol (6mL) and water (0.6mL) was added sodium hydroxide (0.108g, 2.72mmol). The reaction mixture was heated to 50°C for 24h then allowed to cool to rt. The reaction mixture was diluted with diethyl ether (30mL), collected by filtration and washed with diethyl ether. The solid was washed with aqueous acetic acid (2x50mL), then diethyl ether. The solid was air-dried to give the title compound as a white solid. δ_{H} (d₆ DMSO): 7.22 (1H, s), 8.37 (1H, s), 8.88 (1H, s), 12.88 (1H, s).

### Preparation 63 : 2,4-Dimethyl-5-nitropyridine

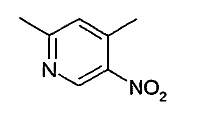

To a stirred suspension of 2-chloro-4-methyl-5-nitropyridine (9.419g, 54.6mmol) in dioxane (110mL) was added *tetrakis*(triphenyl phosphine) palladium (6.330g, 5.46mmol) and the mixture stirred for 15min prior to the addition of trimethyl boroxine (7.68mL, 54.6mmol) and potassium carbonate (22.64g, 164.0mmol). The reaction mixture was heated under reflux for 6h then allowed to cool to rt over 16h. Ethyl acetate (200mL) was added and the mixture stirred vigorously for 1h. The mixture was filtered through celite, washing through with ethyl acetate / THF (1:1, v/v). Brine (100mL) was added and attempted separation of layers resulted in a thick emulsion. After removal of all volatiles, ethyl acetate (300mL) was added and the mixture filtered, giving rise to a biphasic mixture. The layers were separated and the aqueous phase was extracted with ethyl acetate (3 x 100mL). The combined organics were washed with brine (100mL), dried (MgSO₄), filtered and concentrated *in vacuo.* The resulting oil was dissolved in dichloromethane then purified *via* flash column chromatography (SiO₂, ethyl acetate / isohexane, 3:7, v/v) to give the title compound as an orange oil. δ_{H} (CDCl₃): 2.59 (3H, s), 2.60 (3H, s), 7.12 (1H, s), 9.07 (1H, s).

### Preparation 64 : 3-(2-Methyl-5-nitropyridin-4-yl)-2-oxopropionic acid ethyl ester

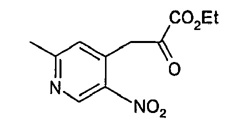

To a solution of potassium ethoxide (0.262g, 2.96mmol) in diethyl ether (10mL) and ethanol (1mL) was added diethyl oxalate (0.404mL, 2.96mmol) in one portion and the resulting solution stirred for 10min at rt. 2,4-Dimethyl-5-nitropyridine (**Preparation 63**, 0.400g, 2.63mmol) was added as a suspension in diethyl ether (1mL) / ethanol (1.5mL) and stirring continued for 16h at rt. The mixture was filtered, washing with cold diethyl ether. The collected precipitate was dissolved in water and the pH adjusted to 4 by the addition of glacial acetic acid. The resulting precipitate was collected by filtration and air dried. The solid was partitioned between ethyl acetate (150mL) and water (50mL) and the layers separated. The aqueous layer was extracted with ethyl acetate (3x20mL) and the combined organics washed with brine (50mL), dried (MgSO₄), filtered and concentrated under reduced pressure to give the title compound as a red solid which required no further purification. δ_{H} (CDCl₃): 1.40 (3H, t), 4.40 (2H, q), 4.52 (2H, s), 7.11 (1H, s), 9.25 (1H, s).

### Preparation 65 : 5-Methyl-1H-pyrrolo[2,3-c]pyridine-2-carboxylic acid ethyl ester

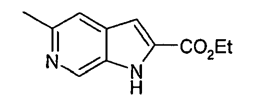

To a solution of the pyruvate (**Preparation 64,** 0.749g, 2.97mmol) in THF (30mL) and ethanol (15mL) was added saturated aqueous ammonium chloride solution (15mL) and the suspension vigorously stirred at rt. Iron powder (1.38g, 24.64mmol) was added portionwise and the mixture heated under reflux for 2h then allowed to cool prior to filtration through a celite plug, and washed through with warm methanol. The mixture was concentrated under reduced pressure, the residue partitioned between ethyl acetate (250mL) and water (250mL) and the layers separated. The aqueous phase was extracted with ethyl acetate (3x50mL) then the combined organics were washed with brine (100mL), dried (MgSO₄) filtered and concentrated *in vacuo.* The residue was adsorbed onto silica gel and purified *via* flash chromatography (SiO₂, ethyl acetate) to give the title compound as a pale orange solid. δ_{H} (CDCl₃): 1.42 (3H, t), 2.63 (3H, s), 4.43 (2H, q), 7.10 (1H, s), 7.40 (1H, s), 8.81 (1H, s), 9.08 (1H, br s).

### Preparation 66 : 5-Methyl-1H-pyrrolo[2,3-c]pyridine-2-carboxylic acid

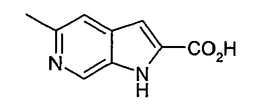

To a stirred solution of ester (**Preparation 65**, 117mg, 0.574mmol) in ethanol (10mL) was added aqueous sodium hydroxide (2M, 0.43mL, 0.861mmol). The resulting solution heated to 55°C for 4h then allowed to cool to room temperature and stirred for 17h. Excess glacial acetic acid was added then all volatiles were removed under reduced pressure. The residue was triturated with water and the resulting solid collected by filtration, washed with water then air dried. The title compound was isolated as a pale yellow solid. m/z (ES⁺) = 177 [*M* + H]⁺; RT = 1.60min.

### Preparation 67 : 4-Methoxypiperidine-1-carboxylic acid tert-butyl ester

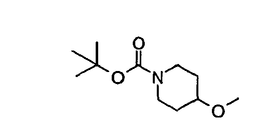

To a vigourosly stirred solution of 4-hydroxypiperidine-1-carboxylic acid *tert-*butyl ester (2.07g, 10.3mmol) in DMF (25mL) was added a 60% sodium hydride dispersion in mineral oil (500mg, 12.5mmol). After stirring for 20min, methyl iodide (0.9mL, 14.5mmol) was added and the resulting mixture stirred for 48h before being added to a mixture of water and brine (250mL, 1:1). Extraction with ethyl acetate (4x50mL), washing of the combined extracts with brine (100mL) and drying (MgSO₄) gave, after concentration, a residue which was purified via flash chromatography (silica gel, ethyl acetate/hexane, 1:1) to give the title compound as a colourless oil. δ_{H} (CDCl₃): 1.50 (9H, s), 1.52, 1.88 (4H, 2m) 3.12 (2H, ddd), 3.18 (1H, m), 3.19 (3H, s), 3.77 (2H, m); R_{f} 0.33 (ethyl acetate/hexane : 1/1).

### Preparation 68 : 4-Methoxypiperidine hydrochloride

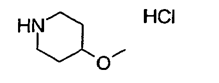

To a solution of 4-methoxypiperidine-1-carboxylic acid *tert*-butyl ester (**Preparation 67**, 1.58g, 7.34mmol) in methanol (20mL) was added hydrochloric acid in 1,4-dioxane (4M, 10mL) and the mixture stirred for 3h at rt. Concentration *in vacuo* gave an oil which was redissolved in water (100mL). The aqueous layer was washed with ethyl acetate (2x30mL) and concentrated to give the title compound as colourless solid. δ_{H} (D₂O): 1.80,2.14 (4H, 2m), 3.13 (2H, m), 3.38 (2H, m), 3.40 (3H, s), 3.68 (m, 1H).

### Preparation 69 : (R)-3-Methoxypyrrolidine-1-carboxylic acid tert-butyl ester

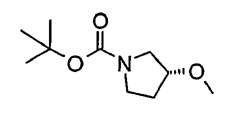

(*R*)-3-Hydroxypyrrolidine-1-carboxylic acid *tert*-butyl ester (Sigma-Aldrich) (1.04g, 5.55mmol) was methylated and purified in a similar way to **Preparation 67** using 60% sodium hydride dispersion (267mg, 6.68mmol) and methyl iodide (0.5mL, 8.06mmol) in DMF (15ml). δ_{H} (CDCl₃): 1.50 (9H, s),1.94-2.02 (2H, m), 3.37 (3H, s), 3.38-3.58 (4H, m), 3.95 (1H, m); R_{f} 0.47 (ethyl acetate/hexane: 1/1).

### Preparation 70 : (R)-3-Methoxypyrrolidine hydrochloride

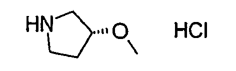

(*R*)-3-Methoxypyrrolidine-1-carboxylic acid *tert*-butyl ester (840mg, 4.17mmol) was deprotected and purified in a similar way to **Preparation 68** using methanol (10mL) and hydrochloric acid in 1,4-dioxane (4 M, 5.0mL). δ_{H} (D₂O): 2.12 (2H, m), 3.10-3.56 (8H, m), 4.20 (1H, m).

### Preparation 71 : (S)-3-Methoxypyrrolidine-1-carboxylic acid tert-butyl ester

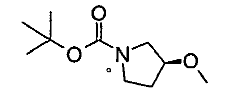

(*S*)-3-Hydroxypyrrolidine-1-carboxylic acid *tert*-butyl ester (Omega Chemical Company) (950mg, 5.55mmol) was methylated and purified in a similar way to **Preparation 67** using 60% sodium hydride dispersion (260mg, 6.50mmol) and methyl iodide (0.5mL, 8.06mmol) in DMF (15ml). ¹H NMR and R_{f} were identical to the (*R*)-enantiomer.

### Preparation 72 : (S)-3-Methoxypyrrolidine hydrochloride

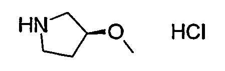

(*S*)-3-Methoxypyrrolidine-1-carboxylic acid *tert*-butyl ester (720mg, 3.58mmol) was deprotected and purified in a similar way to **Preparation 68** using methanol (10mL) and hydrochloric acid in 1,4-dioxane (4 M, 5.0mL). ¹H NMR and R_{f} were identical to (*R*)-enantiomer.

### Preparation 73 : 4-(2-Nitrobenzenesulfonylamino)piperidine-1-carboxylic acid tert-butyl ester

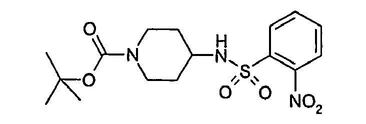

To a solution of 4-aminopiperidine-1-carboxylic acid *tert*-butyl ester (400mg, 200mmol) in anhydrous dichloromethane (14mL) under argon was added triethylamine (340µL, 2.4mmol) and the solution cooled in an ice bath. 2-Nitrophenylsulfonyl chloride (443mg, 2.0mmol) was added and the reaction allowed to stir at rt for 16h. The crude solution was washed with water (2x30mL) before removing the solvent *in vacuo.* The crude material was purified by chromatography using ethyl acetate/petroleum ether (30-60%) as the eluent to give the title compound as an off-white powder. δ_{H} (d₆ DMSO): 8.43 (2H, m), 8.17 (1H, s), 8.09 (2H, m), 3.80-3.69 (2H, m), 3.31-3.23 (1H, m), 2.86-2.69 (2H, m), 1.63-1.51 (2H, m), 1.39 (9H, s), 1.29-1.17 (2H, m).

### Preparation 74 : 4-[Methyl-(2-nitrobenzenesulfonyl)amino]piperidine-1-carboxylic acid tert-butyl ester

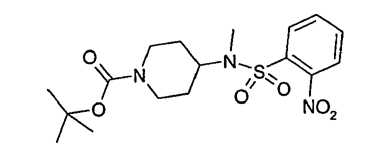

To a solution of 4-(2-nitrobenzenesulfonylamino)piperidine-1-carboxylic acid *tert*-butyl ester (**Preparation 73**) (400mg, 1.04mmol) in DMF (10mL) was added caesium carbonate (507mg, 1.56mmol) and the mixture stirred at rt for 40 min. Iodomethane (323µL, 5.19mmol) was added and the mixture stirred for 16h. Solvent was removed *in vacuo* and the crude residue partitioned between ethyl acetate (20mL) and water (20mL). The organic layer was separated and washed with 1M HCl (2x20mL), water (20mL), then brine (2x20mL) before being dried (MgSO₄). The solvent was removed *in vacuo* to give the title compound as a yellow powder. δ_{H} (d₆ DMSO): 8.43 (2H, m), 8.01 (2H, m), 4.00-3.89 (3H, m), 2.83-2.67 (5H, m), 1.54-1.43 (2H, m), 1.39 (9H, s), 1.34-1.24 (2H, m).

### Preparation 75 : N-Methyl-2-nitro-N-piperidin-4-yl benzenesulfonamide

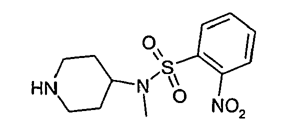

Prepared according to **EXAMPLE 182** from 4-[methyl(2-nitrobenzenesulfonyl)amino]piperidine-1-carboxylic acid *tert*-butyl ester (**Preparation 74**). δ_{H} (d₆-DMSO): 8.44 (2H, m), 8.13 (2H, m), 4.20-7.07 (1H, m), 3.29-3.17 (2H, m), 3.03-2.90 (2H, m), 2.77 (3H, s), 2.00-1.84 (2H, m), 1.51-1.43 (2H, m).

### Preparation 76 : 5-Chloro-1H-pyrrolo[2,3-c]pyridine-2-carboxylic acid (1-(S)-(4-fluorobenzyl)-2-{4-[methyl(2-nitrobenzenesulfonyl)amino]piperidin-1-yl}-2-oxoethyl)amide

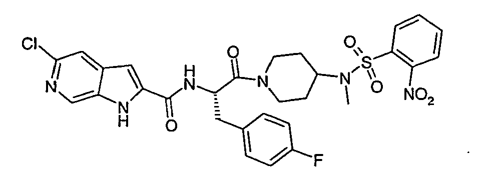

Prepared according to **EXAMPLE 231** from 2-(*S*)-[(5-chloro-1H-pyrrolo[2,3-c]pyridine-2-carbonyl)amino]-3-(4-fluorophenyl)-propionic acid (**EXAMPLE 230**) and N-methyl-2-nitro-N-piperidin-4-yl-benzenesulfonamide hydrochloride (**Preparation 75**). m/z (ES⁺) = 643.36 [*M* + H]⁺.

### Preparation 77 : Thiomorpholine 1,1-dioxide

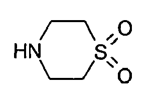

To a solution of thiomorpholine (1.0g, 9.69mmol) in acetic acid (11.5mL) cooled to 0°C (ice bath) was added aqueous hydrogen peroxide solution (30% w/v, 4mL) and the reaction heated to 100°C for 16h. The mixture was cooled and solvent removed *in vacuo* before trituration of the residue with methanol gave a white precipitate. The solid was filtered and washed with methanol to give the title compound as an off-white powder. m/z (ES⁺) = 136.06 [*M* + H]⁺.

### Preparation 78 : Piperidine-4-carboxylic acid methyl ester hydrochloride

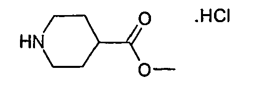

To a cooled solution of anhydrous methanol (100mL) was added acetyl chloride (7.1mL, 0.1mol) and the solution stirred for 75min. Piperidine-4-carboxylic acid (150mg, 1.16mmol) was dissolved in the prepared solution (10mL) and the reaction stirred for 16h. The solvent was removed *in vacuo* to give the title compound as its hydrochloride salt. m/z (ES⁺) = 144.12 [*M* + H]⁺.

### Preparation 79 : 2-(S)-Carbamoyl-piperidine-1-carboxylic acid tert-butyl ester

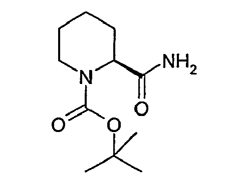

To a solution ofN-Boc-L-pipecolinic acid (200mg, 0.87mmol) in DMF (3.5mL) was added TBTU (336mg, 1.05mmol), ammonium chloride (93mg, 1.74mmol) then DIPEA (182µL, 1.05mmol) and the reaction stirred for 16h. The mixture was partitioned between ethyl acetate (2x30mL) and water (30mL), and the organics combined before being washed with 1M sodium hydroxide (3x30mL) and brine (3x30mL). The organic solution was dried (MgSO₄) and solvent removed *in vacuo* to give the title compound as a white solid. δ_{H} (d₆ DMSO): 7.23 (1H, s), 6.97 (1H, s), 4.56-4.39 (1H, br m), 3.43-3.74 (1H, d), 3.14-2.90 (1H, br m), 2.09-2.00 (1H, d), 1.63-1.47 (3H, m), 1.30-1.17 (2H, m).

### Preparation 80 : (S)-Piperidine-2-carboxylic acid amide hydrochloride

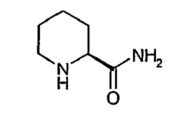

Piperidine-2-carboxylic acid amide was prepared according to the method of Johnson et. al., (J. Med. Chem., 1986, 29, 2100-2104) to give the title compound as an off-white solid. 5_{H} (CD₃OD): 3.87-3.75 (1H, m), 3.43-3.34 (1H, m), 3.09-2.96 (1H, br m), 2.31-2.16 (1H, m), 1.97-1.81 (2H, br m), 1.77-1.57 (3H, br m).

### Preparation 81 : 4-(2-Methoxyethoxy)piperidine-1-carboxylic acid tert-butyl ester

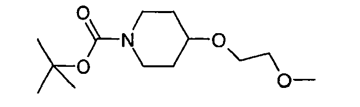

To a solution of *tert*-butyl-4-hydroxy-1-piperidine carboxylate (300mg, 1.49mmol) in DMF (2mL) was added 1-bromo-2-methoxyethane (168µL, 1.79mmol) followed by potassium iodide (25mg, 0.15mmol) and sodium hydride (83.5mg, 2.09mmol) and the reaction stirred at rt for 16h. Solvent was *removed in vacuo* and crude residue was partitioned between ethyl acetate (10mL) and water (10mL). The organic layer was washed with 1M HCl (10mL), 1M NaOH (10mL) then brine (2x10mL) before being dried (MgSO₄) and removing the solvent *in vacuo.* Purification by chromatography using dichloromethane/methanol (97:3) as the eluent gave the title compound as a yellow oil. δ_{H} (CDCl₃): 3.89-3.77 (2H, m), 3.66 (2H, m), 2.57 (2H, m), 3.54-3.46 (1H, m), 3.43 (3H, s), 3.11-3.03 (2H, m), 1.93-1.83 (2H, m), 1.60-1.46 (11H, m).

### Preparation 82 : 4-(2-Methoxyethoxy)piperidine hydrochloride

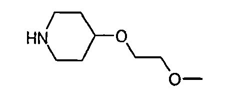

To a solution of 4-(2-methoxyethoxy)-piperidine-1-carboxylic acid tert-butyl ester (**Preparation 81**, 114mag, 0.44mmol) in methanol (3mL) was added 4M HCl in dioxane (550µL, 2.20mmol) and the reaction stirred at rt for 16h. Solvent was *removed in vacuo* and the crude residue dissolved in water (10mL). The aqueous solution was extracted with ethyl acetate (2x10mL) then concentrated *in vacuo.* Purification by trituration in ethyl acetate gave the title compound as the hydrochloride salt. δ_{H} (CD₃OD): 3.74-3.67 (1H, m), 3.64 (2H, m), 3.56 (2H, m), 3.40-2.39 (7H, m), 2.11-1.96 (2H, m), 1.93-1.83 (2H, m).

### Preparation 83 : [1-(R)-(4-Fluorobenzyl)-2-(4-hydroxypiperidin-1-yl)-2-oxoethyl] carbamic acid tert-butyl ester

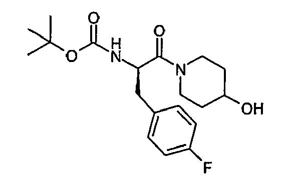

The title compound was prepared according to **EXAMPLE 231** but using Boc-3-(4-fluorophenyl)-(*R*)-alanine and 4-hydroxypiperidine. m/z (ES⁺) = 367.34 [*M*+H]⁺.

### Preparation 84 :2-(R)-Amino-3-(4-fluorophenyl)-1-(4-hydroxypiperidin-1-yl)propan-1-one hydrochloride

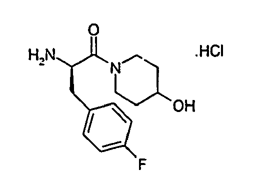

The title compound was prepared according to **Preparation 20** from [1-(*R*)-(4-fluorobenzyl)-2-(4-hydroxypiperidin-1-yl)-2-oxoethyl] carbamic acid *tert*-butyl ester (**Preparation 83**). m/z (ES⁺) = 267.20 [*M* + H]⁺.

### Preparation 85 : 4-(N-Benzyl-N-methylamino)piperidine-1-carboxylic acid tert-butyl ester

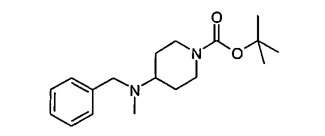

To a solution of *N*-benzylmethylamine (648µL, 5.02mmol) and 1*-tert-*butoxycarbonyl-4-piperidone (500mg, 2.51mmol) in THF (8mL) was added sodium triacetoxyborohydride (798mg, 3.76mmol) followed by acetic acid (144µL, 2.51mmol) and the reaction stirred at rt for 40h. Solvent was *removed in vacuo* and the residue partitioned between ethyl acetate (15mL) and water (15mL). The organic layer was washed with sodium bicarbonate solution (2x15mL) then brine (2x20mL), dried (MgSO₄) and the solvent *removed in vacuo.* The crude material was purified by chromatography using ethyl acetate/petroleum ether (2:1) as the eluent to give the title compound as yellow oil. m/z (ES⁺) = 305.32 [*M* + H]⁺.

### Preparation 86 : 4-Methylaminopiperidine-1-carboxylic acid tert-butyl ester

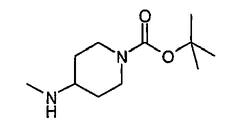

The title compound was prepared according to **Preparation 23** from 4-(benzylmethylamino)piperidine-1-carboxylic acid *tert*-butyl ester (**Preparation 85**). δ_{H} (CD₃OD): 4.13-4.03 (2H, m), 2.90-2.76 (2H, br m), 2.63-2.53 (1H, m), 2.40 (3H, s), 2.97-1.87 (2H, m), 1.49 (9H, s), 1.27-1.16 (2H, m).

### Preparation 87 : Benzylmethyl(tetrahydropyran-4-yl)amine

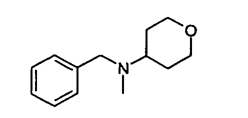

The title compound was prepared according to **Preparation 85** from *N*-benzylmethylamine and tetrahydro-4H-pyran-4-one. Purification of the crude material by chromatography using dichloromethane/methanol (98:2) as the eluent gave the title compound as a yellow oil. δ_{H} (CD₃OD): 7.50-7.40 (5H, m), 4.08 (2H, m), 4.06- (2H, s), 3.49-3.40 (2H, ddd), 3.23-3.14 (1H, m), 2.51 (3H, s), 2.03-1.97 (2H, m), 1.89-1.76 (2H, m).

### Preparation 88 : Methyl(tetrahydropyran-4-yl)amine hydrochloride

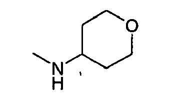

The title compound was prepared according to **Preparation 23** from benzylmethyl(tetrahydropyran-4-yl)amine (**Preparation 87**). Crude material was dissolved in methanol and a solution of 1M HCl in ether was added dropwise to form a precipitate. The product was filtered and washed with ether to give the title compound as the hydrochloride salt as a white crystalline solid. δ_{H} (CD₃OD) 4.11-4.00 (2H, m), 3.53-3.43 (2H, m), 3.37-3.27 (1H, m), 2.74 (3H, s), 2.11-2.03 (2H, m), 1.74-1.60 (2H, m).

### Preparation 89 : 1-Benzylpiperidin-4-yl-dimethylamine

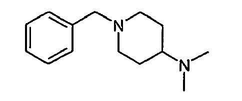

To a solution of 4-amino-1-benzylpiperidine (536µL, 2.63mmol) in formic acid (8.5mL) at 0°C was added formaldehyde solution (37%, 5.5mL) and the mixture heated to reflux for 6h. Solvent was removed *in vacuo* and the crude residue partitioned between ethyl acetate (20mL) and water (20mL). The aqueous layer was separated and taken to pH12 with 2M NaOH solution before being extracted with ethyl acetate (2x20mL). The organic portion was washed with brine (30mL) and dried (MgSO₄) before removing the solvent *in vacuo* to give the title compound as a yellow oil. m/z (ES⁺) = 219.25 [*M*+H]⁺.

### Preparation 90 : Dimethylpiperidin-4-yl amine

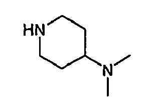

The title compound was prepared according to **Preparation 23** from 1-benzylpiperidin-4-yl-dimethylamine (**Preparation 89**). δ_{H} (CD₃OD) : 3.17-3.09 (2H, m), 2.66-2.54 (2H, m), 2.30 (6H, s), 2.26 (1H, m), 1.94-1.86 (2H, m), 1.49-1.29 (2H, m).

### Preparation 91 : 4-Methanesulfonylamino-piperidine-1-carboxylic acid tert-butyl ester

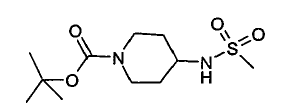

To a solution of 4-amino-1-Boc-piperidine (300mg, 1.50mmol) in dichloromethane (2.0mL) was added a solution of methanesulfonyl chloride (348µL, 4.49mmol) in dichloromethane (1mL) followed by a solution of pyridine (485µL, 5.99mmol) in dichloromethane (1mL), and the reaction stirred at rt for 16h. Water (10mL) was added, the mixture separated and the organic layer washed with 1M HCl (10mL), sodium bicarbonate solution (10mL) then brine (2x10mL). The solution was dried (MgSO₄) and solvent removed *in vacuo.* The crude material was purified by chromatography using dichloromethane/methanol (95:5) as the eluent to give the title compound as an off-white powder. δ_{H} (CD₃OD) 4.46-4.40 (1H, m), 4.09-3.97 (2H, m), 3.53-3.40 (1H, m), 3.0 (3H, s), 2.93-2.81 (2H, m), 2.01-1.93 (2H, m), 1.50-1.37 (2H, m).

### Preparation 92 : N-Piperidin-4-yl methanesulfonamide

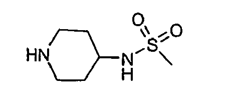

The title compound was prepared from 4-methanesulfonylaminopiperidine-1-carboxylic acid *tert*-butyl ester (**Preparation 91**) according to **Preparation 82** as an off-white powder. δ_{H} (CD₃OD): 3.67-3.57 (1H, m), 3.47-3.40 (2H, m), 3.19-3.10 (2H, m), 3.03 (3H, s), 2.26-2.17 (2H, m), 1.87-1.76 (2H, m).

### Preparation 93 : 2-(S)-Amino-3-pyridin-4-yl propionic acid methyl ester hydrochloride

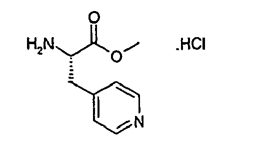

The title compound was prepared according to **Preparation 78**, using Boc-3-(4-pyridyl)-L-alanine as the starting acid. δ_{H} (CD₃OD): 8.90 (2H, br s), 8.16 (2H, br s), 4.73-4.60 (1H, m), 3.84 (3H, s), 3.73-3.50 (2H, m).

### Preparation 94 : 1,4-Dioxa-7-aza-spiro [4.5] decane

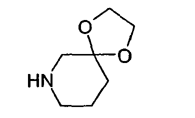

To a solution of 1-Boc-3-piperidone (700mg, 3.51mmol) in toluene (20mL) was added ethylene glycol (588µL, 10.54mmol) followed by p-toluene sulfonic acid hydrate (1.0g, 5.27mmol) and the reaction heated to reflux using Dean-Stark apparatus for 7h. To the mixture was added NaHCO₃ solution, and the organic layer was removed. The aqueous phase was evaporated to dryness and the resulting residue dissolved in THF. Filtration through celite and removal of the solvent *in vacuo* gave the desired product as light brown oil. δ_{H} (CD₃OD): 3.99 (2H, s), 3.73-3.57 (2H, m), 3.63 (4H, m), 2.77 (1H, m), 2.73 (1H, m), 1.76 (2H, m).

### Preparation 95 : 2-Phenyl-1-(S)-(2-phenyl-[1,3]dioxolan-2-yl)ethylamine

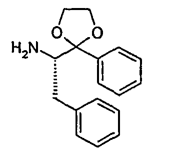

To a solution of commercially available 2-(*S*)-(*N*-tert-butyloxycarbonyl)-amino-1,3-diphenyl-1-propanone (250mg, 0.768mmol) in toluene (100mL) was added ethylene glycol (1.0mL, 17.9mmol) and p-toluenesulfonic acid monohydrate (262mg, 1.38mmol). The resulting mixture was heated under reflux for 48h, removing water with a Dean-Stark trap. After cooling to ambient temperature the mixture was diluted with ethyl acetate (200mL) and successively washed with diluted sodium hydroxide solution (1M, 2 x 50mL) and brine (50mL). The solution was dried (MgSO₄) and concentrated to an oil that was purified by flash chromatography on silica gel (eluent: ethyl acetate) to give the title compound as colourless oil. δ_{H} (CDCl₃): 2.07 (2H, br s), 2.37 (1H, dd), 2.92 (1H, dd), 3.36 (1H, m), 3.85-4.35 (4H, 3m), 7.14-7.59 (10H, m); m/z (ES⁺) = 270.20 [*M*+H]⁺; RT = 2.63min.

### Preparation 96 : 5-Chloro-1H-pyrrolo[2,3-c]pyridine-2-carboxylic acid [2-phenyl-1-(S)-(2-phenyl-[1,3]dioxolan-2-yl)ethyl]amide

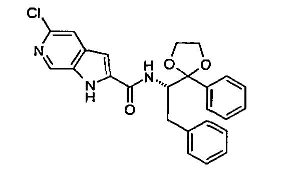

To a solution of 5-chloro-1*H*-pyrrolo[2,3-c]pyridine-2-carboxylic acid (**Preparation 18**, 88mg, 0.403mmol) and 2-phenyl-1-(*S*)-(2-phenyl-[1,3]dioxolan-2-yl)ethylamine (**Preparation 95**, 104mg, 0.386mmol) in DMF (5mL) was added HOBt (65mg, 0.424mmol), DIPEA (0.155mL, 0.890mmol) and EDCI (90mg, 0.469mmol). After stirring at rt for 12h the mixture was added to diluted brine (100mL, water/brine : 1/1). Extraction with ethyl acetate (4 x 25mL), washing of the combined extracts with diluted hydrochloric acid (1M, 30ml), diluted aqueous sodium hydroxide solution (1M, 30ml) and brine (some) followed by drying (MgSO₄) gave after concentration a residue which was purified by flash chromatography on silica gel (eluent: hexane / ethyl acetate : 50 / 50). The title compound was obtained as a colourless oil. δ_{H} (CDCl₃): 2.73 (1H, dd), 3.10 (1H, dd), 3.84-4.22 (4H, 4m), 5.01 (1H, ddd), 6.37 (1H, d), 6.72 (1H, s), 7.05-7.62 (11H, 3m), 8.64 (1H, s), 10.48 (1H, s); m/z (ES⁺) = 448.24 [*M*+H]⁺; RT = 3.64min.

### Preparation 97 : 2-(2-Oxo-2-pyridin-3-yl-ethyl)isoindole-1,3-dione

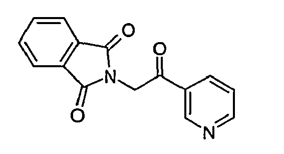

A solution of bromomethylpyridin-3-yl ketone (4.55g, 16.2mmol) and potassium phthalimide (6.0g, 32.4mmol) in DMF (50mL) was stirred for 3 days at rt before the added to diluted brine (500ml, 1 :1). The solution was made acidic (pH 2) with diluted hydrochloric acid (1M) before washed with ethyl acetate (2 x 100ml). The aqueous layer was then made alkaline (pH 12) again with sodium hydroxide solution (2 M) and extracted with DCM (4 x 200ml). The extracts were combined and dried (MgSO₄) before concentrated *in vacuo.* Recrystallisation from methanol (2x) allows to remove crystalline phthalimide and to enrich the title compound in the mother liquer. The crude product was used in **Preparation 98** without further purification. δ_{H} (d₆ DMSO): 5.33 (2H, s), 7.64 (1H, dd), 7.92, 7.97 (4H, 2m), 8.43 (1H, m), 8.88 (1H, m), 9.28 (1H, s); m/z (ES⁺) = 308.13 [*M*+ MeCN + H]⁺; RT = 2.39min.

### Preparation 98 : 2-Amino-1-pyridin-3-ylethanol

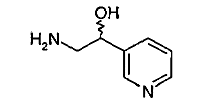

To a solution of crude 2-(2-oxo-2-pyridin-3-ylethyl)isoindole-1,3-dione (**Preparation 97**, 5.0g, ∼19.0mmol) in aqueous isopropanol (210ml, water/IPA : 1/6) was added sodium borohydride (10.2g, 270mmol) in 2 portions. The mixture was stirred at rt for 12h before being carefully acidified (pH 2) with dilute hydrochloric acid (1M). After removal of the solvent the residue was taken up in destillated water (100mL) and passed down a column filled with ion-exchange resin (Amberlite IR 120, H⁺-form, 300g, eluent: 500mL water then 1L of 2 M aqueous ammonia solution). Concentration of the alkaline fractions gave the title compound as a yellow oil. δ_{H} (d₆ DMSO): 2.74, 2.85 (2H, 2m), 4.66 (1H, m), 5.15 (3H, br s), 7.36 (1H, dd), 7.75 (1H, m), 8.46 (1H, m), 8.56 (1H, m); m/z (ES⁺) = 139.11 [*M* + H]⁺; RT = 0.21min.

### Preparation 99 : 2-(S)-Amino-3-(tert-butyldimethylsilanyloxy)-1-(S)-phenylpropan-1-ol

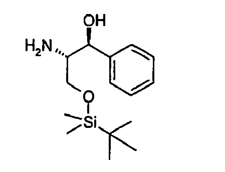

To a solution of (1*S*,2*S*)-2-amino-1-phenyl-1,3-propanediol (2.07g, 12.4mmol) in DMF (10mL) was added imidazole (1.0g, 14.7mmol) and *tert*-butyldimethylsilyl chloride (2.30g, 15.3mmol). After stirring at rt for 12h the mixture was added to diluted brine (150mL, water/brine : 1/1). Extraction with ethyl acetate (4 x 30mL), washing of the combined extracts with brine (30mL) and drying (MgSO₄) gave after concentration a residue which was purified by flash chromatography on silica gel (eluent: ethyl acetate). The title compound was obtained as colourless oil. δ_{H} (CDCl₃): 0.24, 0.26 (6H, 2s), 1.12 (9H, s), 2.50-2.78 (3H, br s), 3.16 (1H, ddd), 3.78 (1H, dd), 3.85 (1H, dd), 4.84 (1H, d), 7.49-7.55 (5H, m); m/z (ES⁺) = 282.32 [*M*+ H]⁺; RT = 2.87min.

### Preparation 100 : 5-Chloro-1H-pyrrolo[2,3-c]pyridine-2-carboxylic acid [1-(S)-(tert-butyldimethylsilanyloxymethyl)-2-(S)-hydroxy-2-phenylethyl]amide

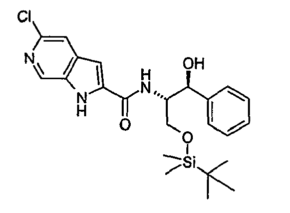

To a solution of 5-chloro-1*H*-pyrrolo[2,3-c]pyridine-2-carboxylic acid (**Preparation 18**, 520mg, 2.65mmol) and 2-amino-3-(*tert*-butyldimethylsilanyloxy)-1-phenylpropan-1-ol (**Preparation 99**, 780mg, 2.77mmol) in DMF (15mL) was added HOBt (411mg, 2.68mmol), DIPEA (0.96mL, 5.51mmol) and EDCI (589mg, 3.07mmol). After stirring at rt for 12h the mixture was added to diluted brine (150mL, water/brine : 1/1). Extraction with ethyl acetate (4 x 50mL), washing of the combined extracts with diluted hydrochloric acid (1M, 50ml), diluted aqueous sodium hydroxide solution (1M, 50ml) and brine (50mL) followed by drying (MgSO₄) gave after concentration a residue which was purified by flash chromatography on silica gel (eluent: hexane / ethyl acetate : 50 / 50). The title compound was obtained as colourless oil. δ_{H} (CD₃OD) 0.00, 0.01 (6H, 2s), 0.84 (9H, s), 3.60 (1H, dd), 3.84 (1H, dd), 4.33 (1H, ddd), 4.98 (1H, d), 7.05 (1H, s), 7.12-7.37 (5H, 3m), 7.60 (1H, s), 8.49 (1H, s); m/z (ES⁺) = 460.36 [*M* + H]⁺; RT = 4.16min.

### Preparation 101 : 5-Chloro-1H-pyrrolo[2,3-c]pyridine-2-carboxylic acid [1-(S)-(tert-butyldimethylsilanyloxymethyl)-2-oxo-2-phenylethyl]amide

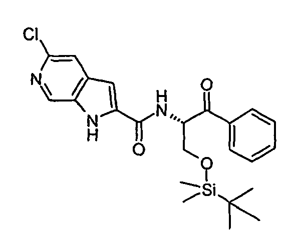

To a solution of 5-chloro-1H-pyrrolo[2,3-c]pyridine-2-carboxylic acid [1-(*S*)-(*tert*-butyldimethylsilanyloxymethyl)-2-(*S*)-hydroxy-2-phenylethyl]amide (**Preparation 100**, 304mg, 0.661mmol) in dry DCM (10mL) was added Dess-Martin periodinane (342mg, 0.806mmol). After stirring for 3h at room temperature alkaline sodium thiosulfate solution was added (5.4g Na₂SO₃ dissolved in 20mL saturated NaHCO₃ solution) and the emulsion vigorously stirred for an additional 30min. Ethyl acetate (150ml) was added and the aqueous layer removed. The organic layer was washed with brine (50ml), dried (MgSO₄) and concentrated to a residue which was purified by flash chromatography on silica gel (eluent: hexane / ethyl acetate : 50/50) to give the title compound as colourless solid. m/z (ES⁺) = 458.34 [*M*+H]⁺; RT = 4.32min.

### Preparation 102 : [1-(S)-(4-Fluorobenzyl)-2-(3-(S)-hydroxypyrrolidin-1-yl)-2-oxoethyl]carbamic acid tert-butyl ester

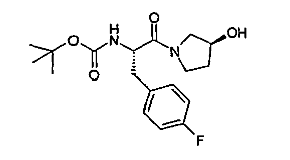

To a stirred solution of (*S*)-*N*-Boc-4-fluorophenylalanine (5.08g, 17.9mmol) and (*S*)-3-hydroxypyrrolidine (1.05mL, 19.7mmol) in anhydrous DMF (200mL), was added DIPEA (6.87mL, 39.5mmol) and HOBt.H₂O (3.02g, 19.7mmol). The reaction mixture was stirred for 10min at room temperature then EDCI (4.13g, 21.5mmol) was added and the resulting mixture stirred for 20h at rt. The volatiles were removed in *vacuo* then the residue partitioned between water (200mL) and ethyl acetate (200mL). The layers were separated and the aqueous layer extracted with ethyl acetate (3x50mL). The combined organics were washed with aqueous sodium hydroxide solution (2M, 3x50mL), brine (100mL), dried (MgSO₄), filtered and concentrated *in vacuo.* The oily residue was purified *via* flash chromatography (SiO₂, methanol / dichloromethane, 1:19, v/v) to give the title compound as a colourless oil which became a white solid on standing. m/z (ES⁺) = 353 [*M*+ H]⁺; RT = 3.17min.

### Preparation 103 : 2-(S)-Amino-3-(4-fluorophenyl)-1-(3-(S)-hydroxypyrrolidin-1-yl)propan-1-one hydrochloride

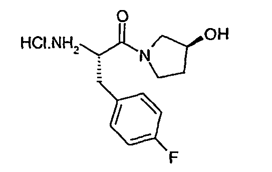

To a solution of ester (**Preparation 102**, 5.25g, 14.9mmol) in methanol (anhydrous, 30mL) was added 4M HCl in dioxane (7.64mL, 30.6mmol) and the resulting solution stirred for 18h at rt then the solvents removed *in vacuo.* The residue was partitioned between ethyl acetate (150mL) and water (100mL). The layers were separated and the organic layer extracted with water (2x50mL). The combined aqueous extracts were washed once with ethyl acetate (30mL), then the combined organic extracts were evaporated to dryness under reduced pressure to give the title compound as a white foam. δ_{H} (CD₃OD) 1.47-1.62 (0.5H, m), 1.67-1.80 (1H, m), 1.83-1.96 (0.5H, m), 2.72-2.87 (1H, m), 2.96-3.12 (2H, m), 3.19-3.29 (1H, m), 3.30-3.62 (2H, m), 4.14-4.39 (2H, m), 6.91-7.08 (2H, m), 7.22 (2H, dd).

### Preparation 104 : (6-Chloropyridin-3-yl)carbamic acid tert-butyl ester

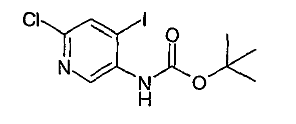

The title compound was prepared according to the method described by Dinnell et al (US 2002/0022624 A1). δ_{H} (CDCl₃): 1.52 (9H, s), 6.52 (1H, s), 7.26 (1H, d), 7.97 (1H, d), 8.23 (1H, d).

### Preparation 105 : (6-Chloro-4-iodopyridin-3-yl)carbamic acid tert-butyl ester

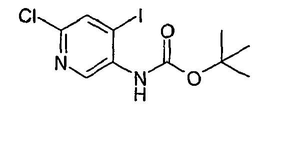

The title compound was prepared according to the method described by Dinnell et al (US 2002/0022624 A1) from the compound of **Preparation 104**. δ_{H} (CDCl₃): 1.54 (9H, s), 6.62 (1H, s), 7.72 (1H, s), 8.93 (1H, s).

### Preparation 106 : 6-Chloro-4-iodopyridin-3-ylamine

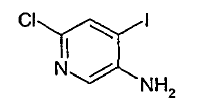

The title compound was prepared according to the method described by Dinnell et al (US 2002/0022624 A1) from the compound of **Preparation 105**. δ_{H} (CDCl₃): 4.12 (2H, br s), 7.60 (1H, s), 7.79 (1H, s).

### Preparation 107 : N-(6-Chloropyridin-2-yl)-2,2-dimethyl propionamide.

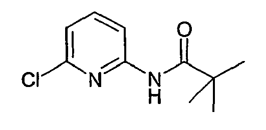

To a solution of 2-amino-6-chloropyridine (3.0g, 23.3mmol) in dichloromethane (45mL) under argon was added triethylamine (4.10mL, 29.2mmol) and the reaction cooled to 0°C (ice bath). A solution of trimethylacetyl chloride (3.16mL, 25.7mmol) in dichloromethane (10mL) was added dropwise over 20min before stirring for 30min at 0°C. The reaction was brought up to rt and stirred for a further 5h, then water (30mL) was added. The organics were separated and washed with Na₂CO₃ solution (2x50mL), dried (MgSO₄) and solvent removed *in vacuo.* Purification by column chromatography (SiO₂, CH₂Cl₂) gave the title compound, m/z (ES⁺) = 213.04 [*M*+H]⁺.

### Preparation 108 : N-(6-Chloro-3-iodopyridin-2-yl)-2,2-dimethyl propionamide.

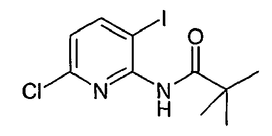

To a dry solution of N-(6-chloropyridin-2-yl)-2,2-dimethyl propionamide (**Preparation 107**, 8.0g, 37.6mmol) in THF (120mL), cooled to -78°C, was added dropwise, a solution of *tert*-butyllithium in pentane (1.7M, 48.7mL, 82.8mmol) over 40min. The reaction was stirred at -78°C for 3h before adding a solution of iodine (11.46g, 45.1mmol) in THF (40mL) dropwise. The mixture was brought up to rt and stirred for 16h. 2M HCl (30mL) was added to the reaction, and after 20min the solvent was removed *in vacuo.* Crude material was partitioned between ethyl acetate (200mL) and water (150mL). Organics were separated and washed with 10% sodium thiosulfate solution (4x100mL) then NaHCO₃ solution (2x100mL), dried (MgSO₄) and the solvent removed *in vacuo.* The residue was purified by column chromatography (SiO₂ CH₂Cl₂) to give the title compound. m/z (ES⁺) = 338.93 [*M*+ H]⁺.

### Preparation 109 : 6-Chloro-3-iodopyridin-2-ylamine.

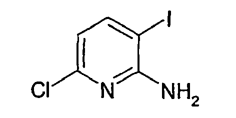

A suspension of N-(6-chloro-3-iodopyridin-2-yl)-2,2-dimethyl propionamide (**Preparation 108**, 5.0g, 14.8mmol) in 1M HCl was heated to reflux for 4.5h. The reaction was cooled to rt and then extracted with diethyl ether (2x50mL). The organics were washed with Na₂CO₃ solution (2x50mL) before being dried (MgSO₄) and the solvent removed *in vacuo.* Purification by column chromatography (SiO₂, CH₂Cl₂) afforded the title compound. δ_{H} (CDCl₃): 7.76 (1H, d), 6.46 (1H, d), 5.43-5.20 (2H, br s).

### Preparation 110 : 6-Chloro-1H-pyrrolo[2,3b]pyridine-2-carboxylic acid.

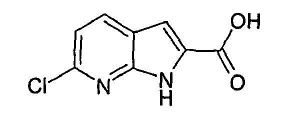

To a dry solution of 6-chloro-3-iodo-pyridin-2-ylamine (**Preparation 109**, 2.80g, 11.0mmol) in DMF (80mL) under argon was added pyruvic acid (2.29mL, 33.0mmol), DABCO (3.70g, 33.0mmol) then palladium(II)acetate (124mg, 0.55mmol) and the mixture purged with argon for 20min. The reaction was heated to 105°C (bath temp.) for 3h before being allowed to cool to rt. Solvent was removed *in vacuo* then crude material partitioned between ethyl acetate (100mL) and water (75mL). The organic layer was separated and washed with water (2x75mL) before being extracted into 2M NaOH (2x75mL). The aqueous layer was acidified to pH 3 with 2M HCl and extracted into ethyl acetate (2x100mL). Organic layers were combined, dried (MgSO₄) and concentrated *in vacuo.* The residue was suspended in water and the filtrate removed to give the title compound. m/z (ES⁺) = 196.91 [*M*+ H]⁺, RT = 3.07min.

### Preparation 111 : [1-(S)-(4-Fluorobenzyl)-2-oxo-2-(5-oxo-[1,4]diazepam-1-yl)ethyl]carbamic acid tert-butyl ester

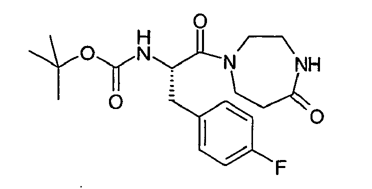

DIPEA (2.08mL, 11.95mmol), BOC-L-phenylalanine (1.128g, 3.98mmol) and HOBt (592mg, 4.38mmol) was added to a solution of [1,4]-diazepan-5-one (500mg, 4.38mmol) in DMF (10mL) and the mixture stirred for 5min. EDCI (992mg, 5.18mmol) was added and the reaction stirred for 16h before removing the solvent *in vacuo.* Purification by column chromatography (SiO₂, 9:1 CH₂Cl₂/MeOH) gave the title compound. m/z (ES⁺) = 380.00 [*M*+H]⁺.

### Preparation 112 : 1-(S)-[2-Amino-3-(4-fluorophenyl)propionyl][1,4]diazepan-5-one

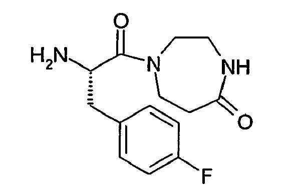

To a solution of [1-(*S*)-(4-fluorobenzyl)-2-oxo-2-(5-oxo-[1,4]diazepam-1-yl)ethyl]carbamic acid *tert*-butyl ester (**Preparation 111**, 1.23g, 3.24mmol) in methanol (15mL) was added a solution of 4M HCl in dioxane (6.48mL, 25.9mmol) and the reaction stirred for 3.5h. Solvent was removed *in vacuo* then crude material taken into water (20mL). The aqueous layer was extracted with ethyl acetate (15mL) then water removed *in vacuo* to afford the title compound as its hydrochloride salt. m/z (ES⁺) = 279.95 [*M* + H]⁺.

### Preparation 113 : 2-(S)-tert-Butoxycarbonylamino-3-(4-fluorophenyl)propionic acid tert-butyl ester

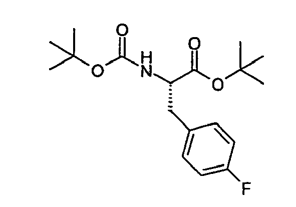

To a stirred solution of (S)-N-Boc-4-fluorophenylalanine (2.83g, 10.0mmol), DMAP (0.12g, 1.0mmol) in DCM (20mL) and 2-methyl-2-propanol (1.05mL, 11.0mmol), was added DCC (2.27g, 11.0mmol). The reaction mixture was stirred at rt for 16h. The reaction mixture was filtered and washed several times with DCM. The filtrate was concentrated *in vacuo* and chromatographed on silica gel eluting with ethyl acetate:isohexane (1:4) to give the title compound. δ_{H} (CDCl₃): 1.39 (9H, s), 1.41 (9H, s), 3.01 (2H, m), 4.41 (1H, m), 4.98 (1H, m), 6.95 (2H, m), 7.12 (2H, m).

### Preparation 114 : 2-(S)-Amino-3-(4-fluorophenyl)propionic acid tert-butyl ester hydrochloride

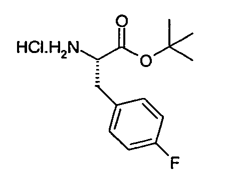

A stirred solution of ethyl acetate (10mL) and methanol (0.60mL, 14.7mmol) was cooled to 0°C under an argon atmosphere. Acetyl chloride (1.05mL, 14.7mmol) was added dropwise, and the solution warmed to rt and stirred for 30min. 2-(*S*)-*tert-*butoxycarbonylamino-3-(4-fluorophenyl)propionic acid tert-butyl ester (Preparation 113, 1g, 2.95mmol) was added, and the reaction mixture stirred at rt for 4h. The reaction mixture was filtered, washed several times with diethyl ether and dried under vacuum to give the title compound. δ_{H} (DMSO): 1.30 (9H, s), 3.01 (1H, dd), 3.20 (1H, dd), 4.08 (1H, m), 7.15 (2H, m), 7.32 (2H, m), 8.64 (3H, br s).

### Preparation 115 : 2-Chloro-5-iodopyridin-4-ylamine

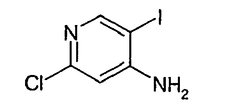

Silver sulfate (7.1g, 22.8mmol) and 4-amino-2-chloropyridine (4.06g, 31.6mmol) were added to a solution of iodine (5.65g, 22.3mmol) in ethanol (100mL) and the reaction mixture stirred at rt for 72h. The bright yellow suspension was filtered, washed with methanol and the filtrate concentrated *in vacuo.* The residue was partitioned between saturated Na₂CO₃ solution (200mL) and ethyl acetate (200ml). After separation the organic layer was washed with Na₂S₂O₃ solution (50mL, 25%) and brine (50mL), dried (MgSO₄), concentrated *in vacuo* and purified by chromatography on silica gel eluting with iso-hexane/ethyl acetate (3:1 to 2.5:1) to give the title compound. δ_{H} (CDCl₃): 4.81 (2H, br s), 6.63 (1H, s), 8.38 (1H, s); m/z (ES⁺) = 254.86 [*M*+H]⁺; RT = 2.51mm.

### Preparation 116 : 6-Chloro-1H-pyrrolo[3,2-c]pyridine-2-carboxylic acid

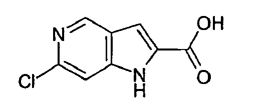

Pyruvic acid (0.86ml, 12.4mmol) was added to a solution of 2-chloro-5-iodo-pyridin-4-ylamine (**Preparation 115**, 1.05mg, 4.13mmol), palladium acetate (56mg, 0.25mmol) and DABCO (1.39g, 12.4mmol) in anhydrous DMF (30ml). The reaction mixture was degassed with argon for 20min, then heated to 145°C for 2h. The solvent was removed *in vacuo* and the residue taken up in water (200mL). The suspension was made alkaline (pH 9-10) with dilute NaOH solution (1M) and filtered through Celite. After washing of the filtrate with ethyl acetate (50 mL) and ether (50mL) the pH was adjusted to 3 with dilute HCl solution (1M). Extraction with ethyl acetate (5 x 50mL), drying of the combined extracts (MgSO₄) and concentration gave the title compound.
δ_{H} (d₆ DMSO): 7.24 (1H, s), 7.42 (1H, s), 8.80 (1H, s); m/z (ES⁻) = 195.02 [*M* - H]⁻; RT = 2.36min.

### Preparation 117 : 4(S)-(4-Fluorobenzyl)oxazolidine-2,5-dione

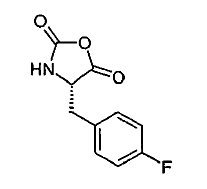

To a solution of 2(*S*)-*tert*-butoxycarbonylamino-3-(4-fluorophenyl)propionic acid (1.5g, 5.29mmol) in ethyl acetate (100mL) under an argon atmosphere was added triphosgene (628mg, 2.12mmol). To the solution was added triethylamine (0.8 mL 5.76mmol,) over 1min, and the reaction stirred for 72h at rt. The reaction mixture was filtered, and the filtrate concentrated *in vacuo* to yield an oily residue. The crude material was crystallised from cold dichloromethane and petroleum ether to give the title compound. δ_{H} (CD₃OD): 7.20 (2H, m), 7.10 (2H, m), 5.86 (1H, s, (NH)), 4.58 (1H, s), 3.33-3.23 (2H, m), 3.11-3.00 (1H, m).

### EXAMPLE 1

### 5-Chloro-1H-pyrrolo[3,2-b]pyridine-2-carboxylic acid 1-dimethylcarbamoyl-2-(S)-phenylethyl)amide

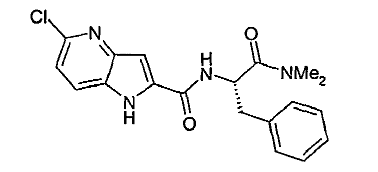

To a solution of 5-chloro-1*H*-pyrrolo[3,2-b]pyridine-2-carboxylic acid (**Preparation 6**, 36mg, 0.18mmol) in DMF (4mL, anhydrous), was added 2-(*S*)-amino-*N,N*-dimethyl-3-phenylpropionamide hydrochloride (**Preparation 8**, 46mg, 0.20mmol), DIPEA (105µL, 6.05mmol) and HOBt (25mg, 0.18mmol) sequentially. The solution was stirred for 5min prior to the addition of EDCI (42mg, 0.22mmol) in one portion. The resulting solution was stirred for 16h at rt. The reaction mixture was partitioned between ethyl acetate (50mL) and brine (20mL). The layers were separated and the aqueous phase extracted with ethyl acetate (3 x 20mL), then the combined organics were washed with water (3 x 10mL) and brine (10mL). The organic phase was dried (MgSO₄), filtered and concentrated *in vacuo.* Purification *via* flash column chromatography eluting with methanol/dichloromethane (1:19) gave an orange oil which was triturated with diethyl ether/hexane to give the title compound as an orange solid. δ_{H} (CDCl₃): 2.71 (3H, s), 2.93 (3H, s), 3.05-3.21 (2H, m), 5.28-5.39 (1H, m), 7.00 (1H, s), 7.17-7.36 (6H, m), 7.69 (1H, d, 9.23Hz), 9.27 (1H, s); m/z (ES⁺) = 371.15 [*M* + H]⁺; RT = 3.28min.

### EXAMPLE 2

### 1H-pyrrolo[3,2-c]pyridine-2-carboxylic acid (1-dimethylcarbamoyl-2(S)-phenylethyl)amide

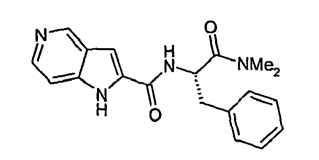

To a solution of 1*H*-pyrrolo[3,2-c]pyridine-2-carboxylic acid (**Preparation 12**, 100mg, 0.62mmol) in DMF (15mL) was added 2-(S)-amino-*N,N*-dimethyl-3-phenylpropionamide hydrochloride (**Preparation 8**, 141mg, 0.62mmol), HOBt (83mg, 0.62mmol) and DIPEA (0.21mL, 1.23mmol). After 30min, EDCI (154mg, 0.80mmol) was added and the mixture was stirred at rt for 72h. The solvent was removed *in vacuo* and the solid partitioned between water (50mL) and ethyl acetate (3 x 50mL). The combined organic layer was dried (MgSO₄), concentrated *in vacuo* and purified by chromatography on silica gel using methanol / dichloromethane (6:94) as eluant to give the title compound as a beige solid. δ_{H} (CD₃OD): 3.06-3.19 (2H, m), 4.83 (6H, s), 5.27 (1H, t), 7.20-7.32 (5H, m), 7.34 (1H, s), 7.45 (1H, d), 8.20 (1H, d), 8.87 (1H, s); m/z (ES⁺) = 337 [*M* + H]⁺*.*

### EXAMPLE 3

### 1H-Pyrrolo[2,3-c]pyridine-2-carboxylic acid (1-dimethylcarbamoyl-2(S)-phenylethyl)amide

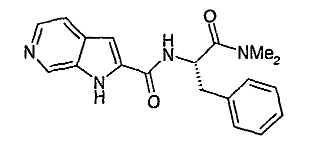

To a solution of 1*H*-pyrrolo[2,3-c]pyridine-2-carboxylic acid (**Preparation 15**, 100mg, 0.62mmol) in DMF (15mL), was added 2-(*S*)-amino-*N,N*-dimethyl-3-phenylpropionamide hydrochloride (**Preparation 8**, 141mg, 0.62mmol), HOBt (83mg, 0.62mmol) and DIPEA (0.21mL, 1.23mmol). The reaction was stirred at rt for 0.5h, followed by addition of EDCI (154mg, 0.80mmol). The mixture was stirred at rt for 72h then partitioned between water (50mL) and ethyl acetate (3 x 50mL). The combined organic fractions were dried (MgSO₄), concentrated *in vacuo* and chromatographed on silica gel eluting with methanol/dichloromethane (1:19) to give the title compound as a yellow solid. δ_{H} (CD₃OD): 2.88 (6H, s), 3.07-3.20 (2H, m), 5.28 (1H, t), 7.20 (1H, s), 7.22-7.31 (5H, m), 7.65 (1H, d), 8.10 (1H, d), 8.76 (1H, s); m/z (ES⁺) = 337 [*M* + H]⁺.

### EXAMPLE 4

### 5-Chloro-1H-pyrrolo[2,3-c]pyridine-2-carboxylic acid (1-dimethylcarbamoyl-2(S)-phenylethyl)amide

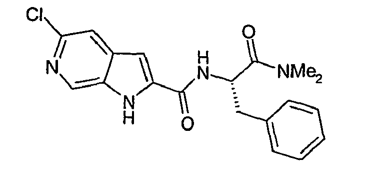

To a solution of 5-chloro-1*H*-pyrrolo[2,3-c]pyridine-2-carboxylic acid (Preparation 18, 100mg, 0.51mmol) in DMF (15mL) was added 2-(*S*)-amino-*N,N-*dimethyl-3-phenylpropionamide hydrochloride (Preparation 8, 116mg, 0.51mmol), HOBt (69mg, 0.51mmol) and DIPEA (0.18mL, 1.02mmol). After 15min, EDCI (127mg, 0.66mmol) was added and the mixture stirred at rt for 15h. The solvent was removed *in vacuo* and the solid partitioned between water (50mL) and ethyl acetate (3 x 50mL). The combined organic phases were dried (MgSO₄) concentrated *in vacuo* and purified by chromatography on silica gel eluting with methanol/dichloromethane (1:19) to give the title compound as a beige solid. δ_{H} (CD₃OD) 2.89 (6H, s), 3.05-3.19 (2H, m), 5.27 (1H, t), 7.16 (1H, s), 7.20-7.32 (5H, m), 7.67 (1H, s), 8.56 (1H, s); m/z (ES⁺) = 371 [*M* + H]⁺.

### EXAMPLE 5

### 5-Chloro-1H-pyrrolo[2,3-c]pyridine-2-carboxylic acid [1-(S)-4-fluorobenzyl)-2-(4-hydroxypiperidin-1-yl)-2-oxoethyl]amide

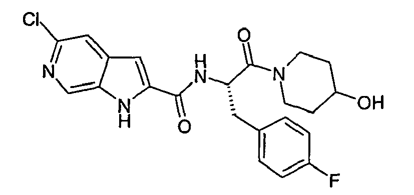

**Route A** : To a solution of 2-(*S*)-[(5-chloro-1*H*-pyrrolo[2,3-c]pyridine-2-carbonyl)amino]-3-(4-fluorophenyl)propionic acid (**EXAMPLE 228**, 1.4g, 3.87mmol) in DMF (35mL) was added HATU (1.77g, 4.64mmol) and the reaction stirred for 10min. 4-Hydroxypiperidine (0.43g, 4.26mmol) was added, followed by DIPEA (0.8mL, 4.64mmol,) and the reaction stirred at rt for 16h. Solvent was removed *in vacuo* and the crude material partitioned between ethyl acetate (50mL) and water (50mL). The organics were washed with sodium bicarbonate (2x30mL) and brine (2x30mL), dried (MgSO₄) and the solvent removed *in vacuo.* Purification by column chromatography (SiO₂, 96:4 dichloromethane/methanol) gave the title compound. m/z (ES⁺) = 445.15 [*M*+H]⁺; RT = 3.24min.
**Route B** : The title compound was prepared as outlined in **EXAMPLE 1** from 5-chloro-1*H*-pyrrolo[2,3-c]pyridine-2-carboxylic acid (Preparation 18) and 2-(*S*)-amino-3-(4-fluorophenyl)-1-(4-hydroxypiperidin-1-yl)propan-1-one hydrochloride (**Preparation 20**). The product was purified by chromatography on silica gel eluting with methanol/dichloromethane (1:19) to give the title compound as a pale yellow solid. δ_{H} (CD₃OD): 1.08-1.19 (0.5H, m), 1.29-1.51 (1.5H, m), 1.54-1.62 (0.5H, m), 1.73-1.84 (1.5H, m), 3.06-3.36 (4H, m), 3.67-3.95 (2.5H, m), 4.03-4.10 (0.5H, m), 5.32 (1H, t), 6.97-7.04 (2H, m), 7.14 (1H, s), 7.26-7.33 (2H, m), 7.66 (1H, s), 8.55 (1H, s); m/z (ES⁺) = 445 [*M* + H]⁺; RT = 3.27min.

### EXAMPLE 6

### 5-Chloro-1H-pyrrolo[2,3-c]pyridine-2-carboxylic acid [1-(S)-benzyl-3-(cis-3,4-dihydroxypyrrolidin-1-yl)-2(R)-hydroxy-3-oxopropyl]amide

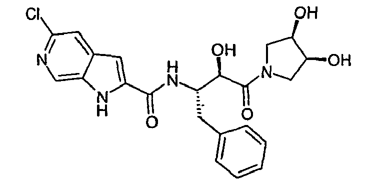

To a solution of (*S*)-3-[(5-chloro-1*H*-pyrrolo[2,3-c]pyridine-2-carbonyl)amino]-2-(*R*)-hydroxy-4-phenylbutyric acid (**EXAMPLE 44**, 50mg, 0.13mmol), *cis*-3,4-dihydroxypyrrolidine (**Preparation 23**, 15mg, 0.15mmol) and HOBt (27mg, 0.20mmol) in DMF (5mL), was added DIPEA (47µL, 0.27mmol). After stirring for 5min, EDCI (28mg, 0.15mmol) was added and the reaction stirred at rt for 72h. The solvent was removed *in vacuo* and the residue partitioned between water (30mL) and ethyl acetate (3 x 30mL). The combined organic fractions were dried (MgSO₄), concentrated *in vacuo* and the residue purified by chromatography on silica gel eluting with methanol/dichloromethane (1:9) to give the title compound as a white solid. m/z (ES⁺) = 459 [*M*+H]⁺. RT = 3.07min.

### EXAMPLE 7

### 5-Bromo-1H-pyrrolo[2,3-c]pyridine-2-carboxylic acid (1-dimethylcarbamoyl-2-(S)-phenylethyl)amide

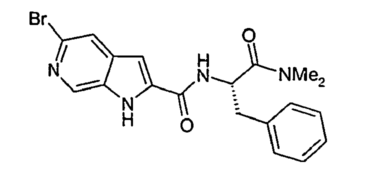

To a solution of 5-bromo-1*H*-pyrrolo[2,3-c]pyridine-2-carboxylic acid (**Preparation 27**, 50mg, 0.21mmol) in DMF (5mL), was added 2-(*S*)-amino-*N,N-*dimethyl-3-phenylpropionamide hydrochloride (**Preparation 8**, 52mg, 0.23mmol), HOBt (31mg, 0.23mmol) and DIPEA (72µL, 0.41mmol). After 5min, EDCI (44mg, 0.23mmol) was added and the reaction was stirred at rt for 16h. The solvent was removed *in vacuo* and the residue partitioned between water (30mL) and ethyl acetate (3 x 30mL). The combined organic fractions were dried (MgSO₄) concentrated in *vacuo* and the residue purified by chromatography on silica gel eluting with methanol/dichloromethane (3:97) to give the title compound as an off-white solid. δ_{H} (CD₃OD): 2.88 (6H, s), 3.06-3.18 (2H, m), 5.27 (1H, t), 7.13 (1H, s), 7.19-7.29 (5H, m), 7.80 (1H, s), 8.53 (1H, s); m/z (ES⁺) = 415 [*M* + +H]⁺.

### EXAMPLE 8

### 1H-Pyrrolo[2,3-b]pyridine-2-carboxylic acid (1-dimethylcarbamoyl-2-(S)-phenylethyl)amide

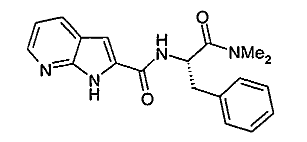

Triethylamine (82µL, 0.59mmol) was added to 2-(*S*)-amino-*N,N*-dimethyl-3-phenylpropionamide hydrochloride (**Preparation 8**, 117mg,0.51mmol) in DCM (5mL) at rt under nitrogen. The mixture was cooled to 0°C and 1*H*-pyrrolo[2,3-b]pyridine-2-carboxylic acid (**Preparation 28**, 75mg, 0.51mmol) was added followed by HOBt (102mg, 0.765mmol) and then EDCI (98mg, 0.51mmol). The reaction mixture was then left to warm to rt, stirred for 4 days and then diluted with ethyl acetate (25mL), washed with aqueous sodium hydroxide solution (2M, 2 x 25mL), aqueous hydrochloric acid (2N, 2 x 25mL) and dried (MgSO₄). The organic solution was concentrated *in vacuo* to give a beige foam which was purified by column chromatography eluting with methanol / dichloromethane (2:98) to give the title compound as a white solid. δ_{H} (CDCl₃): 2.74 (3H, s), 2.95 (3H, s), 3.17 (2H, m), 5.41 (1H, dd), 6.90 (1H, s), 7.08-7.48 (7H, m), 7.98 (1H, d), 8.55 (1H, d); m/z (ES⁺) = 337.2 [*M*+H]⁺, RT = 1.38min.

### EXAMPLE 9

### 5-Chloro-1H-pyrrolo[2,3-c]pyridine-2-carboxylic acid (2-phenoxyethyl)amide

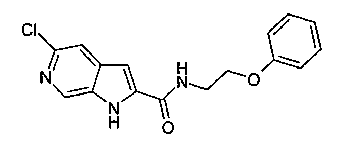

The title compound was prepared as outlined in **EXAMPLE 1** from 5-chloro-1*H*-pyrrolo[2,3-c]pyridine-2-carboxylic acid (**Preparation 18**) and 2-phenoxyethylamine. The product was purified by chromatography on silica gel eluting with methanol/dichloromethane (1:19) to give the title compound as a yellow solid. δ_{H} (CD₃OD): 3.79 (2H, t), 4.17 (2H, t), 6.88-6.97 (3H, m), 7.08 (1H, s), 7.16 (2H, t), 7.67 (1H, s), 8.58 (1H, s); m/z (ES⁺) = 316 [*M* + H]⁺.

### EXAMPLE 10

### 5-Chloro-1H-pyrrolo[2,3-c]pyridine-2-carboxylic acid (2-morpholin-4-ylethyl)amide

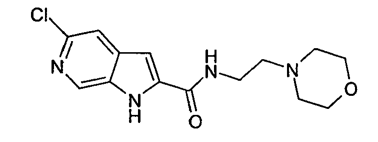

The title compound was prepared as outlined in **EXAMPLE 1** from 5-chloro-1*H*-pyrrolo[2,3-c]pyridine-2-carboxylic acid (**Preparation 18**) and 2-morpholin-4-ylethylamine. The product was purified by chromatography on silica gel eluting with methanol/dichloromethane (1:19) to give the title compound as a yellow solid.
δ_{H} (CD₃OD): 2.54-2.60 (4H, m), 2.64 (2H, t), 3.58 (2H, t), 3.69-3.73 (4H, m), 7.05 (1H, s), 7.66 (1H, s), 8.58 (1H, s); m/z (ES⁺) = 309 [*M*+H]⁺.

### EXAMPLE 11

### 5-Chloro-1H-pyrrolo[2,3-c]pyridine-2-carboxylic acid [2-(4-methoxyphenoxy)ethyl]amide

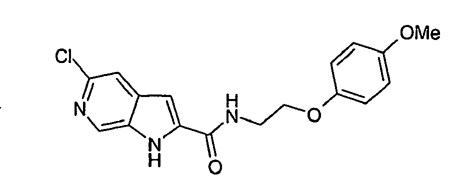

The title compound was prepared as outlined in **EXAMPLE 1** from 5-chloro-1*H*-pyrrolo[2,3-c]pyridine-2-carboxylic acid (**Preparation 18**) and 2-(4-methoxyphenoxy)ethylamine. The product was purified by chromatography on silica gel eluting with methanol/dichloromethane (3:97) to give the title compound as a yellow solid. δ_{H} (CD₃OD): 3.71 (3H, s), 3.77 (2H, t), 4.12 (2H, t), 6.81-6.91 (4H, m), 7.09 (1H, s), 7.67 (1H, s), 8.58 (1H, s); m/z (ES⁺) = 346 [*M*+H]⁺.

### EXAMPLE 12

### 5-Chloro-1H-pyrrolo[2,3-c]pyridine-2-carboxylic acid (2-thiophen-2-ylethyl)amide

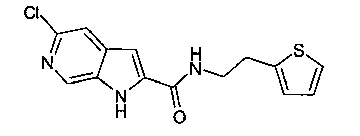

The title compound was prepared as outlined in **EXAMPLE 1** from 5-chloro-1H-pyrrolo[2,3-c]pyridine-2-carboxylic acid (**Preparation 18**) and 2-thiophen-2-ylethylamine. The product was purified by chromatography on silica gel eluting with methanol/dichloromethane (3:97) to give the title compound as a yellow solid. δ_{H} (CD₃OD): 3.16 (2H, t), 3.65 (2H, t), 6.89-6.94 (2H, m), 7.03 (1H, s), 7.20 (1H, d), 7.66 (1H, s), 8.57 (1H, s); m/z (ES⁺) = 306 [*M*+H]⁺.

### EXAMPLE 13

### 5-Chloro-1H-pyrrolo[2,3-c]pyridine-2-carboxylic acid [2-(2-methoxyphenyl)ethyl]amide

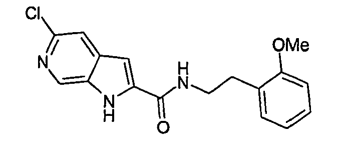

The title compound was prepared as outlined in **EXAMPLE 1** from 5-chloro-1*H*-pyrrolo[2,3-c]pyridine-2-carboxylic acid (**Preparation 18**) and 2-(2-methoxyphenyl)ethylamine. The product was purified by chromatography on silica gel eluting with methanol/dichloromethane (3:97) to give the title compound as a yellow solid. δ_{H} (CD₃OD): 2.94 (2H, t), 3.59 (2H, t), 3.78 (3H, s), 6.84 (1H, t), 6.89 (1H, d), 6.97 (1H, s), 7.12-7.18 (2H, m), 7.60 (1H, s), 8.55 (1H, s); m/z (ES⁺) = 330 [*M*+H]⁺.

### EXAMPLE 14

### 5-Chloro-1H-pyrrolo[2,3-c]pyridine-2-carboxylic acid (2-oxo-2-phenylethyl)amide

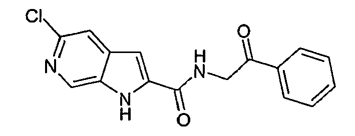

The title compound was prepared as outlined in **EXAMPLE 1** from 5-chloro-1*H*-pyrrolo[2,3-c]pyridine-2-carboxylic acid (**Preparation 18**) and 2-oxo-2-phenylethylamine. The product was purified by mass directed purification to give the title compound as an orange solid. m/z (ES⁺) = 314 [*M* + H]⁺; RT = 3.30min.

### EXAMPLE 15

### 5-Chloro-1H-pyrrolo[2,3-c]pyridine-2-carboxylic acid (1H-benzoimidazol-2-ylmethyl)amide

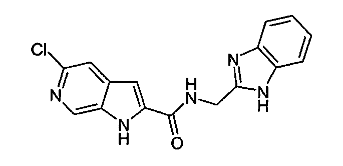

The title compound was prepared as outlined in **EXAMPLE 1** from 5-chloro-1*H*-pyrrolo[2,3-c]pyridine-2-carboxylic acid (**Preparation 18**) and 2-oxo-2-phenylethylamine. The product was purified by mass directed purification to give the title compound as a yellow solid. m/z (ES⁺) = 326 [*M*+H]⁺; RT = 2.66min.

### EXAMPLE 16

### 5-Chloro-1H-pyrrolo[2,3-c]pyridine-2-carboxylic acid phenethylamide

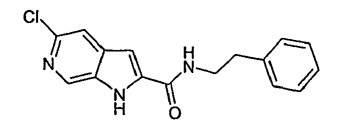

The title compound was prepared as outlined in **EXAMPLE 1** from 5-chloro-1*H*-pyrrolo[2,3-c]pyridine-2-carboxylic acid (**Preparation 18**) and phenethylamine. The product was purified by mass directed purification to give the title compound as an orange solid. δ_{H} (CD₃OD): 2.94 (2H, t), 3.63 (2H, t), 7.00 (1H, s), 7.15-7.30 (5H, m), 7.64 (1H, s), 8.57 (1H, s); m/z (ES⁺) = 300 [*M*+H]⁺.

### EXAMPLE 17

### 5-Chloro-1H-pyrrolo[2,3-c]pyridine-2-carboxylic acid [2-(4-fluorophenyl)ethyl]amide

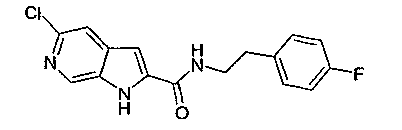

The title compound was prepared as outlined in **EXAMPLE 1** from 5-chloro-1*H*-pyrrolo[2,3-c]pyridine-2-carboxylic acid (**Preparation 18**) and 4-fluorophenethylamine. The product was purified by mass directed purification to give the title compound as an orange solid. δ_{H} (CD₃OD) : 2.93 (2H, t), 3.60 (2H, t), 6.97-7.04 (3H, m), 7.24-7.30 (2H, m), 7.65 (1H, s), 8.56 (1H, s); m/z (ES⁺) = 318 [*M* + H]⁺.

### EXAMPLE 18

### 5-Chloro-1H-pyrrolo[2,3-c]pyridine-2-carboxylic acid [2-(2-chloro-6-fluorobenzylsulfanyl)ethyl]amide

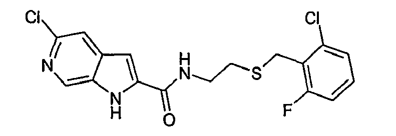

The title compound was prepared as outlined in **EXAMPLE 1** from 5-chloro-1*H*-pyrrolo[2,3-c]pyridine-2-carboxylic acid (**Preparation 18**) and 2-(2-chloro-6-fluorobenzylsulfanyl)ethylamine. The product was purified by mass directed purification to give the title compound as a yellow solid. δ_{H} (CD₃OD): 2.82 (2H, t), 3.64 (2H, t), 3.95 (2H, s), 7.04-7.09 (2H, m), 7.20-7.25 (2H, m), 7.67 (1H, s), 8.58 (1H, s); m/z (ES⁺) = 398 [*M* + H]⁺.

### EXAMPLE 19

### 5-Chloro-1H-pyrrolo[2,3-c]pyridine-2-carboxylic acid (2,3-dihydrobenzo[1,4]dioxin-2-ylmethyl)amide

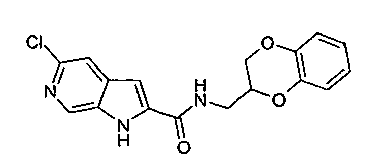

The title compound was prepared as outlined in **EXAMPLE 1** from 5-chloro-1*H-*pyrrolo[2,3-c]pyridine-2-carboxylic acid (**Preparation 18**) and 2,3-dihydrobenzo[1,4]dioxin-2-ylmethylamine. The product was purified by mass directed purification to give the title compound as a yellow solid. δ_{H} (CD₃OD): 3.69-3.73 (2H, m), 3.97-4.03 (1H, m), 4.32-4.42 (2H, m), 6.77-6.88 (4H, m), 7.10 (1H, s), 7.67 (1H, s), 8.58 (1H, s); m/z (ES⁺) = 344 [*M* + H]⁺.

### EXAMPLE 20

### 5-Chloro-1H-pyrrolo[2,3-c]pyridine-2-carboxylic acid [2-(naphthalen-1-ylamino)ethyl]amide

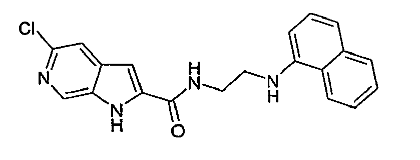

The title compound was prepared as outlined in **EXAMPLE 1** from 5-chloro-1*H-*pyrrolo[2,3-c]pyridine-2-carboxylic acid (**Preparation 18**) and 2-(naphthalen-1-ylamino)ethylamine. The product was purified by mass directed purification to give the title compound as a brown solid. δ_{H} (CD₃OD): 3.55 (2H, t), 3.80 (2H, t), 6.68 (1H, d), 7.04 (1H, s), 7.14 (1H, d), 7.28 (1H, t), 7.36-7.42 (2H, m), 7.65 (1H, s), 7.70-7.74 (1H, m), 7.98-8.02 (1H, m), 8.58 (1H, s); m/z (ES⁺) = 365 [*M* + H]⁺.

### EXAMPLE 21

### 1H-Pyrrolo[2,3-c]pyridine-2-carboxylic acid (2-phenylaminoethyl)amide

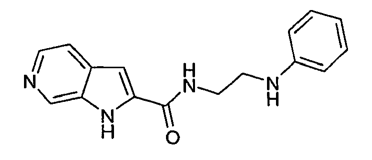

The title compound was prepared as outlined in **EXAMPLE 1** from 1*H-*pyrrolo[2,3-c]pyridine-2-carboxylic acid (**Preparation 15**) and 2-phenylaminoethylamine to give the title compound as a pale yellow solid.
δ_{H} (CD₃OD): 3.37 (2H, t), 3.63 (2H, t), 6.61 (1H, t), 6.69 (2H, d), 7.08-7.13 (2H, m), 7.64 (1H, d), 8.10 (1H, d), 8.78 (1H, s); m/z (ES⁺) = 281 [*M*+H]⁺.

### EXAMPLE 22

### 1H-Pyrrolo[2,3-c]pyridine-2-carboxylic acid (2-phenoxyethyl)amide

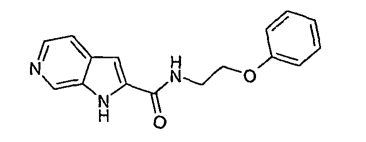

The title compound was prepared as outlined in **EXAMPLE 1** from 1*H-*pyrrolo[2,3-c]pyridine-2-carboxylic acid (**Preparation 15**) and 2-phenoxyethylamine to give the title compound as a pale yellow solid. δ_{H} (CD₃OD): 3.81 (2H, t), 4.18 (2H, t), 6.89-6.97 (3H, m), 7.14 (1H, s), 7.26 (2H, t), 7.65 (1H, d), 8.10 (1H, d), 8.78 (1H, s); m/z (ES⁺) = 282 [*M* + H]⁺.

### EXAMPLE 23

### 5-Methoxy-1H-pyrrolo[3,2-b]pyridine-2-carboxylic acid (1-dimethylcarbamoyl-2-(S)-phenylethyl) amide

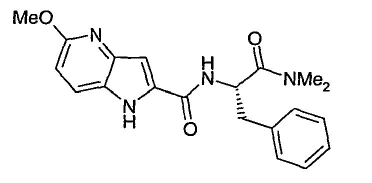

To a solution of 5-methoxy-1*H-*pyrrolo[3,2-b]pyridine-2-carboxylic acid (**Preparation 36**, 50mg, 0.26mmol) in DMF (3mL), was added DIPEA (100µL, 0.57mmol), HOBt (35mg, 0.26mmol) and EDCI (60mg, 0.31mmol) sequentially. The reaction mixture was stirred for 5min prior to the addition of 2-(*S*)-amino-N, *N-*dimethyl-3-phenylpropionamide hydrochloride (**Preparation 8**, 65mg, 0.29mmol) in one portion. The reaction mixture was stirred for 21h at rt then water (15mL) and dichloromethane (30mL) were added. The mixture was stirred vigorously for 10min and the layers separated. The aqueous phase was extracted with dichloromethane (3 x 15mL) and the combined organics washed with brine (30mL), dried (MgSO₄), filtered and concentrated *in vacuo.* Purification *via* flash column chromatography (SiO₂, ethyl acetate/isohexane, 1:1) gave a yellow oil. Trituration with water followed by filtration and drying gave the title compound as a white solid. δ_{H} (CD₃OD): 2.88 (6H, 2 x s), 3.12 (2H, m), 3.94 (3H, s), 5.26 (1H, dd), 6.71 (1H, d), 7.13 (1H, s), 7.25 (5H, m), 7.74 (1H, d); m/z (ES⁺) = 367 [*M* + H]⁺; RT = 3.20min.

### EXAMPLE 24

### 1H-Pyrrolo[3,2-b]pyridine-2-carboxylic acid (1-dimethylcarbamoyl-2-(S)-phenylethyl)amide

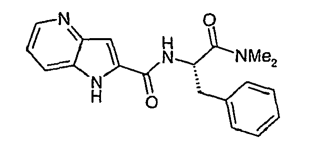

Prepared as outlined in **EXAMPLE 1** from 1*H-*pyrrolo[3,2-b]pyridine-2-carboxylic acid (**Preparation 32**) and 2-(*S*)-amino-N, N-dimethyl-3-phenyl-propionamide hydrochloride (**Preparation 8**, 78mg, 0.34mmol). The title compound was isolated as a white solid. δ_{H} (d₆ DMSO): 2.82 (3H, s), 2.98 (3H, s), 3.03 (2H, m), 5.12 (1H, m), 7.16 (2H, m), 7.24 (2H, m), 7.32 (2H, m), 7.40 (1H, d), 7.74 (1H, d), 8.37 (1H, dd), 8.93 (1H, d); m/z (ES⁺) = 337 [*M* + H]⁺; RT = 3.10min.

### EXAMPLE 25

### 1H-Pyrrolo[3,2-b]pyridine-2-carboxylic acid (2-phenoxyethyl)amide

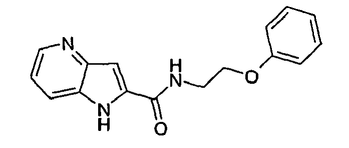

To a solution of 1*H-*pyrrolo[3,2-b]pyridine-2-carboxylic acid (**Preparation 32**, 50mg, 0.31mmol) in DMF (5mL), was added 2-phenoxyethylamine (44µL, 0.34mmol), DIPEA (118µL, 0.68mmol) and HOBt (42mg, 0.31mmol) sequentially. The reaction mixture was stirred for 5min prior to the addition of EDCI (42mg, 0.22mmol) in one portion. The resulting mixture was stirred for 20h at rt and partitioned between ethyl acetate (50mL) and water (20mL). The layers were separated and the aqueous phase extracted with ethyl acetate (2 x 30mL). The combined organic fractions were washed with brine (20mL), dried (MgSO₄), filtered and concentrated in *vacuo* to give an oil. Trituration with diethyl ether / *iso*hexane and collection by filtration gave, after air-drying, the title compound as a cream coloured solid. δ_{H} (d₆ DMSO): 3.48 (2H, m), 4.14 (2H, t), 6.94 (3H, m), 7.17 (1H, dd), 7.28 (3H, m), 7.77 (1H, d), 8.37 (1H, dd), 8.86 (1H, t); m/z (ES⁺) = 282 [*M*+ H]⁺; RT = 2.60min.

### EXAMPLE 26

### 1H-Pyrrolo[3,2]pyridine-2-carboxylic acid (2-phenylaminoethyl)amide

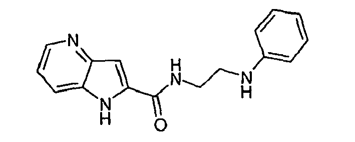

The title compound was prepared as outlined in **EXAMPLE 25** except N-phenylethylenediamine (44µL, 0.34mmol) was used in place of 2-phenoxyethylamine. The title compound was isolated as a cream solid. δ_{H} (d₆ DMSO): 3.23 (2H, m), 3.37 (2H, m), 5.70 (1H, t), 6.52 (1H, t), 6.63 (2H, dd), 7.07 (2H, dd), 7.17 (1H, dd), 7.22 (1H, s), 7.77 (1H, d), 8.37 (1H, dd), 8.73 (1H, t); m/z (ES⁺) = 281 [*M* + H]⁺; RT = 2.36min.

### EXAMPLE 27

### 5-Chloro-1H-pyrrolo[3,2-]pyridine-2-carboxylic acid (2-phenoxyethyl)amide

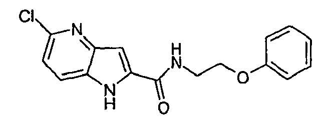

The title compound was prepared as outlined in **EXAMPLE 1** from 5-chloro-1*H-*pyrrolo[3,2-b]pyridine-2-carboxylic acid (**Preparation 6)** and 2-phenoxyethylamine. On completion of the reaction, the mixture was partitioned between water and dichloromethane on a hydrophobic frit, washing with dichloromethane. The organic filtrate was concentrated *in vacuo* then triturated with dichloromethane/methanol/ethyl acetate to give the title compound as a white solid. δ_{H} (d₆ DMSO): 3.68 (2H, m), 4.13 (2H, m), 6.94 (3H, m), 7.25 (4H, m), 7.83 (1H, d), 8.95 (1H, t), 12.09 (1H, s). m/z (ES⁺) = 316 [*M* + H]⁺; RT = 3.45min.

### EXAMPLE 28

### 5-Chloro-1H-pyrrolo[2,3-c]pyridine-2-carboxylic acid [2-(4-benzylpiperazin-1-yl)ethyl]amide

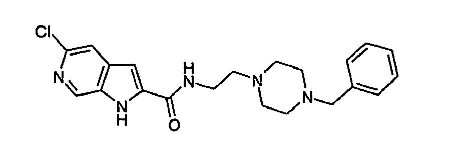

The title compound was prepared as outlined in **EXAMPLE 1** from 5-chloro-1*H-*pyrrolo[2,3-c]pyridine-2-carboxylic acid (**Preparation 18**) and 4-benzylpiperazin-lylethylamine. The product was purified by mass directed purification to give the title compound as an orange solid. m/z (ES⁺) = 398 [*M* + H]⁺; RT = 2.75min.

### EXAMPLE 29

### 5-Chloro-1H-pyrrolo[2,3-c]pyridine-2-carboxylic acid (2-benzylaminoethyl)amide

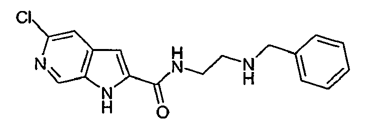

The title compound was prepared as outlined in **EXAMPLE 1** from 5-chloro-1*H-*pyrrolo[2,3-c]pyridine-2-carboxylic acid (**Preparation 18**) and 2-benzylaminoethylamine. The product was purified by mass directed purification to give the title compound as an off-white solid. m/z (ES⁺) = 329 [*M*+ H]⁺; RT = 2.75min.

### EXAMPLE 30

### 5-Chloro-1H-pyrrolo[2,3-c]pyridine-2-carboxylic acid phenylcarbamoylmethylamide

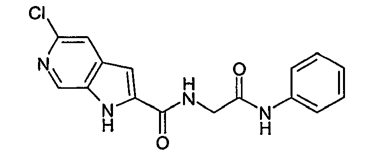

To a solution of [(5-chloro-1*H-*pyrrolo[2,3-c]pyridine-2-carbonyl)amino] acetic acid (**EXAMPLE 40**, 30mg, 0.12mmol) in DMF (2mL) was added aniline (12µL, 0.13mmol), HOBt (16mg, 0.12mmol) and DIPEA (41µL, 0.24mmol). After 5min, EDCI (29mg, 0.15mmol) was added, and the reaction stirred at rt for 16h. The solvent was removed *in vacuo* and the solid partitioned between water (20mL) and ethyl acetate (3 x 20mL). The combined organic fractions were dried (MgSO₄), concentrated *in vacuo* and the residue purified by chromatography on silica gel eluting with methanol/dichloromethane (1:19) to give the title compound as a yellow solid. m/z (ES⁺) = 329 [*M* + H]⁺; RT = 3.17min.

### EXAMPLE 31

### 5-Chloro-1H-pyrrolo[2,3-c]pyridine-2-carboxylic acid [(tetrahydropyran-4-ylcarbamoyl)methyl]amide

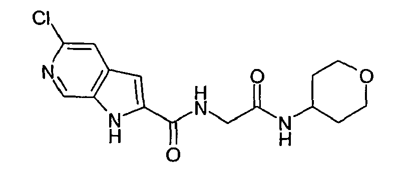

The title compound was prepared according to the procedure of **EXAMPLE 30** except 4-aminotetrahydropyran was used in place of aniline. After 16h the reaction mixture was poured into water and left for a further 16h. The solid was filtered and dried to give the title compound as a white crystalline solid. m/z (ES⁺) = 337 [*M*+H]⁺; RT = 2.72min.

### EXAMPLE 32

### 5-Chloro-1H-pyrrolo[2,3-c]pyridine-2-carboxylic acid {[(thiophen-2-ylmethyl)carbamoyl]methyl} amide

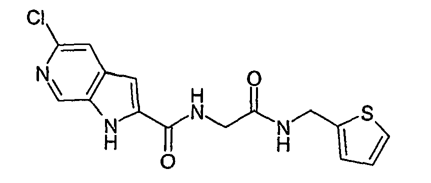

The title compound was prepared according to **EXAMPLE 30** except 2-aminomethylthiophene was used in place of aniline. After stirring for 16h the reaction mixture was poured into water and the precipitate was filtered and dried to give the title compound as a white solid. m/z (ES⁺) = 349 [*M*+H]⁺; RT = 3.07min.

### EXAMPLE 33

### 5-Chloro-1H-pyrrolo[2,3-c]pyridine-2-carboxylic acid [(4-methoxyphenylcarbamoyl)methyl]amide

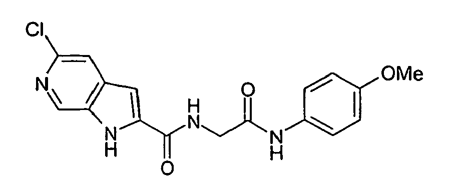

The title compound was prepared according to **EXAMPLE 30** except p-anisidine was used in place of aniline. After stirring for 16h, the reaction mixture was poured into water and the precipitate was filtered and dried to give the title compound as a beige solid. m/z (ES⁺) = 359 [*M*+ H]⁺; RT = 3.22min.

### EXAMPLE 34

### 5-Chloro-1H-pyrrolo[2,3-c]pyridine-2-carboxylic acid (1-(S)-benzyl-2-oxo-2-pyrrolidin-1-ylethyl)amide

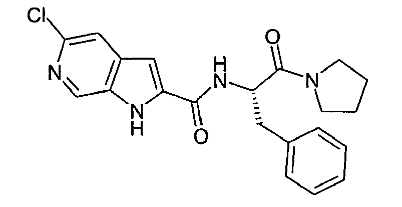

To a solution of 2-(S)-[(5-chloro-1*H-*pyrrolo[2,3-c]pyridine-2-carbonyl)amino]-3-phenylpropionic acid (**EXAMPLE 42**, 50mg, 0.15mmol) in DMF (3mL) was added pyrrolidine (13µL, 0.16mmol), HOBt (20mg, 0.15mmol) and DIPEA (51µL, 0.29mmol). After 5min, EDCI (36mg, 0.19mmol) was added and the reaction was stirred at rt for 16h. The solvent was removed *in vacuo* and the solid was triturated with water, filtered and dried to give the title compound as a beige solid. m/z (ES⁺) = 397 [*M*+H]⁺; RT = 3.38min.

### EXAMPLE 35

### 5-Chloro-1H-pyrrolo[2,3-c]pyridine-2-carboxylic acid [1-(S)-benzyl-2-(3-(S)-hydroxypyrrolidin-1-yl)-2-oxoethyl]amide

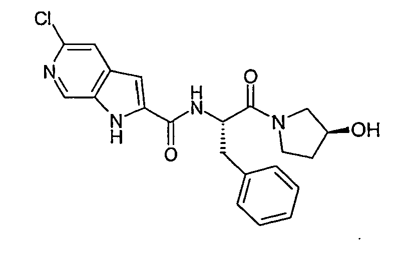

To a solution of 2-(*S*)-[(5-chloro-1*H-*pyrrolo[2,3-c]pyridine-2-carbonyl)amino]-3-phenylpropionic acid (**EXAMPLE 42**, 50mg, 0.15mmol) in DMF (3mL) was added 3-(*S*)-hydroxypyrrolidine (13.9mg, 0.16mmol), HOBt (20mg, 0.15mmol) and DIPEA (51µL, 0.29mmol). After 5min, EDCI (36mg, 0.19mmol) was added and the reaction stirred at rt for 16h. The solvent was removed *in vacuo* and the residue partitioned between water (20mL) and ethyl acetate (3 x 20mL), dried (MgSO₄) and concentrated *in vacuo.* Purification *via* chromatography on silica gel eluting with methanol / dichloromethane (4:96) gave the title compound as a white solid. m/z (ES⁺) = 413 [*M* + H]⁺; RT = 3.20min.

### EXAMPLE 36

### 5-Chloro-1H-pyrrolo[2,3-c]pyridine-2-carboxylic acid [1-(S)-benzyl-2-(3,4-dihydroxypyrrolidin-1-yl)-2-oxo-ethyl] amide

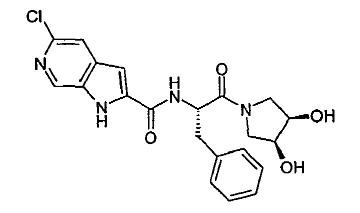

The title compound was prepared according to **EXAMPLE 35** except *cis-*3,4-dihydroxypyrrolidine was used in place of 3-(*S*)-hydroxypyrrolidine. Purification *via* chromatography on silica gel eluting with a gradient of methanol/dichloromethane (4:96 to 1:9) gave the title compound as a white solid. m/z (ES⁺) = 429 [*M*+H]⁺; RT = 3.12min.

### EXAMPLE 37

### 5-Chloro-1H-pyrrolo[2,3-c]pyridine-2-carboxylic acid (1-(S)-benzyl-2-oxo-2-thiomorpholin-4-ylethyl)amide

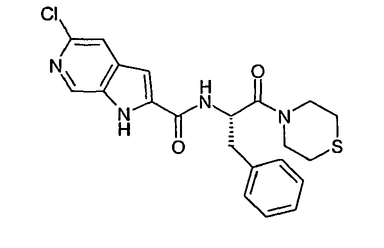

The title compound was prepared according to the procedure used for **EXAMPLE 35** except thiomorpholine was used in place of 3-(*S*)-hydroxypyrrolidine. Purification *via* chromatography on silica gel eluting with methanol/dichloromethane (3:97) gave the title compound as a white solid. m/z (ES⁺) = 429 [*M* + H]⁺; RT = 3.54min.

### EXAMPLE 38

### 5-Chloro-1H-pyrrolo[2,3-c]pyridine-2-carboxylic acid[2-phenyl-1-(S)-(tetrahydropyran-4-ylcarbamoyl)ethyl] amide

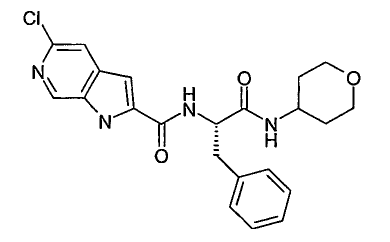

The title compound was prepared according to **EXAMPLE 35** except 4-aminotetrahydropyran was used in place of 3-(*S*)-hydroxypyrrolidine. The product was recrystallised from methanol/dichloromethane (3:97) to give the title compound as a white solid. m/z (ES⁺) = 427 [*M*+ H]⁺; RT = 3.23min.

### EXAMPLE 39

### [(5-Chloro-1H-pyrrolo[2,3-c]pyridine-2-carbonyl)amino]acetic acid ethyl ester

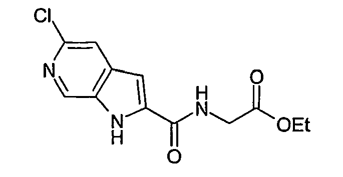

To a solution of 5-chloro-1*H-*pyrrolo[2,3-c]pyridine-2-carboxylic acid (**Preparation 18**, 800mg, 4.1mmol) in DMF (40mL) was added glycine ethyl ester hydrochloride (625mg, 4.5mmol), HOBt (0.55g, 4.1mmol) and DIPEA (2.13mL, 12.2mmol). After 5min, EDCI (1.01g, 5.3mmol) was added and the reaction stirred at rt for 16h. The solvent was removed *in vacuo* and the solid partitioned between water (100mL) and ethyl acetate (3 x 80mL). The combined organic fractions were dried (MgSO₄), concentrated *in vacuo* and purified by chromatography on silica gel eluting with methanol/dichloromethane (4:96) to give the title compound as a yellow solid.
δ_{H} (CD₃OD): 1.28 (3H, t), 4.14 (2H, s), 4.23 (2H, q), 7.10 (1H, s), 7.68 (1H, s), 8.59 (1H, s); m/z (ES⁺) = 282 [*M*+ H]⁺.

### EXAMPLE 40

### [(5-Chloro-1H-pyrrolo[2,3-c]pyridine-2-carbonyl)amino]acetic acid

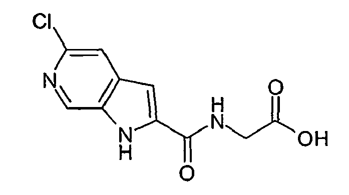

To a solution of [(5-chloro-1*H-*pyrrolo[2,3-c]pyridine-2-carbonyl)amino] acetic acid ethyl ester (**EXAMPLE 39**, 500mg, 1.8mmol) in THF (30mL) was added sodium hydroxide solution (1.8mL, 2M, 3.6mmol) and the reaction stirred at rt for 4h. The solvent was removed *in vacuo* and the solid partitioned between hydrochloric acid (1M, 100mL) and ethyl acetate (2 x 100mL). The aqueous layer was concentrated *in vacuo* and the solid residue suspended in water (10mL), filtered and dried to give the title compound as an off-white solid. δ_{H} (d₆ DMSO): 3.97 (2H, d), 7.18 (1H, s), 7.76 (1H, s), 8.57 (1H, s), 9.17 (1H, t), 12.32 (1H, s); m/z (ES⁺) = 254 [*M*+H]⁺.

### EXAMPLE 41

### 2-(S)-[(5-Chloro-1H-pyrrolo[2,3-c]pyridine-2-carbonyl)amino]-3-phenylpropionic acid ethyl ester

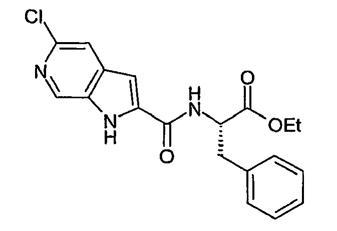

To a solution of 5-chloro-1*H-*pyrrolo[2,3-c]pyridine-2-carboxylic acid (**Preparation 18**, 2.00g, 10.2mmol) in DMF (50mL) was added L-phenylalanine ethyl ester hydrochloride (2.45g, 10.7mmol), HOBt (1.37g, 10.2mmol) and DIPEA (5.3mL, 30.5mmol). After 5min, EDCI (2.54g, 13.2mmol) was added and the reaction mixture stirred at rt for 16h. The solvent was removed *in vacuo* and the solid dissolved in ethyl acetate (150mL) and washed with water (200mL). The organic phase was dried (MgSO₄), concentrated *in vacuo* and purified by chromatography on silica gel eluting with methanol/dichloromethane (3:97) to give the title compound as a pale yellow solid. δ_{H} (CD₃OD): 1.21 (3H, t), 3.13 (1H, dd), 3.28 (1H, dd), 4.17 (2H, q), 4.86 (1H, m), 7.09 (1H, s), 7.16-7.26 (5H, m), 7.65 (1H, s), 8.55 (1H, s); m/z (ES⁺) = 372 [*M*+ H]⁺.

### EXAMPLE 42

### 2-(S)-[(5-Chloro-1H-pyrrolo[2,3-c]pyridine-2-carbonyl)amino]-3-phenylpropionic acid

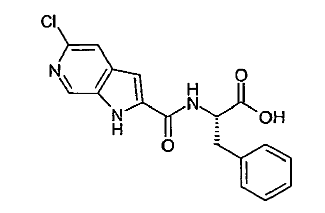

Sodium hydroxide solution (2.5ml, 2M, 5.1mmol) was added to a solution of 2-(*S*)-[(5-chloro-1*H*-pyrrolo[2,3-c]pyridine-2-carbonyl)amino]-3-phenylpropionic acid ethyl ester (**EXAMPLES 41**, 940mg, 2.5mmol) in THF (30mL) and the reaction mixture was stirred at rt for 16h. The solvent was removed *in vacuo* and the solid partitioned between hydrochloric acid (2M, 40mL) and ethyl acetate (3 x 40mL). The combined organic fractions were dried (MgSO₄) and concentrated *in vacuo* to give the title compound as a yellow solid. m/z (ES⁺) = 344 [*M* + H]⁺; RT = 3.29min.

### EXAMPLE 43

### (S)-3-[(5-Chloro-1H-pyrrolo[2,3-c]pyridine-2-carbonyl)amino]-(R)-2-hydroxy-4-phenylbutyric acid methyl ester

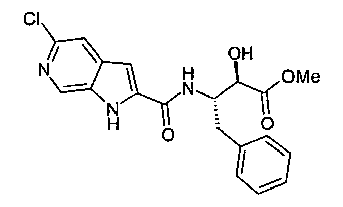

To a solution of 5-chloro-1*H-*pyrrolo[2,3-c]pyridine-2-carboxylic acid (**Preparation 18**, 165mg, 0.84mmol) and (3*S*,2*R*)-3-amino-2-hydroxy-4-phenylbutyric acid methyl ester (**Preparation 21**, 175mg, 0.84mmol) in DMF (10mL) was added HOBt (125mg, 0.92mmol), DIPEA (0.29mL, 1.68mmol) and EDCI (177mg, 0.92mmol) and the reaction stirred at rt for 72h. The reaction solvent was removed *in vacuo,* and the residue partitioned between water (40mL) and ethyl acetate (3 x 40mL). The combined organic fractions were dried (MgSO₄), concentrated in *vacuo* and the residue purified by chromatography on silica gel eluting with methanol/dichloromethane (3:97) to give the title compound as a yellow solid. δ_{H} (CD₃OD): 2.97-3.12 (2H, m), 3.67 (1H, s), 4.25 (1H, d), 4.70-4.75 (1H, m), 7.08 (1H, s), 7.15-7.21 (1H, m), 7.25-7.34 (4H, m), 7.65 (1H, s), 8.55 (1H, s); m/z (ES⁺) = 388 [*M* + H]⁺.

### EXAMPLE 44

### (S)-3[(5-Chloro-1H-pyrrolo[2,3-c]pyridine-2-carbonyl)amino]-(R)-2-hydroxy-4-phenylbutyric acid

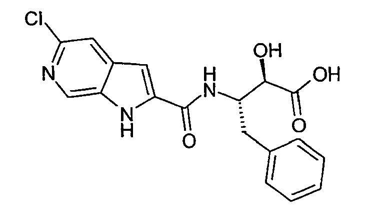

Sodium hydroxide solution (0.24mL, 2M, 0.48mmol) was added to a solution of (*S*)-3-[(5-chloro-1*H*-pyrrolo[2,3-c]pyridine-2-carbonyl)amino]-(*R*)-2-hydroxy-4-phenylbutyric acid methyl ester (**EXAMPLE 43**, 170mg, 0.44mmol) in methanol (5mL) and the reaction stirred at rt for 24h. The solvent was removed *in vacuo* and the residue partitioned between hydrochloric acid (1N, 30mL) and ethyl acetate (3 x 30mL). The combined organic fractions were dried (MgSO₄) and concentrated *in vacuo* to give the title compound as a yellow solid. δ_{H} (DMSO): 2.71 (1H, dd), 2.91 (1H, dd), 3.56 (1H, d), 4.38-4.46 (1H, m), 4.77 (1H, s), 6.76 (1H, s), 7.11-7.16 (1H, m), 7.19-7.27 (4H, m), 7.45 (1H, s), 8.44-8.52 (2H, m); m/z (ES⁺) = 374 [*M*+ H]⁺.

### EXAMPLE 45

### 5-Chloro-1H-pyrrolo[2,3-c]pyridine-2-carboxylic acid 12-(4-methoxyphenyl)-2-oxoethyl]amide

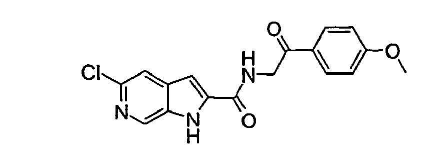

Aqueous hydrochloric acid (2.1mL, 2M) was added to a solution of 5-chloro-1*H-*pyrrolo[2,3-c]pyridine-2-carboxylic acid [2-(4-methoxyphenyl)[1,3]dioxolan-2-ylmethyl]amide (**Preparation 49**, 160mg, 0.41mmol) in acetone (20mL). The mixture was heated under reflux for 90min then allowed to cool to rt. The suspension was filtered then washed with acetone and air dried, to give the title compound as a pale yellow solid. δ_{H} (d₆ DMSO): 3.86 (3H, s), 4.79 (2H, d), 7.08 (2H, d), 7.23 (1H, s), 7.77 (1H, s), 8.03 (2H, d), 8.58 (1H, s), 9.14 (1H, t), 12.31 (1H, br s). m/z (ES⁺) = 344 [*M* + H]⁺; RT = 3.34min.

The following compounds were synthesised according to the method or **EXAMPLE 45** using the appropriate ketal and aqueous hydrochloric acid.

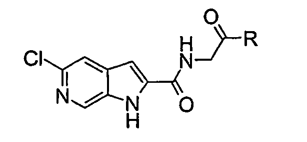

| **Example** | **R** | **m/z** |
|---|---|---|
| **46** | 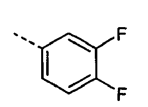 | m/z (ES⁺) = 350 [*M* + H]⁺; RT = 3.20min |
| **47** | 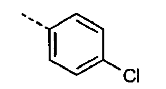 | m/z (ES⁺) = 348 [M + H]⁺; RT = 3.32min |
| **48** | 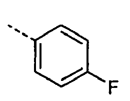 | m/z (ES⁺) = 332 [*M* + H]⁺; RT = 3.25min |

### EXAMPLE 49

### 5-Chloro-1H-pyrrolo[2,3-c]pyridine-2-carboxylic acid [2-(4-fluorophenyl)-2-hydroxyethyl]amide

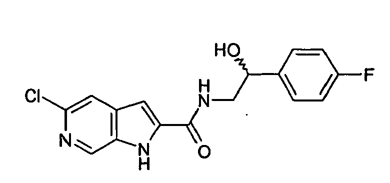

To a stirred suspension of 5-chloro-1H-pyrrolo[2,3-c]pyridine-2-carboxylic acid [2-(4-fluorophenyl)-2-oxoethyl]amide **EXAMPLE 48** (0.05g, 151mmol) in ethanol (5mL, absolute) was added polymer-supported borohydride (2.5mmol/g, 0.09g, 226mmol) and the mixture sonicated with gentle warming until the ketone had dissolved. The reaction mixture was stirred for 2 days at rt then filtered, washing with methanol. The filtrate was evaporated to dryness *in vacuo* to give a colourless oil. Purification *via* flash column chromatography (SiO₂, ethyl acetate : isohexane, 1:1, v/v) gave the title compound as a white solid. m/z (ES⁺) = 334 [*M* + H]⁺; RT = 2.93min.

### EXAMPLE 50

### 5-Chloro-1H-pyrrolo[2,3-c]pyridine-2-carboxylic acid (1-dimethylcarbamoyl-2-(S)-pyridin-3-yl-ethyl)amide

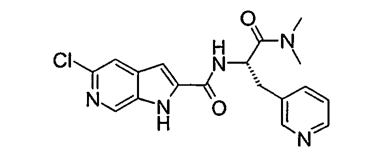

To a solution of (*S*)-2-amino-*N,N*-dimethyl-3-pyridin-3-ylpropionamide hydrochloride (**Preparation 53**, 170mg, 0.74mmol) in DMF (5mL) was added DIPEA (0.45mL, 2.58mmol), 5-chloro-1*H*-pyrrolo[2,3-c]pyridine-2-carboxylic acid (**Preparation 18**, 145mg, 0.74mmol) and TBTU (262mg, 0.82mmol). The reaction mixture was stirred at rt for 16h. The solvent was removed *in vacuo* and the remainder was purified by preparative HPLC, to give the title compound as an off-white solid. δ_{H} (CD₃OD): 2.95 (3H, s), 3.08 (3H, s), 3.11-3.18 (1H, m), 3.22-3.28 (1H, m), 5.31-5.37 (1H, m), 7.12 (1H, s), 7.34-7.38 (1H, m), 7.65 (1H, s), 7.79-7.84 (1H, m), 8.37-8.41 (1H, m), 8.45-8.51 (1H, m), 8.54 (1H, s); m/z (ES⁺) = 372 [*M*+ H]⁺.

### EXAMPLE 51

### 5-Chloro-1H-pyrrolo[2,3-c]pyridine-2-carboxylic acid (2-oxo-1-(S)-pyridin-3-ylmethyl-2-pyrrolidin-1-yl-ethyl)amide

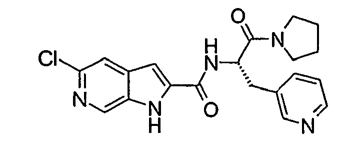

To a solution of (*S*)-2-amino-3-pyridin-3-yl-1-pyrrolidin-1-ylpropan-1-one hydrochloride (**Preparation 54**, 239mg, 0.94mmol) in DMF (5mL) was added DIPEA (0.60mL, 3.28mmol), 5-chloro-1*H-*pyrrolo[2,3-c]pyridine-2-carboxylic acid (**Preparation 18**, 184mg, 0.94mmol) and TBTU (330mg, 1.03mmol). The reaction mixture was stirred at rt for 16h. The solvent was removed *in vacuo* and the residue partitioned between ethyl acetate (100mL) and sodium hydroxide solution (2 x 100mL, 1N). The organic phase was dried (MgSO₄) and evaporated to dryness give the title compound as a brown solid. δ_{H} (CD₃OD): 1.74-1.94 (4H, m), 3.12-3.39 (4H, m), 3.41-3.48 (1H, m), 3.69-3.75 (1H, m), 5.07-5.12 (1H, m), 7.16 (1H, s), 7.34-7.39 (1H, m), 7.67 (1H, s), 7.79-7.83 (1H, m), 8.37-8.42 (1H, m), 8.45-8.51 (1H, m), 8.56 (1H, s); m/z (ES⁺) = 398 [*M*+ H]⁺.

### EXAMPLE 52

### 5-Chloro-1H-pyrrolo[2,3-c] pyridine-2-carboxylic acid (1-dimethylcarbamoyl-2-(S)-pyridin-2-yl-ethyl)amide

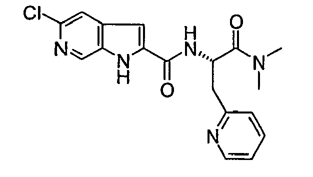

To a solution of 2-(*S*)-amino-*N,N*-dimethylamino-3-pyridin-2-yl-propionamide hydrochloride (**Preparation 53**, 0.15g, 0.66mmol) in DMF (5mL) was added DIPEA (0.4mL, 2.31mmol), TBTU (0.212g, 0.66mmol) and 5-chloro-1*H*-indole-2-carboxylic acid (**Preparation 18**, 0.130g, 0.66mmol). The reaction mixture was stirred at rt for 16h then concentrated under reduced pressure. The residue was dissolved in ethyl acetate (200mL) and washed with sodium hydroxide solution (100mL, 1N). The organic extract was dried and concentrated *in vacuo.* Purification by preparative hplc gave the title compound as a white solid. m/z (ES⁺) = 372 [*M* + H]⁺; RT = 2.47min.

### EXAMPLE 53

### 5-Chloro-1H-pyrrolo [2,3-c] pyridine-2-carboxylic acid [1-(S)-(4-fluorobenzyl)-2-(3-(S)-hydroxypyrrolidin-1-yl)-2-oxoethyl]amide

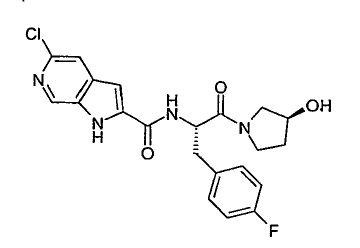

To a solution of carboxylic acid (**Preparation 18**, 2.66g, 13.6mmol) in DMF (anhydrous, 120mL) was added amine.HCl (**Preparation 103**, 4.30g, 14.9mmol), DIPEA (7.79mL, 44.7mmol) and HOBt.H₂O (2.280g, 14.9mmol). The resulting solution was stirred at rt for 10min prior to the addition of EDCI (3.12g, 16.3mmol) and the reaction mixture stirred for 17h at rt. The volatiles were removed *in vacuo* then the residue was partitioned between ethyl acetate (200mL) and water (200mL). The aqueous phase was extracted with ethyl acetate (3x50mL) then the combined organics washed with sodium hydroxide solution (2M, 3x50mL), hydrochloric acid (2M, 2x50mL), brine (100mL), dried (MgSO₄), filtered and concentrated *in vacuo.* The isolated solid was dissolved in methanol / dichloromethane (15:185, v/v) then purified *via* flash chromatography (SiO₂, dissolved in methanol / dichloromethane, 15:185, v/v) to give the title compound as a pale yellow powder. δ_{H} (CD₃OD): 1.72-2.02 (2H, m), 3.01-3.13 (1H, m), 3.13-3.26 (2H, m), 3.35-3.52 (1.5H, m), 3.55-3.65 (0.5H, m), 3.71-3.81 (0.5H, m), 3.84 (0.5H, dd), 4.23-4.33 (0.5H, m), 4.37-4.45 (0.5H, m), 4.99 (0.5H, t), 5.07 (0.5H, t), 6.90-7.07 (2H, m), 7.13 (1H, d), 7.31 (2H, dd), 7.66 (1H, s), 8.54 (1H, d); m/z (ES⁺) = 431 [*M* + H]⁺; RT = 3.17min.

### EXAMPLE 54

### 5-Chloro-1H-pyrrolo [2,3-c] pyridine-2-carboxylic acid [2-(S)-(4-fluorophenyl)-1-isopropylcarbamoylethyl] amide

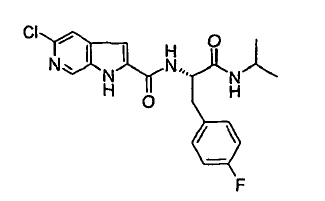

To a solution of 2-(S)-[(5-chloro-1H-pyrrolo[2,3-c]pyridine-2-carbonyl)-amino]-3-(4-fluorophenyl)propionic acid (30mg, 0.08mmol) in DMF (3mL) was added DIPEA (17.3µL, 0.10mmol) and HATU (37.8mg, 0.10mmol). After 15min, isopropylamine (7.1µL, 0.08mmol) was added. The reaction mixture was stirred at rt for 24h then concentrated under reduced pressure (genevac). Purification by mass directed purification gave the title compound as a yellow solid. m/z (ES⁺) = 403 [*M*+H] ⁺; RT = 3.39min.

### EXAMPLES 55-98

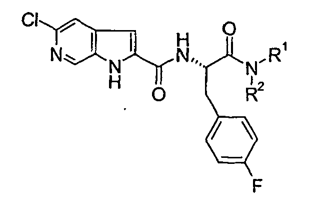

The following compounds were prepared according to the method of **EXAMPLE 54** from 2-(*S*)-[(5-chloro-1H-pyrrolo[2,3-c]pyridine-2-carbonyl)amino]-3-(4-fluorophenyl)propionic acid and the appropriate amine.

| **Example** | **NR¹R²** | **m/z** | **RT (min)** |
|---|---|---|---|
| **55** | 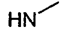 | 375 | 3.11 |
| **56** | 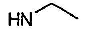 | 389 | 3.24 |
| **57** | 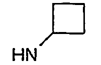 | 415 | 3.36 |
| **58** | 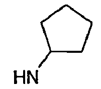 | 429 | 3.51 |
| **59** | 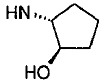 | 443/445 | 3.11 |
| **60** | 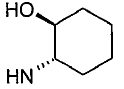 | 457/459 | 3.22 |
| **61** | 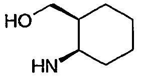 | 471/473 | 3.29 |
| **62** | 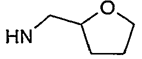 | 445 | 3.20 |
| **63** | 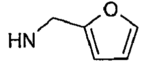 | 441 | 3.44 |
| **64** | 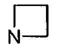 | 401 | 3.19 |
| **65** | 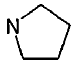 | 415 | 3.31 |
| **66** | 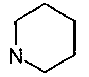 | 429 | 3.51 |
| **67** | 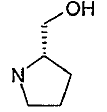 | 445 | 3.24 |
| **68** | 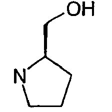 | 445 | 3.14 |
| **69** | 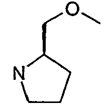 | 459 | 3.39 |
| **70** | 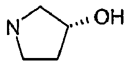 | 431 | 3.07 |
| **71** | 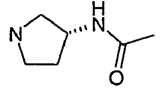 | 472 | 3.04 |
| **72** | 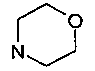 | 431 | 3.17 |
| **73** | 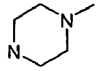 | 444 | 2.69 |
| **74** | 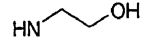 | 405 | 3.06 |
| **75** | 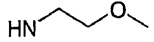 | 419 | 3.19 |
| **76** | 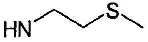 | 435 | 3.36 |
| **77** | 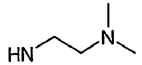 | 432 | 2.77 |
| **78** | 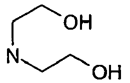 | 449 | 2.99 |
| **79** | 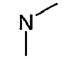 | 389 | 3.24 |
| **80** | 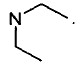 | 417 | 3.37 |
| **81** | 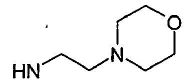 | 474 | 2.67 |
| **82** | 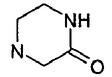 | 444 | 3.04 |
| **83** | 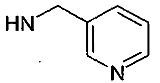 | 452 | 2.65 |
| **84** | 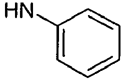 | 437 | 3.57 |
| **85** | 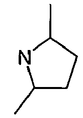 | 443 | 3.52 |
| **86** | 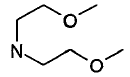 | 477 | 3.31 |
| **87** | 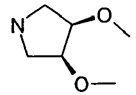 | 475 | 3.11 |
| **88** | 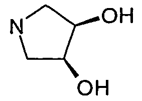 | 447 | 2.99 |
| **89** | 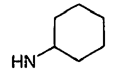 | 443 | 3.51 |
| 90 | 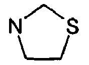 | 433 | 3.32 |
| 91 | 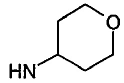 | 445 | 3.19 |
| 92 | 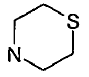 | 447 | 3.37 |
| 93 | 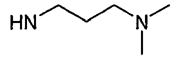 | 446 | 2.64 |
| 94 | 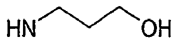 | 419 | 3.04 |
| 95 | 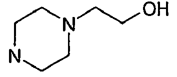 | 474 | 2.64 |
| 96 | 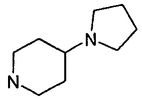 | 498 | 2.70 |
| 97 | 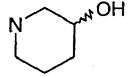 | 445 | 3.14 |
| 98 | 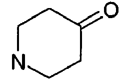 | 443 | 3.20 |

### EXAMPLE 99

### 5-Chloro-1H-pyrrolo[2,3-c]pyridine-2-carboxylic acid [1-cyclopropylcarbamoyl-2-(S)-(4-fluorophenyl)ethyl] amide

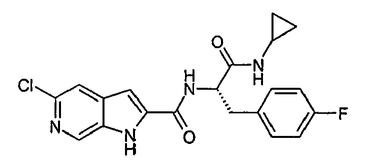

To a solution of 2-(*S*)-[(5-chloro-1*H*-pyrrolo[2,3-c]pyridine-2-carbonyl)-amino]-3-(4-fluorophenyl)propionic acid (100mg, 0.28mmol) in DMF (5ml) was added cyclopropylamine (19.2µl, 0.28mmol), HOBt (37mg, 0.28mmol) and DIPEA (96µl, 0.55mmol). After 5min, EDCI (69mg, 0.36mmol) was added and the reaction mixture stirred at rt for 16h. The solvent was removed *in vacuo* and the residue triturated with water and chromatographed on silica gel eluting with methanol:dichloromethane (1:24) to give the title compound as an off-white solid. m/z (ES⁺) = 401 [*M*+ H]⁺; RT = 3.22min.

### EXAMPLES 100-106

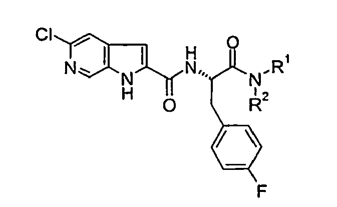

The following compounds were prepared according to the method of **EXAMPLE 99** from 2-(*S*)-[(5-chloro-1*H-*pyrrolo[2,3-c]pyridine-2-carbonyl)amino]3-(4-fluorophenyl)propionic acid and the appropriate amine. All compounds were purified by chromatography on silica gel eluting with the described solvent system.

| **Example** | **NR¹R²** | **Eluent** | **m/z** | **RT** (min) |
|---|---|---|---|---|
| **100** | 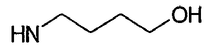 | MeOH:DCM 1:19 | 433 | 3.06 |
| **101** | 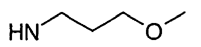 | MeOH:DCM 3:97 | 433 | 3.20 |
| **102** | 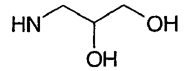 | MeOH:DCM 6:94 | 435 | 2.95 |
| **103** | 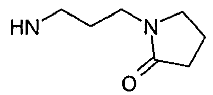 | MeOH:DCM 3:97 | 486 | 3.15 |
| **104** | 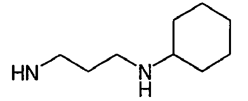 | NH₄OH:MeOH:DCM 1:9:90 | 500 | 2.82 |
| **105** | 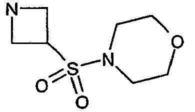 | MeOH:DCM 1:19 | 550 | 3.24 |
| **106** | 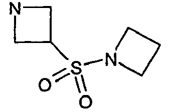 | MeOH:DCM 1:19 | 520 | 3.36 |

### EXAMPLE 107

### 5-Chloro-1H-pyrrolo[2,3-c]pyridine-2-carboxylic acid [1-(S)-(4-fluorobenzyl)-2-(3-hydroxyazetidin-1-yl)-2-oxoethyl]amide

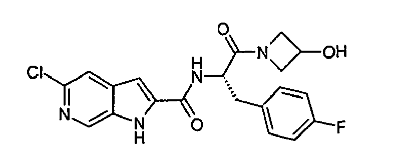

2-(*S*)-[(5-Chloro-1H-pyrrolo[2,3-c]pyridine-2-carbonyl)amino]-3-(4-fluorophenyl)propionic acid (100mg, 0.28mmol), 3-hydroxyazetidine hydrochloride (Heterocycles, 2002, 56(1-2), 433-442; 30mg, 0.28mmol) and HOBt (37mg, 0.28mmol) were dissolved in DMF (5ml) and DIPEA (0.14ml, 0.83mmol). After 5min, EDCI (69mg, 0.36mmol) was added and the reaction mixture stirred at rt for 16h. The solvent was removed *in vacuo* and the residue triturated with water. Purification by chromatography on silica gel eluting with methanol:dichloromethane (1:49 to 3:97) gave the title compound as a yellow solid. δ_{H} (CD₃OD): 3.08-3.21 (2H, m), 3.58-3.63 (0.5H, m), 3.84-3.91 (0.5H, m), 4.03-4.09 (0.5H, m), 4.25-4.32 (0.5H, m), 4.38-4.94 (4H, m), 7.03-7.12 (2H, m), 7.15-7.19 (1H, m), 7.30-7.38 (2H, m), 7.68 (1H, s), 8.59 (1H, s); RT = 3.32min.

### EXAMPLE 108

### 5-Chloro-1H-pyrrolo[2,3-c]pyridine-2-carboxylic acid [1-(S)-benzyl-2-(3-hydroxazetidin-1-yl)-2-oxoethyl]amide

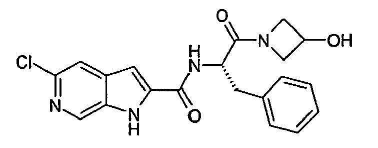

2-(*S*)-[(5-Chloro-1*H*-pyrrolo[2,3-c]pyridine-2-carbonyl)amino]-3-phenylpropionic acid (100mg, 0.29mmol), 3-hydroxyazetidine hydrochloride (32mg, 0.29mmol) and HOBt (39mg, 0.29mmol) were dissolved in DMF (5ml) and DIPEA (0.15ml, 0.87mmol). After 5min, EDCI (72mg, 0.38mmol) was added and the reaction mixture stirred at rt for 16h. The solvent was removed *in vacuo* and the residue triturated with water. Purification by chromatography on silica gel eluting with methanol:dichloromethane (1:49 to 3:97) gave the title compound as an off-white solid. δ_{H} (CD₃OD): 3.12-3.20 (2H, m), 3.45-3.51 (0.5H, m), 3.83-3.90 (0.5H, m), 4.00-4.13 (1H, m), 4.35-4.52 (2H, m), 4.57-4.94 (2H, m), 7.18-7.22 (1H, m), 7.27-7.40 (5H, m), 7.68 (1H, s), 8.59 (1H, s); RT = 3.27min.

### EXAMPLE 109

### 5-Chloro-1H-pyrrolo[2,3-c]pyridine-2-carboxylic acid [2-(3-hydroxyazetidin-1-yl)-2-oxoethyl]amide

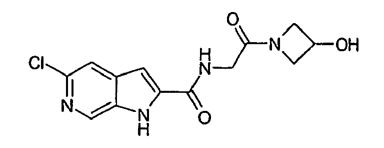

[(5-Chloro-1*H-*pyrrolo[2,3-c]pyridine-2-carbonyl)amino] acetic acid (30mg, 0.12mmol), 3-hydroxyazetidine hydrochloride (13mg, 0.12mmol) and HOBt (16mg, 0.12mmol) were dissolved in DMF (3ml) and DIPEA (43µl, 0.25mmol). After 5min, EDCI (30mg, 0.15mmol) was added and the reaction mixture stirred at rt for 16h. The solvent was removed *in vacuo* and the residue partitioned between water (20ml) and DCM (3 x 20ml). The combined organics were dried (MgSO₄), concentrated *in vacuo* and the residue purified by chromatography on silica gel eluting with methanol:dichloromethane (1:24) to give the title compound as a white solid. δ_{H} (CD₃OD): 4.06-4.15 (3H, m), 4.40-4.46 (1H, m), 4.55-4.63 (1H, m), 4.77-4.94 (2H, m), 7.14 (1H, s), 7.71 (1H, s), 8.62 (1H, s); RT = 2.82min.

### EXAMPLES 110-111

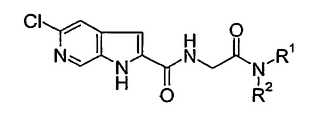

The following compounds were prepared according to the method of **EXAMPLE 109** from [(5-chloro-1*H-*pyrrolo[2,3-c]pyridine-2-carbonyl)amino] acetic acid and the appropriate amine. All compounds were purified by chromatography on silica gel eluting with the described solvent system.

| **Example** | **NR¹R²** | **Eluent** | **m/z** | **RT (min)** |
|---|---|---|---|---|
| **110** | 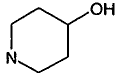 | MeOH:DCM 6:94 to 8:92 | 337 | 2.61 |
| **111** | 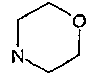 | MeOH:DCM 1:24 | 323 | 2.65 |

### EXAMPLE 112

### 2-[(5-Chloro-1H-pyrrolo[2,3-c]pyridine-2-carbonyl)amino]3-oxo-3-phenylpropionic acid ethyl ester

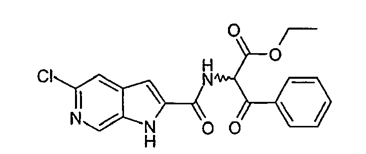

5-Chloro-1*H-*pyrrolo[2,3-c]pyridine-2-carboxylic acid (100mg, 0.51mmol) and 4-(4,6-dimethoxy[1.3.5]triazin-2-yl)-4-methylmorpholinium chloride (DMTMM) (183mg, 0.66mmol) were dissolved in THF (20ml). After 5min, 2-amino-3-oxo-3-phenylpropionic acid ethyl ester hydrochloride (Tetrahedron Lett., 1993, 34(2), 211-214; 124mg, 0.51mmol) and 4-methylmorpholine (56µl, 0.51mmol) were added and the reaction mixture stirred at rt for 72h. The solvent was removed *in vacuo* and the residue partitioned between water (40ml) and EtOAc (3 x 30ml). The combined organics were dried (MgSO₄), concentrated *in vacuo* and purified by chromatography on silica gel eluting with methanol:dichloromethane (1:19). This material was further triturated with methanol:diethyl ether (1:19) to give the title compound as a pale yellow solid. m/z (ES⁺) = 386 [*M* + H]⁺; RT = 3.56min.

### EXAMPLE 113

### 5-Chloro-1H-pyrrolo[2,3-c]pyridine-2-carboxylic acid [2-(S)-(4-fluorophenyl)-1-(methoxymethylcarbamoyl)ethyl]amide

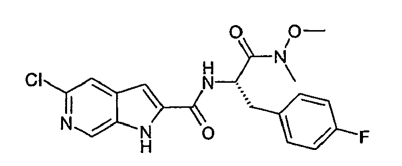

2-(*S*)-[(5-Chloro-1H-pyrrolo[2,3-c]pyridine-2-carbonyl)amino]-3-(4-fluorophenyl)propionic acid (80mg, 0.22mmol) and *N,O*-dimethylhydroxylamine hydrochloride (24mg, 0.24mmol) were dissolved in ethanol (10ml) and 4-methylmorpholine (27µl, 0.24mmol). To this was added DMTMM (67mg, 0.24mmol) and the reaction mixture was stirred at rt for 16h. Further *N,O*-dimethylhydroxylamine hydrochloride (12mg, 0.12mmol), 4-methylmorpholine (14µl, 0.12mmol) and DMTMM (34mg, 0.12mmol) were added and the reaction mixture was stirred at rt for 96h. The solvent was removed *in vacuo* and the residue partitioned between water (40ml) and EtOAc (2 x 40ml). The combined organics were dried (MgSO₄), concentrated *in vacuo* and purified by chromatography on silica gel eluting with methanol:dichloromethane (1:19) to give the title compound as a white solid. m/z (ES⁺) = 405 [*M* + H]⁺; RT = 3.36min.

### EXAMPLE 114

### 5-Chloro-1H-pyrrolo[2,3-b]pyridine-2-carboxylic acid [1-(S)-(4-fluorobenzyl)-2-(4-hydroxypineridin-1-yl)-2-oxoethyl]amide

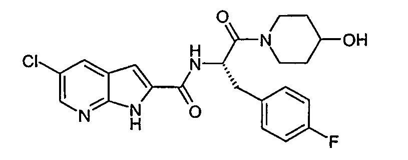

5-Chloro-1H-pyrrolo[2,3-b]pyridine-2-carboxylic acid (**Preparation 57,** 50mg, 0.25mmol), 2-(*S*)-amino-3-(4-fluorophenyl)-1-(4-hydroxypiperidin-1-yl)propan-1-one hydrochloride (85mg, 0.25mmol) and DMTMM (85mg, 0.31mmol) were dissolved in ethanol (5ml) and 4-methylmorpholine (31µl, 0.28mmol). The reaction mixture was stirred at rt for 16h. The solvent was removed *in vacuo* and the residue partitioned between water (30ml) and EtOAc (3 x 25ml). The combined organics were dried (MgSO₄), concentrated *in vacuo* and purified by chromatography on silica gel eluting with methanol:dichloromethane (1:19) to give the title compound as a beige solid. m/z (ES⁺) = 445 [*M* + H]⁺; RT = 3.31min.

### EXAMPLE 115

### 5-Chloro-1H-pyrrolo[2,3-b]pyridine-2-carboxylic acid (1-dimethylcarbamoyl-2-(S)-phenylethyl)amide

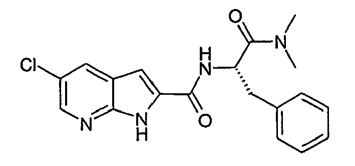

5-Chloro-1H-pyrrolo[2,3-b]pyridine-2-carboxylic acid (**Preparation 57**, 55mg, 0.28mmol), 2-(*S*)-amino-*N,N*-dimethyl-3-phenylpropionamide hydrochloride (70mg, 0.31mmol) and DMTMM (93mg, 0.34mmol) were dissolved in ethanol (5ml) and 4-methylmorpholine (34µl, 0.31mmol). The reaction mixture was stirred at rt for 24h. The solvent was removed *in vacuo* and the residue partitioned between water (40ml) and EtOAc (2 x 40ml). The combined organics were washed with 2N NaOH solution (40ml), brine (40ml), dried (MgSO₄) concentrated *in vacuo* and purified by chromatography on silica gel eluting with methanol:dichloromethane (3:97) to give the title compound as a yellow solid. m/z (ES⁺) = 371 [*M*+ H]⁺; RT = 3.49min.

### EXAMPLE 116

### 5-Ethynyl-1H-pyrrolo[2,3-c]pyridine-2-carboxylic acid (1-(S)-dimethylcarbamoyl-2-phenylethyl)amide

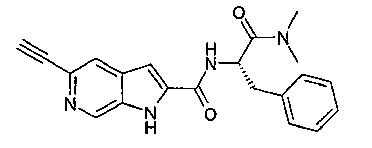

To a solution of 2-(*S*)-amino-*N,N*-dimethyl-3-phenylpropionamide hydrochloride (**Preparation 8**, 0.0135g, 0.059mmol) in DMF (anhydrous, 3mL) was added DIPEA (0.031mL, 0.177mmol) then carboxylic acid (**Preparation 60**, 0.010g, 0.054mmol). To the stirred solution was added HOBt.H₂O (0.008g, 0.059mmol) then, after 10min, EDCI (0.012g, 0.065mmol). The reaction mixture was stirred for 18h then all volatiles were removed *in vacuo.* The residue was partitioned between ethyl acetate (30mL) and water (20mL). The layers were separated and the aqueous layer extracted with ethyl acetate (3x20mL). The combined organics were washed with brine (30mL), dried (MgSO₄), filtered and concentrated *in vacuo.* The residue was dissolved in methanol then adsorbed onto silica gel. Purification *via* flash column chromatography (SiO₂, CH₂Cl₂: MeOH, 19 : 1, v/v) gave the title compound as an off-white solid. δ_{H} (CD₃OD): 2.88 (3H, s), 2.89 (3H, s), 3.00-3.23 (2H, m), 3.50 (1H, s), 5.27 (1H, t), 7.10-7.37 (6H, m), 7.86 (1H, s), 8.70 (1H, s). m/z (ES⁺) = 361 [*M* + H]⁺; RT = 2.65min.

### EXAMPLE 117

### 5-Cyano-1H-pyrrolo[2,3-c]pyridine-2-carboxylic acid (1-(S)-dimethylcarbamoyl-2-phenylethyl)amide

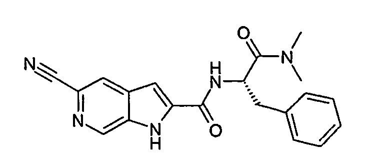

To a solution of 2-(*S*)-amino-*N,N*-dimethyl-3-phenylpropionamide hydrochloride (**Preparation 8**, 0.023g, 0.100mmol) in DMF (anhydrous, 4mL) was added DIPEA (0.052mL, 0.300mmol) then carboxylic acid (**Preparation 62**, 0.017g, 0.091mmol). To the stirred solution was added HOBt.H₂O (0.0135g, 0.100mmol) then, after 10min, EDCI (0.021g, 0.109mmol). The reaction mixture was stirred for 18h then all volatiles were removed *in vacuo.* The residue was partitioned between ethyl acetate (30mL) and water (20mL). The layers were separated and the aqueous layer extracted with ethyl acetate (3x20mL). The combined organics were washed with brine (30mL), dried (MgSO₄) filtered and concentrated *in vacuo.* The residue was dissolved in ethyl acetate then adsorbed onto silica gel. Purification *via* flash column chromatography (SiO₂, ethyl acetate : isohexane, 1:1, v/v) gave the title compound as a pale brown solid. δ_{H} (CDCl₃): 2.77 (3H, s), 2.96 (3H, s), 3.08-8.20 (2H, m), 5.23-5.41 (1H, m), 6.99 (1H, s), 7.14-7.36 (5H, m), 7.65 (1H, d), 8.01 (1H, s), 8.87 (1H, s), 10.01 (1H, s); m/z (ES⁺) = 362 [*M* + H]⁺; RT = 3.11min.

### EXAMPLE 118

### 5-Cyano-1H-pyrrolo[2,3-c]pyridine-2-carboxylic acid [1-(S)-(4-fluorobenzyl)-2-(4-hydroxypiperidin-1-yl)-2-oxoethyl]amide

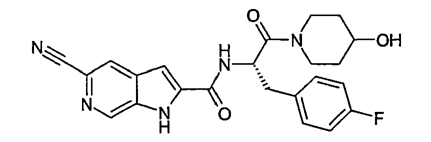

To a solution of 2-(*S*)-amino-3-(4-fluorophenyl)-1-(4-hydroxypiperidin-1-yl)propan-1-one hydrochloride (**Preparation 20**, 0.036g, 0.118mmol) in DMF (anhydrous, 5mL) was added DIPEA (0.061mL, 0.353mmol) then carboxylic acid (**Preparation 62**, 0.020g, 0.107mmol). To the stirred solution was added HOBt.H₂O (0.016g, 0.107mmol) then, after 10min, EDCI (0.025g, 0.128mmol). The reaction mixture was stirred for 18h then all volatiles were removed *in vacuo.* The residue was partitioned between CH₂Cl₂ (30mL) and water (20mL). The layers were separated then the aqueous was extracted with CH₂Cl₂ (2x30mL). The combined organics were washed with brine (50mL), dried (MgSO₄), filtered and concentrated *in vacuo.* The residue was dissolved in methanol then adsorbed onto silica gel. Purification *via* flash column chromatography (SiO₂, methanol : dichloromethane, 7:93, v/v) gave the title compound as a white solid. δ_{H} (d₆ DMSO): 1.05-1.32 (2H, m), 1.49-1.72 (2H, m), 2.86-3.42 (4H, m), 3.53-3.87 (2.5H, m), 3.93-4.05 (0.5H, m), 4.69 (1H, d), 5.07-5.21 (1H, m), 6.97-7.12 (2H, m), 7.26-7.39 (2H, m), 7.47 (1H, s), 8.41 (1H, s), 8.81 (1H, s), 9.01-9.41 (1H, m), 12.64 (1H, s); m/z (ES⁺) = 436 [*M* + H]⁺; RT = 1.51min.

### EXAMPLE 119

### 5-Methyl-1H-pyrrolo[2,3-c]pyridine-2-carboxylic acid (1-dimethylcarbamoyl-2-(S)-phenylethyl) amide

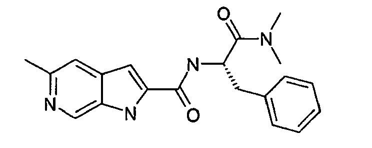

To a solution of 2-(*S*)-amino-*N,N*-dimethyl-3-phenylpropionamide hydrochloride (**Preparation 8**, 0.0286g, 0.125mmol) in DMF (anhydrous, 5mL) was added DIPEA (0.071mL, 0.409mmol) then carboxylic acid (**Preparation 66**, 0.020g, 0.114mmol). To the stirred solution was added HOBt.H₂O (0.017g, 0.125mmol) then, after 10min, EDCI (0.026g, 0.136mmol). The reaction mixture was stirred for 16h at room temperature then all volatiles were removed *in vacuo.* The residue was partitioned between CH₂Cl₂ (50mL) and water (50mL). The layers were separated then the aqueous layer extracted with CH₂Cl₂ (3x20mL). The combined organics were washed with brine (30mL), dried (MgSO₄), filtered and concentrated *in vacuo.* The residue was dissolved in CH₂Cl₂ then purified *via* flash column chromatography (SiO₂, CH₂Cl₂ then methanol : CH₂Cl₂, 1:19, v/v) to give the title compound as a pale yellow solid. δ_{H} (CDCl₃): 2.63 (3H, s), 2.77 (3H, s), 2.97 (3H, s), 3.11-3.25 (2H, m), 5.34-5.44 (1H, m), 6.85 (1H, s), 7.20-7.33 (6H, m), 7.34 (1H, s), 7.83 (1H, d), 8.78 (1H, s); m/z (ES⁺) = 351 [*M*+ H]⁺; RT = 2.36min.

### EXAMPLE 120

### 5-Chloro-1H-pyrrolo[2,3-c]pyridine-2-carboxylic acid [1-(S)-(4-fluorobenzyl)-2-(4-methoxypiperidin-1-yl)-2-oxoethyl] amide

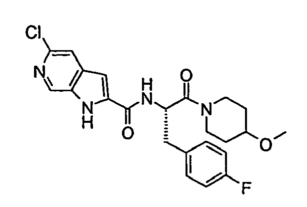

To a solution of 2-(*S*)-[(5-chloro-1*H-*pyrrolo[2,3-c]pyridine-2-carbonyl)amino]-3-phenylpropionic acid (**EXAMPLE 42**, 150mg, 0.42mmol) and 4-methoxypiperidine hydrochloride (**Preparation 68**, 86mg, 0.57mmol) in DMF (5mL) was added HOBt (66mg, 0.43mmol), DIPEA (0.23mL, 1.34mmol) and EDCI (102mg, 0.53mmol). After stirring at rt for 12h the mixture was added to diluted brine (100mL, water/brine : 1/1). Extraction with ethyl acetate (4 x 25mL), washing of the combined extracts with brine (50mL) and drying (MgSO₄) gave, after concentration, a residue which was purified by recrystallisation from methanol to give the title compound as a colourless solid. m/z (ES⁺) = 459.38 [*M*+H]⁺; RT = 3.40min.

### EXAMPLE 121

### 5-Chloro-1H-pyrrolo[2,3-c]pyridine-2-carboxylic acid [1-(S)-(4-fluorobenzyl)-2-(3-(R)-methoxypyrrolidin-1-yl)-2-oxoethyl]amide

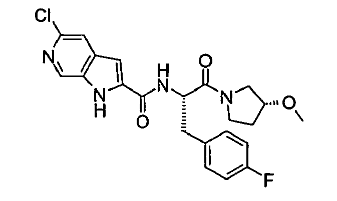

To a solution of 2-(*S*)-[(5-chloro-1*H-*pyrrolo[2,3-c]pyridine-2-carbonyl)amino]-3-phenylpropionic acid (**EXAMPLE 42**, 104mg, 0.29mmol) and(R)-3-methoxypyrrolidine hydrochloride (**Preparation 70**, 40mg, 0.29mmol) in DMF (5mL) was added HOBt (44mg, 0.29mmol), DIPEA (0.15mL, 0.88mmol) and EDCI (66mg, 0.344mmol). After stirring at rt for 12h the mixture was added to diluted brine (100mL, water/brine : 1/1). Extraction with ethyl acetate (4 x 25mL), washing of the combined extracts with brine (50mL) and drying (MgSO₄) gave, after concentration, a residue which was recrystallised from acetonitrile to give the title compound as a colourless solid. m/z (ES⁺) = 445.31 [*M*+H]⁺; RT = 3.36min.

### EXAMPLE 122

### 5-Chloro-1H-pyrrolo[2,3-c]pyridine-2-carboxylic acid [(1-(S)-(4-fluorobenzyl)-2-(3-(S)-methoxypyrrolidin-1-yl)-2-oxoethyl]amide

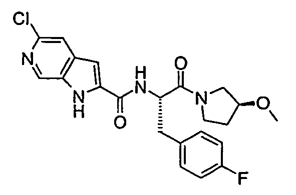

To a solution of 2-(*S*)-[(5-chloro-1*H-*pyrrolo[2,3-c]pyridine-2-carbonyl)amino]-3-phenylpropionic acid (**EXAMPLE 42**, 53mg, 0.15mmol) and (*S*)-3-methoxypyrrolidine hydrochloride (**Preparation 72**, 20mg, 0.15mmol) in DMF (5mL) was added HOBt (25mg, 0.16mmol), DIPEA (76µL, 0.44mmol) and EDCI (34mg, 0.18mmol). After stirring at rt for 12h the mixture was added to diluted brine (100mL, water/brine : 1/1). Extraction with ethyl acetate (4 x 25mL), washing of the combined extracts with brine (50mL) and drying (MgSO₄) gave, after concentration, a residue which was purified *via* flash chromatography (silica gel, dichloromethane/methanol, 95:5) to give the title compound as a colourless solid. m/z (ES⁺) = 445.34 [*M*+H]⁺; RT = 3.34min.

### EXAMPLE 123

### 3-(S)-[(5-Chloro-1H-pyrrolo[2,3-c]pyridine-2-carbonyl)amino]-2-(S)-hydroxy-4-phenylbultyric acid methyl ester

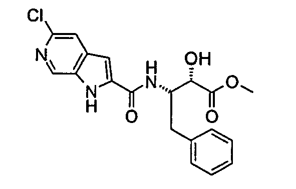

5-Chloro-1*H-*pyrrolo[2,3-c]pyridine-2-carboxylic acid (**Preparation 18**, 170mg, 0.86mmol) and (3*S*,2*S*)-3-amino-2-hydroxy-4-phenylbutyric acid methyl ester (**Preparation 21A**, 174mg, 0.83mmol) were coupled under similar conditions to **EXAMPLE 43** using HOBt (142mg, 0.93mmol), EDCI (200mg, 1.04mmol), DIPEA (0.32ml, 1.87mmol) in DMF (10ml). The crude product was purified by chromatography on silica gel eluting with hexane / ethyl acetate (25:75) to give the title compound as a colourless oil. δ_{H} (CDCl₃): 3.04 (4H, 2dd), 3.74 (3H, s), 4.36 (1H, br s), 4.63 (1H, m), 4.98 (1H, ddd), 6.66 (1H, s), 6.96 (1H, d), 7.18-7.35 (5H, m), 7.48 (1H, s), 8.63 (1H, s).

### EXAMPLE 124

### 3-(S)-[(5-Chloro-1H-pyrrolo[2,3-c]pyridine-2-carbonyl)amino]-2-(S)-hydroxy-4-phenylbutyric acid

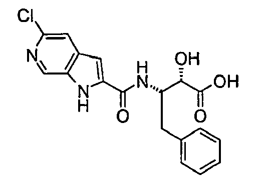

3-(*S*)-[(5-Chloro-1H-pyrrolo[2,3-c]pyridine-2-carbonyl)amino]-2-(*S*)-hydroxy-4-phenylbutyric acid methyl ester (152mg, 0.39mmol) was hydrolysed in a similar way to **EXAMPLE 44** using sodium hydroxide solution (0.44ml, 1N, 0.44mmol) in methanol (10mL). δ_{H} (d₆ DMSO): 2.83,2.95 (2H, 2dd), 4.19 (1H, d), 4.52 (1H, m), 5.75 (1H, br s), 7.10-7.33 (6H, 2m), 7.68 (1H, s), 8.54 (1H, s), 8.72 (1H, d).

### EXAMPLE 125

### 5-Chloro-1H-pyrrolo[2,3-c]pyridine-2-carboxylic acid (1-(S)-benzyl-2-dimethylcarbamoyl-2-(S)-hydroxyethyl)amide

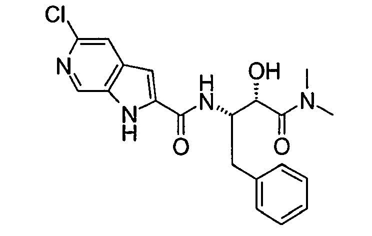

Dimethylamine hydrochloride (7mg, 0.085mmol) was added to a solution of 3-(*S*)-[(5-chloro-1H-pyrrolo[2,3-c]pyridine-2-carbonyl)amino]-2-(*S*)-hydroxy-4-phenylbutyric acid (**EXAMPLE 124**, 30mg, 0.080mmol), HOBt (14mg, 0.091mmol), DIPEA (31µL, 0.18mmol) and EDCI (18mg, 0.094mmol) in DMF (3mL). After the addition of DIPEA (14ml, 0.080mmol) the mixture was stirred for 12h before adding to diluted brine (100mL, water/brine : 1/1). Extraction with ethyl acetate (4 x 25mL), washing of the combined extracts with brine (50mL) and drying (MgSO₄) gave, after concentration, a residue which was purified by preparative LCMS to give the title compound as a colourless solid. m/z (ES⁺) = 401.28 [*M* + H]⁺; RT = 3.06min.

### EXAMPLE 126

### 5-Chloro-1H-pyrrolo[2,3-c]pyridine-2-carboxylic acid (1-(S)-benzyl-2-(S)-hydroxy-3-oxo-3-pyrrolidin-1-ylpropyl)amide

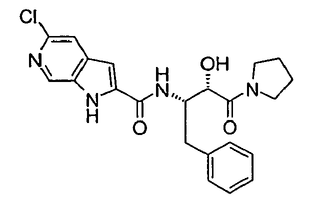

Pyrrolidine (7µg, 0.084mmol) was added to a solution of 3-(*S*)-[(5-chloro-1H-pyrrolo[2,3-c]pyridine-2-carbonyl)amino]-2-(*S*)-hydroxy-4-phenylbutyric acid (**EXAMPLE 124**, 30mg, 0.080mmol), HOBt (14mg, 0.091mmol), DIPEA (31µL, 0.18mmol) and EDCI (18mg, 0.094mmol) in DMF (3mL). After stirring for 12h the mixture was added to diluted brine (100mL, water/brine : 1/1). Extraction with ethyl acetate (4 x 25mL), washing of the combined extracts with brine (50mL) and drying (MgSO₄) gave, after concentration, a residue which was purified by preparative LCMS to give the title compound as a colourless solid. m/z (ES⁺) = 427.31 [*M*+H]⁺; RT = 3.27min.

### EXAMPLE 127

### 5-Chloro-1H-pyrrolo[2,3-c]pyridine-2-carboxylic acid [1-(S)-benzyl-3-(3,4-dihydroxypyrrolidin-1-yl)-2-(S)-hydroxy-3-oxopropyl]amide

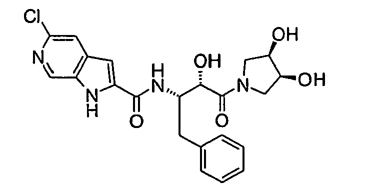

cis-3,4-Dihydroxypyrrolidine (**Preparation 23**, 9mg, 0.087mmol) was added to a solution of 3-(*S*)-[(5-chloro-1H-pyrrolo[2,3-c]pyridine-2-carbonyl)amino]-2-(*S*)-hydroxy-4-phenylbutyric acid (**EXAMPLE 124**, 30mg, 0.080mmol), HOBt (14mg, 0.091mmol), DIPEA (31µL, 0.18mmol) and EDCI (18mg, 0.094mmol) in DMF (3mL). After stirring for 12h the mixture was added to diluted brine (100mL, water/brine : 1/1). Extraction with ethyl acetate (4 x 25mL), washing of the combined extracts with brine (50mL) and drying (MgSO₄) gave, after concentration, a residue which was purified by preparative LCMS to give the title compound as a colourless solid. m/z (ES⁺) = 459.29 [*M* + H]⁺; RT = 2.87min.

### EXAMPLE 128

### 5-Chloro-1H-pyrrolo[2,3-c]pyridine-2-carboxylic acid (1-(S)-benzyl-2-(R)-hydroxy-2-propylcarbamoylethyl)amide

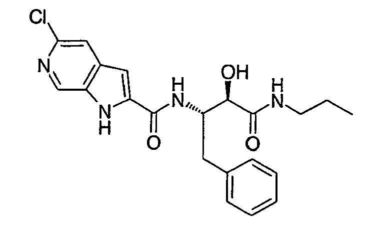

*n-*Propylamine (16µL, 0.19mmol) was added to a solution of 3-(*S*)-[(5-chloro-1H-pyrrolo[2,3-c]pyridine-2-carbonyl)amino]-2-(*R*)-hydroxy-4-phenylbutyric acid (**EXAMPLE 44**, 40mg, 0.11mmol), HOBt (16.4mg, 0.11mmol), DIPEA (41µL, 0.24mmol) and EDCI (25mg, 0.13mmol) in DMF (3mL). After stirring for 72h at room temperature the solvent was removed *in vacuo* and the remaining residue was purified by preparative LCMS to give the title compound as colourless solid. m/z (ES⁺) = 415.34 [*M*+H]⁺; RT = 3.10mm.

### EXAMPLES 129-147

The following compounds were prepared according to the method of **EXAMPLE 128** from 3-(*S*)-[(5-chloro-1H-pyrrolo[2,3-c]pyridine-2-carbonyl)-amino]-2-(*R*)-hydroxy-4-phenylbutyric acid and the appropriate amine.

| **Example** | **Structure** | **RT (min)** | **m/z** |
|---|---|---|---|
| **129** | 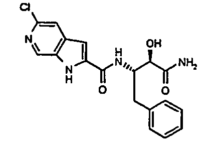 | 2.87 | 373.26 |
| **130** | 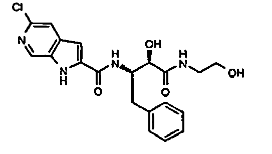 | 2.89 | 417.31 |
| **131** | 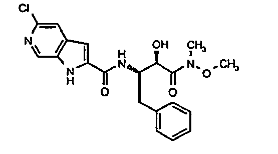 | 3.09 | 417.32 |
| **132** | 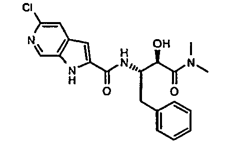 | 3.23 | 401.29 |
| **133** | 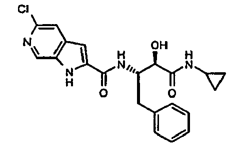 | 3.03 | 413.33 |
| **134** | 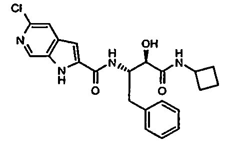 | 3.20 | 427.34 |
| **135** | 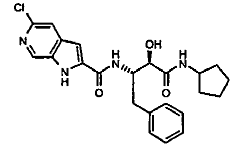 | 3.22 | 441.37 |
| **136** | 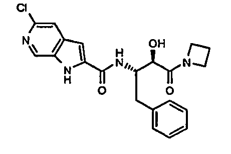 | 3.19 | 413.32 |
| **137** | 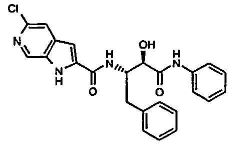 | 3.65 | 449.34 |
| **138** | 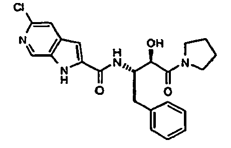 | 3.09 | 427.30 |
| **139** | 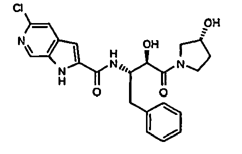 | 3.12 | 443.33 |
| **140** | 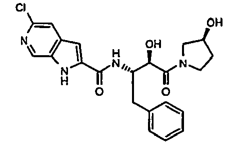 | 2.87 | 443.33 |
| **141** | 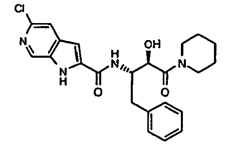 | 3.41 | 441.32 |
| **142** | 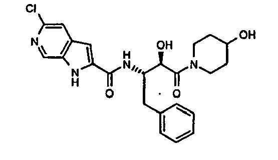 | 2.91 | 457.30 |
| **143** | 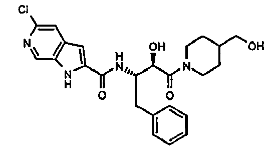 | 3.21 | 471.40 |
| **144** | 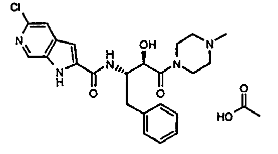 | 2.63 | 456.39 |
| **145** | 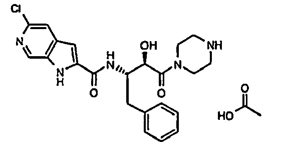 | 2.57 | 442.37 |
| **146** | 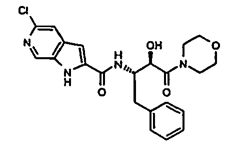 | 3.06 | 443.35 |
| **147** | 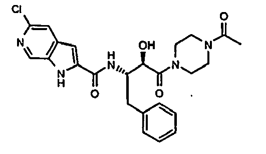 | 3.16 | 484.37 |

### EXAMPLES 148-174

The following compounds were prepared according to the procedure outlined below from 5-chloro-1*H*-pyrrolo[2,3-c]pyridine-2-carboxylic acid (**Preparation 18**) and the appropriate amine.

To a solution of 5-chloro-1*H-*pyrrolo[2,3-c]pyridine-2-carboxylic acid (0.12 mmol, 1.5 eq) in 1:1.2 DMF / DCM (2 ml), HOBT (0.16 mmol, 2 eq) was added, followed by PS-carbodiimide (0.16 mmol, 2 eq). The reaction mixture was shaken for 15min and a solution of amine (0.08 mmol, 1 eq) in DCM (0.5 ml) was added. The reaction mixture was shaken overnight. HOBT and unreacted starting material acid were scavenged with MP-Trisamine (0.36 mmol, 4.5 eq). MP-Trisamine was added to the reaction mixture and the mixture shaken for 5h. The resin was filtered and washed with a solution of 1:1 DMF/DCM (2x4 ml). The filtrate was concentrated to give the product amide.

| **Example** | **Structure** | **RT (min)** | **m/z (ES⁺)** |
|---|---|---|---|
| **148** | 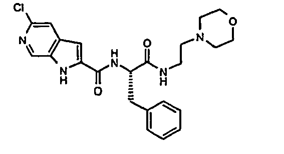 | 2.47 | 456.2 [M+H]⁺ |
| **149** | 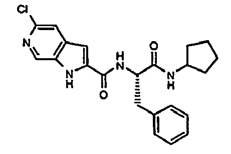 | 3.51 | 411.3 [M+H]⁺ |
| **150** | 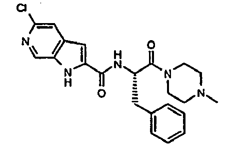 | 2.57 | 426.3 [M+H]⁺ |
| **151** | 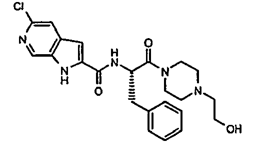 | 2.53 | 456.3 [M+H]⁺ |
| **152** | 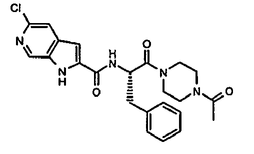 | 3.26 | 454.4 [M+H]⁺ |
| **153** | 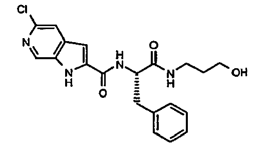 | 2.94 | 401.3 [M+H]⁺ |
| **154** | 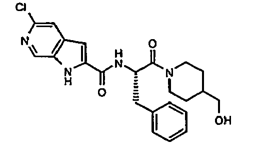 | 3.11 | 441.2 [M+H]⁺ |
| **155** | 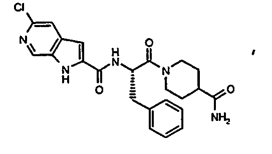 | 2.97 | 454.4 [M+H]⁺ |
| **156** | 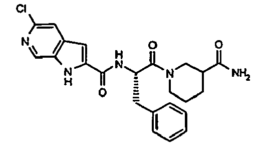 | 2.9 | 454.4 [M+H]⁺ |
| **157** | 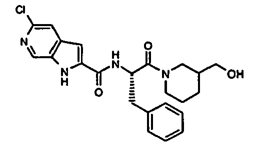 | 3.04 | 441.4 [M+H]⁺ |
| **158** | 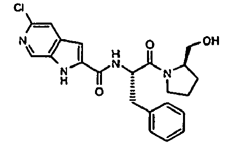 | 2.99 | 427.3 [M+H]⁺ |
| **159** | 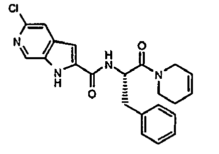 | 3.42 | 409.3 [M+H]⁺ |
| **160** | 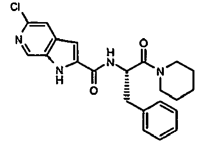 | 3.7 | 411.3 [M+H]⁺ |
| **161** | 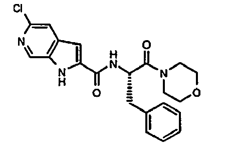 | 3.17 | 413.3 [M+H]⁺ |
| **162** | 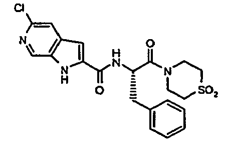 | 3.15 | 461.3 [M+H]⁺ |
| **163** | 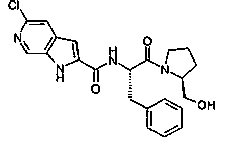 | 3.12 | 427.3 [M+H]⁺ |
| **164** | 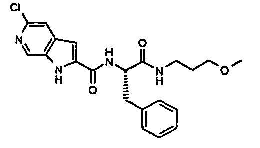 | 3.34 | 415.3 [M+H]⁺ |
| **165** | 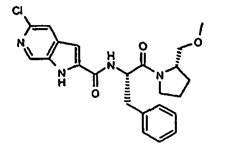 | 3.45 | 441.3 [M+H]⁺ |
| **166** | 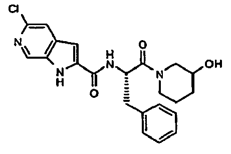 | 3.06 | 427.4 [M+H]⁺ |
| **167** | 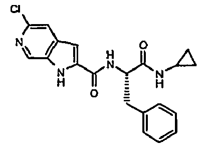 | 3.14 | 383.3 [M+H]⁺ |
| **168** | 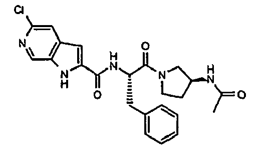 | 3.17 | 454.3 [M+H]⁺ |
| **169** | 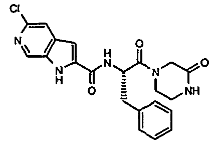 | 2.94 | 426.3 [M+H]⁺ |
| **170** | 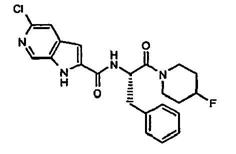 | 3.37 | 429.4 [M+H]⁺ |
| **171** | 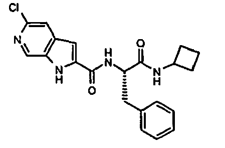 | 3.57 | 397.3 [M+H]⁺ |
| **172** | 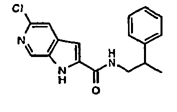 | 3.39 | 314.2 [M+H]⁺ |
| **173** | 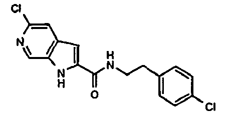 | 3.7 | 334.2 [M+H]⁺ |
| **174** | 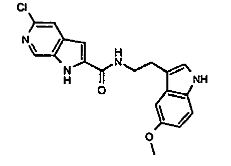 | 3.24 | 369.3 [M+H]+ |

### EXAMPLE 175

### 5-Chloro-1H-pynolo[2,3-c]pyridine-2-carboxylic acid [2-(4-benzyloxypiperidin-1-yl)-1-(S)-(4-fluorobenzyl)-2-oxoethyl] amide

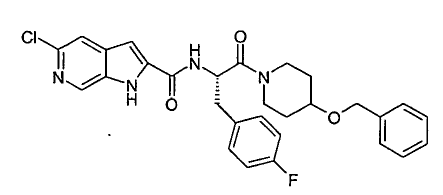

To 5-chloro-1*H-*pyrrolo[2,3-c]pyridine-2-carboxylic acid [1-(*S*)-(4-fluorobenzyl)-2-(4-hydroxypiperidin-1-yl)-2-oxoethyl]amide (**EXAMPLE 5**, 50mg, 0.11mmol) in anhydrous DMF (3mL) under argon was added benzylbromide (16µl, 0.13mmol) followed by sodium hydride (6.3mg, 0.16mmol) and the reaction stirred for 16h. Solvent was removed *in vacuo* and the residue partitioned between ethyl acetate (2 x 20mL) and water (20mL). The organic fractions were washed with 1M HCl (20mL), NaHCO₃ (2 x 20mL) then brine (2 x 20mL), dried (MgSO₄) and the solvent removed *in vacuo.* Crude material was purified by chromatography on silica gel with dichloromethane/methanol (9:1) as the eluent to give the title compound as an off-white powder. m/z (ES⁺) = 535.33 [*M*+H]⁺; RT = 3.44min.

### EXAMPLE 176

### 1-[2-(S)-[5-Chloro-1H-pyrrolo[2,3-c]pyridine-2-carbonyl)amino]-3-(4-fluorophenyl)propionyl]piperidine-4-carboxylic acid

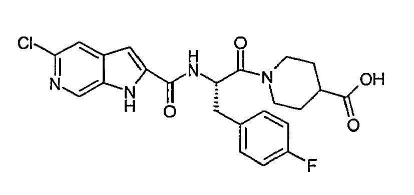

To a solution of 1-[2-(*S*)-[5-chloro-1H-pyrrolo[2,3-c]pyridine-2-carbonyl)amino]-3-(4-fluorophenyl)propionyl]piperidine-4-carboxylic acid methyl ester (**EXAMPLE 239**, 48mg, 0.1mmol) in THF (2.0mL) was added 1M sodium hydroxide solution (0.11mL, 0.11mmol) and the reaction stirred at rt for 3h. Solvent was removed *in vacuo* and the crude material partitioned between diethyl ether (20mL) and water (2 x 20mL). The aqueous layers were combined and acidified to pH2 with 2M HCl, and organics were extracted into ethyl acetate (2 x 20mL). Organic layers were combined and washed with brine (2x15mL), dried (MgSO₄) and solvent removed *in vacuo.* The crude material was crystallised from ethyl acetate/petroleum ether to give the title compound as a white powder. m/z (ES⁺) = 473.30 [*M* + H]⁺; RT = 3.20min.

### EXAMPLES 177 and 178 were prepared in a similar way to EXAMPLE 176:

| **EXAMPLE** | **Amine** | **m/z** | **RT (min)** |
|---|---|---|---|
| **177** | 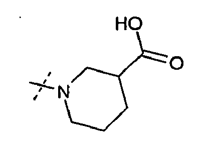 | 473.29 | 3.26 |
| **178** | 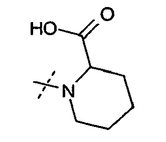 | 473.3 | 3.33 |

### EXAMPLE 179

### Acetic acid 1-[2-(S)-[5-chloro-1H-pyrrolo[23-c]pyridine-2-carbonyl)amino]-3-(4-fluorophenyl)propionyl]piperidin-4-yl ester

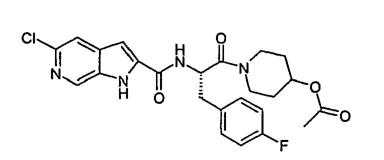

The title compound was prepared from 5-chloro-1*H-*pyrrolo[2,3-c]pyridine-2-carboxylic acid [1-(*S*)-(4-fluorobenzyl)-2-(4-hydroxypiperidin-1-yl)-2-oxoethyl] amide (**EXAMPLE 5**). To a solution of the starting amide (50mg, 0.14mmol) in anhydrous pyridine (2mL) under argon was added acetic anhydride (1mL) and the reaction stirred at rt over 4h. Solvent was removed *in vacuo* and the crude material dissolved in ethyl acetate (30mL). Organics were washed with 1M HCl (2x15mL), water (15mL) then brine (2x15mL), dried (MgSO₄) and solvent removed *in vacuo.* The residue was purified by chromatography with dichloromethane/methanol (99:1) as the eluent to give the title compound as a white powder. m/z (ES⁺) = 487.26 [*M*+ H]⁺; RT = 3.38min.

### EXAMPLE 180

### 5-Chloro-1H-pyrrolo[2,3-c]pyridine-2-carboxylic acid [2-(4-aminopiperidin-1-yl)-1-(S)-(4-fluorobenzyl)-2-oxoethyl]amide

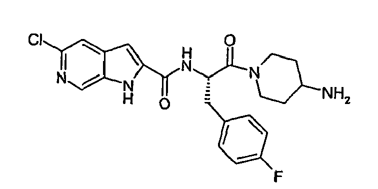

To a suspension of {1-[2-(*S*)-[(5-chloro-1H-pyrrolo[2,3-c]pyridine-2-carbonyl)amino]-3-(4-fluorophenyl)propionyl]piperidin-4-yl}carbamic acid *tert*-butyl ester (**EXAMPLE 230**, 500mg, 0.92mmol) in methanol (12mL) was added a solution of 4M HCl in dioxane (0.69mL, 2.76mmol) and the reaction stirred for 48h. The resulting precipitate was filtered and washed with ethyl acetate to give the title compound as the hydrochloride salt as a pale yellow powder. m/z (ES⁺) = 444.16 [*M* + H]⁺; RT = 2.65min. The product was dissolved in saturated sodium bicarbonate solution. Solvent was removed *in vacuo* and the crude material dissolved in THF (30mL). The solution was filtered through celite and solvent removed *in vacuo* to give the title compound as a yellow powder. m/z (ES⁺) = 444.32 (*M*+H]⁺; RT = 2.59min.

### EXAMPLES 181-189 were prepared according to EXAMPLE 180 from the appropriate Boc-protected amine:

| **EXAMPLE** | **Structure** | **m/z** | **RT (min)** |
|---|---|---|---|
| **181** | 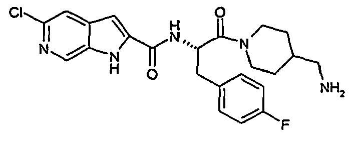 | 458.3 | 2.66 |
| **182** | 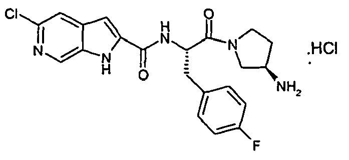 | 430.35 | 2.66 |
| **183** | 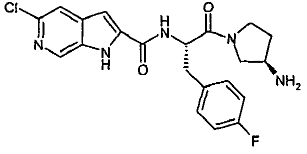 | 430.39 | 2.62 |
| **184** | 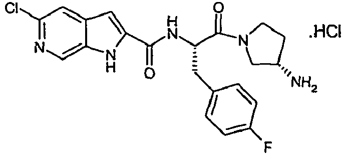 | 430.34 | 2.73 |
| **185** | 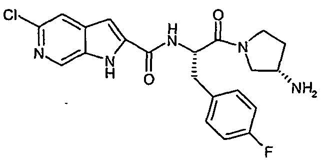 | 430.38 | 2.69 |
| **186** | 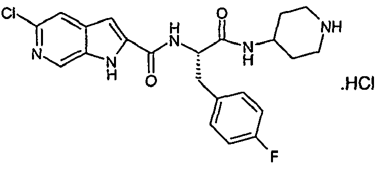 | 444.37 | 2.65 |
| **187** | 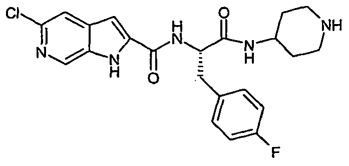 | 444.41 | 2.59 |
| **188** | 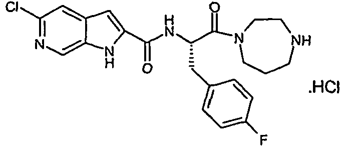 | 444.37 | 2.84 |
| **189** | 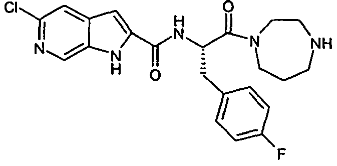 | 444.4 | 2.78 |

### EXAMPLE 190

### 5-Chloro-1H-pyrrolo[2,3-c]pyridine-2-carboxylic acid [2-(4-diacetylaminopiperidin-1-yl)-1-(S)-(4-fluorobenzyl)-2-oxoethyl]amide

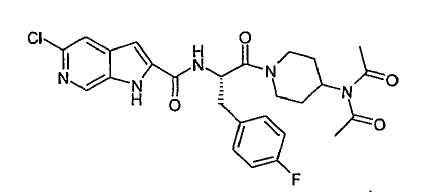

Prepared from 5-chloro-1H-pyrrolo[2,3-c]pyridine-2-carboxylic acid [2-(4-aminopiperidin-1-yl)-1-(*S*)-(4-fluorobenzyl)-2-oxoethyl]amide (**EXAMPLE 180**). The compound was synthesised according to **EXAMPLE 179** and purified by preparative HPLC to give the title compound as a white powder. m/z (ES⁺) = 486.28 [*M*- CH₃CO₂H + NH₄]⁺; RT = 3.18min.

### EXAMPLE 191

### 5-Chloro-1H-pyrrolo[2,3-c]pyridine-2-carboxylic acid [1-(S)-(4-fluorobenzyl)-2-(4-methylaminopiperidin-1-yl)-2-oxoethyl]amide

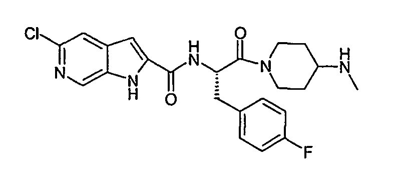

To a solution of 5-chloro-1H-pyrrolo[2,3-c]pyridine-2-carboxylic acid (1-(*S*)-(4-fluorobenzyl)-2-{4-[methyl-(2-nitrobenzenesulfonyl)amino]piperidin-1-yl}-2-oxoethyl)amide (**Preparation 76**, 82mg, 0.13mmol) in acetonitrile (6mL) was added phenylthiol (145µL, 1.4mmol) followed by potassium carbonate (230mg, 1.66mmol) and the reaction heated to 50°C for 24h. To the mixture was added diethyl ether (10mL) and 1M HCl (15mL) and stirred for 5min. The organic layer was separated and washed with 1M HCl (15mL) before combining the aqueous layers. The solution was basified to pH9 with solid potassium carbonate and extracted with ethyl acetate (2x20mL). Organic layers were combined, washed with brine (2x20mL) and dried (MgSO₄) Removal of the solvent *in vacuo* provided the desired product as an off-white powder. m/z (ES⁺) = 458.40 [*M* + H]⁺; RT = 2.67min. A small portion of the product (10mg) was dissolved in methanol (2mL) and 1M HCl added to bring the solution to pH 1-2. After stirring for 20min the solvent was removed *in vacuo* to give the title compound as the hydrochloride salt as a yellow powder. m/z (ES⁺) = 458.38 [*M*+H]⁺; RT = 2.64min.

### EXAMPLE 192

### 5-Chloro-1H-pyrrolo[2,3-c]pyridine-2-carboxylic acid[1-(S)-(4-fluorobenzyl)-2-(4-methylaminomethylpiperidin-1-yl)-2-oxoethyl]amide

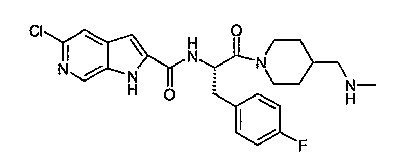

Prepared according to **EXAMPLE 191** from 5-chloro-1H-pyrrolo[2,3-c]pyridine-2-carboxylic acid [1-(*S*)-(4-fluorobenzyl)-2-(4-{[methyl-(2-nitrobenzenesulfonyl)amino]methyl}piperidin-1-yl)-2-oxoethyl]amide (synthesised according to **Preparations 73-76** from the 4-aminomethylpiperidine-1-carboxylic acid *tert*-butyl ester starting material). m/z (ES⁺) free base = 472.33 [*M+*H]⁺; RT = 2.80min. m/z (ES⁺) HCl salt = 430.43 [*M*+H]⁺; RT = 2.72min.

### EXAMPLE 193

### 5-Chloro-1H-pyrrolo[2,3-c]pyjdine-2-carboxylic acid [1-(S)-benzyl-2-(3-(R)-hydroxypyrrolidin-1-yl)-2-oxoethyl]amide

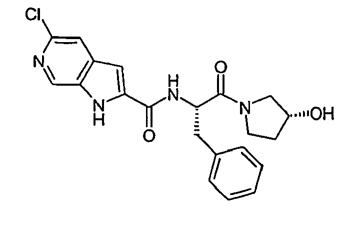

Prepared according to **EXAMPLE 35,** using 3-(*R*)-hydroxypyrrolidine in place of 3-(S)-hydroxypyrrolidine. Purification via chromatography using dichloromethane/methanol (95:5) as the eluent gave the title compound as an off-white powder. m/z (ES⁺) = 413.22 [*M*+H]⁺; RT = 3.13min.

### EXAMPLE 194

### 5-Chloro-1H-pyrrolo[2,3-c]pyridine-2-carboxylic acid [1-(S)-(4-fluorobenzyl)-2-oxo-2-(4-trifluoromethylpiperidin-1-yl)ethyl] amide

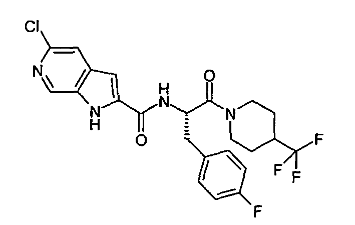

To 4-trifluoromethyl piperidine (17mg, 0.11mmol) was added a solution of 2-(*S*)-[(5-chloro-1H-pyrrolo[2,3-c]pyridine-2-carbonyl)amino]-3-(4-fluorophenyl)propionic acid (**EXAMPLE 228**, 40mg, 0.11mmol) in DMF (400µL) followed by a solution of HATU (50mg, 0.13mmol) in DMF (400µL) and finally a solution of DIPEA (23µL, 0.13mmol) in DMF (200µL). The resulting mixture was stirred at rt for 96h then solvent was removed *in vacuo.* The crude material was purified by crystallisation from THF/petroleum ether to give the title compound as a yellow powder. (ES⁺) = 497.23 [*M*+H]⁺; RT = 3.50min.

### EXAMPLES 195-225 were prepared in the same way as EXAMPLE 194:

| **EXAMPLE** | **Amine** | **Purification** | **m/z** | **RT (min)** |
|---|---|---|---|---|
| **195** | 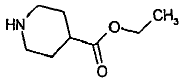 | Crystallisation (THF/PE) | 501.26 | 3.43 |
| **196** | 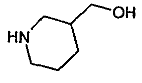 | MDP | 459.3 | 3.13 |
| **197** | 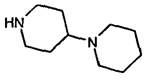 | MDP | 512.37 | 2.77 |
| **198** | 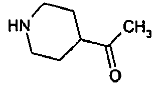 | MDP | 471.3 | 3.24 |
| **199** | 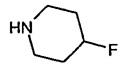 | MDP | 447.27 | 3.37 |
| **200** | 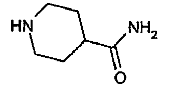 | MDP | 472.3 | 3.03 |
| **201** | 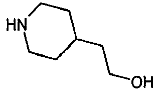 | MDP | 473.32 | 3.09 |
| **202** | 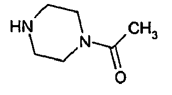 | MDP | 472.29 | 3.08 |
| **203** | 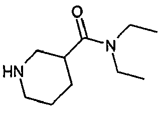 | MDP | 528.38 | 3.3 |
| **204** | 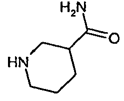 | MDP | 472.3 | 3.09 |
| **205** | 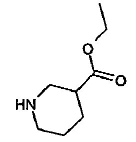 | MDP | 501.32 | 3.55 |
| **206** | 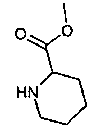 | MDP | 487.3 | 3.57 |
| **207** | 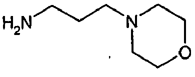 | MDP | 488.34 | 2.75 |
| **208** | 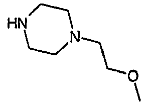 | MDP | 488.4 | 2.59 |
| **209** | 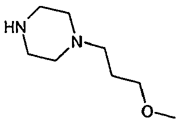 | MDP | 502.38 | 2.77 |
| **210** | 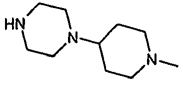 | MDP | 527.37 | 2.47 |
| **211** | 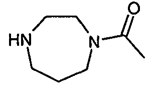 | MDP | 486.29 | 3.09 |
| **212** | 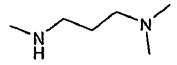 | MDP | 460.4 | 2.65 |
| **213** | 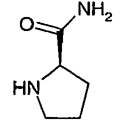 | MDP | 458.36 | 3.18 |
| **214** | 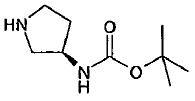 | MDP | 530.45 | 3.52 |
| **215** | 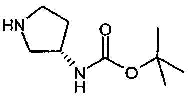 | MDP | 530.45 | 3.46 |
| **216** | 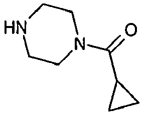 | MDP | 498.4 | 3.23 |
| **217** | 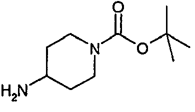 | MDP | 544.48 | 3.61 |
| **218** | 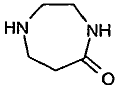 | MDP | 458.35 | 3 |
| **219** | 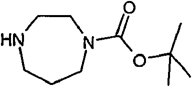 | MDP | 544.46 | 3.61 |
| **220** | 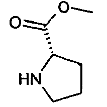 | Chromatography (DCM/MeoH 98:2) | 473.33 | 3.48 |
| **221** | 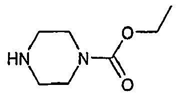 | Chromatography (DCM/MeoH 98:2) | 502.38 | 3.43 |
| **222** | 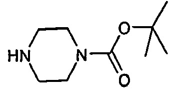 | Trituration (EtOAc) | 530.39 | 3.65 |
| **223** | 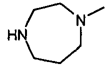 | Trituration (EtOAc) | 458.32 | 2.8 |
| **224** | 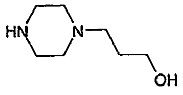 | Prep HPLC | 488.35 | 2.63 |
| **225** | 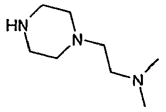 | Crystallisation (MeOH) | 501.4 | 2.62 |

### EXAMPLE 226

### 5-Chloro-1H-pyrrolo[2,3-c]pyridine-2-carboxylic acid [1-(S)-(4-fluorobenzyl)-2-oxo-2-piperazin-1-ylethyl]amide

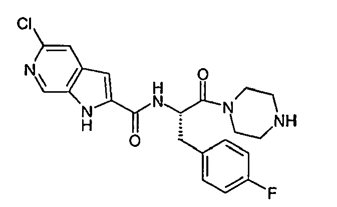

Prepared according to **EXAMPLE 180** from 4-[2-(*S*)-[5-chloro-1H-pyrrolo[2,3-c]pyridine-2-carbonyl)amino]-3-(4-fluorophenyl)propionyl]piperazine-1-carboxylic acid *tert*-butyl ester (prepared according to **EXAMPLE 222**). Purification gave the title compound as a yellow powder. m/z (ES⁺) = 430.34 [*M* + H]⁺; RT = 2.56min.

### EXAMPLE 227

### 2-(S)-[(5-Chloro-1H-pvrrolo[2,3-c]pyridine-2-carbonyl)amino]-3-(4-fluorophenyl)propionic acid ethyl ester

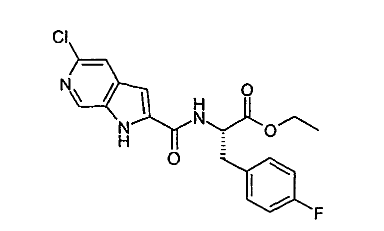

Prepared according to **EXAMPLE 41** using p-fluoro-L-phenylalanine ethyl ester hydrochloride instead of L-phenylalanine ethyl ester hydrochloride. Chromatography gave the title compound as a yellow powder. m/z (ES⁺) = 390.27 [*M* + H]⁺; RT = 3.71min.

### EXAMPLE 228

### 2-(S)-[(5-Chloro-1H-pyrrolo[2,3-c]pyridine-2-carbonyl)amino]-3-(4-fluorophenyl)propionic acid

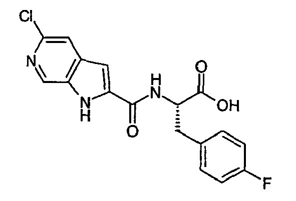

The title compound was prepared according to **EXAMPLE 42** using 2-(*S*)-[(5-chloro-1H-pyrrolo[2,3-c]pyridine-2-carbonyl)amino]-3-(4-fluorophenyl)propionic acid ethyl ester (**EXAMPLE 227**). Solvent was removed *in vacuo* and the residue taken into water. The aqueous layer was extracted with ethyl acetate (3x) then acidified with 2M HCl solution to pH 2. The precipitate was filtered and washed thoroughly with water to give the title compound as a cream-coloured powder. (ES⁺) = 3 62.24 [*M* + H]⁺; RT = 3.21min.

### EXAMPLE 229

### 5-Chloro-1H-pyrrolo[2,3-c]pyridine-2-carboxylic acid [2-(1,4-dioxa-8-azaspiro[4.5]dec-8-yl)-1-(S)-(4-fluorobenzyl)-2-oxoethyl]amide

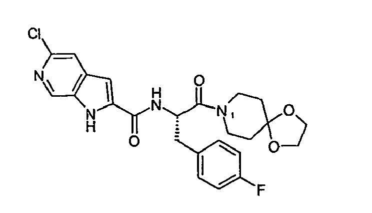

To a solution of 2-(*S*)-[(5-chloro-1H-pyrrolo[2,3-c]pyridine-2-carbonyl)amino]-3-(4-fluorophenyl)propionic acid (**EXAMPLE 228**, 40mg, 0.11mmol) in DMF (4mL) was added HATU (50mg, 0.13mmol) and the reaction stirred for 10 min. 4-Piperidone ethylene ketal (19mg, 0.13mmol) was added, followed by DIPEA (23µL, 0.13mmol) and the reaction stirred at rt for 16h. Solvent was removed *in vacuo* and the crude material partitioned between ethyl acetate (15mL) and water (15mL). The organic layer was washed with 1M HCl solution (15mL), sodium bicarbonate solution (2x20mL) then brine (2x20mL), dried (MgSO₄) and the solvent removed *in vacuo.* Purification by chromatography using dichloromethane/methanol (9:1) as the eluent gave the title compound as a pale yellow powder. (ES⁺) = 487.30 [*M* + H]⁺; RT = 3.28min.

**EXAMPLES 230-237** were prepared in a similar way to **EXAMPLE 229:**

| **EXAMPLE** | **Amine** | **Purification** | **m/z** | **RT (min)** |
|---|---|---|---|---|
| **230** | 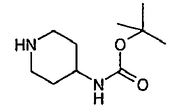 | Chromatography (DCM/MeOH 9:1) | 544.45 | 5.39 |
| **231** | 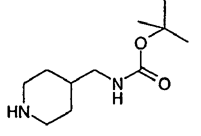 | Chromatography (DCM/MeOH 9:1) | 558.22 | 3.67 |
| **232** | 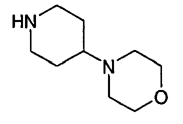 | Chromatography (DCM/MeOH 9:1) | 514.28 | 2.67 |
| **233** | 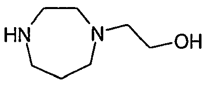 | Chromatography (DCM/MeOH 9:1) | 488.38 | 2.69 |
| **234** | 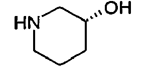 | Chromatography (DCM/MeOH 9:1) | 445.28 | 3.17 |
| **235** | 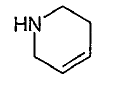 | Chromatography (EtOAc/PE 3:1) | 427.35 | 3.5 |
| 236 | 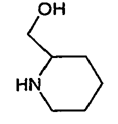 | prep. HPLC | 459.4 | 3.2 |
| 237 | 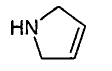 | Chromatography (EtOAc) | 413.2 | 3.31 |

### EXAMPLE 238

### 5-Chloro-1H-pyrrolo[2,3-c]pyridine-2-carboxylic acid [2-(1,1-dioxo-1,6-thiomorpholin-4-yl)-1-(S)-(4-fluorobenzyl)-2-oxoethyl]amide

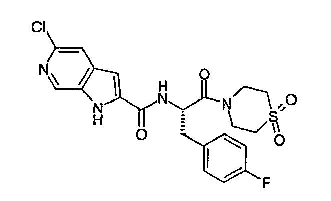

Prepared according to **EXAMPLE 229** from 2-(*S*)-[(5-chloro-1H-pyrrolo[2,3-c]pyridine-2-carbonyl)amino]-3-(4-fluorophenyl)propionic acid (**EXAMPLE 228**) and thiomorpholine-1,1-dioxide (**Preparation 77**). Purification by trituration with methanol gave the title compound as a pale yellow powder. (ES⁺) = 479.24 [*M*+H]⁺; RT = 3.17min.

### EXAMPLE 239

### 1-[2-(S)-[(5-Chloro-1H-pyrrolo[2,3-c]pyridine-2-carbonyl)amino]-3-(4-fluorophenyl)propionly]piperidine-4-carboxylic acid methyl ester

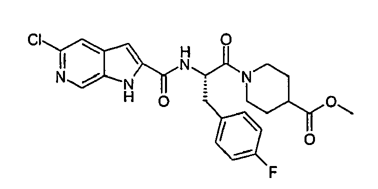

Prepared according to **EXAMPLE 229** from 2-(*S*)-[(5-chloro-1H-pyrrolo[2,3-c]pyridine-2-carbonyl)amino]-3-(4-fluorophenyl)propionic acid (**EXAMPLE 228**) and piperidine-4-carboxylic acid methyl ester hydrochloride (**Preparation 78**). Purification by chromatography using ethyl acetate/petroleum ether (70:30) as the eluent gave the title compound as a pale yellow powder. m/z (ES⁺) = 487.32 [*M*+ H]⁺; RT = 3.44min.

### EXAMPLE 240 was prepared in a similar way to EXAMPLE 239:

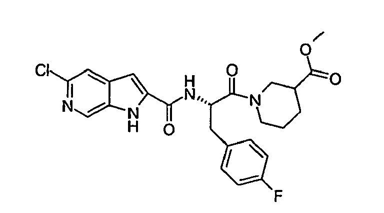

m/z (ES⁺) = 487.35 [*M*+H]⁺; RT = 3.88min.

### EXAMPLE 241

### 5-Chloro-1H-pyrrolo[2,3-c]pyridine-2-carboxylic acid [1-(S)-benzyl-2-(4-hydroxypiperidin-1-yl)-2-oxoethyl]amide

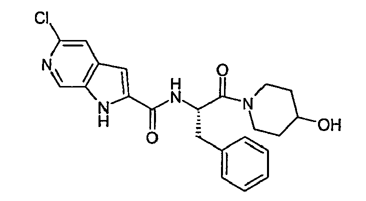

The title compound was prepared according to **EXAMPLE 229** using 2-(*S*)-[(5-chloro-1*H-*pyrrolo[2,3-c]pyridine-2-carbonyl)amino]-3-phenylpropionic acid (**EXAMPLE 42**) and 4-hydroxypiperidine. Purification by chromatography using dichloromethane/methanol (9:1) as the eluent gave the title compound as an orange powder. m/z (ES⁺) = 427.35 [*M* + H]⁺; RT = 2.99min.

### EXAMPLE 242

### 5-Chloro-1H-pyrrolo[2,3-c]pyridine-2-carboxylic acid [2-(2-carbamoylpiperidin-1-yl)-1-(S)-(4-fluorobenzyl)-2-oxoethyl] amide

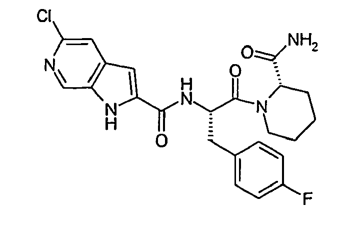

To a solution of piperidine-2-carboxylic acid amide hydrochloride (**Preparation 80**, 24mg, 0.17mmol) in DMF (3mL) was added 2-(*S*)-[(5-chloro-1H-pyrrolo[2,3-c]pyridine-2-carbonyl)amino]-3-(4-fluorophenyl)propionic acid (**EXAMPLE 230**, 52mg, 0.14mmol), followed by HATU (65.6mg, 0.17mmol) and DIPEA (63µL, 0.36mmol), and the reaction stirred at rt for 16h. Solvent was removed *in vacuo,* purification via preparative HPLC gave the title compound as an off-white powder. m/z (ES⁺) = 472.31 [*M*+H]⁺; RT = 3.19min.

### EXAMPLE 243

### 5-Chloro-1H-pyrrolo[2,3-c]pyridine-2-carboxylic acid {1-(S)-(4-fluorobenzyl)-2-[4-(2-methoxyethoxy)piperidin-1-yl]-2-oxoethyl}amide

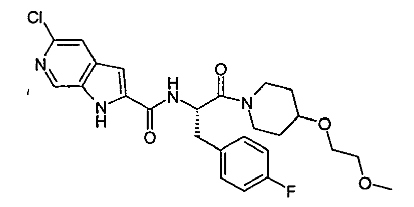

The title compound was prepared according to **EXAMPLE 229** from 2-(*S*)-[(5-chloro-1H-pyrrolo[2,3-c]pyridine-2-carbonyl)amino]-3-(4-fluorophenyl)propionic acid (**EXAMPLE 228**) and 4-(2-methoxyethoxy)piperidine hydrochloride (**Preparation 82**). Purification by chromatography using dichloromethane/methanol (95:5) as the eluent gave the title compound as an off-white powder. m/z (ES⁺) 503.26 [*M*+H]⁺; RT = 3.31min.

### EXAMPLE 244

### 5-Chloro-1H-pyrrolo[2,3-c]pyridine-2-carboxylic acid {1-(S)-(4-fluorobenzyl)-2-[4-(3-methoxypropoxy)piperidin-1-yl]-2-oxoethyl} amide

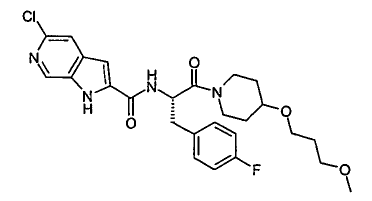

The title compound was prepared according to **EXAMPLE 229** using 4-(3-methoxypropoxy)piperidine hydrochloride, synthesised from the appropriate starting materials (**Preparations 81 and 82**). Purification by chromatography using dichloromethane/methanol (9:1) as the eluent gave the title compound as an off-white powder. m/z (ES⁺) = 517.36 [*M*+H]⁺; RT = 3.41min.

### EXAMPLE 245

### 5-Chloro-1H-pyrrolo[2,3-c]pyridine-2-carboxylic acid [2-(4-acetylaminopiperidin-1-yl)-1-(S)-4-fluorobenzyl)-2-oxoethyl]amide

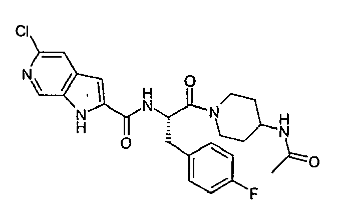

To a solution of acetic acid (7.5µL, 0.13mmol) in DMF (5mL) was added EDCI (33mg, 0.17mmol), HOBt (19.5mg, 0.14mmol), 5-chloro-1H-pyrrolo[2,3-c]pyridine-2-carboxylic acid [2-(4-aminopiperidin-1-yl)-1-(*S*)-(4-fluorobenzyl)-2-oxoethyl]amide (**EXAMPLE 180**, 70mg, 0.16mmol) and DIPEA (57µL, 0.33mmol), and the reaction stirred at rt for 16h. Solvent was removed *in vacuo* then crude material partitioned between ethyl acetate (15mL) and water (15mL). The organic layer was washed with NaHCO₃ (2x20mL) and brine (2x30mL), dried (MgSO₄) and the solvent removed *in vacuo.* Purification by chromatography using dichloromethane/methanol (95:5) as the eluent gave the title compound as an off-white powder. m/z (ES⁺) = 486.27 [*M*+H]⁺; RT = 3.16min.

### EXAMPLE 246

### 5-Chloro-1H-pyrrolo[2,3-c]pyridine-2-carboxylic acid [2-[4-(acetylaminomethyl)piperidin-1-yl]-1-(S)-(4- fluorobenzyl)-2-oxoethyl]amide

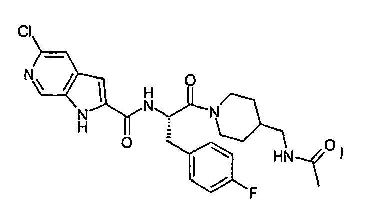

The title compound was prepared from 5-chloro-1*H-*pyrrolo[2,3-c]pyridine-2-carboxylic acid [2-(4-aminomethylpiperidin-1-yl)-1-(*S*)-(4-fluorobenzyl)-2-oxyethyl]amide (**EXAMPLE 229** then **180** from the appropriate piperidin-4-ylmethylcarbamic acid *tert*-butyl ester). Purification by preparative HPLC gave the title compound as a white powder. m/z (ES⁺) = 500.38 [*M*+H]⁺; RT = 3.14min.

### EXAMPLE 247

### 5-Chloro-1H-pyrrolo[2,3-c]pyridine-2-carboxylic acid [1-(R)-(4-fluorobenzyl)-2-(4-hydroxypiperidin-1-yl)-2-oxoethyl]amide

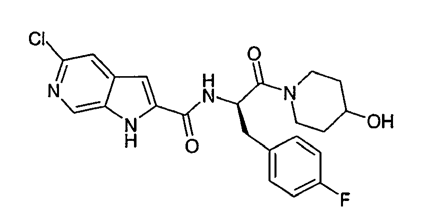

The title compound was prepared according to **EXAMPLE 1** from 5-chloro-1*H-*pyrrolo[2,3-c]pyridine-2-carboxylic acid (**Preparation 18**) and 2-(*R*)-amino-3-(4-fluorophenyl)-1-(4-hydroxypiperidin-1-yl) propan-1-one hydrochloride (**Preparation 84**). Purification by chromatography using dichloromethane/methanol (92:8) as the eluent gave the title compound as a pale yellow powder. m/z (ES⁺) = 445.34 [*M* + H]⁺; RT = 3.10min.

### EXAMPLE 248

### 4-{[2-(S)-[(5-Chloro-1H-pyrrolo[2,3-c]pyridine-2-carbonyl)amino]-3-(4-fluorophenyl)propionyl]methylamino}piperidine-1-carboxylic acid tert-butyl ester

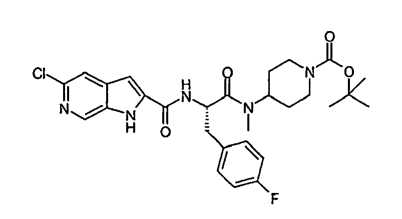

The title compound was prepared according to **EXAMPLE 229** from 2-(*S*)-[(5-chloro-1H-pyrrolo[2,3-c]pyridine-2-carbonyl)amino]-3-(4-fluorophenyl)-propionic acid (**EXAMPLE 228**) and 4-methylaminopiperidine-1-carboxylic acid *tert*-butyl ester (**Preparation 86**). Purification by chromatography using dichloromethane/methanol (95:5) as the eluent gave the title compound as a pale yellow powder. m/z (ES⁺) = 558.48 [*M*+H]⁺; RT = 3.82min.

### EXAMPLE 249

### 5-Chloro-1H-pyrrolo[2,3-c]pyridine-2-carboxylic acid [2-(S)-(4-fluorophenyl)-1-(methylpiperidin-4-yl carbamoyl)ethyl]amide

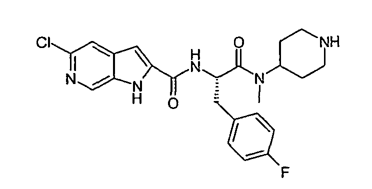

The title compound was prepared according to **Preparation 82**, from 4-{[2-(*S*)-[(5-chloro-1H-pyrrolo[2,3-c]pyridine-2-carbonyl)amino]-3-(4-fluorophenyl)propionyl]methylamino}piperidine-1-carboxylic acid *tert*-butyl ester (**EXAMPLE 248**), to give the title compound as the hydrochloride salt as a yellow crystalline solid, m/z (ES⁺) = 458.30 [*M*+H]⁺; RT = 2.86min. The product was dissolved in saturated sodium bicarbonate solution and extracted into ethyl acetate. Organic solvent was removed *in vacuo* to give the title compound as the free base as a yellow powder. m/z (ES⁺) = 458.33 [*M*+H]⁺; RT = 2.82min.

### EXAMPLE 250

### 5-Chloro-1H-pyrrolo[2,3-c] pyridine-2-carboxylic acid {2-(S)-(4-fluorophenyl)-1-[methyl(tetrahydropyran-4-yl)carbamoyl]ethyl}amide

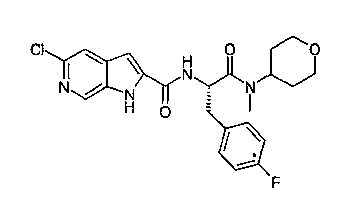

The title compound was prepared according to **EXAMPLE 229** from 2-(*S*)-[(5-chloro-1H-pyrrolo[2,3-c]pyridine-2-carbonyl)amino]-3-(4-fluorophenyl)propionic acid (**EXAMPLE 228**) and methyl(tetrahydropyran-4-yl) amine hydrochloride (**Preparation 88**). m/z (ES⁺) = 459.27 [*M*+H]⁺; RT = 3.41min.

### EXAMPLE 251

### 5-Chloro-1H-pyrrolo[2,3-c]pyridine-2-carboxylic acid [2-(4-dimethylaminopiperidin-1-yl)-1-(S)-(4-fluorobenzyl)-2-oxoethyl] amide

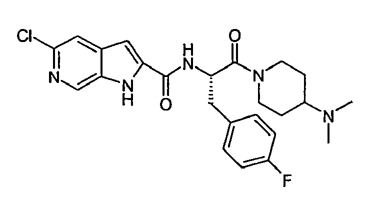

The title compound was prepared according to **EXAMPLE 35** from 2-(*S*)-[(5-chloro-1H-pyrrolo[2,3-c]pyridine-2-carbonyl)amino]-3-(4-fluorophenyl)propionic acid (**EXAMPLE 228**) and dimethylpiperidin-4-yl amine (**Preparation 90**). Purification by preparative HPLC gave the title compound as a yellow crystalline solid. m/z (ES⁺) = 472.34 [*M* + H]⁺; RT = 2.64min.

### EXAMPLE 252

### 5-Chloro-1H-pyrrolo[2,3-c]pyridine-2-carboxylic acid [1-(S)-(4-fluorobenzyl)-2-(4-methanesulfonylaminopiperidin-1-yl)-2-oxoethyl] amide

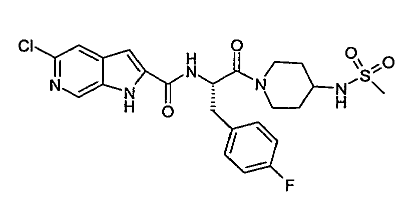

The title compound was prepared according to **EXAMPLE 229** from 2-(*S*)-[(5-chloro-1H-pyrrolo[2,3-c]pyridine-2-carbonyl)amino]-3-(4-fluorophenyl)propionic acid (**EXAMPLE 228**) and N-piperidin-4-yl methanesulfonamide hydrochloride (Preparation 92). Purification gave the title compound as an off-white powder. m/z (ES⁺) = 522.30 [*M*+H]⁺; RT = 3.29min.

### EXAMPLE 253

### 2-(S)-[(5-Chloro-1H-pyrrolo[2,3-c]pyridine-2-carbonyl)-amino]-3-pyridin-4-yl-propionic acid methyl ester

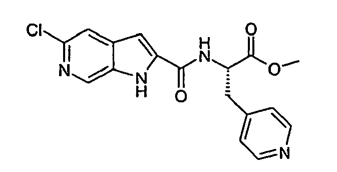

To a solution of 5-chloro-1*H-*pyrrolo[2,3-c]pyridine-2-carboxylic acid (**Preparation 18**, 185mg, 0.94mmol) in DMF (7mL) was added 2-(*S*)-amino-3-pyridin-4-yl propionic acid methyl ester hydrochloride (**Preparation 93**, 204mg, 0.94mmol) followed by TBTU (333mg, 1.04mmol) and DIPEA (740µL, 4.24mmol), and the reaction stirred at rt for 16h. Solvent was removed *in vacuo* and the residue partitioned between ethyl acetate (30mL) and water (30mL). The organic layer was washed with water (2x30mL), NaHCO₃ solution (3x40mL) then brine (3x50mL), dried (MgSO₄) and the solvent removed *in vacuo.* Purification by chromatography (SiO₂, EtOAc) gave the title compound as a pink powder. m/z (ES⁺) = 359.11 [*M*+ H]⁺; RT = 2.42min.

### EXAMPLE 254

### 5-Chloro-1H-pyrrolo[2,3-c]pyridine-2-carboxylic acid (1-dimethylcarbamoyl-2-(S)-pyridin-4-yl-ethyl)amide

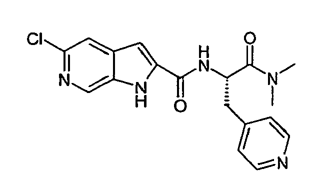

The title compound was prepared according to **EXAMPLE 253** from 2-(*S*)-[(5-chloro-1H-pyrrolo[2,3-c]pyridine-2-carbonyl)amino]-3-pyridin-4-yl-propionic acid (**EXAMPLE 259**) and dimethylamine hydrochloride. Purification by preparative HPLC gave the title compound as an off-white powder. m/z (ES⁺) = 372.13 [*M* + H]⁺; RT = 2.31 min.

**EXAMPLE 255** was prepared in a similar way to **EXAMPLE 254**: 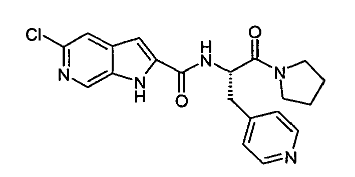 m/z (ES⁺) = 398.15 [*M*+H]⁺; RT = 2.53min.

### EXAMPLE 256

### 5-Chloro-1H-pyrrolo[2,3-c]pyridine-2-carboxylic acid [2-(1,4-dioxa-7-aza-spiro[4.5]dec-7-yl)-1-(S)-(4-fluorobenzyl)-2-oxoethyl]amide

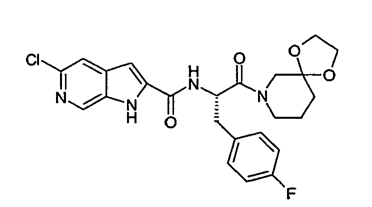

To a suspension of 2-(*S*)-[(5-chloro-1H-pyrrolo[2,3-c]pyridine-2-carbonyl)amino]-3-(4-fluorophenyl)propionic acid (**EXAMPLE 228**, 80mg, 0.22mmol) in ethanol (6mL) was added DMTMM (78mg, 0.27mmol) and the reaction stirred for 5min. To the mixture was added 1,4-dioxa-7-aza-spiro[4.5]decane (**Preparation 94**, 35mg, 0.24mmol) and stirring continued for 16h. Solvent was removed *in vacuo* and the crude material partitioned between ethyl acetate and water. The organic layer was washed with NaHCO₃ solution (2x20mL) then brine (2x20mL), dried (MgSO₄) and the solvent removed *in vacuo.* Purification by chromatography using dichloromethane/methanol (95:5) as the eluent gave the title compound as a pale yellow powder. m/z (ES⁺) = 487.21 [*M*+H]⁺; RT = 3.50min.

### EXAMPLE 257

### 5-Chloro-1H-pyrrolo[2,3-c]pyridine-2-carboxylic acid [2-(3R,4R) dihydroxypyrrolidin-1-yl)-1-(S)-(4-fluorobenzyl)-2-oxoethyl]amide

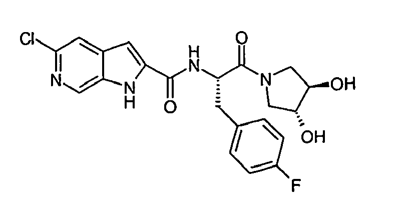

The title compound was prepared according to **EXAMPLE 229** using (3*R*,4*R*)-dihydroxypyrrolidine (prepared according to **Preparation 23** from the commercially available benzyl derivative). Additional crystallisation from methanol gave the title compound as colourless crystals. m/z (ES⁺) = 447.33 [*M* + H]⁺; RT = 2.99min.

### EXAMPLE 258

### 5-Chloro-1H-pyrrolo[2,3-c]pyridine-2-carboxylic acid [2-(3-(S)-4-(S) dihydroxypyrrolidin-1-yl)-1-(S)-(4-fluorobenzyl)-2-oxoethyl] amide

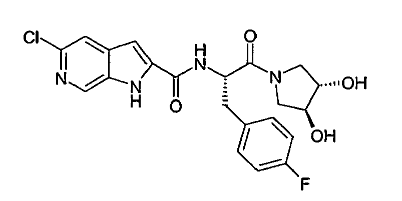

The title compound was prepared according to **EXAMPLE 229** using 3-(*S*)-4-(*S*)-dihydroxypyrrolidine (prepared according to **Preparation 23**). Additional crystallisation from methanol gave the title compound as colourless crystals. m/z (ES⁺) = 447.33 [*M* + H]⁺; RT = 3.07min.

### EXAMPLE 259

### 2-(S)-[(5-Chloro-1H-pyrrolo[2,3-c]pyridine-2-carbonyl)amino]-3-pyridin-4-yl-propionic acid

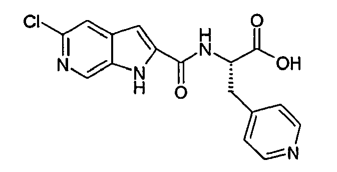

The title compound was prepared according to **EXAMPLE 42** from 2-(*S*)-[(5-chloro-1H-pyrrolo[2,3-c]pyridine-2-carbonyl)amino]-3-pyridin-4-yl-propionic acid methyl ester (**EXAMPLE 253**). m/z (ES⁺) = 345.09 [*M* + H]⁺.

### EXAMPLE 260

### 5-Chloro-1H-pyrrolo[2,3-c]pyridine-2-carboxylic acid (1-(S)-benzyl-2-oxo-2-phenylethyl)amide

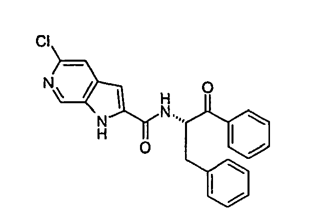

To a solution of 5-chloro-1H-pyrrolo[2,3-c]pyridine-2-carboxylic acid [2-phenyl-1-(*S*)-(2-phenyl-[1,3]dioxolan-2-yl)ethyl]amide (**Preparation 96**, 47mg, 0.105mmol) in acetone (20mL) was added aqueous hydrochloric acid (1mL, 1M). After stirring under reflux for 3 days the solvent was removed *in vacuo.* The residue was distributed between ethyl acetate (100mL) and saturated sodium carbonate solution (50mL). After separation the organic layer was washed with brine (50ml), dried (MgSO₄) and concentrated to a residue which was purified by flash chromatography on silica gel (eluent: hexane / ethyl acetate : 50 / 50) to give the title compound as colourless solid. m/z (ES⁺) = 404.21 [*M*+H]⁺; RT = 3.58min.

### EXAMPLE 261

### 5-Chloro-1H-pyrrolo[2,3-c]pyridine-2-carboxylic acid (2-hydroxy-2-pyridin-3-yl-ethyl) amide

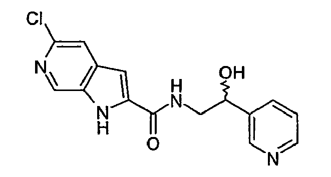

To a solution of 5-chloro-1*H-*pyrrolo[2,3-c]pyridine-2-carboxylic acid (**Preparation 18**, 296mg, 1.51mmol) and 2-amino-1-pyridin-3-ylethanol (**Preparation 98**, 214mg, 1.55mmol) in DMF (10mL) was added HOBt (225mg, 1.47mmol), DIPEA (0.55mL, 3.16mmol) and EDCI (340mg, 1.77mmol). After stirring at rt for 12h the solvent was removed *in vacuo* and the residue then taken up in THF (150mL) and washed with diluted sodium hydroxide solution (1M, 50mL) and brine (2 x 50mL). The solution was dried (MgSO₄) and concentrated to an oil that was further purified by flash chromatography on silica gel (eluent: DCM / methanol : 90 / 10 + 0.5% triethylamine) to give the title compound as off-white solid. δ_{H} (d₆ DMSO): 3.53 (2H, m), 4.85 (1H, m), 5.75 (1H, d), 7.15 (1H, s), 7.37 (1H, dd), 7.75 (2H, m), 8.47 (1H, m), 8.57 (1H, s), 8.89 (1H, appt), 12.24 (1H, s); m/z (ES⁺) 317.17 [*M*+H]⁺; RT = 2.71min.

### EXAMPLE 262

### 5-Chloro-1H-pyrrolo[2,3-c]pyridine-2-carboxylic acid (2-(S)-hydroxy-1-(S)-methoxymethyl-2-phenylethyl)amide

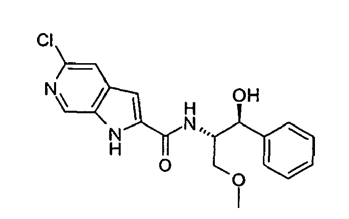

To a solution of 5-chloro-1*H-*pyrrolo[2,3-c]pyridine-2-carboxylic acid (**Preparation 18**, 223mg, 1.13mmol) and commercially available (1*S*,2S)-(+)-2-amino-3-methoxy-1-phenyl-1-propanol (200mg, 1.10mmol) in DMF (5mL) was added HOBt (173mg, 1.13mmol), DIPEA (0.42mL, 2.41mmol) and EDCI (260mg, 1.36mmol). After stirring at rt for 12h the mixture was added to diluted brine (100mL, water/brine : 1/1). Extraction with ethyl acetate (4 x 25mL), washing of the combined extracts with diluted hydrochloric acid (1M, 30ml), diluted aqueous sodium hydroxide solution (1M, 30ml) and brine (30mL) followed by drying over magnesium sulphate gave after concentration a residue which was purified by flash chromatography on silica gel (eluent: ethyl acetate). The title compound was obtained as colourless solid. δ_{H} (CD₃OD): 3.37 (3H, s), 3.34 (1H, dd), 3.66 (1H, dd), 4.53 (1H, ddd), 5.03 (1H, d), 7.15 (1H, s), 7.25-7.45 (5H, 3m), 7.68 (1H, s), 8.58 (1H, s); m/z (ES⁺) = 360.22 [*M*+ H]⁺; RT = 3.12min.

### EXAMPLE 263

### 5-Chloro-1H-pyrrolo[2,3-c]pyridine-2-carboxylic acid (1-(S)-hydroxymethyl-2-oxo-2-phenylethyl)amide

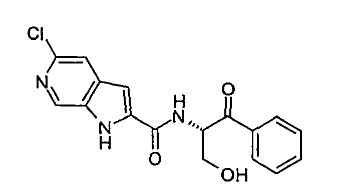

To a solution of 5-chloro-1H-pyrrolo[2,3-c]pyridine-2-carboxylic acid [1-(*S*)-(*tert*-butyldimethylsilanyloxymethyl)-2-oxo-2-phenylethyl]amide (**Preparation 101**, 220mg, 0.48mmol) in THF (10mL) was added acetic acid (60µL) and tetrabutylammonium fluoride solution (1ml, 1M in THF) at rt. After stirring for 3h the reaction mixture was distributed between ethyl acetate (100mL) and water (30mL). The organic layer was separated, then washed with brine (50ml), dried (MgSO₄) and concentrated to a solid residue. Recrystallisation from THF gave the title compound. m/z (ES⁺) = 344.21 [*M*+H]⁺; RT = 3.02min.

### EXAMPLE 264

### 5-Chloro-1H-pyrrolo[2,3-c]pyridine-2-carboxylic acid (1-(S)-methoxymethyl-2-oxo-2-phenylethyl)amide

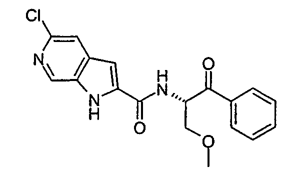

5-Chloro-1H-pyrrolo[2,3-c]pyridine-2-carboxylic acid (2-(*S*)-hydroxy-1-(*S*)-methoxymethyl-2-phenylethyl)amide (**EXAMPLE 262**, 101mg, 0.281mmol) was oxidised and isolated in a similar way to **Preparation 101** using Dess-Martin periodinane (240mg, 0.566mmol) in DCM (10mL). The title compound was obtained by recrystallisation of the crude product from methanol. m/z (ES⁺) = 358.24 [*M* + H]⁺; RT=3.18min.

### EXAMPLE 265

### 5-Chloro-1H-pryrrolo[2,3-c]pyridine-2-carboxylic acid (2-oxo-2-pyridin-3-ylethyl)amide

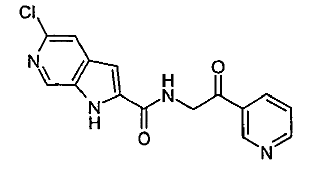

To a solution of racemic 5-chloro-1H-pyrrolo[2,3-c]pyridine-2-carboxylic acid (2-hydroxy-2-pyridin-3-ylethyl)amide (**EXAMPLE 261**, 80mg, 0.253mmol) in dry THF (20mL) was added Dess-Martin periodinane (307mg, 0.724mmol). After stirring for 4h at rt alkaline sodium thiosulfate solution was added (5.4g Na₂SO₃ dissolved in 20mL saturated NaHCO₃ solution) and the emulsion was vigorously stirred for additional 30min before further diluted with water (~150mL). Extraction with THF (4 x 50mL), washing of the combined extracts with saturated sodium hydrogen carbonate (50mL) and brine (50mL) gave a solution which was concentrated after drying (MgSO₄). Purification of the residue by flash chromatography on silica gel (eluent: DCM / methanol : 90 /10) gave the title compound as off-white solid. m/z (ES⁺) = 315.19 [*M*+H]⁺; RT = 2.65min.

### EXAMPLE 266

### 6-Chloro-1H-pyrrolo[2,3-b]pyridine-2-carboxylic acid (1-dimethylcarbamoyl-2-(S)-phenethyl)amide

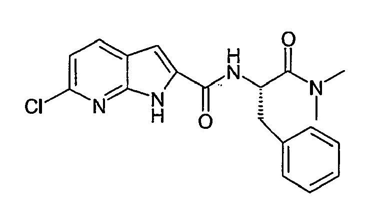

DIPEA (155µL, 0.89mmol), HOBt (43mg, 0.28mmol) and 6-chloro-1H-pyrrolo[2,3b]pyridine-2-carboxylic acid (**Preparation 110**, 50mg, 0.25mmol) was added to a stirred solution of 2-(*S*)-amino-N,N-dimethyl-3-phenyl propionamide hydrochloride (**Preparation 8**, 61mg, 0.27mmol) in DMF (4mL). After 5min EDCI (63mg, 0.33mmol) was added and the reaction stirred for 22h. Purification by column chromatography (SiO₂, 95:5 CH₂Cl₂/MeOH) afforded the title compound. m/z (ES⁺) = 370.93 [*M*+ H]⁺; RT = 3.62min.

### EXAMPLE 267

### 6-Chloro-1H-pyrrolo[2,3-b]pyridine-2-carboxylic acid [1-(S)-(4-fluorobenzyl)-2-(3-(S)-hydroxypyrrolidin-1-yl)-2-oxoethyl]amide

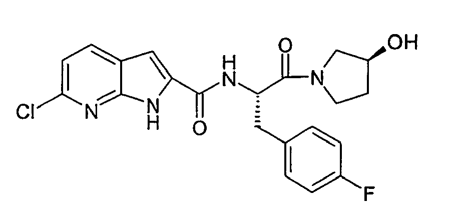

The title compound was prepared according to **EXAMPLE 266** but using 2-(*S*)-amino-3-(4-fluorophenyl)-1-(3-(S)-hydroxypyrrolidin-1-yl)-propan-1-one hydrochloride (**Preparation 103**) instead of 2-(*S*)-amino-N,N-dimethyl-3-phenyl propionamide hydrochloride. m/z (ES⁺) = 430.94 [*M*+H]⁺; RT = 4.31min.

### EXAMPLE 268

### 6-Chloro-1H-pyrrolo[2,3-b]pyridine-2-carboxylic acid [1-(S)-fluorobenzyl)-2-(4-hydroxypiperidin-1-yl)-2-oxoethyl]amide

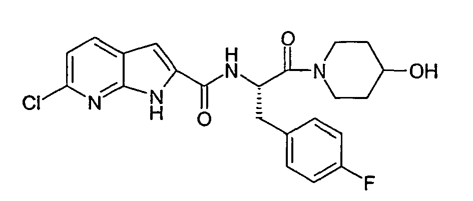

The title compound was prepared according to **EXAMPLE 266** but using 2-(*S*)-amino-3-(4-fluorophenyl)-1-(4-hydroxypiperidin-1-yl)-propan-1-one hydrochloride (**Preparation 20**) instead of 2-(*S*)-amino-N,N-dimethyl-3-phenyl propionamide hydrochloride. Purification by column chromatography (SiO₂, 9:1 CH₂Cl₂/MeOH) gave the title compound. m/z (ES⁺) = 444.91 [*M*+H]⁺; RT = 3.55min.

### EXAMPLE 269

### 6-Chloro-1H-pyrrolo[2,3-b]pyridine-2-carboxylic acid [1-(S)-(4-fluorobenzyl)-2-oxo-2-(5-oxo-[1-4]diazepam-1-yl)ethyl]amide

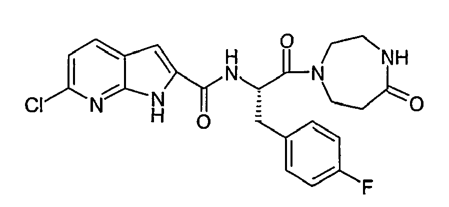

The title compound was prepared according to **EXAMPLE 266** but using 1-[2-(*S*)-amino-3-(4-fluorophenyl)propionyl]-[1,4]diazepan-5-one hydrochloride (**Preparation 112**) instead of 2-(*S*)-amino-N,N-dimethyl-3-phenyl propionamide hydrochloride. Purification by column chromatography (SiO₂ 94:6 CH₂Cl₂/MeOH) gave the title compound. m/z (ES⁺) = 457.91 [*M*+H]⁺; RT = 3.74min.

### EXAMPLE 270

### 6-Chloro-1H-pyrrolo[2,3-b]pyridine-2-carboxylic acid (2-oxo-2 phenethyl)amide

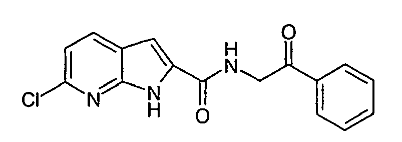

To a solution of 6-chloro-1H-pyrrolo[2,3b]pyridine-2-carboxylic acid (**Preparation 110**, 60mg, 0.31mmol) in ethanol (5mL) was added 2-aminoacetophenone hydrochloride (58mg, 0.34mmol), N-methylmorpholine (74µL, 0.67mmol) and DMTMM (198mg, 0.67mmol) and the reaction stirred at rt for 16h. Solvent was removed *in vacuo* and the resulting residue partitioned between ethyl acetate (20mL) and water (20mL). Organics were washed with 1M HCl (20mL), water (20mL), NaHCO₃ solution (2x20mL) then brine (20mL) before being dried (MgSO₄) and solvent concentrated *in vacuo.* Purification by column chromatography (SiO₂, 2:1 Pet. Ether/ EtOAc then 97:3 CH₂Cl₂/MeOH) gave the title compound.

### EXAMPLE 271

### 2-(S)-[6-Chloro-1H-pyrrolo[2,3-b]pyridine-2-carbonyl)amino]-3-(4-fluorophenyl) propionic acid ethyl ester

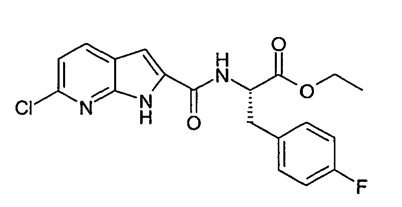

To a solution of 6-chloro-1H-pyrrolo[2,3b]pyridine-2-carboxylic acid (**Preparation 110**, 450mg, 2.29mmol) in DMF (20mL) was added 4-fluoro phenylalanine ethyl ester hydrochloride (624mg, 2.52mmol), DIPEA (1.40mL, 8.01mmol) and HOBt. (386mg, 2.52mmol) and the reaction stirred. After 5min EDCI (570mg, 2.98mmol) was added and stirring continued for 16hr. Solvent was removed *in vacuo* then crude material partitioned between ethyl acetate (75mL) and water (50mL). Organics were washed with NaHCO₃ solution (3x50mL) then brine (2x50mL), dried (MgSO₄) and THE solvent removed *in vacuo* to give the title compound. m/z (ES⁺) = 389.90 [*M*+H]⁺; RT = 3.79min.

### EXAMPLE 272

### 2-(S)-[(6-Chloro-1H-pyrrolo[2,3-b]pyridine-2-carbonyl)amino]-3-(4-fluorophenyl)propionic acid

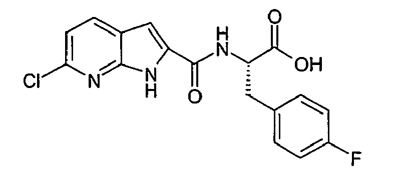

To a solution of 2-[(6-chloro-1H-pyrrolo[2,3-b]pyridine-2-carbonyl)amino]-3-(4-fluorophenyl)propionic acid ethyl ester (**EXAMPLE 271**, 780mg, 2.0mmol) in methanol (15mL) was added 2M NaOH (2mL, 4.0mmol) and the reaction was stirred for 16hr. Solvent was removed *in vacuo* and crude residue dissolved in water (20mL). The aqueous phase was washed with ethyl acetate (2x20mL), then acidified to Ph 3 with 2M HCl. The organics were extracted into ethyl acetate (2x30mL), and then dried (MgSO₄) before concentrating the solvent *in vacuo* to provide the title compound. m/z (ES⁺) = 361.88 [*M* + *H*]⁺.

### EXAMPLE 273

### 6-Chloro-1H-pyrrolo[2,3-b]pyridine-2-carboxylic acid [1-(S)-(4-fluorobenzyl)-2-oxo-2-pyrrolidin-1-yl-ethyl]amide

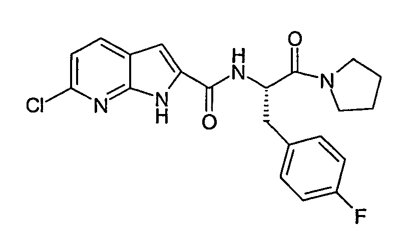

To pyrrolidine (11.5mg, 0.14mmol) was added a solution of DIPEA (60µL, 0.35mmol) in DMF (500µL) followed by 2-(*S*)-[(6-chloro-1H-pyrrolo[2,3-b]pyridine-2-carbonyl)amino]-3-(4-fluorophenyl) propionic acid (**EXAMPLE 272**, 50mg, 0.14mmol) in DMF (500µL) and HOBt (23mg, 0.15mmol) in DMF (500µL) and the mixture stirred. After 5min EDCI (34.5mg, 0.18mmol) in DMF (500µL) was added and the reaction stirred for 16hr. Solvent was removed *in vacuo* then crude material purified by mass-directed purification to give the title compound. m/z (ES⁺) = 414.93 [*M* + H]⁺; RT = 3.77min.

**EXAMPLES 274-**276 were prepared in the same way:

| **Example** | **Structure** | **m/z** | **RT (min)** |
|---|---|---|---|
| **274** | 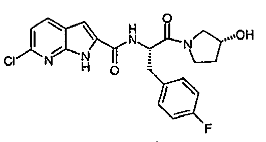 | 430.92 | 3.46 |
| **275** | 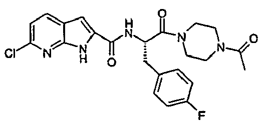 | 471.92 | 3.51 |
| **276** | 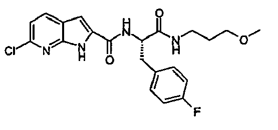 | 432.92 | 3.79 |

**EXAMPLES 277-280** were prepared in the same way, but purification was by trituration from methanol:

| **Example** | **Structure** | **m/z** | **RT (min)** |
|---|---|---|---|
| **277** | 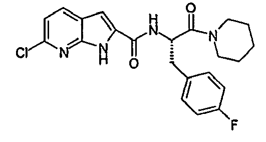 | 428.95 | 5.06 |
| **278** | 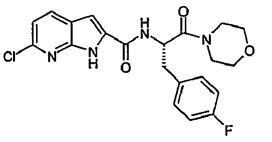 | 430.93 | 3.65 |
| **279** | 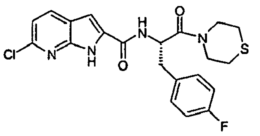 | 446.9 | 3.9 |
| **280** | 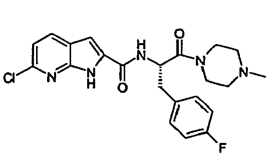 | 443.92 | 3.05 |

### EXAMPLE 281

### 2-(S)-[(5-Chloro-1H-pyrrolo[2,3-b]pyridine-2-carbonyl)amino]-3-(4-fluorophenyl)-propionic acid tert-butyl ester

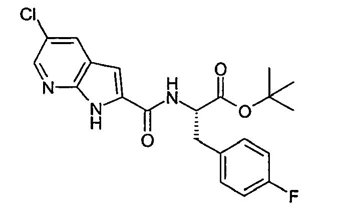

To a solution of 5-chloro-1H-pyrrolo[2,3-b]pyridine-2-carboxylic acid (**Preparation 57**, 500mg, 2.54mmol) in DMF (20mL) was added 2-(*S*)-amino-3-(4-fluorophenyl)propionic acid tert-butyl ester hydrochloride (**Preparation 114**, 701mg, 2.54mmol), HOBt (344mg, 2.54mmol) and DIPEA (1.4mL, 7.88mmol). After 5min, EDCI (634mg, 3.31mmol) was added and the reaction mixture stirred at rt for 72h. The solvent was removed *in vacuo* and the solid partitioned between water (50mL) and ethyl acetate (3 x 40mL). The combined organic phase was washed with brine (20mL), dried (MgSO₄) concentrated *in vacuo* and purified by chromatography on silica gel eluting with methanol:dichloromethane (1:99) to give the title compound. δ_{H} (CD₃OD): 1.42 (9H, s), 3.09 (1H, dd), 3.22 (1H, dd), 4.76 (1H, m), 6.98 (2H, m), 7.07 (1H, s), 7.27 (2H, m), 8.06 (1H, d), 8.28 (1H, d); m/z (ES⁺) = 418 [*M*+ H]⁺.

### EXAMPLE 282

### 2-(S)-[(5-Chloro-1H-pyrrolo[2,3-b]pyridine-2-carbonyl)amino]-3-(4-fluorophenyl)-propionic acid

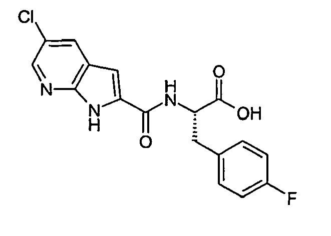

Trifluoroacetic acid (1.9mL, 24.3mmol) was added to a suspension of 2-(*S*)-[(5-chloro-1H-pyrrolo[2,3-b]pyridine-2-carbonyl)amino]-3-(4-fluorophenyl)-propionic acid *tert*-butyl ester (**EXAMPLE 281**, 507mg, 1.21mmol) in DCM (25mL) and the reaction mixture was stirred at rt for 16h. Further trifluoroacetic acid (2mL) was added and the reaction was stirred at rt for 48h. The solvent was removed *in vacuo* and the solid partitioned between 1N hydrochloric acid (50mL) and ethyl acetate (3 x 30mL). The combined organic fractions were washed with brine (20mL), dried (MgSO₄) and concentrated *in vacuo* to give the title compound. δ_{H} (CD₃OD): 3.11 (1H, dd), 3.33 (1H, dd), 4.88 (1H, m), 6.97 (2H, m), 7.06 (1H, s), 7.28 (2H, m), 8.08 (1H, d), 8.29 (1H, d); m/z (ES⁺) = 362 [*M* + H]⁺; RT = 3.67min.

### EXAMPLE 283

### 5-Chloro-1H-pyrrolo[2,3-b]pyridine-2-carboxylic acid [1-(S)-(4-fluoro-benzyl)-2-(3-hydroxy-pyrrolidin-1-yl)-2-oxo-ethyl]-amide

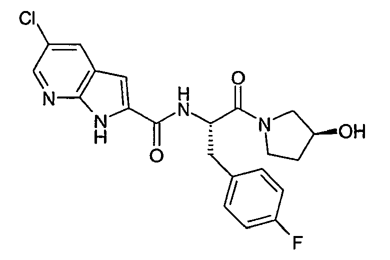

To a solution of 5-chloro-1H-pyrrolo[2,3-b]pyridine-2-carboxylic acid (**Preparation 57**, 34mg, 0.17mmol) in DMF (5mL) was added 2-(*S*)-amino-3-(4-fluorophenyl)-1-(3-(*S*)-hydroxypyrrolidin-1-yl)propan-1-one hydrochloride (**Preparation 103**, 50mg, 0.17mmol), HOBt (23mg, 0.17mmol) and DIPEA (94µL, 0.54mmol). After 5min, EDCI (43mg, 0.23mmol) was added and the reaction stirred at rt for 16h. The solvent was removed *in vacuo* and the residue partitioned between water (25mL) and ethyl acetate (3 x 20mL). The combined organic layer was washed with 2N sodium hydroxide solution (2 x 10mL), brine (10mL), dried (MgSO₄) and concentrated *in vacuo.* Purification *via* chromatography on silica gel eluting with methanol:dichloromethane (7:93) gave the title compound. m/z (ES⁺) = 431 [*M*+ H]⁺; RT = 3.49min.

### EXAMPLE 284

### 5-Chloro-1H-pyrrolo[2,3-b]pyridine-2-carboxylic acid (2-oxo-2-phenylethyl)amide

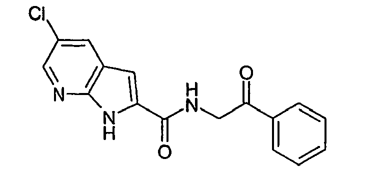

5-Chloro-1H-pyrrolo[2,3-b]pyridine-2-carboxylic acid (**Preparation 57**, 50mg, 0.25mmol), 2-aminoacetophenone hydrochloride (48mg, 0.28mmol) and DMTMM (85mg, 0.31mmol) were dissolved in THF (5mL) and 4-methylmorpholine (31µL, 0.28mmol). The reaction mixture was stirred at rt for 16h. The solvent was removed *in vacuo* and the residue partitioned between water (20mL) and EtOAc (3 x 20mL). The combined organics were dried (MgSO₄) concentrated *in vacuo* and purified by chromatography on silica gel eluting with methanol:dichloromethane (1:19) to give the title compound. m/z (ES⁺) = 314 [*M* + H]⁺; RT = 3.47min.

### EXAMPLE 285

### 5-Chloro-1H-pyrrolo[2,3-b]pyridine-2-carboxylic acid [2-(S)-(4-fluorophenyl)-1-(methoxymethylcarbamoyl)ethyl]amide

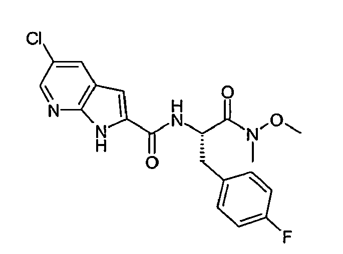

2-(*S*)-[(5-Chloro-1H-pyrrolo[2,3-b]pyridine-2-carbonyl)amino]-3-(4-fluorophenyl)propionic acid (**EXAMPLE 282**, 300mg, 0.08mmol) and *N,O-*dimethylhydroxylamine hydrochloride (8mg, 0.08mmol) were dissolved in THF (5mL) and 4-methylmorpholine (9µL, 0.08mmol). To this was added DMTMM (28mg, 0.10mmol) and the reaction mixture was stirred at rt for 16h. The solvent was removed *in vacuo* and the residue partitioned between water (30mL) and EtOAc (3 x 30mL). The combined organics were washed with 2N sodium hydroxide solution (2 x 20ml), brine (20ml), dried (MgSO₄) and concentrated *in vacuo.* The crude residue was purified by chromatography on silica gel eluting with methanol:dichloromethane (1:24) to give the title compound. m/z (ES⁺) = 405 [*M*+ H]⁺; RT = 3.69min.

### EXAMPLE 286

### 5-Chloro-1H-pyrrolo[2,3-b]pyridine-2-carboxylic acid [1-(S)-(4-fluorobenzyl)-2-(3-hydroxy-pyrrolidin-1-yl)-2-oxo-ethyl]-amide

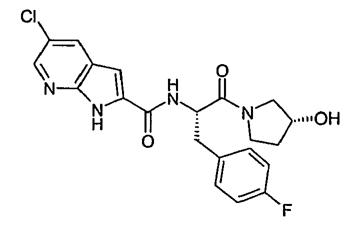

To a solution of 2-(*S*)-[(5-chloro-1H-pyrrolo[2,3-b]pyridine-2-carbonyl)amino]-3-(4-fluorophenyl)propionic acid (**EXAMPLE 286**, 30mg, 0.08mmol) in DMF (3mL) was added (*R*)-(+)-3-hydroxypyrrolidine (7.2mg, 0.08mmol), HOBt (11.2mg, 0.08mmol) and DIPEA (30.3µL, 0.17mmol). After 5min, EDCI (20.7mg, 0.11mmol) was added and the reaction was stirred at rt for 16h. The solvent was removed *in vacuo* and the residue partitioned between water (20mL) and ethyl acetate (3 x 20mL). The combined organic fractions were washed with 2N sodium hydroxide solution (20mL), brine (20mL), dried (MgSO₄) and concentrated *in vacuo.* The crude product was triturated from methanol to give the title compound. m/z (ES⁺) = 431 [*M*+H]⁺; RT = 3.44min.

### EXAMPLES 287 - 294

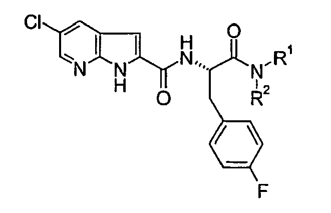

The following compounds were prepared according to the method of **EXAMPLE 286** from 2-(*S*)-[(5-chloro-1H-pyrrolo[2,3-c]pyridine-2-carbonyl)amino]-3-(4-fluorophenyl)propionic acid (**EXAMPLE 282**) and the appropriate amine, with the exception that all compounds were purified by mass directed purification.

| **Example** | **NR¹R²** | **m/z** | **RT (min)** |
|---|---|---|---|
| **287** | 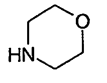 | 431 | 3.47 |
| **288** | 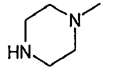 | 444 | 2.89 |
| **289** | 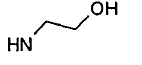 | 405 | 3.20 |
| **290** | 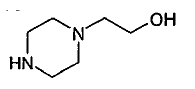 | 474 | 2.92 |
| **291** | 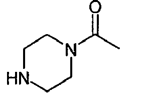 | 472 | 3.37 |
| **292** | 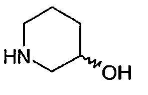 | 445 | 3.61 |
| **293** | 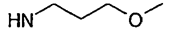 | 433 | 3.51 |
| **294** | 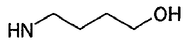 | 433 | 3.34 |

### EXAMPLE 295

### 6-Chloro-1H-pyrrolo[3,2-c]pyridine-2-carboxylic acid (1-dimethylcarbamoyl-2-(S)-phenylethyl)amide

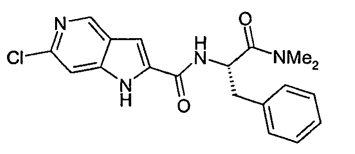

To a solution of 6-chloro-1*H*-pyrrolo[3,2-c]pyridine-2-carboxylic acid (**Preparation 116**, 47mg, 0.24mmol) in DMF (5mL, anhydrous) was added 2-(*S*)-amino-*N,N*-dimethyl-3-phenylpropionamide hydrochloride (**Preparation 8**, 56mg, 0.25mmol), DIPEA (131µL, 0.75mmol) and HOBt (40mg, 0.26mmol) sequentially. The solution was stirred for 5min prior to the addition of EDCI (55mg, 0.29mmol) in one portion. The resulting solution was stirred for 12h at rt. The reaction mixture was partitioned between ethyl acetate (50mL) and water/brine (150mL, 1:1). The layers were separated and the aqueous phase extracted with ethyl acetate (3 x 50mL), then the combined organics were washed with dilute HCl solution (1M, 50mL), dilute NaOH solution (1M, 50mL) and brine (50mL). The organic phase was dried (MgSO₄) filtered and concentrated *in vacuo.* Purification *via* flash column chromatography eluting with toluene/acetone (3:1) gave the title compound. δ_{H} (CDCl₃): 2.88, 3.05 (6H, 2s), 3.18, 3.28 (2H, 2 dd), 5.41 (2H, m), 7.02 (1H, s), 7.28-7.34 (6H, m), 8.17 (1H, d), 8.68 (1H, s), 10.58 (1H, br s); m/z (ES⁺) = 371.13 [*M*+ H]⁺; RT = 3.28min.

### EXAMPLE 296

### 6-Chloro-1H-pyrrolo[3,2-c]pyridine-2-carboylic acid [1-(S)-(4-fluorobenzyl)-2-(4-hydroxypiperidin-1-yl)-2-oxoethyl]amide

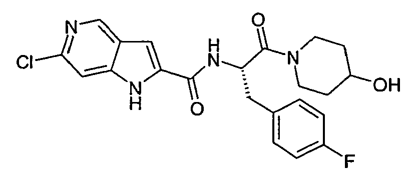

The title compound was prepared as outlined in **EXAMPLE 295** from 6-chloro-1*H*-pyrrolo[3,2-c]pyridine-2-carboxylic acid (**Preparation 116**) and 2-(*S*)-amino-3-(4-fluorophenyl)-1-(4-hydroxypiperidin-1-yl)propan-1-one hydrochloride (**Preparation 20**). The product was purified by chromatography on silica gel eluting with methanol/dichloromethane (1:19) to give the title compound. δ_{H} (CD₃OD): 1.16-1.88 (4H, 3m), 3.04-3.19 (4H, m), 3.69-4.16 (3H, 2m), 5.31 (1H, m), 7.00 (2H, m), 7.29 (3H, m), 7.42 (1H, s), 8.67 (1H, s); m/z (ES⁺) = 444.89 [*M*+ H]⁺; RT = 3.27min.

### EXAMPLE 297

### 6-Chloro-1H-pyrrolo [3,2-c] pyridine-2-carboxylic acid [1-(S)-(4-fluorobenzyl)-2-(3-(S)-hydroxypyrrolidin-1-yl)-2-oxoethyl]amide

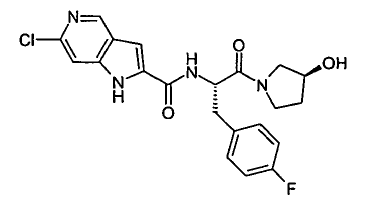

The title compound was prepared as outlined in **EXAMPLE 295** from 6-chloro-1*H*-pyrrolo[3,2-c]pyridine-2-carboxylic acid (**Preparation 116**) and 2-(*S*)-amino-3-(4-fluorophenyl)-1-(3-(*S*)-hydroxypyrrolidin-1-yl)propan-1-one hydrochloride (**Preparation 103**). The crude product was recrystallised from ethyl acetate to give the title compound. δ_{H} (CD₃OD): 1.77-1.98 (2H, m), 3.08-3.88 (6H, 6m), 4.30, 4.42 (1H, 2m), 5.07, 5.09 (1H, 2m), 7.00 (2H, m), 7.30 (3H, m), 7.43 (1H, m), 8.67 (1H, s); m/z (ES⁺) = 430.90 [*M*+ H]⁺; RT = 3.29min.

### EXAMPLE 298

### 5-Cyano-1H-pyrrolo[2,3-c]pyridine-2-carboxylic acid (2-oxo-2-phenylethyl)amide

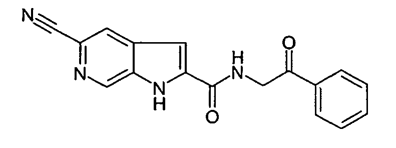

To a solution of 5-cyano-1*H*-pyrrolo[2,3-c]pyridine-2-carboxylic acid (**Preparation 62**, 0.035g, 0.19mmol) in THF (15mL) under argon was added 2-aminoacetophenone hydrochloride (0.036g, 0.21mmol), N-methylmorpholine (25µM, 0.23mmol) and DMTMM (0.072g, 0.27mmol). The reaction mixture was stirred at rt for 16h. The reaction mixture was concentrated to dryness *in vacuo.* The residue was partitioned between ethyl acetate (100mL) and water (50mL). The organic phase was separated and the aqueous phase was further extracted with ethyl acetate (100mL). The combined organic extract was washed with 1N NaOH (40mL), saturated sodium chloride (40mL), dried (MgSO₄) filtered and concentrated *in vacuo.* Crude material was purified by chromatography in hexane / ethyl acetate (1:2) and recrystallised from methanol to give the title compound. m/z (ES+) = 304 [*M*+ H]⁺; RT = 4.37.

### EXAMPLE 299

### 2-[(5-Cyano-1H-pyrrolo[2,3-c]pyridine-2-carbonyl)amino]-3 (S)-(4-fluorophenyl)propionic acid ethyl ester

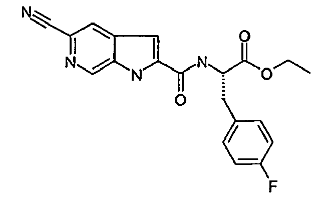

To a solution of 2-amino-3-(*S*)-(4-fluorophenyl)propionic acid ethyl ester hydrochloride (0.530g, 2.14mmol) in DMF (15mL) was added DIPEA (1.3mL, 7.49mmol), HOBt (0.290g, 2.14mmol), 5-cyano-1*H*-pyrrolo[2,3-c]pyridine-2-carboxylic acid (**Preparation 62**, 0.400g, 2.14mmol) and EDCI (0.492g, 2.57mmol). The reaction mixture was stirred at rt for 16h. Reaction mixture was concentrated *in vacuo* and the residue was partitioned between water (150mL) and ethyl acetate (200mL). Organic phase was separated and the aqueous phase was further extracted with ethyl acetate (200mL). The combined organic extracts were washed with saturated NaHCO₃ (75mL), saturated sodium chloride (100mL), dried (MgSO₄) filtered and concentrated *in vacuo* to give the title compound. m/z (ES+) = 381 [*M*+ H]⁺; RT = 4.79min.

### EXAMPLE 300

### 2-[(5-Cyano-1H-pyrrolo[2,3-c]pyridine-2-carbonyl)amino]-3-(S)-(4-fluorophenyl)propionic acid

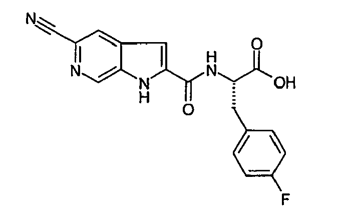

To a solution of 2-[(5-cyano-1H-pyrrolo[2,3-c]pyridine-2-carbonyl)amino]-3-(*S*)-(4-fluorophenyl)propionic acid ethyl ester (**EXAMPLE 299**, 0.800g, 2.1mmol) in methanol / water (2:1) was added 1N NaOH (4.2mL, 4.2mmol) and stirred at rt for 16h. Reaction mixture was concentrated *in vacuo* to remove methanol. Water (150mL) was added to the residue and washed with ethyl acetate (2 x 75ml). The aqueous phase was cooled in an ice bath and acidified to pH 4 using 2N HCl. The precipitate formed was isolated and washed with water and ether to give the title compound. m/z (ES+) = 353 [*M*+ H]⁺; RT = 3.27 min.

### EXAMPLE 301

### 5-Cyano-1H-pyrrolo[2,3-c]pyridine-2-carboxylic acid [1-dimethylcarbamoyl-2-(S)-(4-fluorophenyl)ethyl]amide

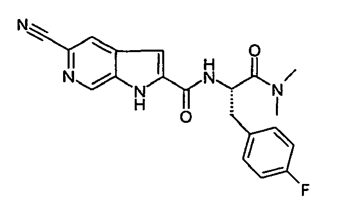

To a solution of N-dimethylamine hydrochloride (0.006g, 0.07mmol) in DMF (6mL) was added DIPEA (37µL, 0.21mmol), HOBt (0.009g, 0.07mmol), 2-[(5-cyano-1H-pyrrolo[2,3-c]pyridine-2-carbonyl)amino]-3-(*S*)-(4-fluorophenyl)propionic acid (**EXAMPLE 300**, 0.025g, 0.07mmol) and EDCI (0.016g, 0.084mmol). The reaction mixture was stirred at rt for 16h then concentrated *in vacuo* and the residue was partitioned between water (50mL) and ethyl acetate (100mL). The organic phase was separated and the aqueous phase was further extracted with ethyl acetate (100mL). The combined organic extracts were washed with saturated NaHCO₃ (50mL), saturated sodium chloride (75mL), dried (MgSO₄) filtered and concentrated *in vacuo* to give the title compound. m/z (ES+) = 380; RT = 3.45.

### EXAMPLES 302 - 307

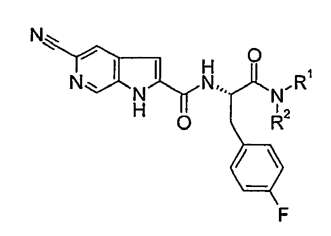

The following compounds were prepared according to the method of **EXAMPLE 301** from 2-[(5-cyano-1H-pyrrolo[2,3-c]pyidine-2-carbonyl)amino]-3-(*S*)-(4-fluorophenyl)propionic acid (**EXAMPLE 300**) and the appropriate amine.

| **Example** | **Amine** | **m/z** | **RT (min)** |
|---|---|---|---|
| **302** | 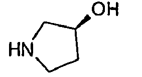 | 422 | 3.36 |
| **303** | 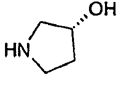 | 422 | 3.32 |
| **304** | 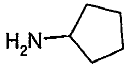 | 420 | 3.92 |
| **305** | 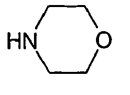 | 422 | 3.44 |
| **306** | 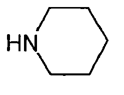 | 420 | 3.72 |
| **307** | 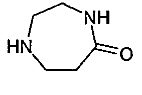 | 449 | 3.26 |

### In vitro GP activity

### Materials

α-D-Glucose-1-phosphate (disodium salt), Glycogen, D-Glucose, Malachite Green Hydrochloride, Ammonium Molybdate tetrahydrate, BSA, HEPES and rabbit muscle phosphorylase a (P1261) were purchased from Sigma. All other reagents were analytical grade.

### Method

### Glycogen phosphorylase assay in vitro:

An assay for glycogen phosphorylase activity in the reverse direction was developed based on the method described by Engers et al., Can. J. Biochem., 1970, 48, 746-754]. Rabbit muscle glycogen phosphorylase a (Sigma) was reconstituted at a stock concentration of 100µg/mL in 25mM Tris/HCl. The pH was measured in a 96-well plate in a final volume of 100µL containing 50mM Hepes pH 7.2, 7.5mM glucose, 0.5mM glucose-1-phosphate and 1mg/mL glycogen. After incubation at 30°C for 30min, the inorganic phosphate released from glucose-1-phosphate was measured by the addition of 150µL of malachite green/molybdate solution prepared as follows: 5mL of 4.2% ammonium molybdate in 4N HCl, 15mL of 0.045% malachite green, 50µL of Tween 20. Following a 30min incubation at rt, the absorbance was measured at 620nm. For IC₅₀ determination, 10µL of a serial dilution of compound (100µM to 0.004µM) in DMSO was added to each reaction in duplicate with the equivalent concentration of DMSO added to the control uninhibited reaction. Dose response curves were then obtained by plotting % inhibition versus log₁₀ compound concentration. IC₅₀ is defined as the concentration of compound achieving 50% inhibition under the assay conditions described.

The **EXAMPLES** have an IC₅₀ of < 1mM. For example, **EXAMPLES 1-20**, 22, 27, and 30-48 demonstrated efficacy by measuring values of IC₅₀ in the range of 62.8-0.07µM. Examples 21, 23-26, 28, and 29 yielded IC₅₀ 100µM or higher. It is advantageous that the measured IC₅₀ be lower than 100µM. It is still more advantageous for the IC₅₀ to be lower than 50µM. It is even more advantageous for the IC₅₀ to be lower than 5µM. It is yet more advantageous for the IC₅₀ to be lower than 0.5µM.

## Claims

1. A compound of Formula (I): 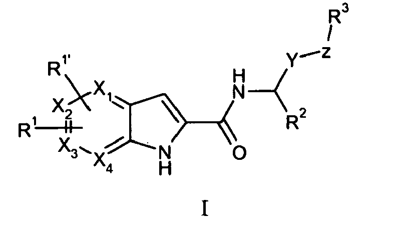 or a stereoisomer, or a pharmaceutically acceptable salt thereof, wherein:
one of X₁, X₂, X₃ and X₄ must be N and the others must be C;
R¹ and R¹' are each independently, hydrogen, halogen, hydroxy, cyano, C₁₋₄-alkyl, C₁₋₄alkoxy, fluoromethyl, difluoromethyl, trifluoromethyl, ethenyl, or ethynyl;
R² is hydrogen, C₁₋₄alkyl, COOR⁶, COR⁶, C₁₋₄alkoxyC₁₋₄alkyl-, hydroxyC₁₋₄-alkyl-, cycloalkyl, cycloalkylC₁₋₄alkyl-, aryl, arylC₁₋₄alkyl-, hetaryl, or hetarylC₁₋₄-alkyl-, wherein any of the aryl or hetaryl rings are optionally substituted with 1-2 independent halogen, cyano, C₁₋₄alkyl, C₁₋₄alkoxy, -NR¹³R¹⁴, -SO₂C₁₋₄alkyl, - SO₂NR¹³R¹⁴, hydroxy, fluoromethyl, difluoromethyl, or trifluoromethyl substituents;
Y is a direct bond, C₁₋₂alkyl, or -CH(OH)-;
Z is CH₂, -C(O)-, -O-, >NH, >N(C₁₋₄alkyl), >N(C₃₋₆Cycloalkyl), or absent; but when Y is -CH(OH)-, Z or R³ must be bonded to Y through a carbon-carbon bond;
R³ is hydrogen, -COOH, -COOC₁₋₄alkyl, C₁₋₄alkoxy, C₁₋₄alkyl, arylC₁₋₄-alkylthio-, aryl, -C₁₋₄alkylaryl, hetaryl, -C₁₋₄alkylhetaryl, cycloalkyl, -C₁₋₄-alkylcycloalkyl, heterocyclyl or -C₁₋₄alkylheterocyclyl, wherein any of the rings is optionally substituted with 1-3 independent halogen, cyano, C₁₋₄alkyl, fluoromethyl, difluoromethyl, trifluoromethyl, -NHC(O)O(C₁₋₄alkyl), -C₁₋₄alkylNHC(O)O(C₁₋₄-alkyl), -NR⁷R⁸, -C₁₋₄alkylNR⁷R⁸, -C(O)R⁹, C₁₋₄alkoxy-, C₁₋₄alkoxyC₁₋₄alkyl-, - COOH, -COOC₁₋₄alkyl, -NHC(O)R⁹, -C₁₋₄alkylNHC(O)R⁹, -C(O)N(R¹⁰)₂, -C₁₋₄-alkylC(O)N(R¹⁰)₂, -C₁₋₄alkoxyC₁₋₄alkoxy, hydroxy, hydroxyC₁₋₄alkyl-, -NHSO₂R¹⁰, -SO₂(C₁₋₄alkyl), -SO₂NR¹¹R¹², 5- to 6-membered heterocyclyl, phenyl, phenylC₁₋₄-alkoxy, or phenylC₁₋₂alkyl substituents, wherein phenyl is optionally substituted with 1-2 independent halogen, cyano, C₁₋₄alkyl, C₁₋₄alkoxy, -NR¹³R¹⁴, -SO₂C₁₋₄alkyl, - SO₂NR¹³R¹⁴, hydroxy, fluoromethyl, difluoromethyl or trifluoromethyl substituents, or two bonds on a ring carbon of the heterocyclyl group optionally can form an oxo ( =O) substituent;
or R³ is -NR⁴R⁵ or -NR⁴(-C₁₋₄alkylR⁵);
R⁴ is hydrogen, C₁₋₃alkyl, -C₂₋₃alkyl-NR⁷R⁸, C₃₋₆cycloalkyl optionally substituted by hydroxy or hydroxyC₁₋₄alkyl- further optionally substituted by hydroxy, C₁₋₂alkoxyC₂₋₄alkyl-, or C₁₋₂alkyl-S(O)ₙ-C₂₋₃alkyl-;
n is 0, 1, or 2;
R⁵ is hydrogen, hydroxyC₂₋₃alkyl-, C₁₋₂alkoxy, C₁₋₂alkoxyC₁₋₄alkyl-, or aryl, hetaryl, or heterocyclyl;
wherein a heterocyclic nitrogen-containing R⁵ ring optionally is mono-substituted on the ring nitrogen with C₁₋₄alkyl, benzyl, benzoyl, C₁₋₄alkyl-C(O)-, -SO₂C₁₋₄alkyl, -SO₂NR¹³R¹⁴, C₁₋₄alkoxycarbonyl, or aryl(C₁₋₄alkoxy)carbonyl; and wherein the R⁵ rings are optionally mono-substituted on a ring carbon with halogen, cyano, C₁₋₄alkyl-C(O)-, C₁₋₄alkyl-SO₂-, C₁₋₄alkyl, C₁₋₄alkoxy, hydroxy, -NR¹³R¹⁴, hydroxy, hydroxyC₁₋₄alkyl-, carbamoyl, or C₁₋₄alkylcarbamoyl-, provided that no quatemised nitrogen is included; or two bonds on a ring carbon of the heterocyclyl group optionally can form an oxo ( =O ) substituent;
R⁶ is C₁₋₄alkyl, aryl, or hetaryl;
R⁷ and R⁸ are independently hydrogen, C₁₋₄alkyl, C₃₋₆cycloalkyl, or CO(C₁₋₄-alkyl);
R⁹ is C₁₋₄alkyl, or C₃₋₆cycloalkyl;
R¹⁰ is hydrogen, C₁₋₄alkyl, or C₃₋₆cycloalkyl;
R¹¹ and R¹² are independently hydrogen or C₁₋₄alkyl, or together with the nitrogen to which they are attached may form a 4- to 6-membered heterocycle;
R¹³ and R¹⁴ are independently hydrogen or C₁₋₄alkyl; and
wherein there are no nitrogen-oxygen, nitrogen-nitrogen or nitrogen-halogen bonds in linking the three components -Y-Z-R³ to each other;
provided that the compound is not 2R,4S,5S-6-cyclohexyl-5-(6'-azaindol-2'-ylcarbonylamino)-2-(2'-methylpropyl)-gamma-hexanolactone.

2. A compound according to claim 1, or a stereoisomer, or a pharmaceutically acceptable salt thereof, wherein X₃ is N.

3. A compound according to claim 1, or a stereoisomer, or a pharmaceutically acceptable salt thereof, wherein X₁ is N.

4. A compound according to claim 1, or a stereoisomer, or a pharmaceutically acceptable salt thereof, wherein X₂ is N.

5. A compound according to claim 1, or a stereoisomer, or a pharmaceutically acceptable salt thereof, wherein X₄ is N.

6. A compound according to any one of the preceding claims, or a stereoisomer, or a pharmaceutically acceptable salt thereof, wherein R¹ and R^{1'} are each independently, halogen, cyano or hydrogen.

7. A compound according to any one of the preceding claims, or a stereoisomer, or a pharmaceutically acceptable salt thereof, wherein one of R¹ and R^{1'} is hydrogen and the other is a 5-halo or 5-cyano group.

8. A compound according to any one of the preceding claims, or a stereoisomer, or a pharmaceutically acceptable salt thereof, wherein Y is a direct bond or C₁₋₂alkyl.

9. A compound according to any one of the preceding claims, wherein Z is -C(O)-.

10. A compound according to any one of claims 1, 2 or 6 to 9, or a stereoisomer, or a pharmaceutically acceptable salt thereof, wherein
X₃ is N;
Y is a direct bond or C₁₋₂alkyl; and
Z is -C(O)-.

11. A compound according to any one of the preceding claims, or a stereoisomer, or a pharmaceutically acceptable salt thereof, wherein Y is a direct bond.

12. A compound according to any one of the preceding claims, or a stereoisomer, or a pharmaceutically acceptable salt thereof, wherein R² is hydrogen, C₁₋₄alkyl, aryl or arylC₁₋₄alkyl-, wherein the aryl ring is optionally substituted with 1-2 independent halogen, cyano, C₁₋₄alkyl, C₁₋₄alkoxy, NR¹³R¹⁴, -SO₂C₁₋₄alkyl, -SO₂NR¹³R¹⁴, hydroxy, fluoromethyl, difluoromethyl, or trifluoromethyl, substituents.

13. A compound according to claim 12, or a stereoisomer, or a pharmaceutically acceptable salt thereof, wherein R² is benzyl optionally substituted with 1-2 halogen substituents.

14. A compound according to any one of the preceding claims, or a stereoisomer, or a pharmaceutically acceptable salt thereof, wherein R³ is heterocyclyl, -C₁₋₄-alkylheterocyclyl optionally substituted with 1-3 independent halogen, cyano, -C₁₋₄-alkyl, fluoromethyl, difluoromethyl, trifluoromethyl, -NHC(O)O(C₁₋₄alkyl), -C₁₋₄-alkylNHC(O)O(C₁₋₄alkyl), -NR⁷R⁸, -C₁₋₄alkylNR⁷R⁸, -C(O)R⁹, C₁₋₄alkoxy-, C₁₋₄-alkoxyC₁₋₄alkyl-, -COOH, -COOC₁₋₄alkyl, -NHC(O)R⁹, -C₁₋₄alkylNHC(O)R⁹, - C(O)N(R¹⁰)₂, -C₁₋₄alkylC(O)N(R¹⁰)₂, -C₁₋₄alkoxyC₁₋₄alkoxy, hydroxy, hydroxyC₁₋₄-alkyl, -NHSO₂R¹⁰, -SO₂(C₁₋₄alkyl), -SO₂NR¹¹R¹², 5- to 6-membered heterocyclyl, phenyl, phenylC₁₋₂alkoxy or phenylC₁₋₂alkyl substituents, wherein phenyl is optionally substituted with 1-2 independent halogen, cyano, C₁₋₄alkyl, C₁₋₄alkoxy, - NR¹³R¹⁴, -SO₂C₁₋₄alkyl, -SO₂NR¹³R¹⁴, hydroxy, fluoromethyl, difluoromethyl, or trifluoromethyl substituents, or two bonds on a ring carbon of the heterocyclyl group optionally can form an oxo (=O) substituent; or R³ is -NR⁴R⁵ or -NR⁴(-C₁₋₄alkylR⁵).

15. A compound according to claim 14, wherein R³ is an optionally substituted nitrogen containing heterocyclyl group, linked to Z via a ring nitrogen atom; or R³ is -NR⁴R⁵ or -NR⁴(-C₁₋₄alkylR⁵).

16. A compound according to claim 15, wherein R³ is an optionally substituted 4-8-membered nitrogen containing heterocyclyl group.

17. A compound according to claim 16, or a stereoisomer, or a pharmaceutically acceptable salt thereof, wherein R³ is pyrrolidin-1-yl or piperidin-1-yl optionally substituted with hydroxy.

18. A compound according to claim 1 selected from the following: 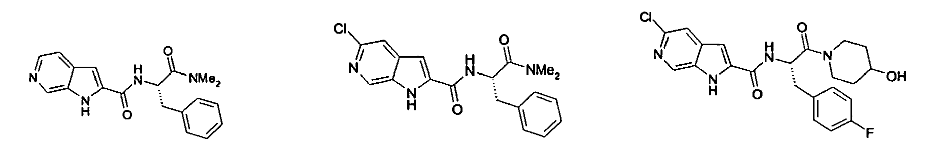 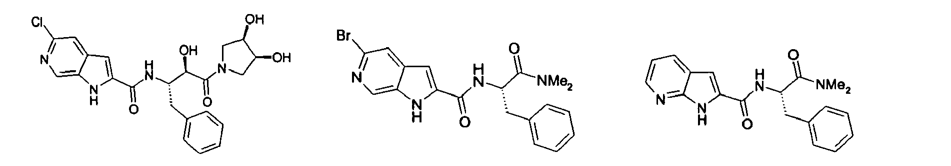 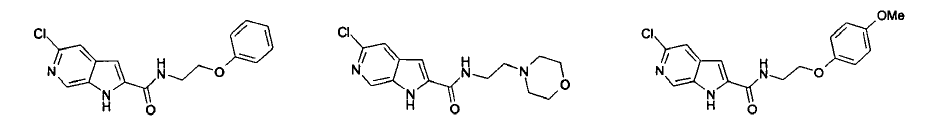 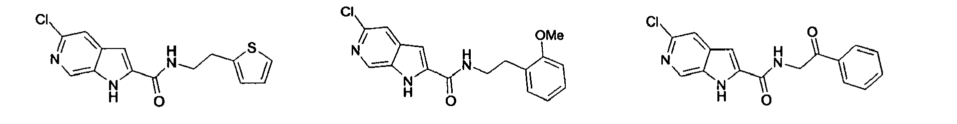 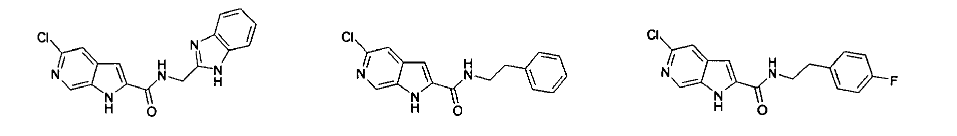 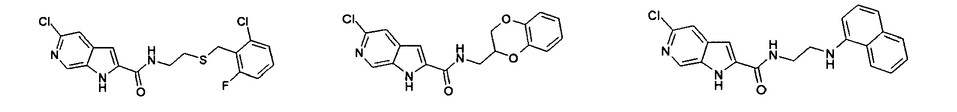 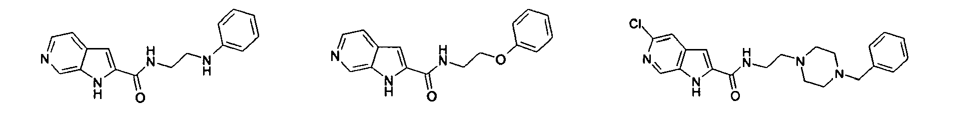 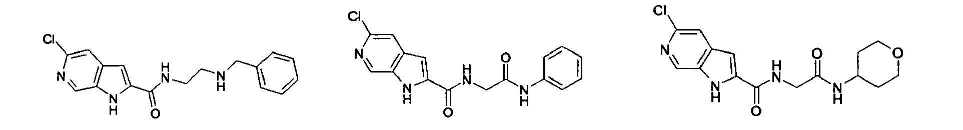 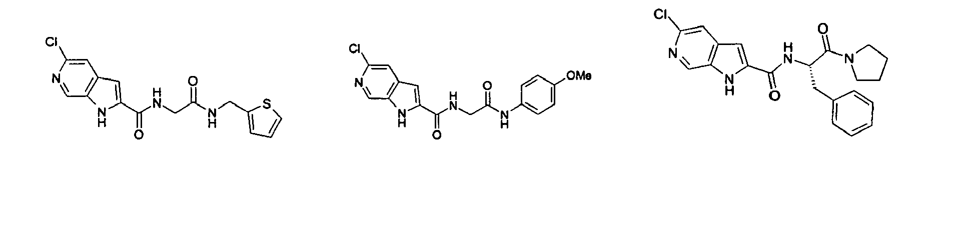 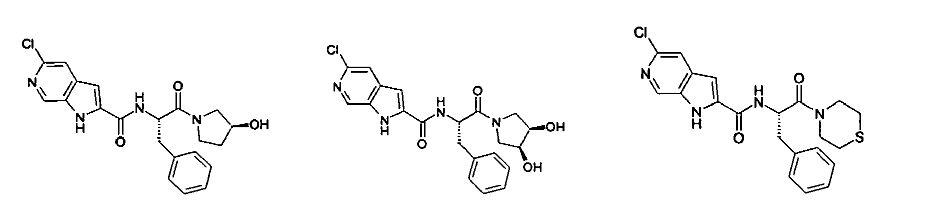 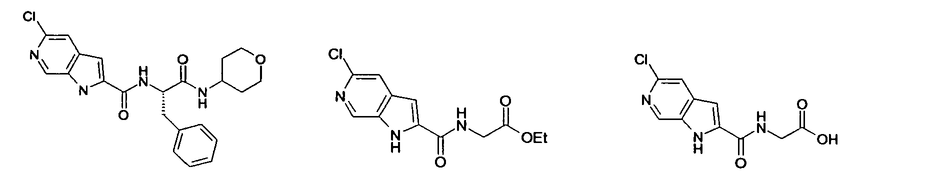 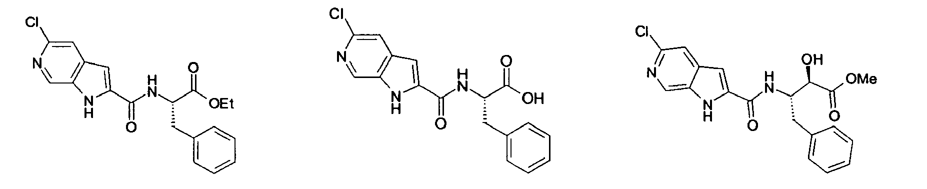 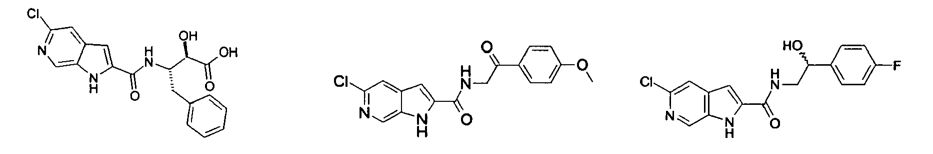 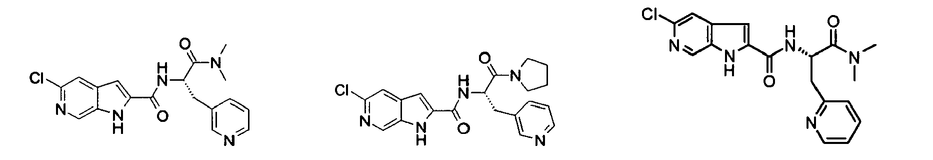 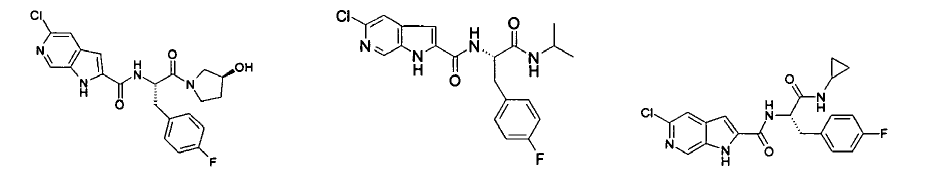 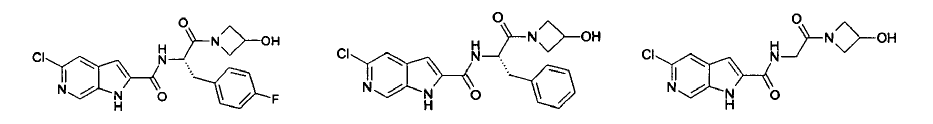 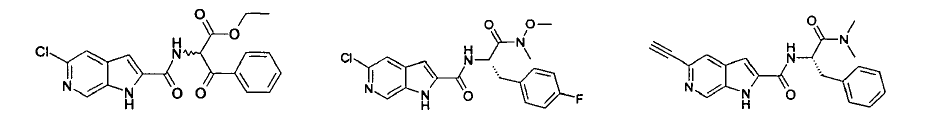 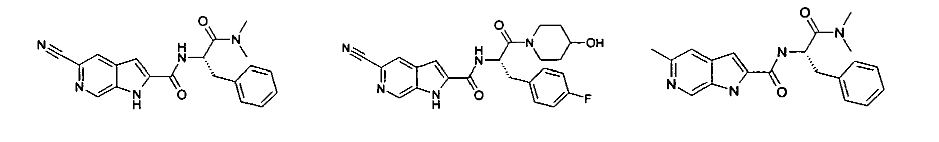 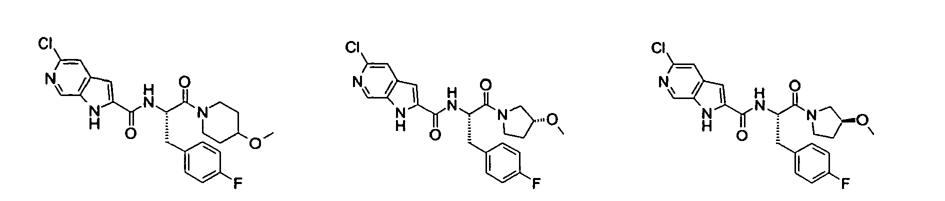 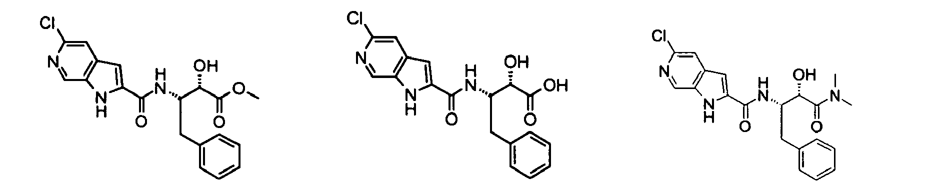 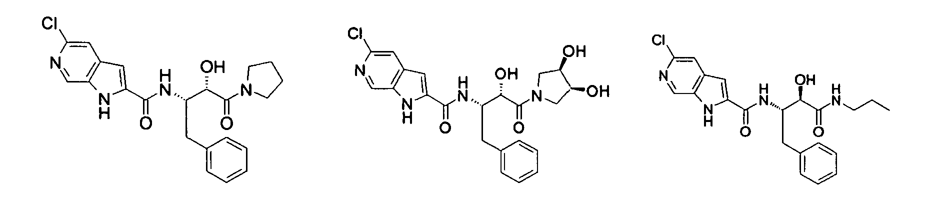 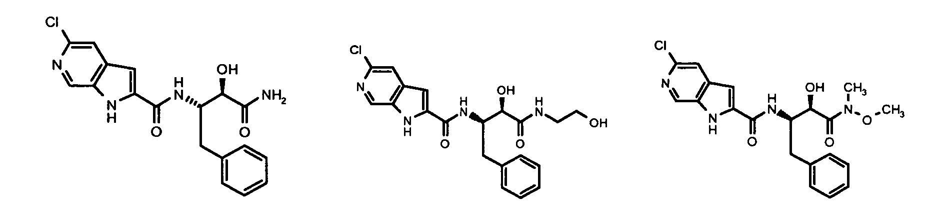 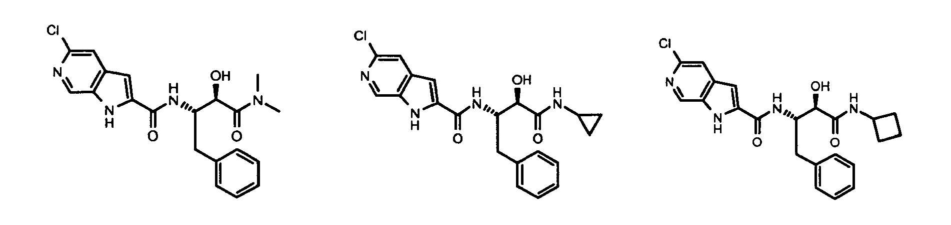 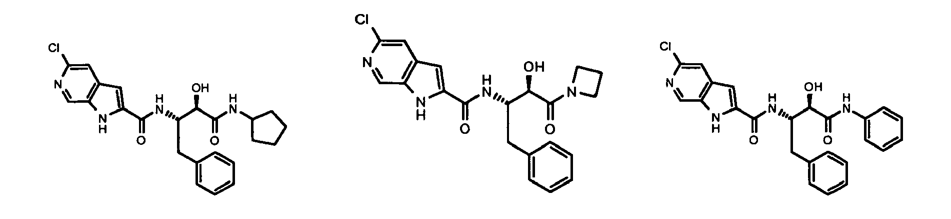 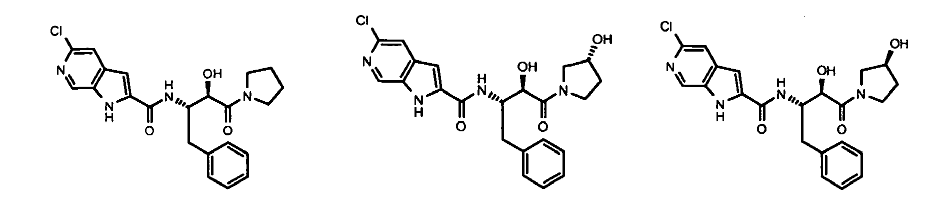 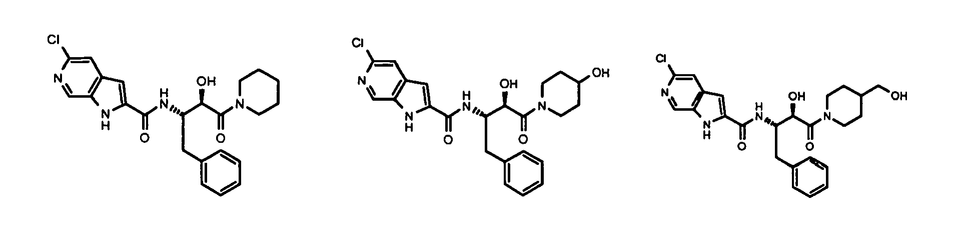 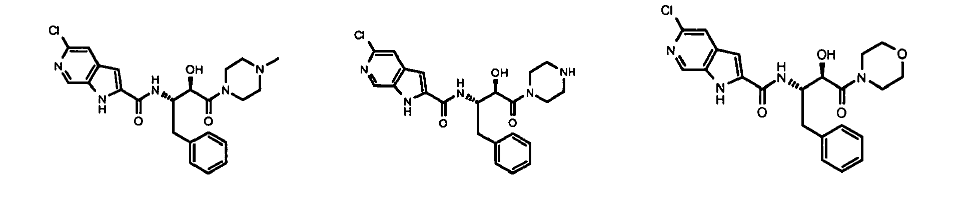 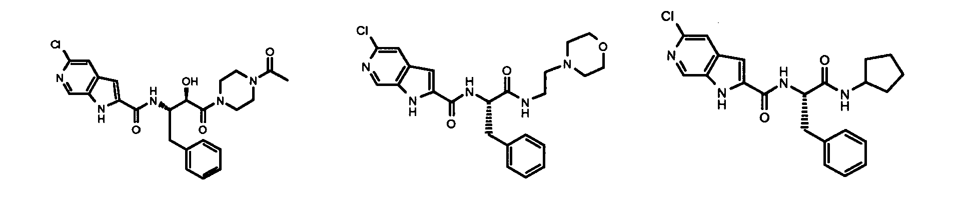 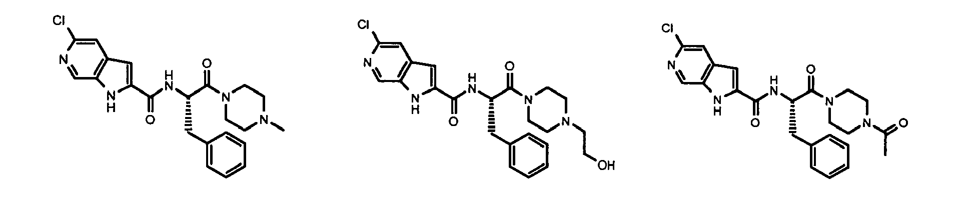 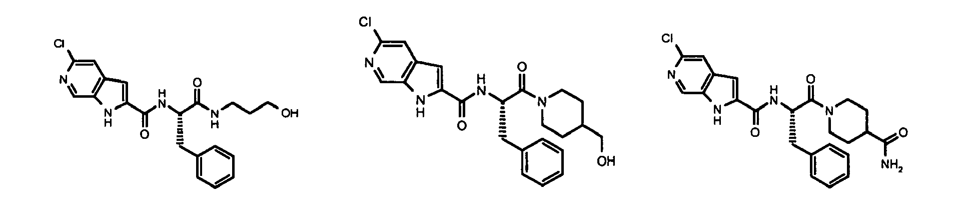 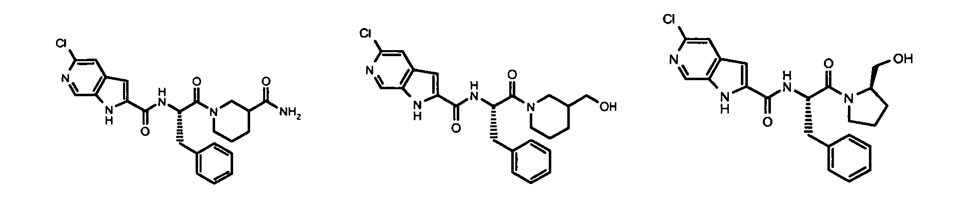 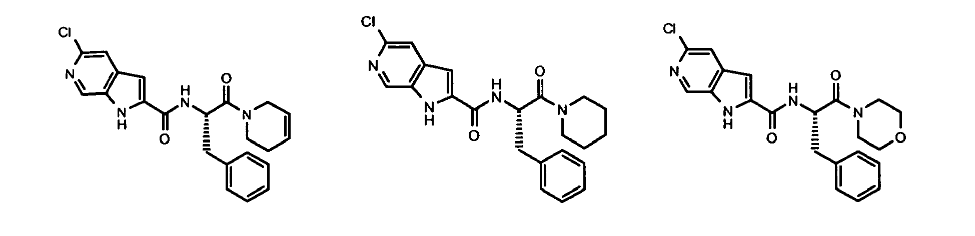 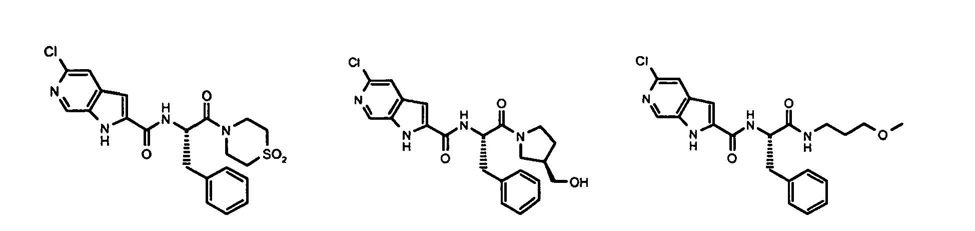 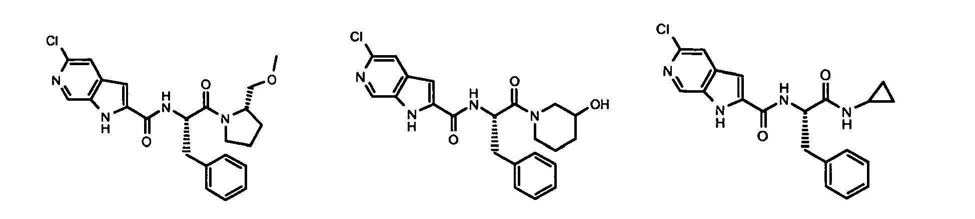 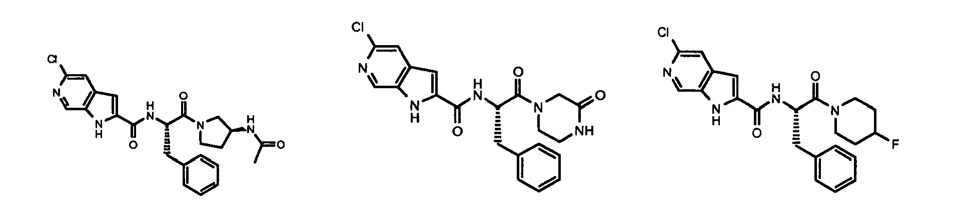 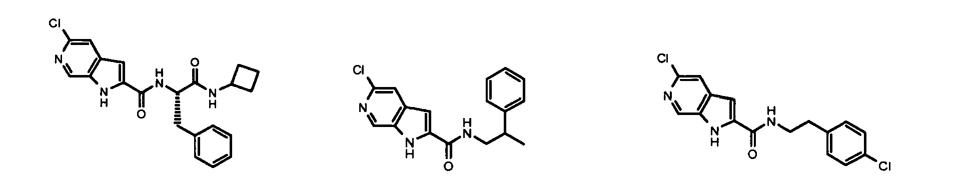 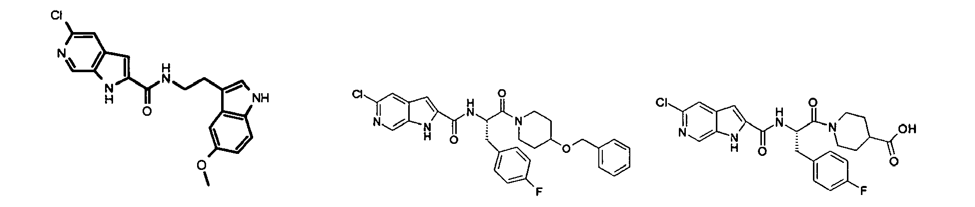 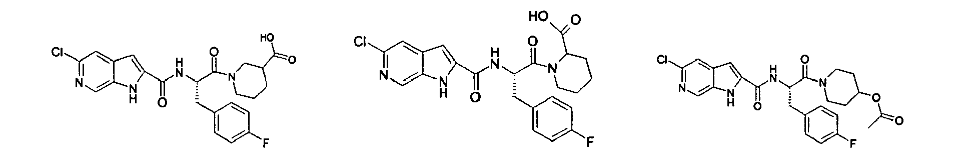 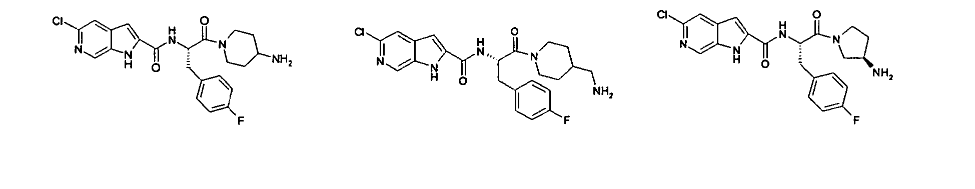 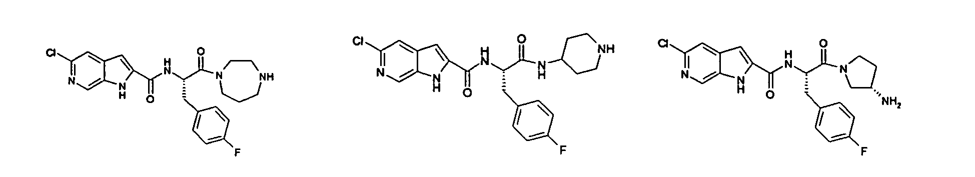 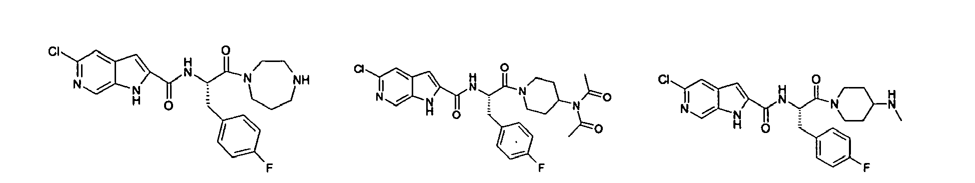 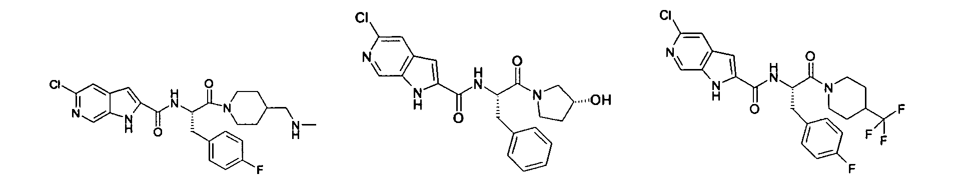 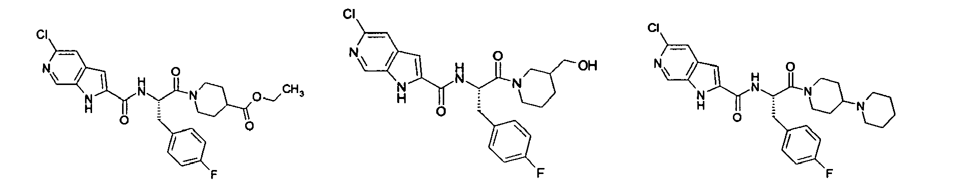 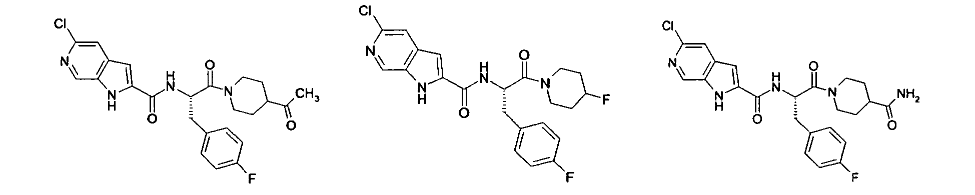 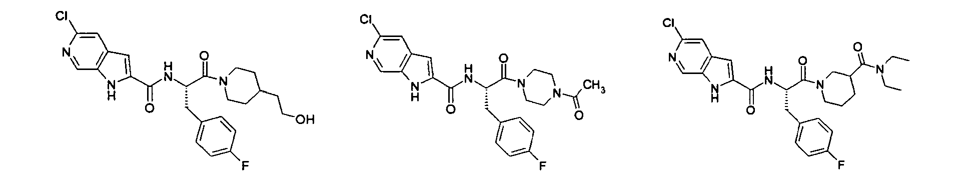 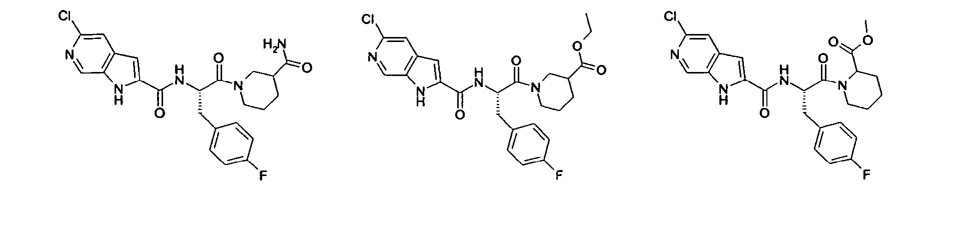 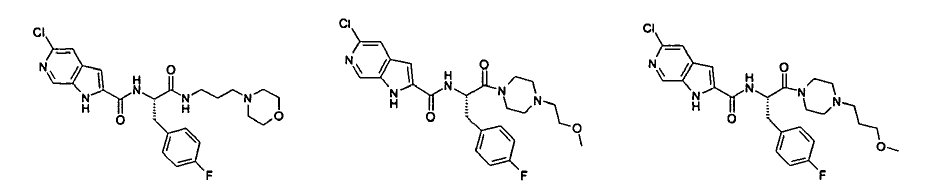 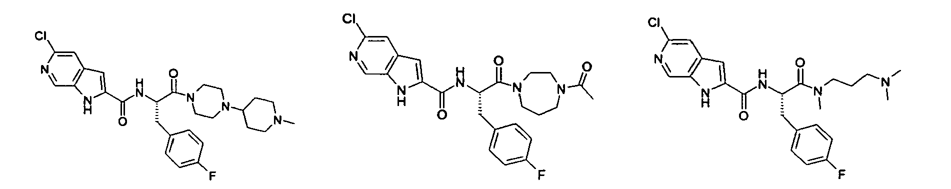 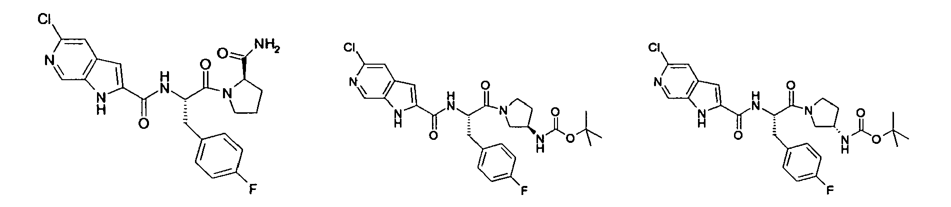 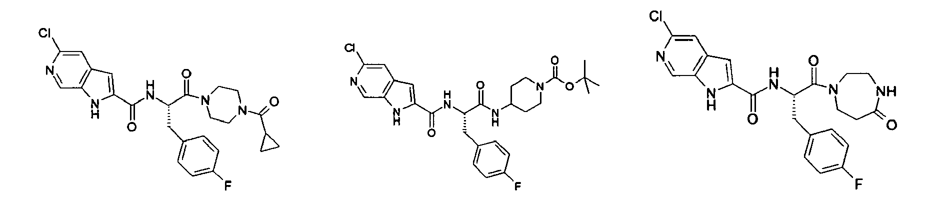 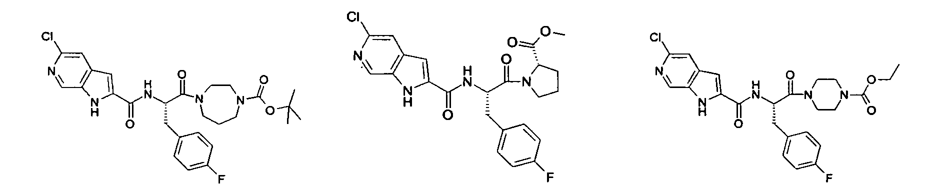 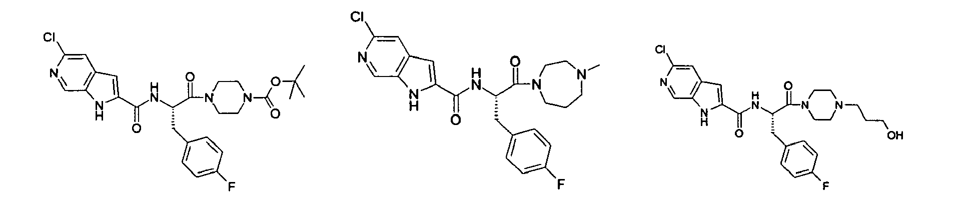 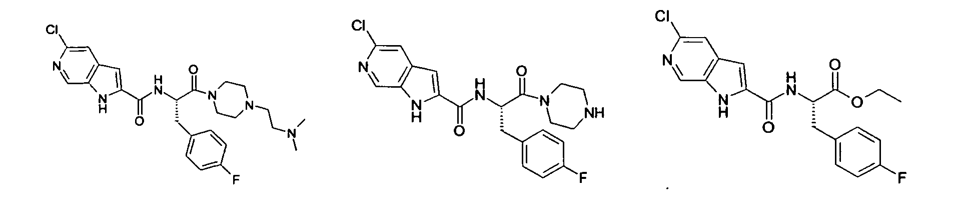 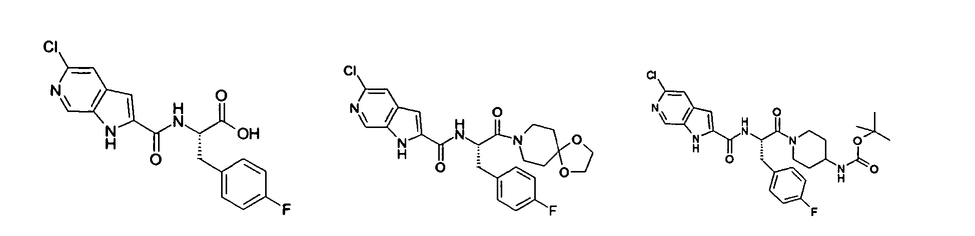 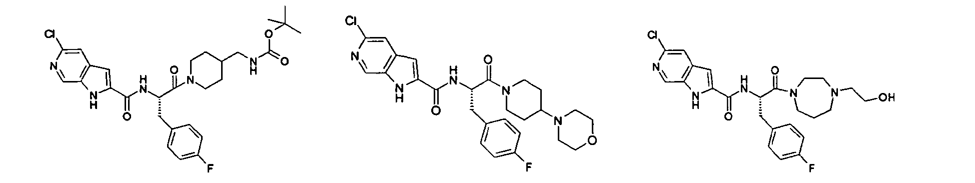 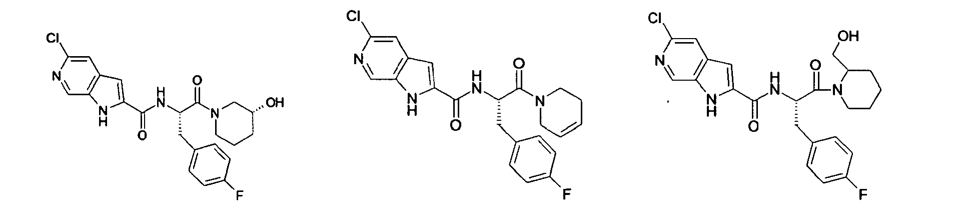 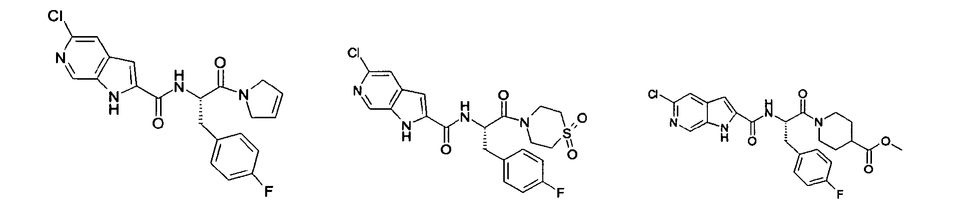 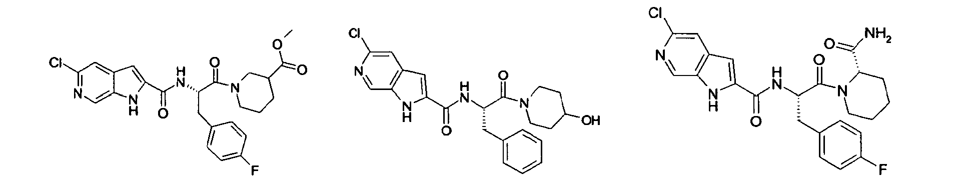 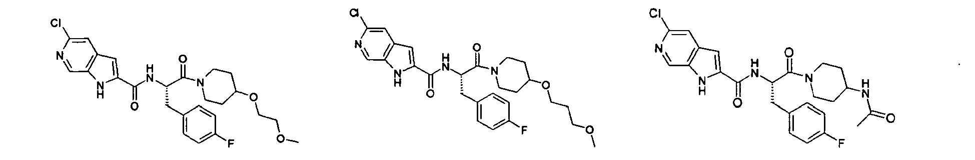 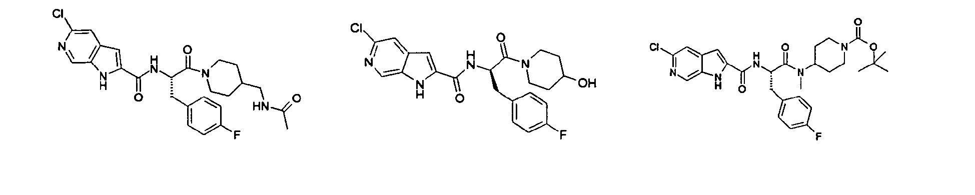 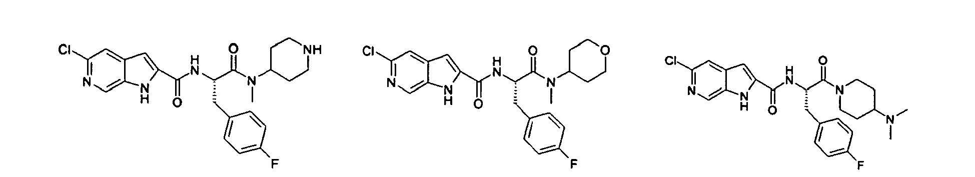 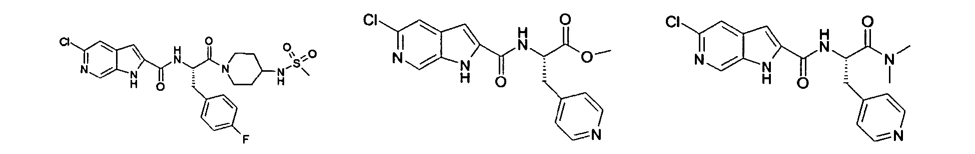 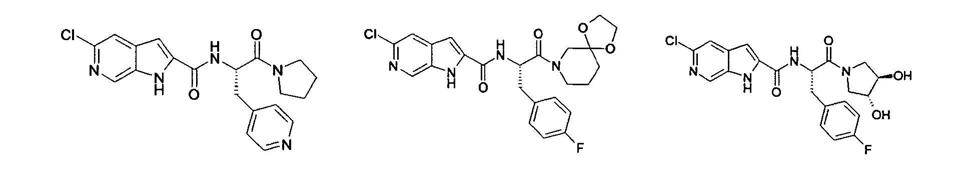 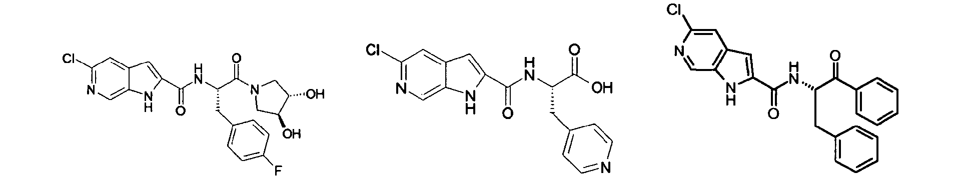 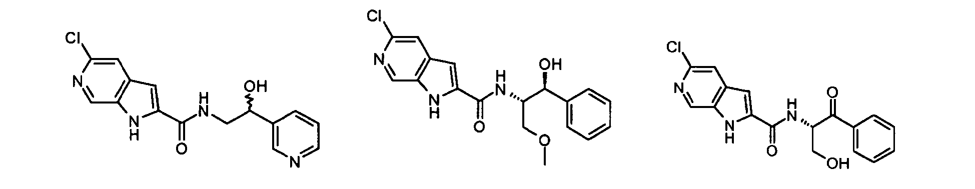 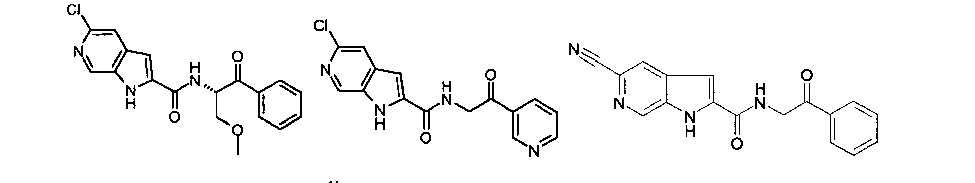 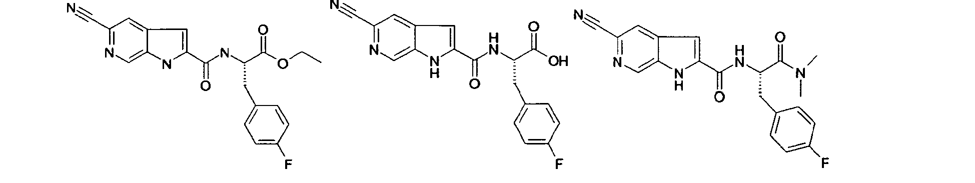 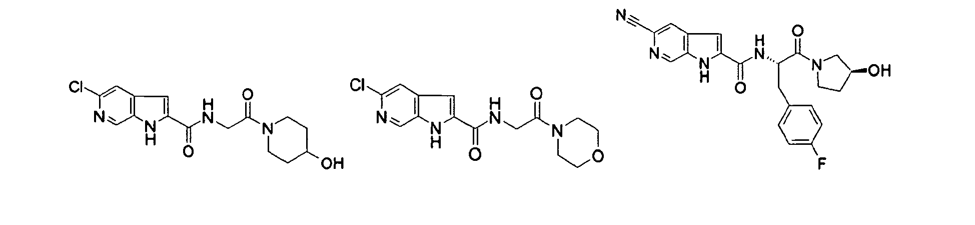 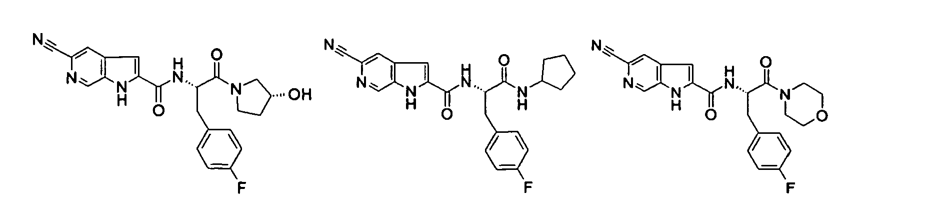 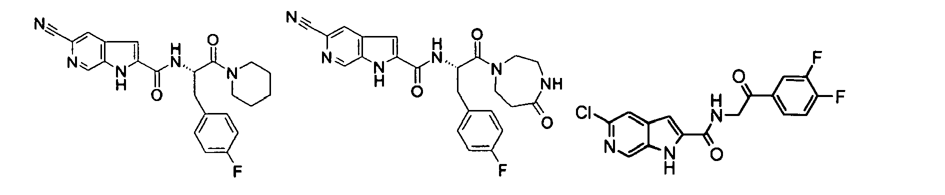 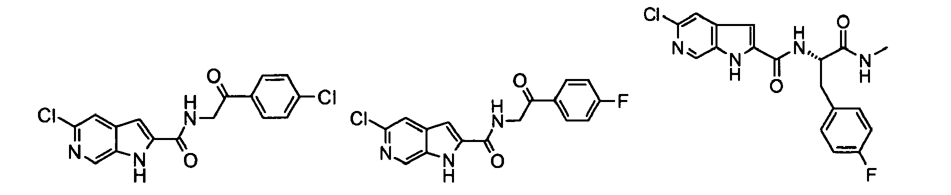 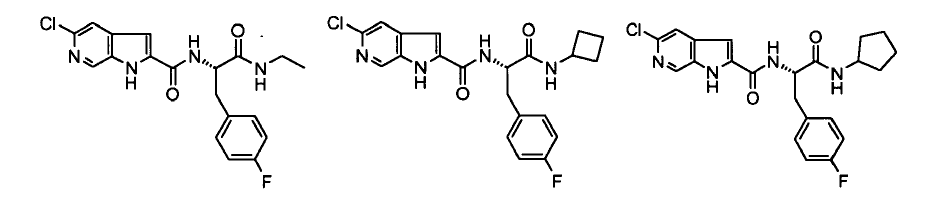 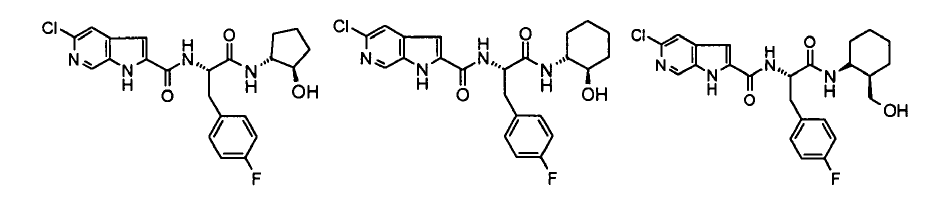 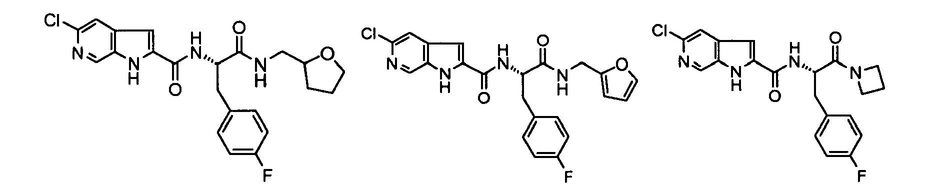 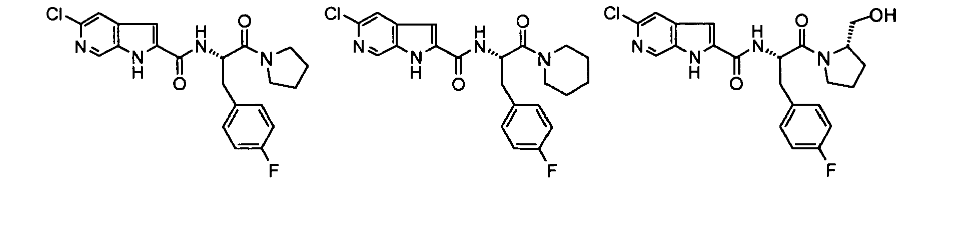 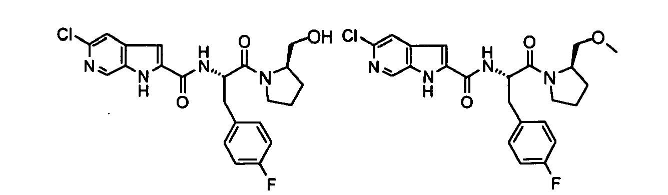 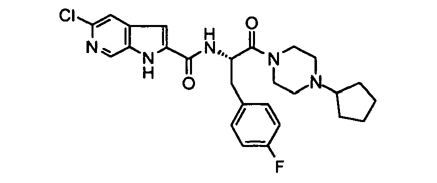 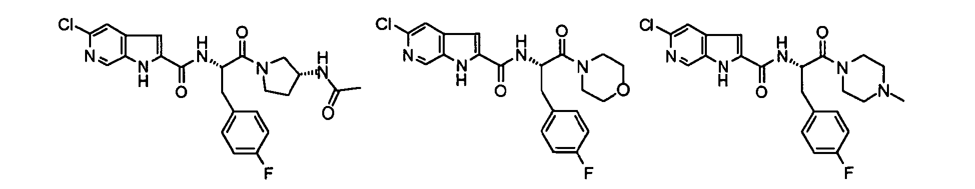 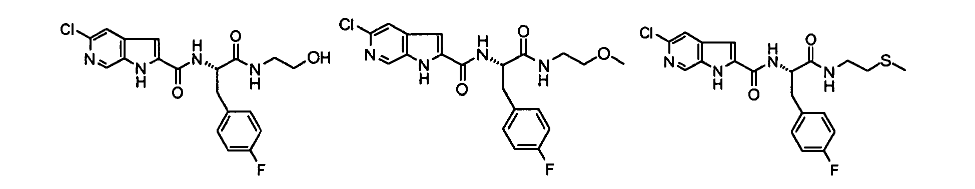 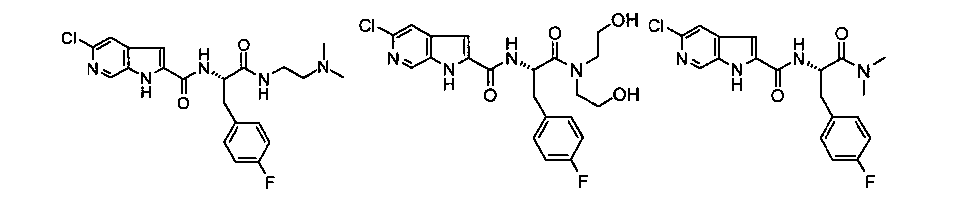 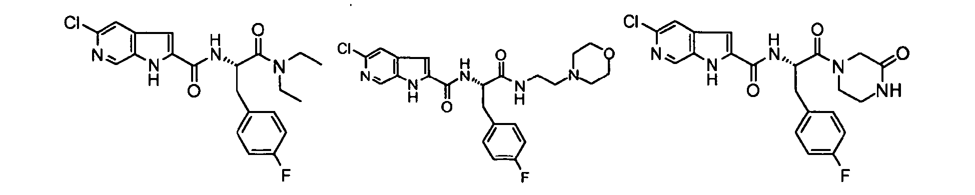 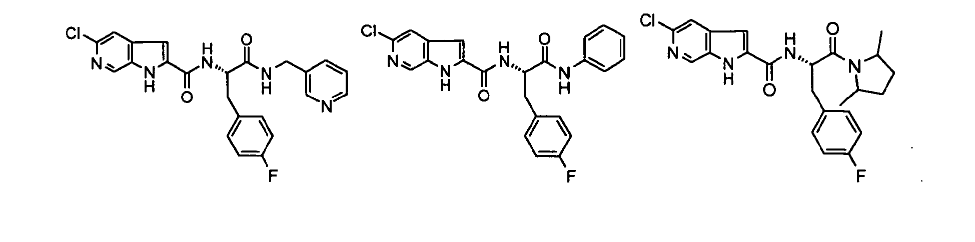 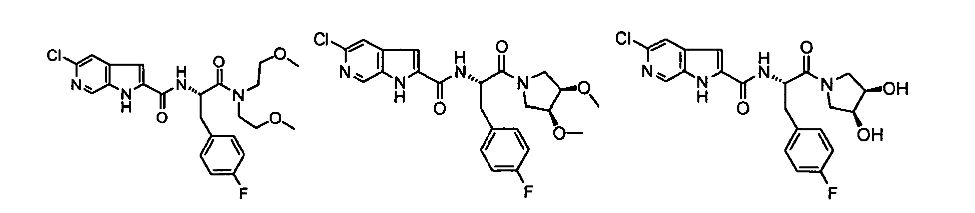 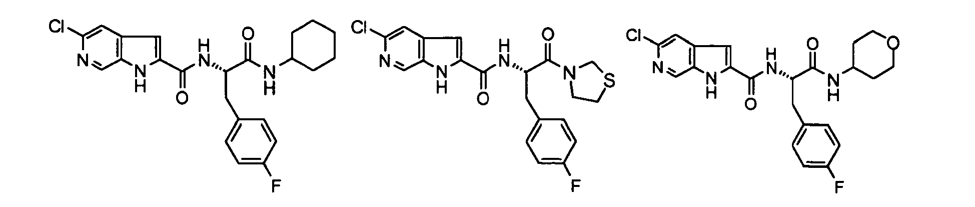 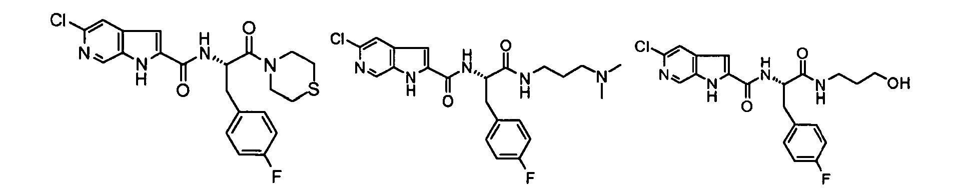 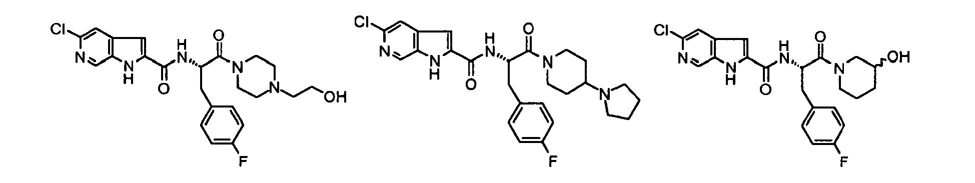 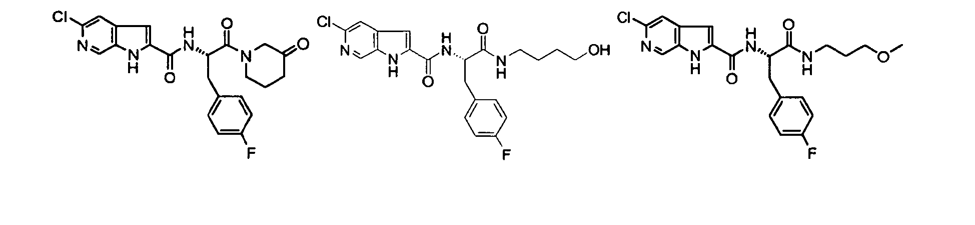 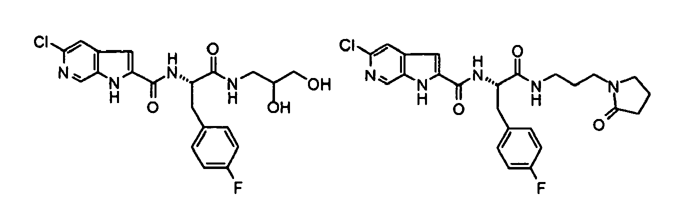 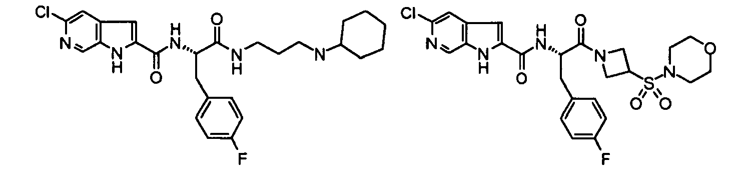 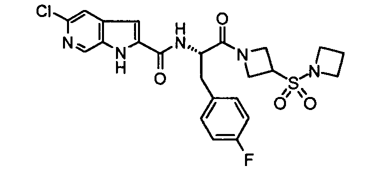 or a pharmaceutically acceptable salt thereof.

19. A compound according to claim 1 selected from the following: 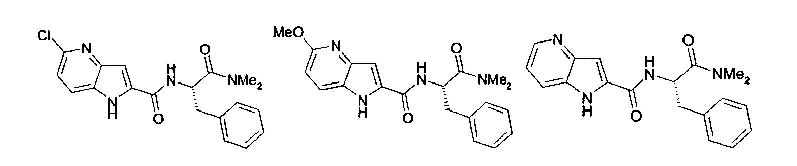 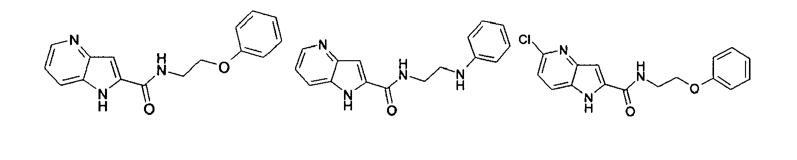 or a pharmaceutically acceptable salt thereof.

20. A compound according to claim 1 selected from the following: 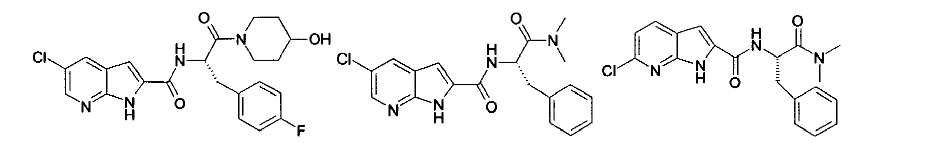 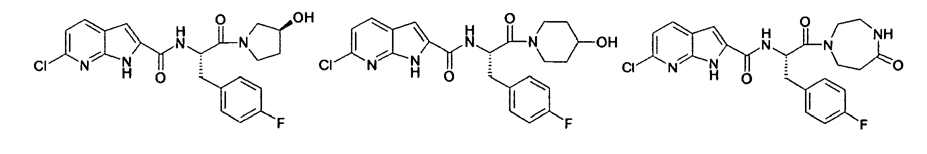 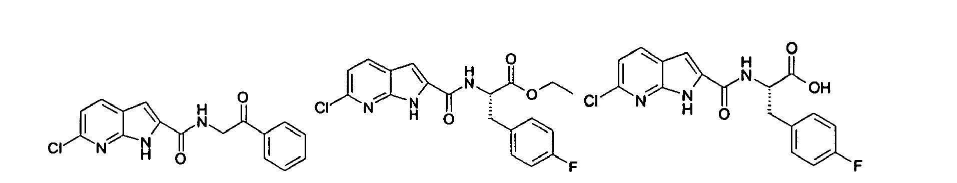 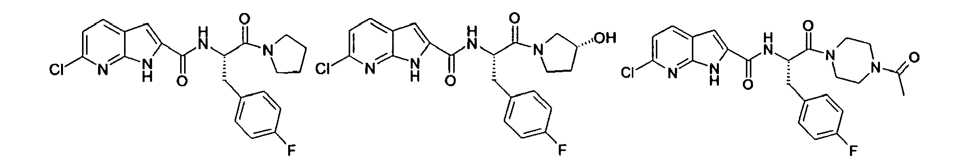 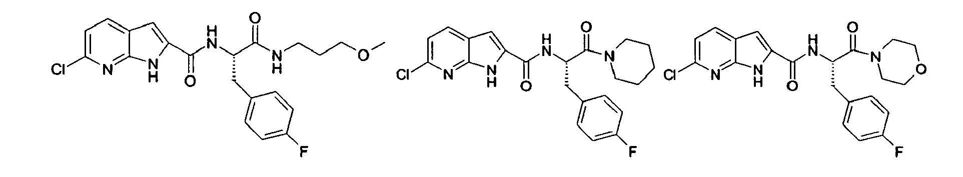 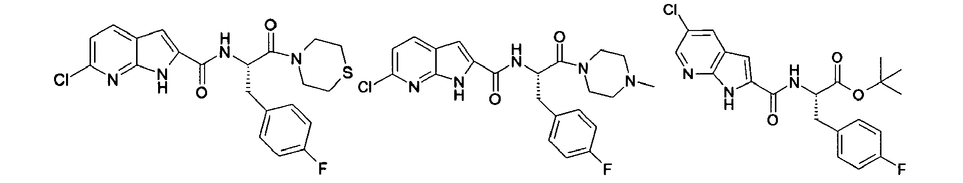 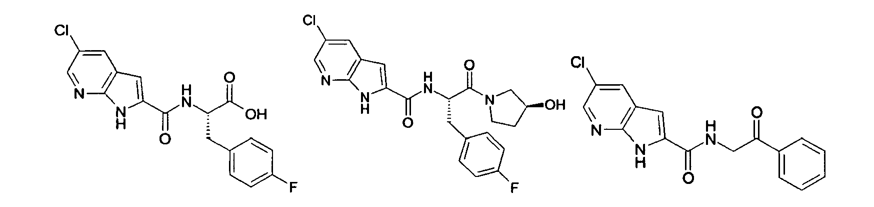 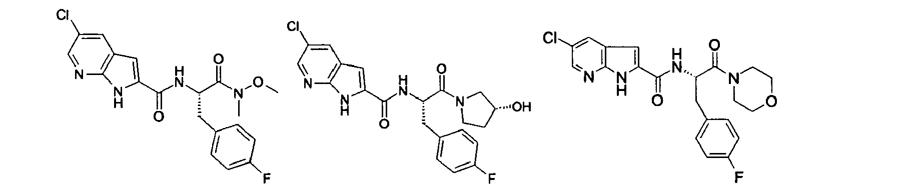 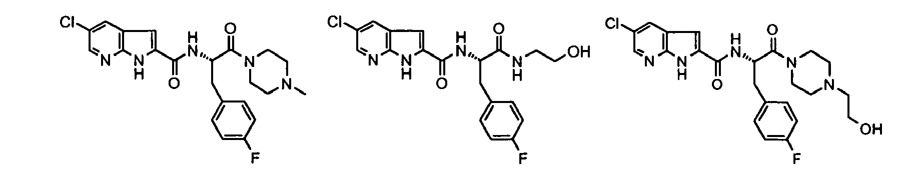 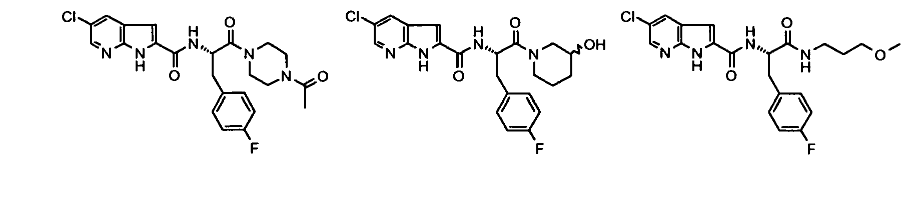 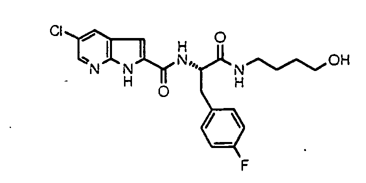 or a pharmaceutically acceptable salt thereof.

21. A compound according to claim 1 selected from the following: 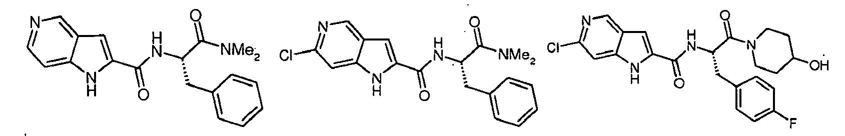 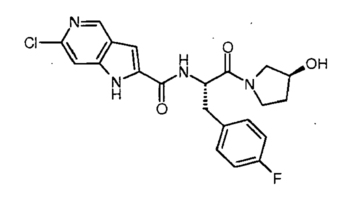 or a pharmaceutically acceptable salt thereof.

22. The compound according to claim 1: 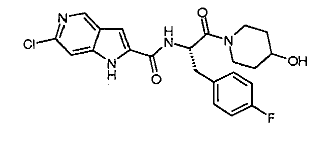 or a pharmaceutically acceptable salt thereof.

23. A composition comprising a compound according to any one of claims 1 to 22, or a stereoisomer, or a pharmaceutically acceptable salt thereof; and a pharmaceutically acceptable carrier.

24. A compound according to any one of claims 1 to 22, or a stereoisomer, or a pharmaceutically acceptable salt thereof, for use in the prophylactic or therapeutic treatment of hyperglycemia or diabetes.

25. A compound according to any one of claims 1 to 22, or a stereoisomer, or a pharmaceutically acceptable salt thereof, for use in the prevention of diabetes in a human demonstrating pre-diabetic hyperglycemia or impaired glucose tolerance.

26. A compound according to any one of claims 1 to 22, or a stereoisomer, or a pharmaceutically acceptable salt thereof, for use in the prophylactic or therapeutic treatment of hypercholesterolemia, hyperinsulinemia, hyperlipidemia, atherosclerosis or myocardial ischemia.

27. A compound according to any one of claims 1 to 22, or a stereoisomer; or a pharmaceutically acceptable salt thereof, for use in cardioprotection.

28. The use of a compound according to any one of claims 1 to 22, or a stereoisomer, or a pharmaceutically acceptable salt thereof, in the manufacture of a medicament for the treatment of a disease or condition as defined in any one of claims 24 to 27.

29. A process for the production of a compound of Formula (I) according to claim 1, comprising:
a) 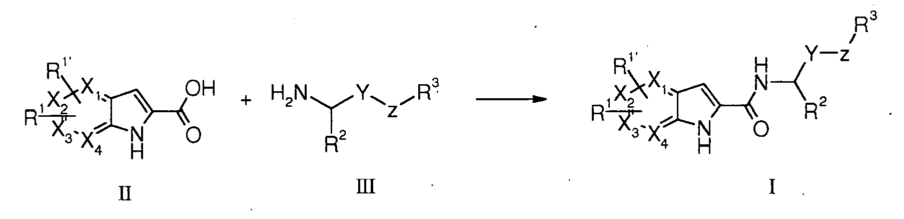 coupling a pyrrolopyridine-2-carboxylic acid of Formula (II), or a protected or activated derivative thereof, with an amine of Formula (III); or
b) 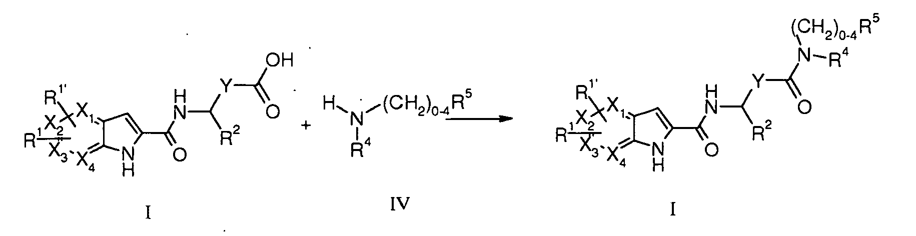 for compounds of Formula (I) wherein Z is C=O and R³ is -NR⁴R⁵ or -NR⁴(-C₁₋₄alkylR⁵), coupling a carboxylic acid of Formula (I), or a protected or activated derivative thereof, wherein Z is absent and R³ is -CO₂H, with an amine of Formula (IV); or
c) 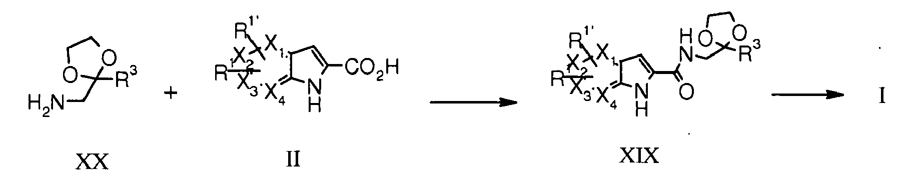 for compounds of Formula (III) wherein R² is H, Y is a direct bond, Z is - C(O)- and R³ is -aryl or -hetaryl coupling of a compound of Formula (XX) with a compound of Formula (II) under standard coupling conditions to give a compound of Formula (XIX), followed by removal of the ketal group in the presence of acid; or
d) 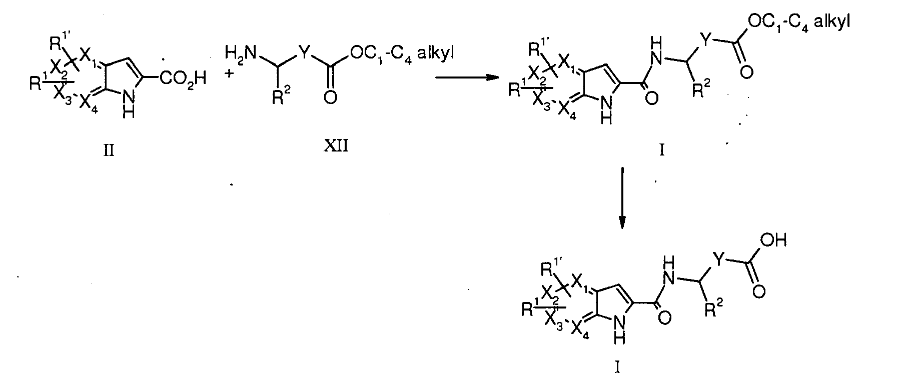 for compounds of Formula (I) wherein Z is C=O and R³ is C₁₋₄alkoxy, coupling a compound of Formula (II), or a protected or activated derivative thereof, and a compound of Formula (XII); or
e) for compounds of Formula (I) wherein Z is absent and R³ is -CO₂H, ester hydrolysis of compounds of Formula (I) where Z is C=O and R³ is a C₁₋₄alkoxy group.

30. A compound selected from:
5-Chloro-1*H*-pyrrolo[3,2-b]pyridine-2-carboxylic acid;
5-Bromo-1*H*-pyrrolo[3,2-b]pyridine-2-carboxylic acid;
5-Cyano-1*H*-pywolo[3,2-b]pyridine-2-carboxylic acid;
5-Methoxy-1*H*-pyrrolo[3,2-b]pyridine-2-carboxylic acid;
1*H*-Pyrrolo[3,2-c]pyridine-2-carboxylic acid;
6-Chloro-1*H*-pyrrolo[3,2-c]pyridine-2-carboxylic acid;
6-Cyano-1*H*-pyrrolo[3,2-c]pyridine-2-carboxylic acid;
5-Chloro-1*H*-pyrrolo[2,3-c]pyridine-2-carboxylic acid;
5-Bromo-1*H*-pyrrolo[2,3-c]pyridine-2-carboxylic acid;
5-Ethynyl-1*H*-pyrrolo[2,3-c]pyridine-2-carboxylic acid;
5-Cyano-1*H*-pyrrolo[2,3-c]pyridine-2-carboxylic acid;
5-Methyl-1*H*-pyrrolo[2,3-c]pyridine-2-carboxylic acid;
5-Chloro-1*H*-pyrrolo[2,3-b]pyridine-2-carboxylic acid;
6-Chloro-1*H*-pyrrolo[2,3-b]pyridine-2-carboxylic acid; and
6-Cyano-1*H*-pyrrolo[2,3-b]pyridine-2-carboxylic acid;
or a C₁₋₄alkyl ester of any one thereof.

31. A compound of Formula (XIX): 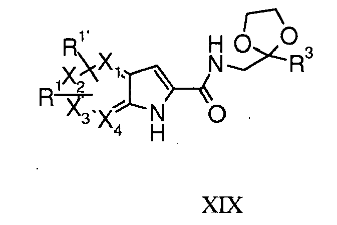 wherein R¹, R^{1'}, X₁, X₂, X₃, and X₄ are as defined in claim 1 and R³ is -aryl or -hetaryl.

32. 4(*S*)-(4-Fluorobenzyl)oxazolidine-2,5-dione.

## Patentansprüche

1. Verbindungen der Formel (I): 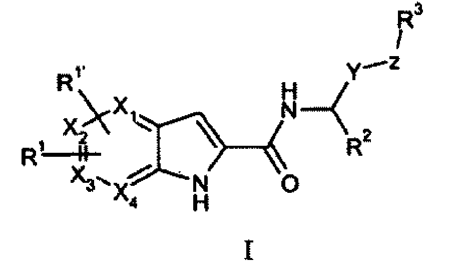 und deren Stereoisomere und deren pharmazeutisch unbedenkliche Salze, wobei:
einer der Reste X₁, X₂, X₃ und X₄ für N steht und die anderen für C stehen;
R¹ und R^{1'} jeweils unabhängig voneinander für Wasserstoff, Halogen, Hydroxy, Cyano, C₁₋₄-Alkyl, C₁₋₄-Alkoxy, Fluormethyl, Difluormethyl, Trifluormethyl, Ethenyl oder Ethinyl stehen;
R² für Wasserstoff, C₁₋₄-Alkyl, COOR⁶, COR⁶, C₁₋₄-Alkoxy-C₁₋₄-alkyl-, Hydroxy-C₁₋₄-alkyl-, Cycloalkyl, Cycloalkyl-C₁₋₄-alkyl-, Aryl, Aryl-C₁₋₄-alkyl-, Hetaryl oder Hetaryl-C₁₋₄-alkyl- steht, wobei die Aryl- und Hetarylringe gegebenenfalls durch 1-2 unabhängig ausgewählte Halogen-, Cyano-, C₁₋₄-Alkyl-, C₁₋₄-Alkoxy-, -NR¹³R¹⁴-, -SO₂-C₁₋₄-Alkyl-, -SO₂NR¹³R¹⁴-, Hydroxyl-, Fluormethyl-, Difluormethyl- oder Trifluormethylsubstituenten substituiert sind;
Y für eine direkte Bindung, C₁₋₂-Alkyl oder -CH(OH)- steht;
Z für CH₂, -C(O)-, -O-, >NH, >N(C₁₋₄-Alkyl), >N(C₃₋₆-Cycloalkyl) steht oder fehlt;
wobei jedoch, wenn Y für -CH(OH)- steht, Z oder R³ über eine Kohlenstoff-Kohlenstoff-Bindung an Y gebunden ist;
R³ für Wasserstoff, -COOH, -COO-C₁₋₄-Alkyl, C₁₋₄-Alkoxy, C₁₋₄-Alkyl, Aryl-C₁₋₄-alkylthio-, Aryl, -C₁₋₄-Alkylaryl, Hetaryl, -C₁₋₄-Alkylhetaryl, Cycloalkyl, -C₁₋₄-Alkylcycloalkyl, Heterocyclyl oder -C₁₋₄-Alkylheterocyclyl steht, wobei die Ringe jeweils gegebenenfalls durch 1-3 unabhängig ausgewählte Halogen-, Cyano-, C₁₋₄-Alkyl-, Fluormethyl-, Difluormethyl-, Trifluormethyl-, -NHC(O)O-(C₁₋₄-Alkyl)-, -C₁₋₄-Alkyl-NHC(O)O-(C₁₋₄-alkyl)-, -NR⁷R⁸-, -C₁₋₄-Alkyl-NR⁷R⁸-, -C(O)R⁹-, C₁₋₄-Alkoxy-, C₁₋₄-Alkoxy-C₁₋₄-alkyl-, -COOH-, -COO-C₁₋₄-Alkyl-, -NHC(O)R⁹-, -C₁₋₄-Alkyl-NHC(O)R⁹-, -C(O)N(R¹⁰)₂-, -C₁₋₄-Alkyl-C(O)N(R¹⁰)₂-, -C₁₋₄-Alkoxy-C₁₋₄-alkoxy-, Hydroxyl-, Hydroxy-C₁₋₄-alkyl-, -NHSO₂R¹⁰-, -SO₂(C₁₋₄-Alkyl)-, -SO₂NR¹¹R¹²-, 5- oder 6-gliedrige Heterocyclyl-, Phenyl-, Phenyl-C₁₋₂-alkoxy- oder Phenyl-C₁₋₂-alkylsubstituenten substituiert sind, wobei Phenyl gegebenenfalls durch 1-2 unabhängig ausgewählte Halogen-, Cyano-, C₁₋₄-Alkyl-, C₁₋₄-Alkoxy-, -NR¹³R¹⁴-, -SO₂-C₁₋₄-Alkyl-, -SO₂NR¹³R¹⁴-, Hydroxyl-, Fluormethyl-, Difluormethyl- oder Trifluormethylsubstituenten substituiert ist, oder zwei Bindungen an einem Ringkohlenstoff der Heterocyclylgruppe gegebenenfalls einen Oxo-(=O)-Substituenten bilden können;
oder R³ für -NR⁴R⁵ oder -NR⁴(-C₁₋₄-Alkyl-R⁵) steht;
R⁴ für Wasserstoff, C₁₋₃-Alkyl, -C₂₋₃-Alkyl-NR⁷R⁸, C₃₋₆-Cycloalkyl, gegebenenfalls substituiert durch Hydroxy oder Hydroxy-C₁₋₄-alkyl-, weiterhin gegebenenfalls substituiert durch Hydroxyl, C₁₋₂-Alkoxy-C₂₋₄-alkyl- oder C₁₋₂-Alkyl-S(O)ₙ-C₂₋₃-alkyl-, steht;
n für 0, 1 oder 2 steht;
R⁵ für Wasserstoff, Hydroxy-C₂₋₃-alkyl-, C₁₋₂-Alkoxy, C₁₋₂-Alkoxy-C₁₋₄-alkyl- oder Aryl, Hetaryl oder Heterocyclyl steht;
wobei ein heterocyclischer, stickstoffhaltiger R⁵-Ring gegebenenfalls am Ringstickstoff durch C₁₋₄-Alkyl, Benzyl, Benzoyl, C₁₋₄-Alkyl-C(O)-, -SO₂-C₁₋₄-Alkyl, -SO₂NR¹³R¹⁴, C₁₋₄-Alkoxycarbonyl oder Aryl-(C₁₋₄-alkoxy)carbonyl monosubstituiert ist; und wobei die R⁵-Ringe gegebenenfalls an einem Ringkohlenstoff durch Halogen, Cyano, C₁₋₄-Alkyl-C(O) -, C₁₋₄-Alkyl-SO₂-, C₁₋₄-Alkyl, C₁₋₄-Alkoxy, Hydroxy, -NR¹³R¹⁴, Hydroxy-C₁₋₄-alkyl-, Carbamoyl oder C₁₋₄-Alkylcarbamoyl-monosubstituiert sind, mit der Maßgabe, daß kein quaternisierter Stickstoff eingeschlossen ist; oder zwei Bindungen an einem Kohlenstoffatom der Heterocyclylgruppe gegebenenfalls einen Oxo-(=O)-Substituenten bilden können;
R⁶ für C₁₋₄-Alkyl, Aryl oder Hetaryl steht;
R⁷ und R⁸ unabhängig voneinander für Wasserstoff, C₁₋₄-Alkyl, C₃₋₆-Cycloalkyl oder CO-(C₁₋₄-Alkyl) stehen;
R⁹ für C₁₋₄-Alkyl oder C₃₋₆-Cycloalkyl steht;
R¹⁰ für Wasserstoff, C₁₋₄-Alkyl oder C₃₋₆-Cycloalkyl steht;
R¹¹ und R¹² unabhängig voneinander für Wasserstoff oder C₁₋₄-Alkyl stehen oder zusammen mit dem Stickstoff, an den sie gebunden sind, einen 4- bis 6gliedrigen Heterocyclus bilden können;
R¹³ und R¹⁴ unabhängig voneinander für Wasserstoff oder C₁₋₄-Alkyl stehen; und
wobei die drei Komponenten -Y-Z-R³ nicht über Stickstoff-Sauerstoff-, Stickstoff-Stickstoff- oder Stickstoff-Halogen-Bindungen miteinander verbunden sind;
mit der Maßgabe, daß es sich bei der Verbindung nicht um 2R,4S,5S-6-Cyclohexyl-5-(6'-azaindol-2'-ylcarbonylamino)-2-(2'-methylpropyl)-gamma-hexanolacton handelt.

2. Verbindungen nach Anspruch 1 und deren Stereoisomere und deren pharmazeutisch unbedenkliche Salze, wobei X₃ für N steht.

3. Verbindungen nach Anspruch 1 und deren Stereoisomere und deren pharmazeutisch unbedenkliche Salze, wobei X₁ für N steht.

4. Verbindungen nach Anspruch 1 und deren Stereoisomere und deren pharmazeutisch unbedenkliche Salze, wobei X₂ für N steht.

5. Verbindungen nach Anspruch 1 und deren Stereoisomere und deren pharmazeutisch unbedenkliche Salze, wobei X₄ für N steht.

6. Verbindungen nach einem der vorhergehenden Ansprüche und deren Stereoisomere und deren pharmazeutisch unbedenkliche Salze, wobei R¹ und R^{1'} jeweils unabhängig voneinander für Halogen, Cyano oder Wasserstoff stehen.

7. Verbindungen nach einem der vorhergehenden Ansprüche und deren Stereoisomere und deren pharmazeutisch unbedenkliche Salze, wobei einer der Reste R¹ und R^{1'} für Wasserstoff und der andere für eine 5-Halogen- oder 5-Cyanogruppe steht.

8. Verbindungen nach einem der vorhergehenden Ansprüche und deren Stereoisomere und deren pharmazeutisch unbedenkliche Salze, wobei Y für eine direkte Bindung oder C₁₋₂-Alkyl steht.

9. Verbindungen nach einem der vorhergehenden Ansprüche, wobei Z für -C(O)- steht.

10. Verbindungen nach einem der Ansprüche 1, 2 oder 6 bis 9 und deren Stereoisomere und deren pharmazeutisch unbedenkliche Salze, wobei
X₃ für N steht;
Y für eine direkte Bindung oder C₁₋₂-Alkyl steht; und
Z für -C(O)- steht.

11. Verbindungen nach einem der vorhergehenden Ansprüche und deren Stereoisomere und deren pharmazeutisch unbedenkliche Salze, wobei Y für eine direkte Bindung steht.

12. Verbindungen nach einem der vorhergehenden Ansprüche und deren Stereoisomere und deren pharmazeutisch unbedenkliche Salze, wobei R² für Wasserstoff, C₁₋₄-Alkyl, Aryl oder Aryl-C₁₋₄-alkyl-steht, wobei der Arylring gegebenenfalls durch 1-2 unabhängig ausgewählte Halogen-, Cyano-, C₁₋₄-Alkyl-, C₁₋₄-Alkoxy-, -NR¹³R¹⁴-, -SO₂-C₁₋₄-Alkyl-, -SO₂NR¹³R¹⁴-, Hydroxyl-, Fluormethyl-, Difluormethyl- oder Trifluormethylsubstituenten substituiert ist.

13. Verbindungen nach Anspruch 12 und deren Stereoisomere und deren pharmazeutisch unbedenkliche Salze, wobei R² für gegebenenfalls durch 1-2 Halogensubstituenten substituiertes Benzyl steht.

14. Verbindungen nach einem der vorhergehenden Ansprüche und deren Stereoisomere und deren pharmazeutisch unbedenkliche Salze, wobei R³ für Heterocyclyl, -C₁₋₄-Alkylheterocyclyl, gegebenenfalls substituiert durch 1-3 unabhängig ausgewählte Halogen-, Cyano-, C₁₋₄-Alkyl-, Fluormethyl-, Difluormethyl-, Trifluormethyl-, -NHC(O)O-(C₁₋₄-Alkyl)-, -C₁₋₄-Alkyl-NHC(O)O-(C₁₋₄-alkyl)-, -NR⁷R⁸-, -C₁₋₄-Alkyl-NR⁷R⁸-, -C(O)R⁹, C₁₋₄-Alkoxy-, C₁₋₄-Alkoxy-C₁₋₄-alkyl-, -COOH-, -COO-C₁₋₄-Alkyl-, -NHC(O)R⁹-, -C₁₋₄-Alkyl-NHC(O)R⁹-, -C(O)N(R¹⁰)₂-, -C₁₋₄-Alkyl-C(O)N(R¹⁰)₂-, -C₁₋₄-Alkoxy-C₁₋₄-alkoxy-, Hydroxyl-, Hydroxy-C₁₋₄-alkyl-, -NHSO₂R¹⁰ -, -SO₂(C₁₋₄-Alkyl)-, -SO₂NR¹¹R¹²-, 5- oder 6gliedrige Heterocyclyl-, Phenyl-, Phenyl-C₁₋₂-alkoxy- oder PhenylC₁₋₂-alkylsubstituenten, steht, wobei Phenyl gegebenenfalls substituiert ist durch 1-2 unabhängig ausgewählte Halogen-, Cyano-, C₁₋₄-Alkyl-, C₁₋₄-Alkoxy-, -NR¹³R¹⁴-, -SO₂-C₁₋₄-Alkyl-, -SO₂NR¹³R¹⁴-, Hydroxyl-, Fluormethyl-, Difluormethyl- oder Trifluormethylsubstituenten, oder zwei Bindungen an einem Ringkohlenstoff der Heterocyclylgruppe können gegebenenfalls einen Oxo-(=O)-Substituenten bilden; oder R³ steht für -NR⁴R⁵ oder -NR⁴(-C₁₋₄-Alkyl-R⁵).

15. Verbindungen nach Anspruch 14, wobei R³ für eine einen gegebenenfalls substituierten Stickstoff enthaltende Heterocyclylgruppe steht, die über ein Ringstickstoffatom an Z gebunden ist; oder R³ steht für -NR⁴R⁵ oder -NR⁴(-C₁₋₄-Alkyl-R⁵).

16. Verbindungen nach Anspruch 15, wobei R³ für eine gegebenenfalls substituierte 4- bis 8gliedrige stickstoffhaltige Heterocyclylgruppe steht.

17. Verbindungen nach Anspruch 16 und deren Stereoisomere und deren pharmazeutisch unbedenkliche Salze, wobei R³ für Pyrrolidin-1-yl oder Piperidin-1-yl, gegebenenfalls substituiert durch Hydroxy, steht.

18. Verbindungen nach Anspruch 1, ausgewählt aus den folgenden: 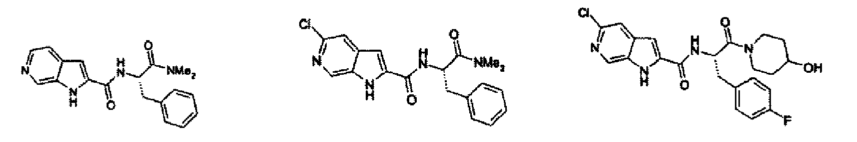 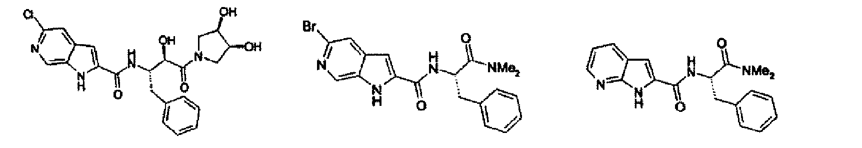 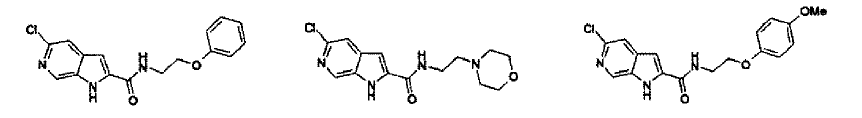 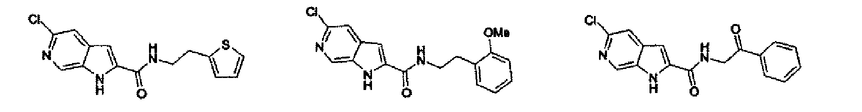 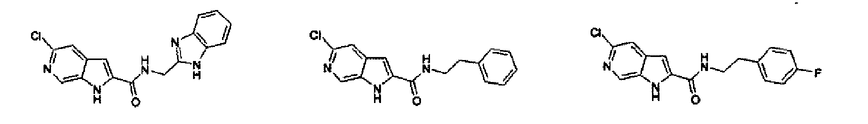 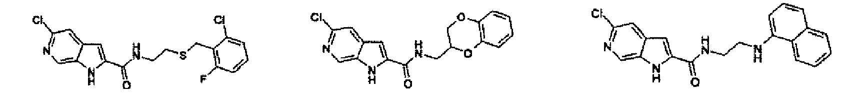 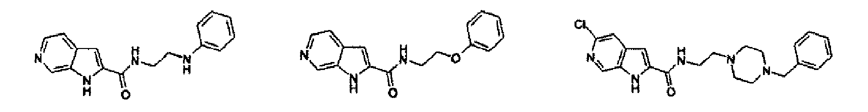 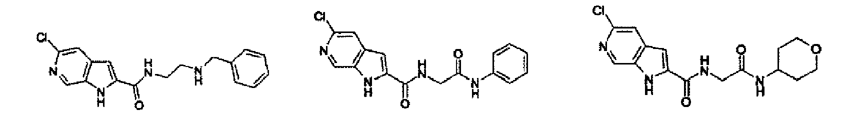 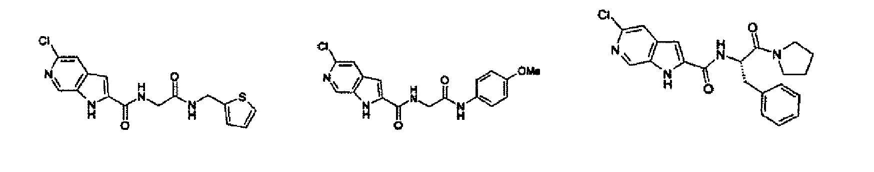 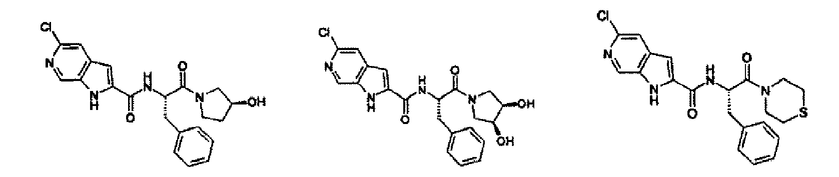 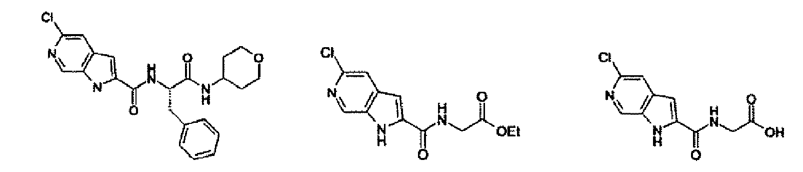 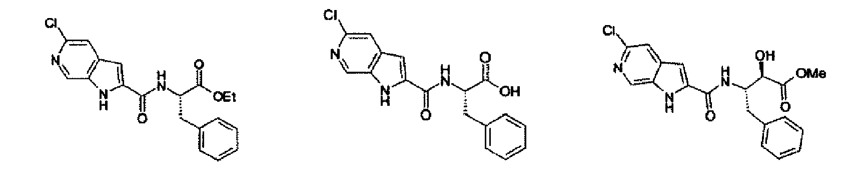 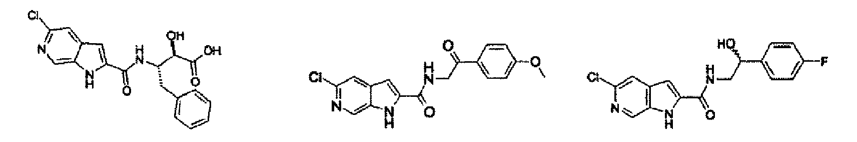 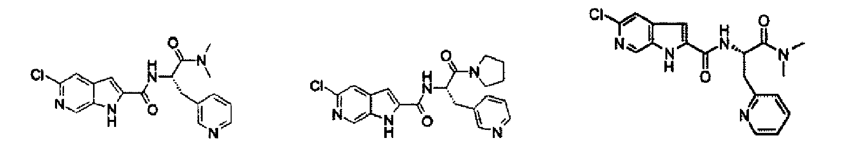 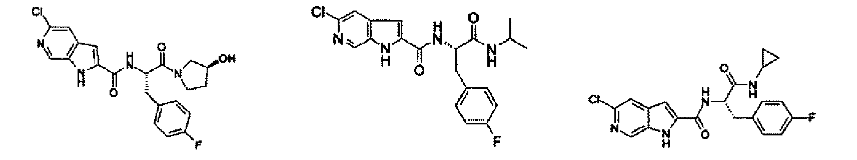 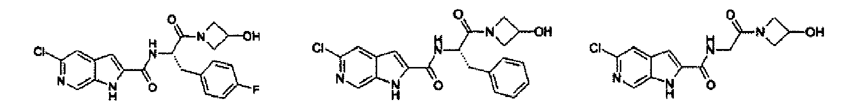 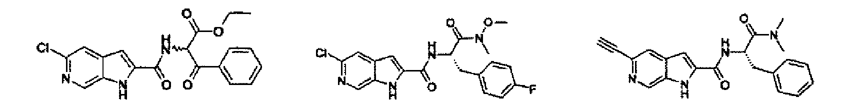 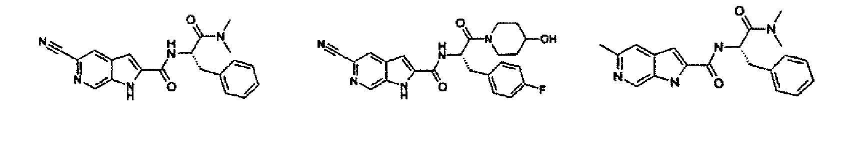 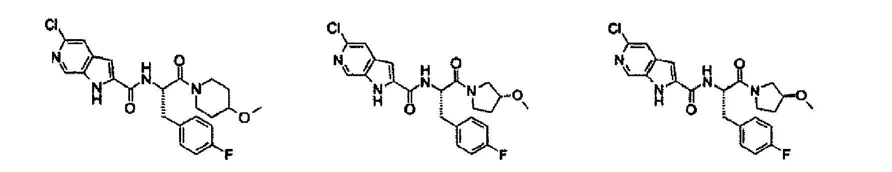 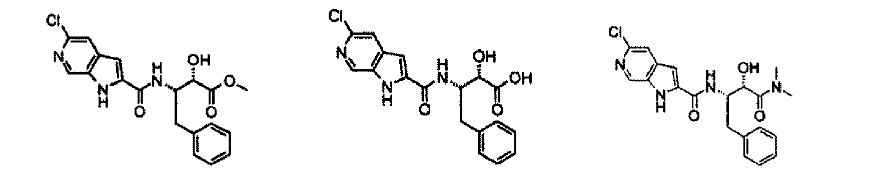 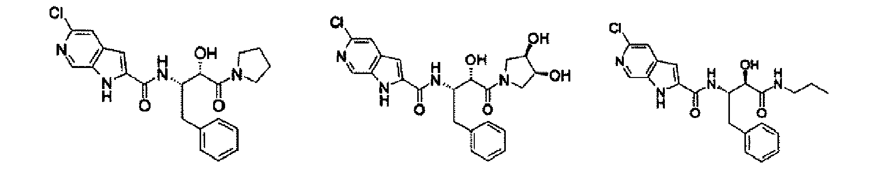 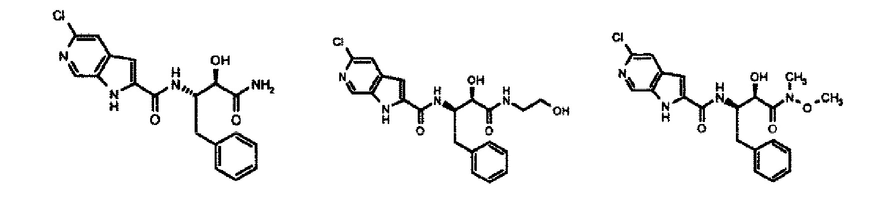 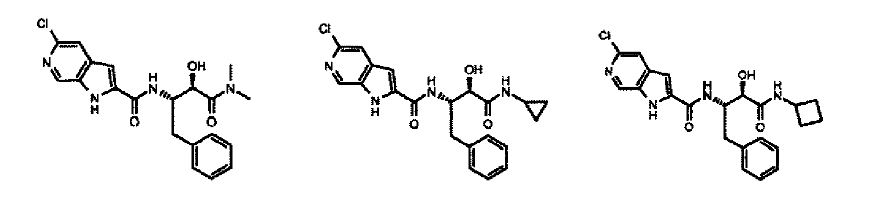 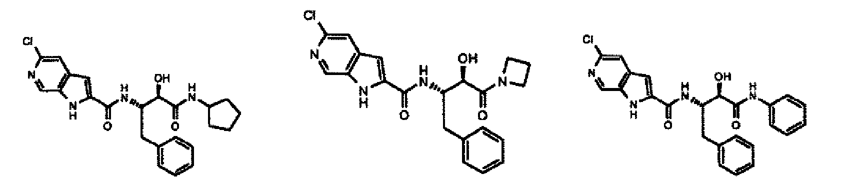 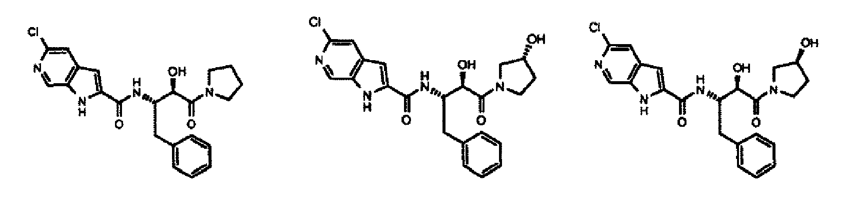 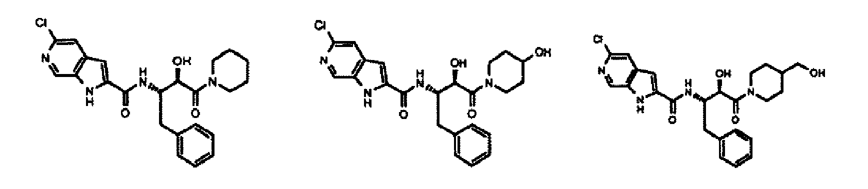 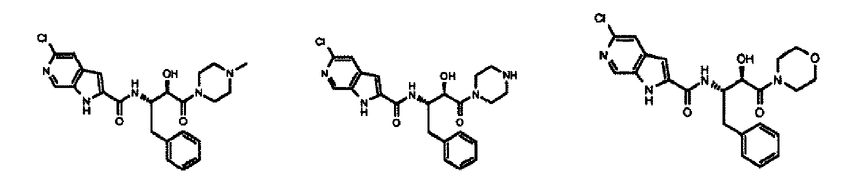 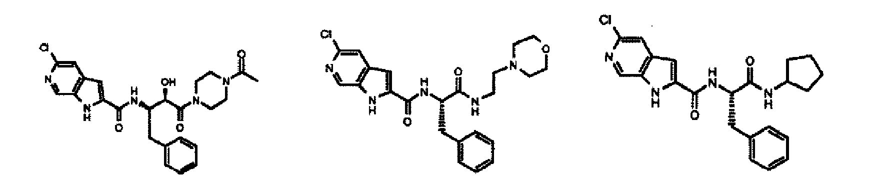 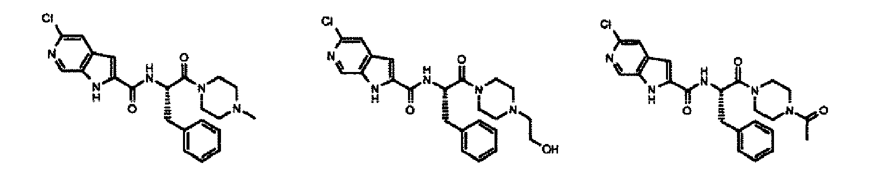 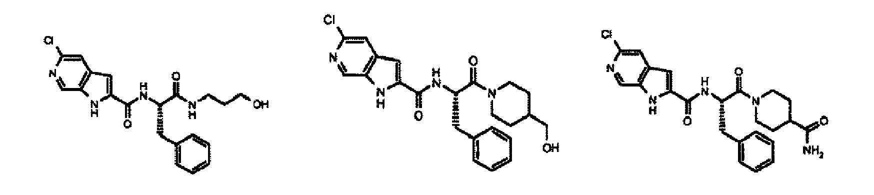 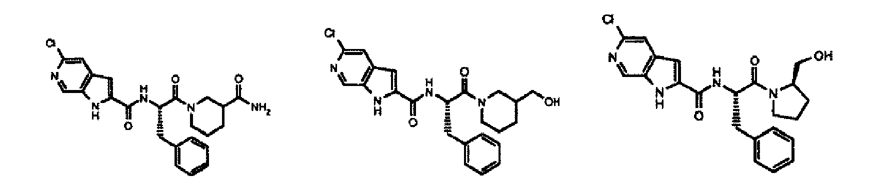 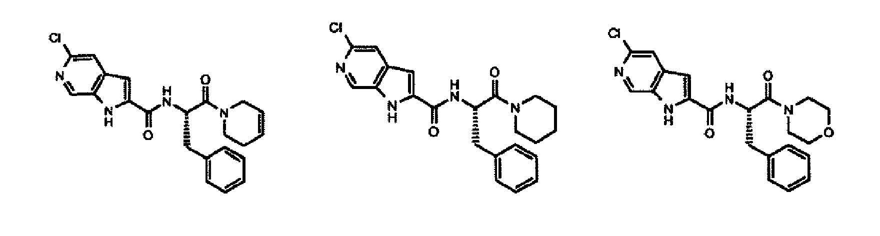 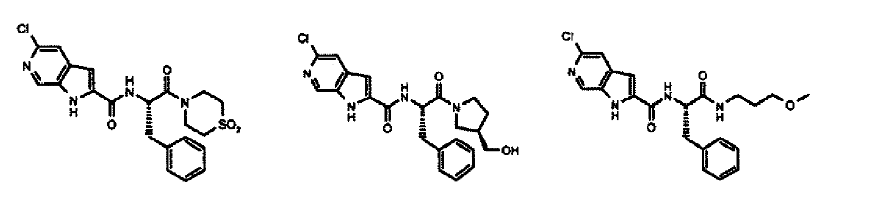 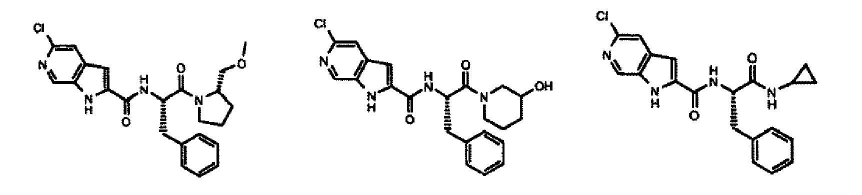 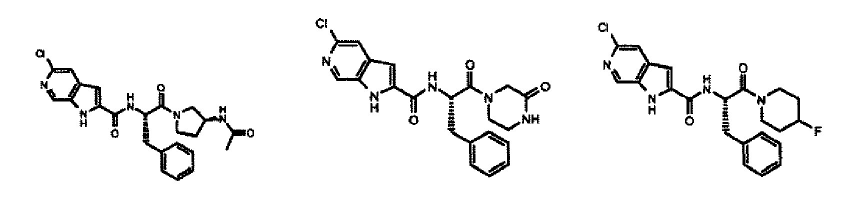 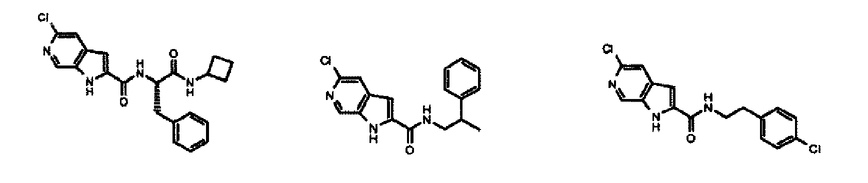 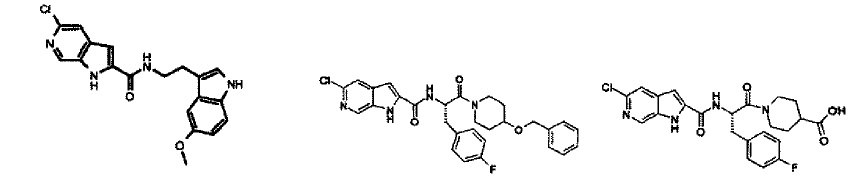 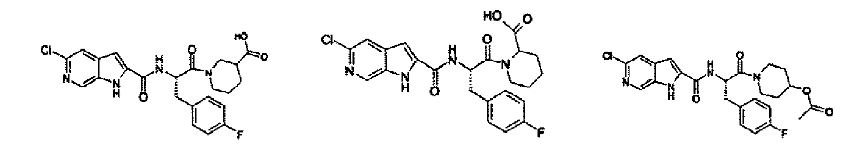 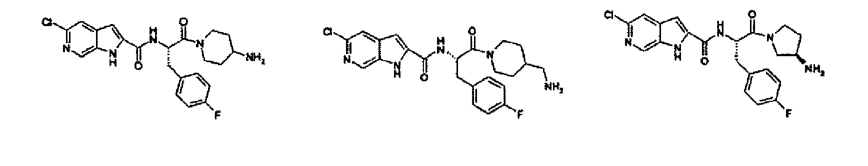 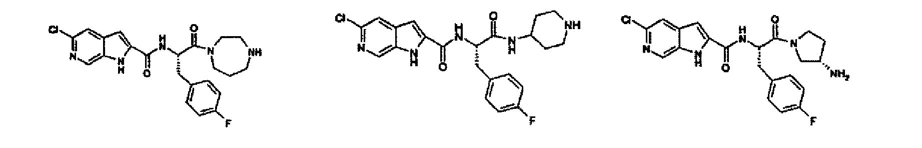 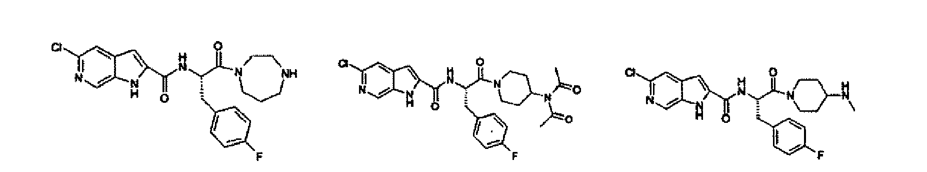 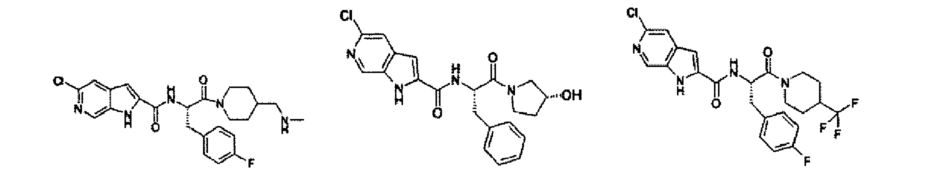 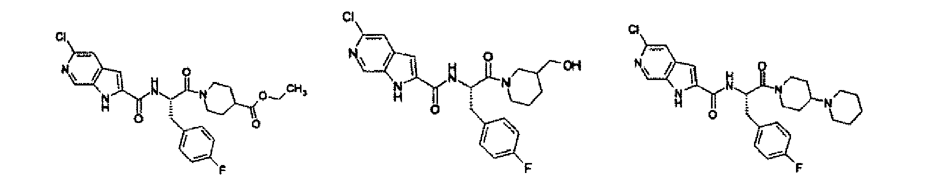 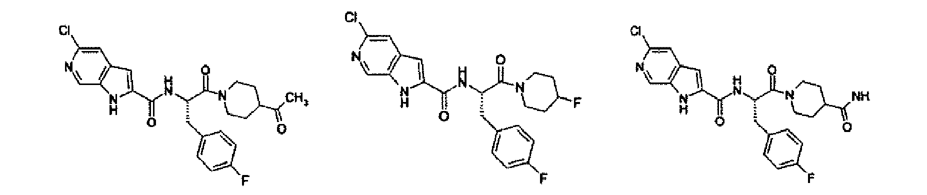 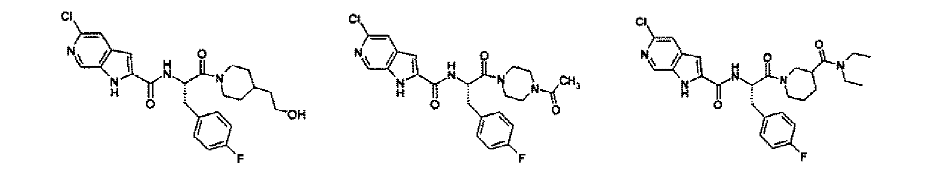 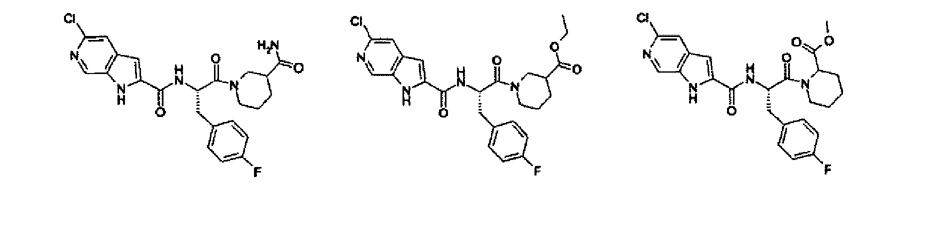 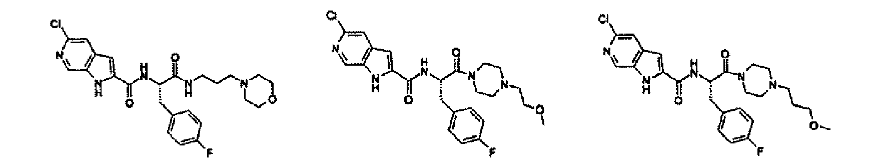 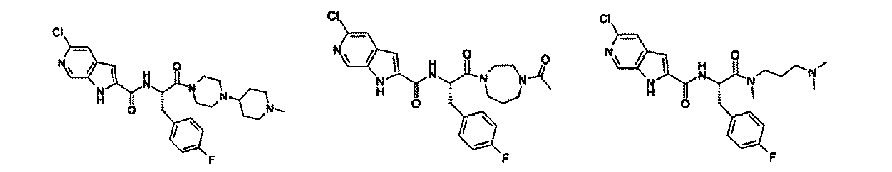 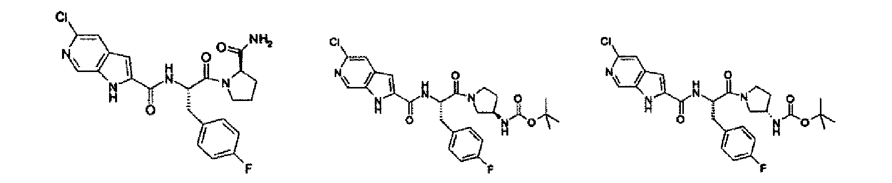 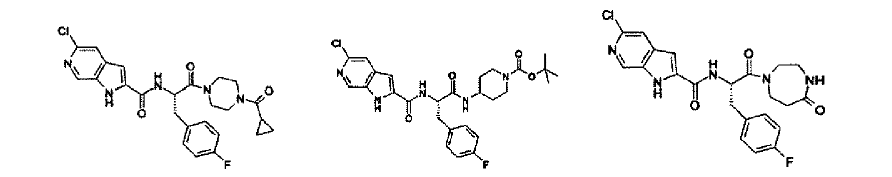 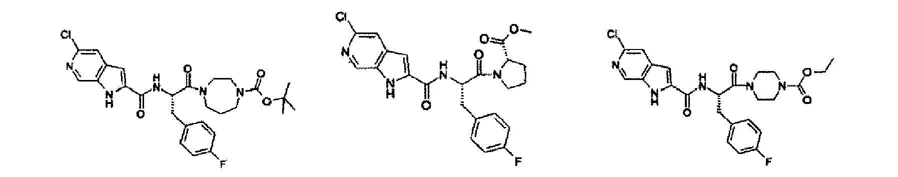 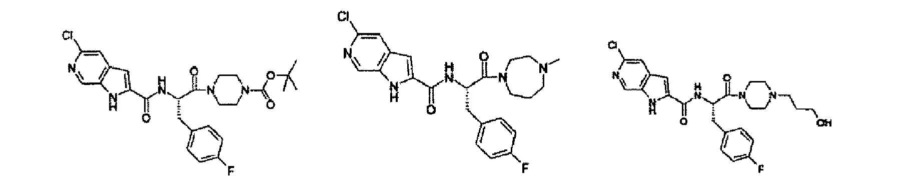 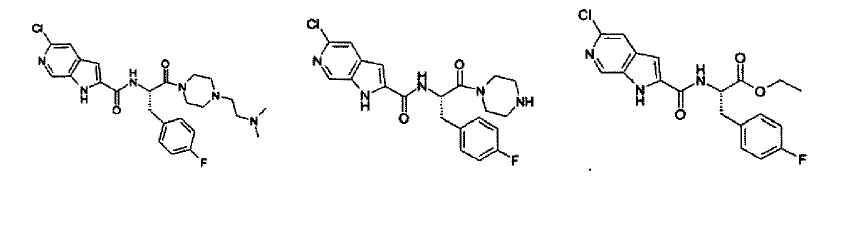 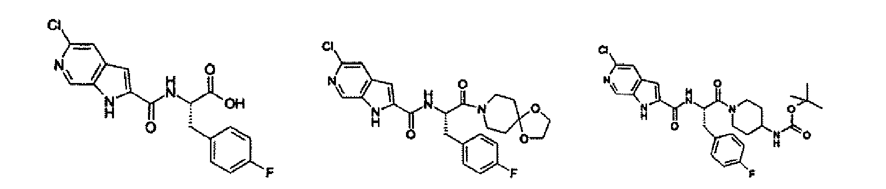 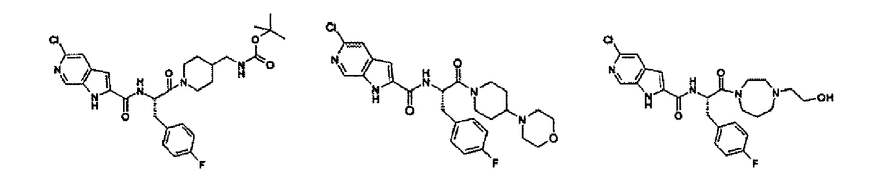 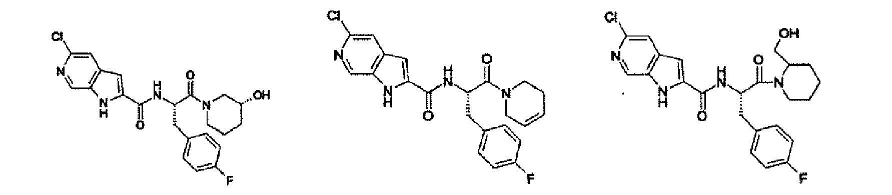 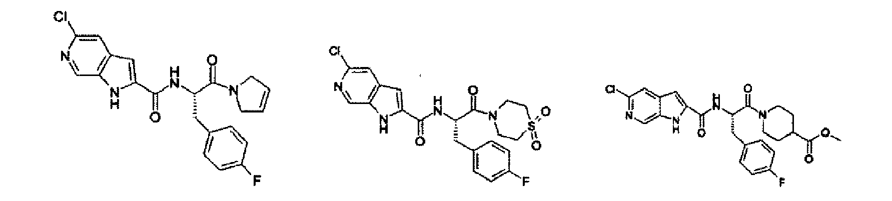 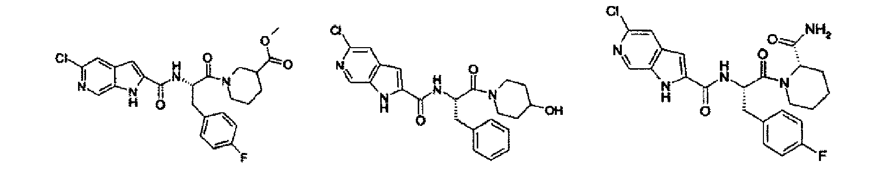 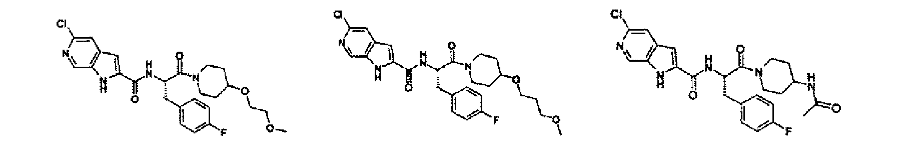 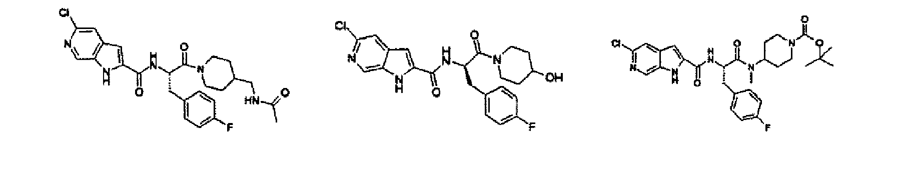 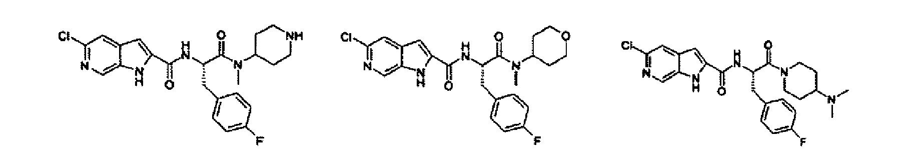 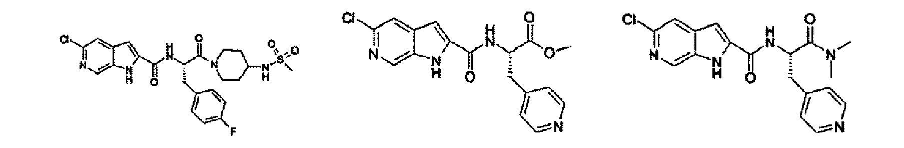 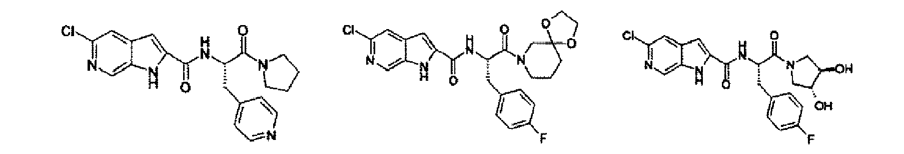 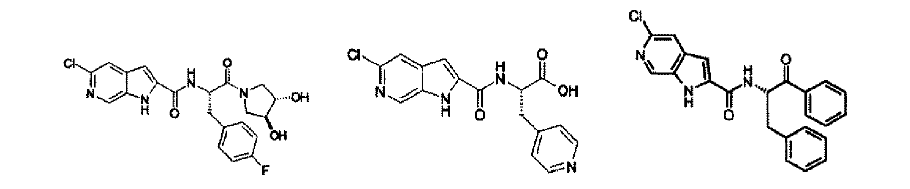 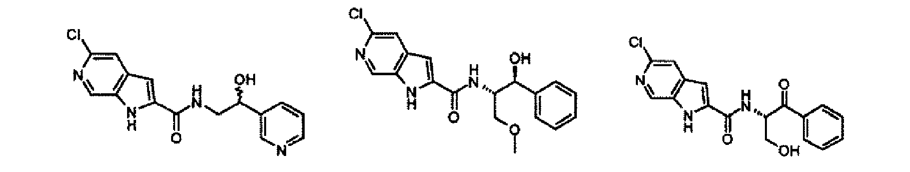 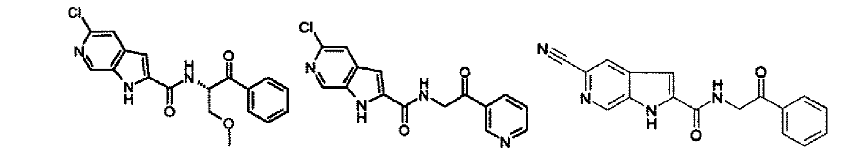 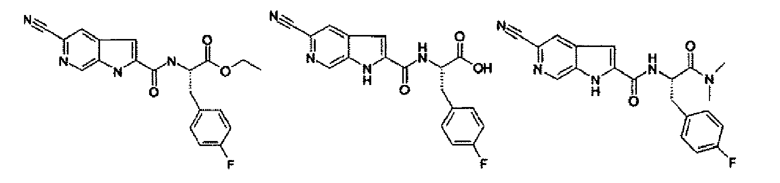 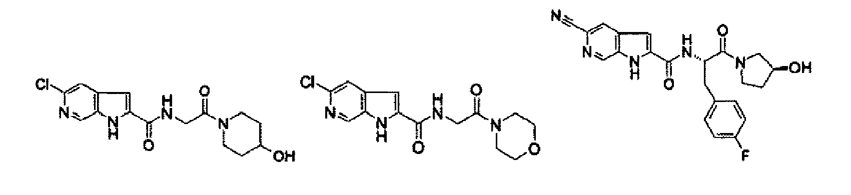 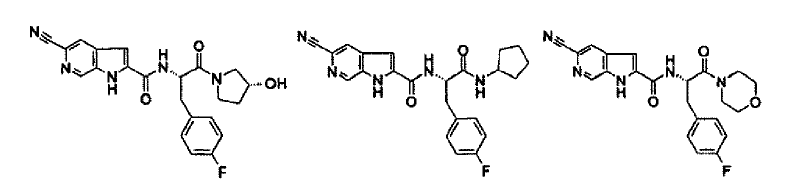 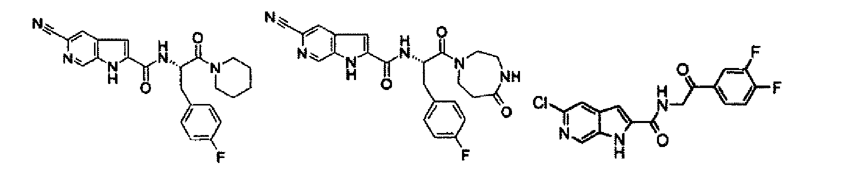 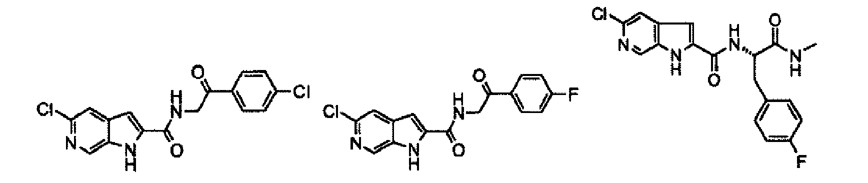 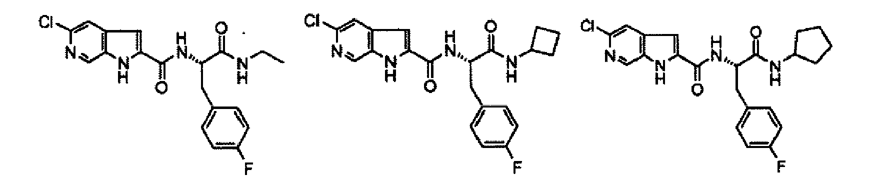 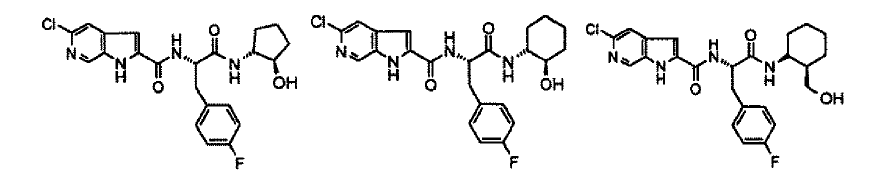 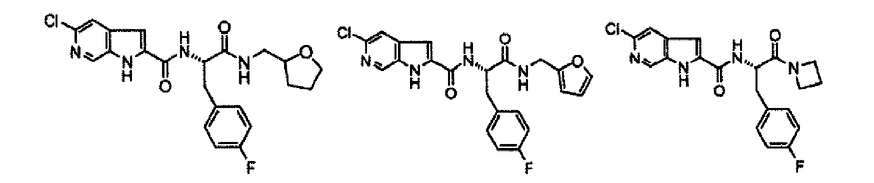 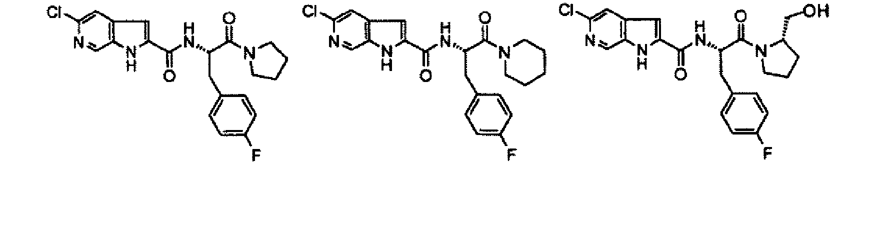 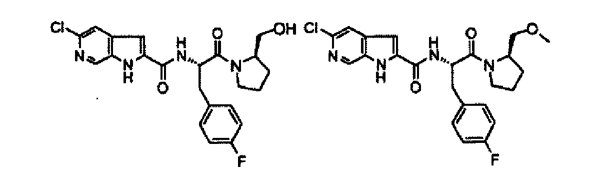 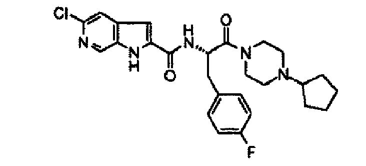 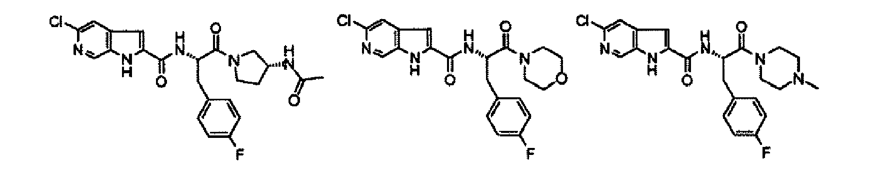 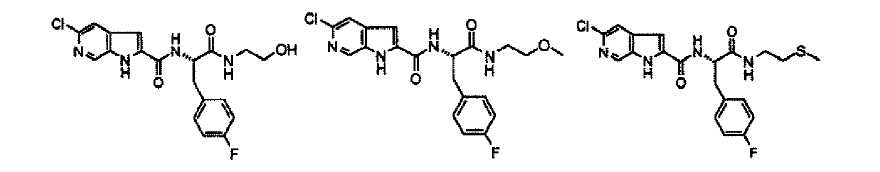 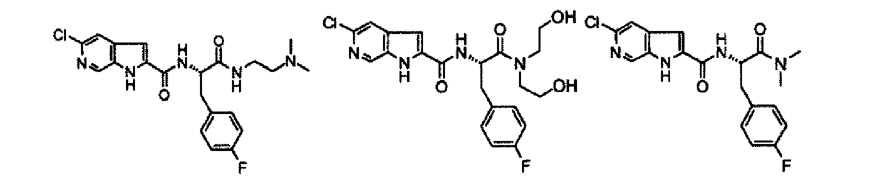 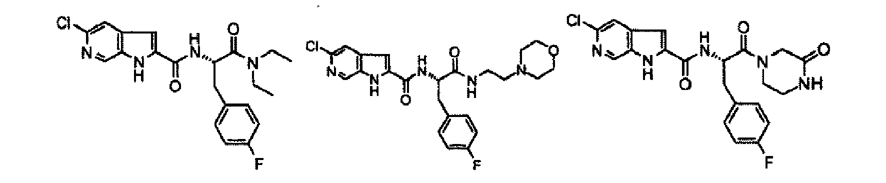 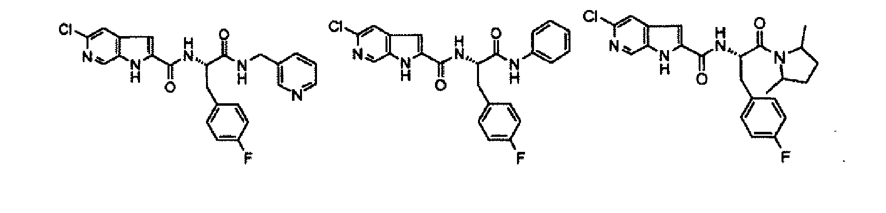 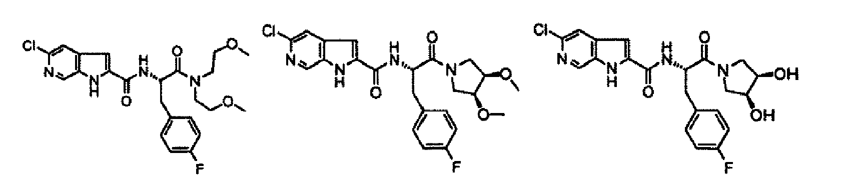 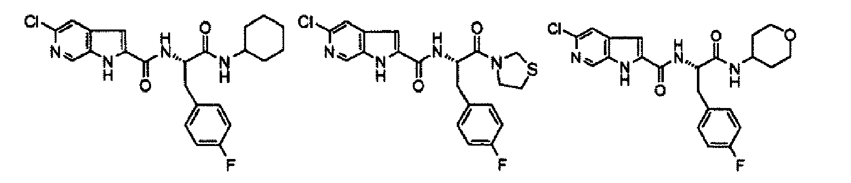 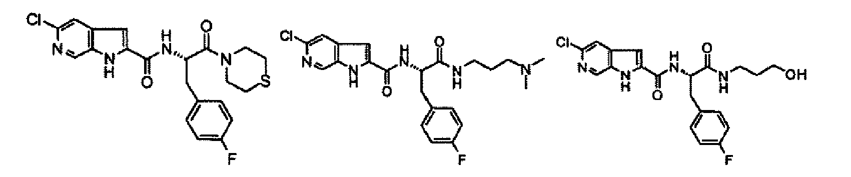 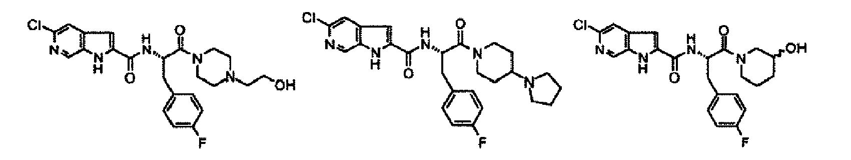 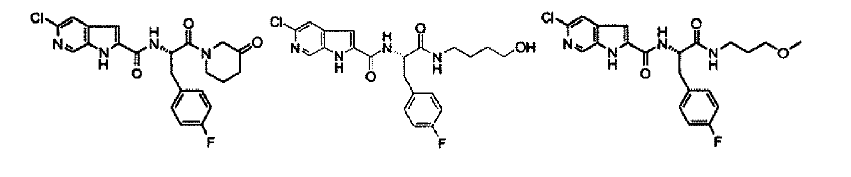 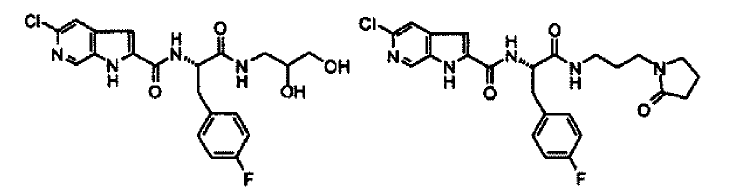 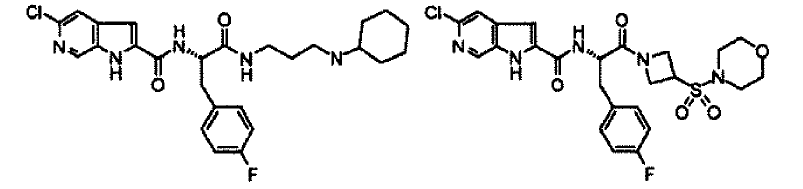 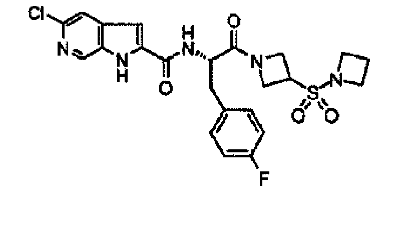 und deren pharmazeutisch unbedenkliche Salze.

19. Verbindungen nach Anspruch 1, ausgewählt aus den folgenden: 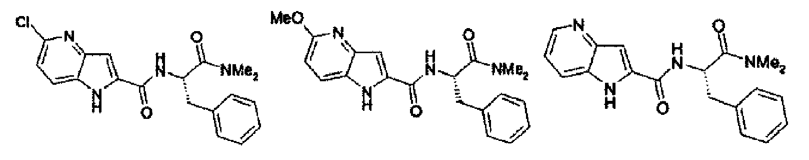 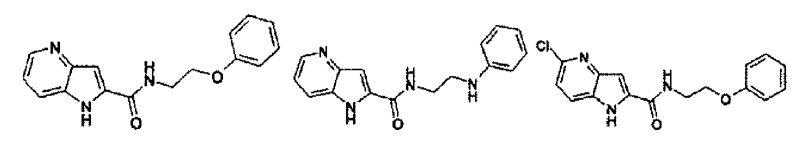 und deren pharmazeutisch unbedenkliche Salze.

20. Verbindungen nach Anspruch 1, ausgewählt aus den folgenden: 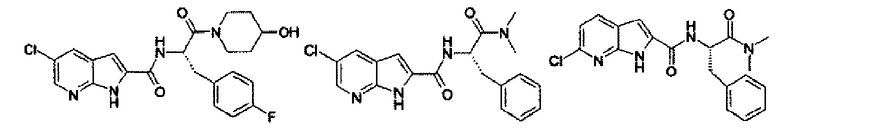 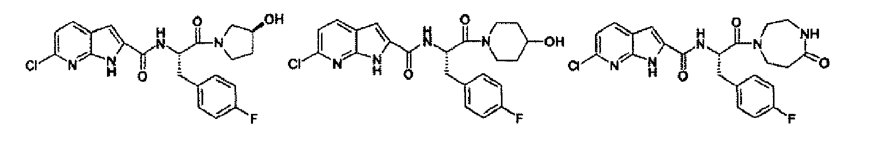 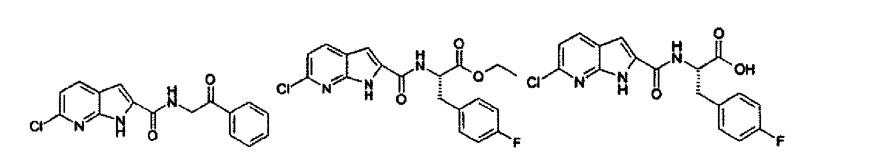 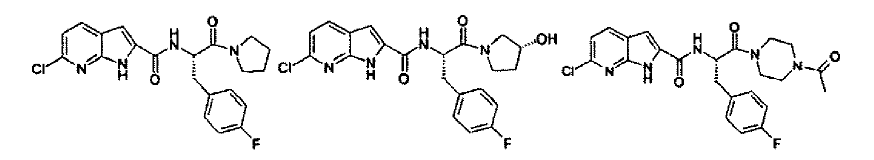 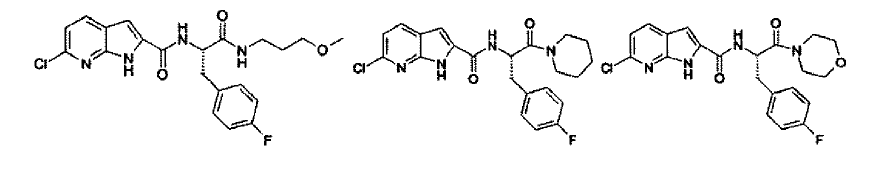 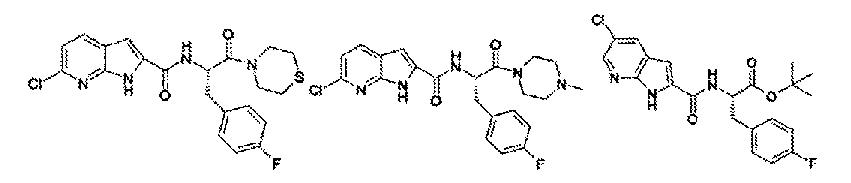 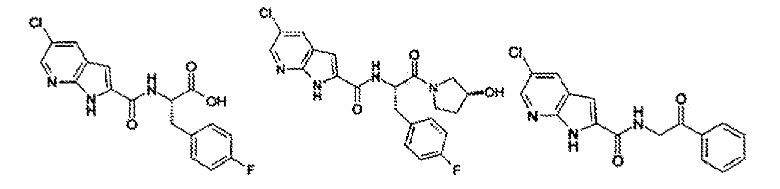 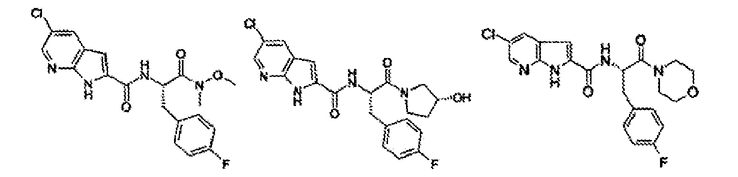 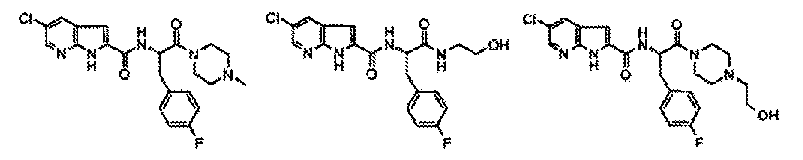 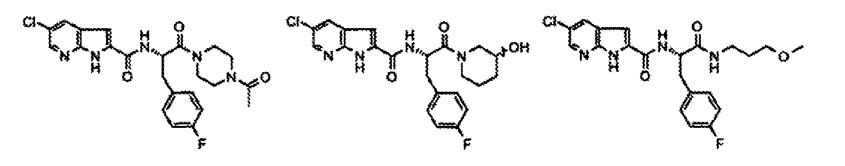 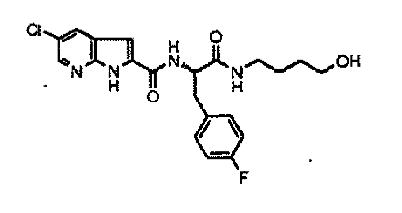 und deren pharmazeutisch unbedenkliche Salze.

21. Verbindungen nach Anspruch 1, ausgewählt aus den folgenden: 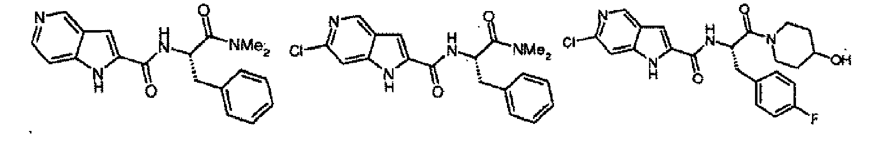 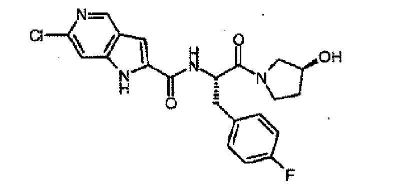 und deren pharmazeutisch unbedenkliche Salze.

22. Verbindung nach Anspruch 1: 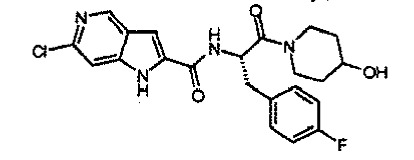 und deren pharmazeutisch unbedenkliche Salze.

23. Zusammensetzung, enthaltend, eine Verbindung nach einem der Ansprüche 1 bis 22 oder ein Stereoisomer oder ein pharmazeutisch unbedenkliches Salz davon und einen pharmazeutisch unbedenklichen Träger.

24. Verbindungen nach einem der Ansprüche 1 bis 22 und deren Stereoisomere und deren pharmazeutisch unbedenkliche Salze zur Verwendung bei der prophylaktischen oder therapeutischen Behandlung von Hyperglykämie oder Diabetes.

25. Verbindungen nach einem der Ansprüche 1 bis 22 und deren Stereoisomere und deren pharmazeutisch unbedenkliche Salze zur Verwendung bei der Prävention von Diabetes in einem Menschen, der prädiabetische Hyperglykämie oder eine gestörte Glucosetoleranz zeigt.

26. Verbindungen nach einem der Ansprüche 1 bis 22 und deren Stereoisomere und deren pharmazeutisch unbedenkliche Salze zur Verwendung bei der prophylaktischen oder therapeutischen Behandlung von Hypercholesteinämie, Hyperinsulinämie, Hyperlipidämie, Atherosklerose oder myokardialer Ischämie.

27. Verbindungen nach einem der Ansprüche 1 bis 22 und deren Stereoisomere und deren pharmazeutisch unbedenkliche Salze zur Verwendung bei der Kardioprotektion.

28. Verwendung einer Verbindung nach einem der Ansprüche 1 bis 22 oder eines Stereoisomers oder eines pharmazeutisch unbedenklichen Salzes davon bei der Herstellung eines Medikaments zur Behandlung einer wie in einem der Ansprüche 24 bis 27 definierten Krankheit bzw. eines wie in einem der Ansprüche 24 bis 27 definierten Leidens.

29. Verfahren zur Herstellung einer Verbindung der Formel (I) nach Anspruch 1, bei dem man:
a) 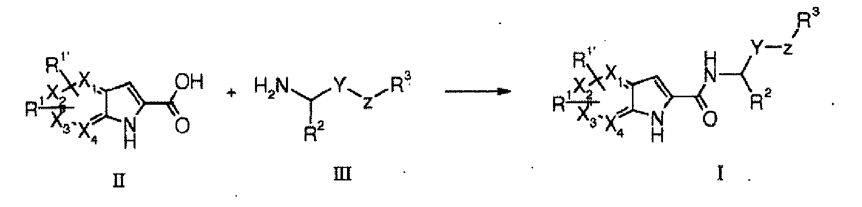 eine Pyrrolopyridin-2-carbonsäure der Formel (II) oder ein geschütztes oder aktiviertes Derivat davon mit einem Amin der Formel (III) kuppelt; oder
b) 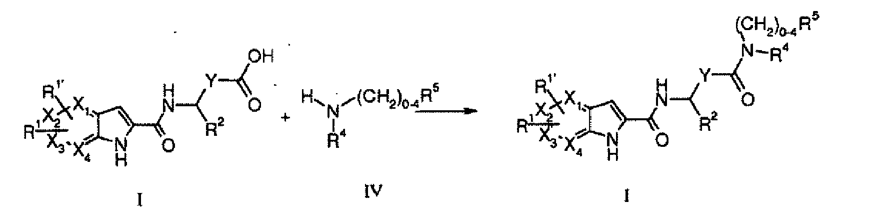 bei Verbindungen der Formel (I), in denen Z für C=O steht und R³ für -NR⁴R⁵ oder -NR⁴(-C₁₋₄-Alkyl-R⁵) steht, eine Carbonsäure der Formel (I) oder ein geschütztes oder aktiviertes Derivat davon, in dem Z fehlt und R³ für -CO₂H steht, mit einem Amin der Formel (IV) kuppelt; oder
c) 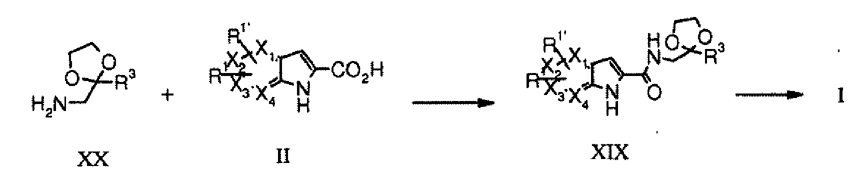 bei Verbindungen der Formel (III), in denen R² für H steht, Y für eine direkte Bindung steht, Z für -C(O)-steht und R³ für -Aryl oder -Hetaryl steht, eine Verbindung der Formel (XX) unter Standardkupplungsbedingungen mit einer Verbindung der Formel (II) zu einer Verbindung der Formel (XIX) kuppelt und anschließend die Ketalgruppe in Gegenwart von Säure entfernt; oder
d) 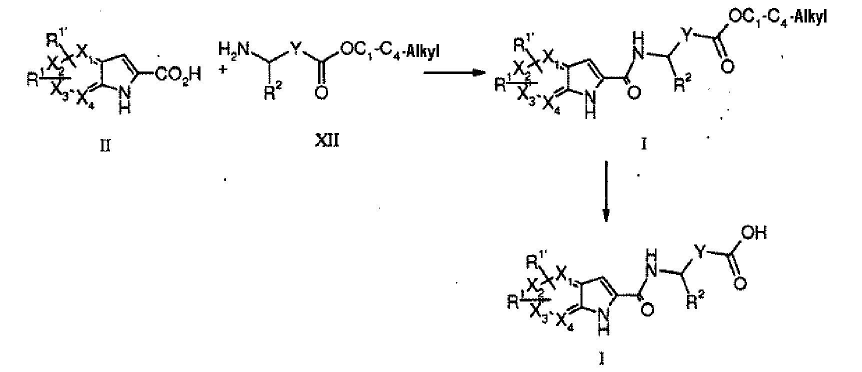 bei Verbindungen der Formel (I), in denen Z für C=O steht und R³ für C₁₋₄-Alkoxy steht, eine Verbindung der Formel (II) oder ein geschütztes oder aktiviertes Derivat davon mit einer Verbindung der Formel (XII) kuppelt; oder
e) bei Verbindungen der Formel (I), in denen Z fehlt und R³ für -CO₂H steht, eine Verbindung der Formel (I), in welcher Z für C=O steht und R³ für eine C₁₋₄-Alkoxygruppe steht, einer Esterhydrolyse unterzieht.

30. Verbindungen, ausgewählt aus:
5-Chlor-1*H*-pyrrolo[3,2-b]pyridin-2-carbonsäure;
5-Brom-1*H-*pyrrolo[3,2-b]pyridin-2-carbonsäure;
5-Cyano-1*H-*pyrrolo[3,2-b]pyridin-2-carbonsäure;
5-Methoxy-1*H*-pyrrolo[3,2-b]pyridin-2-carbonsäure;
1*H*-Pyrrolo[3,2-c]pyridin-2-carbonsäure;
6-Chlor-1*H*-pyrrolo[3,2-c]pyridin-2-carbonsäure;
6-Cyano-1*H*-pyrrolo[3,2-c]pyridin-2-carbonsäure;
5-Chlor-1*H*-pyrrolo[2,3-c]pyridin-2-carbonsäure;
5-Brom-1*H*-pyrrolo[2,3-c]pyridin-2-carbonsäure;
5-Ethinyl-1*H*-pyrrolo[2,3-c]pyridin-2-carbonsäure;
5-Cyano-1*H*-pyrrolo[2,3-c]pyridin-2-carbonsäure;
5-Methyl-1*H*-pyrrolo[2,3-c]pyridin-2-carbonsäure;
5-Chlor-1*H*-pyrrolo[2,3-b]pyridin-2-carbonsäure;
6-Chlor-1*H*-pyrrolo[2,3-b]pyridin-2-carbonsäure; und
6-Cyano-1*H*-pyrrolo[2,3-b]pyridin-2-carbonsäure;
und deren jeweilige C₁₋₄-Alkylester.

31. Verbindungen der Formel (XIX): 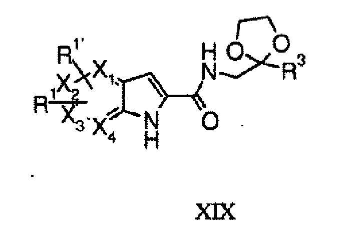 in welcher R¹, R^{1'}, X₁, X₂, X₃ und X₄ wie in Anspruch 1 definiert sind und R³ für -Aryl oder -Hetaryl steht.

32. 4(S)-(4-Fluorbenzyl)oxazolidin-2,5-dion.

## Revendications

1. Composé de la formule (I) : 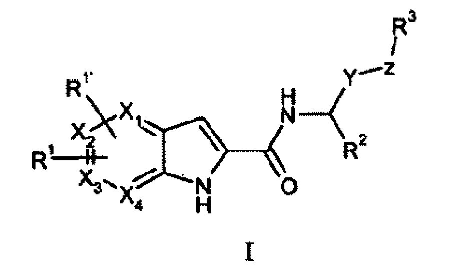 ou un stéréoisomère, ou un sel pharmaceutiquement acceptable de celui-ci, dans lequel :
un parmi X₁, X₂, X₃ et X₄ doit être un atome de N et les autres doivent être un atome de C ;
R¹ et R^{1'} représentent chacun indépendamment un atome d'hydrogène, un atome d'halogène, un groupe hydroxy, cyano, alkyle de C₁ à C₄, alcoxy de C₁ à C₄, fluorométhyle, difluorométhyle, trifluorométhyle, éthényle ou éthynyle ;
R² représente un atome d'hydrogène, un groupe alkyle de C₁ à C₄, COOR⁶, COR⁶, alcoxy de C₁ à C₄-alkyle de C₁ à C₄-, hydroxyalkyle de C₁ à C₄-, cycloalkyle, cycloalkylalkyle de C₁ à C₄-, aryle, arylalkyle de C₁ à C₄-, hétaryle ou hétarylalkyle de C₁ à C₄-, dans lequel l'un quelconque des noyaux aryle ou hétaryle est éventuellement substitué par un ou deux substituants indépendants halogène, cyano, alkyle de C₁ à C₄, alcoxy de C₁ à C₄, -NR¹³R¹⁴, -SO₂-alkyle de C₁ à C₄, -SO₂NR¹³R¹⁴, hydroxy, fluorométhyle, difluorométhyle, ou trifluorométhyle ;
Y représente une liaison directe, un groupe alkyle de C₁ à C₂ ou un groupe -CH(OH)- ;
Z représente un groupe CH₂, un groupe -C(O)-, un groupe -O-, un groupe >NH, un groupe >N (alkyle de C₁ à C₄) , un groupe >N(cycloalkyle de C₃ à C₆), ou est absent ; mais lorsque Y représente un groupe -CH(OH)-, Z ou R³ doit être lié à Y par une liaison carbone-carbone ;
R³ représente un atome d'hydrogène, un groupe -COOH, - COO-alkyle de C₁ à C₄, alcoxy de C₁ à C₄, alkyle de C₁ à C₄, arylalkylthio de C₁ à C₄-, aryle, -alkylaryle de C₁ à C₄, hétaryle, -alkylhétaryle de C₁ à C₄, cycloalkyle, - alkylcycloalkyle de C₁ à C₄, hétérocyclyle ou - alkylhétérocyclyle de C₁ à C₄, dans lequel l'un quelconque des noyaux est éventuellement substitué par un à trois substituants indépendants halogène, cyano, alkyle de C₁ à C₄, fluorométhyle, difluorométhyle, trifluorométhyle, -NHC(O)O(alkyle de C₁ à C₄), -(alkyl de C₁ à C₄)NHC(O)O(alkyle de C₁ à C₄), -NR⁷R⁸, -alkyle de C₁ à C₄-NR⁷R⁸, -C(O)R⁹, alcoxy de C₁ à C₄-, alcoxy de C₁ à C₄-alkyle de C₁ à C₄-, -COOH, -COO-alkyle de C₁ à C₄, - NHC(O)R⁹, -alkyle de C₁ à C₄-NHC(O)R⁹, -C(O)N(R¹⁰)₂, -alkyle de C₁ à C₄-C(O)N(R¹⁰)₂, -alcoxy de C₁ à C₄-alcoxy de C₁ à C₄, hydroxy, hydroxyalkyle de C₁ à C₄-, -NHSO₂R¹⁰, -SO₂ (alkyle de C₁ à C₄) , -SO₂NR¹¹R¹², hétérocyclyle à 5 à 6 chaînons, phényle, phénylalcoxy de C₁ à C₂, ou phénylalkyle de C₁ à C₂, dans lesquels le groupe phényle est éventuellement substitué par un à deux substituants indépendants halogène, cyano, alkyle de C₁ à C₄, alcoxy de C₁ à C₄, -NR¹³R¹⁴, -SO₂-alkyle de C₁ à C₄, -SO₂NR¹³R¹⁴, hydroxy, fluorométhyle, difluorométhyle ou trifluorométhyle, ou deux liaisons sur un atome de carbone du noyau du groupe hétérocyclyle peuvent éventuellement former un substituant oxo (=O) ;
ou R³ représente un groupe -NR⁴R⁵ ou un groupe -NR⁴(-alkyle de C₁ à C₄-R⁵) ;
R⁴ représente un atome d'hydrogène, un groupe alkyle de C₁ à C₃, un groupe -alkyle de C₂ à C₃-NR⁷R⁸, un groupe cycloalkyle de C₃ à C₆ éventuellement substitué par un groupe hydroxy ou un groupe hydroxyalkyle de C₁ à C₄- en outre éventuellement substitué par un groupe hydroxy, un groupe alcoxy de C₁ à C₂-alkyle de C₂ à C₄- ou un groupe alkyle de C₁ à C₂-S(O)ₙ-alkyle de C₂ à C₃- ;
n vaut 0, 1 ou 2 ;
R⁵ représente un atome d'hydrogène, un groupe hydroxyalkyle de C₂ à C₃-, alcoxy de C₁ à C₂, alcoxy de C₁ à C₂-alkyle de C₁ à C₄- ou un groupe aryle, hétaryle ou hétérocyclyle ;
dans lequel un noyau R⁵ hétérocyclique azoté est éventuellement monosubstitué sur l'atome d'azote du noyau par un groupe alkyle de C₁ à C₄, benzyle, benzoyle, alkyle de C₁ à C₄-C(O)-, SO₂-alkyle de C₁ à C₄, -SO₂NR¹³R¹⁴, alcoxycarbonyle de C₁ à C₄ ou aryl(alcoxy de C₁ à C₄)carbonyle ; et dans lequel les noyaux R⁵ sont éventuellement monosubstitués sur un atome de carbone du noyau par un atome d'halogène, un groupe cyano, alkyle de C₁ à C₄-C(O)-, alkyle de C₁ à C₄-SO₂, alkyle de C₁ à C₄, alcoxy de C₁ à C₄, hydroxy, -NR¹³R¹⁴, hydroxy, hydroxyalkyle de C₁ à C₄-, carbamoyle ou alkyle de C₁ à C₄-carbamoyl-, à condition qu'aucun azote quaternisé ne soit inclus ; ou deux liaisons sur un atome de carbone du noyau du groupe hétérocyclyle peuvent éventuellement former un substituant oxo (=O) ;
R⁶ représente un groupe alkyle de C₁ à C₄, un groupe aryle ou un groupe hétaryle ;
R⁷ et R⁸ représentent indépendamment un atome d'hydrogène, un groupe alkyle de C₁ à C₄, un groupe cycloalkyle de C₃ à C₆ ou un groupe CO(alkyle de C₁ à C₄) ;
R⁹ représente un groupe alkyle de C₁ à C₄ ou un groupe cycloalkyle de C₃ à C₆ ;
R¹⁰ représente un atome d'hydrogène, un groupe alkyle de C₁ à C₄ ou un groupe cycloalkyle de C₃ à C₆ ;
R¹¹ et R¹² représentent indépendamment un atome d'hydrogène ou un groupe alkyle de C₁ à C₄ ou, avec l'atome d'azote auquel ils sont fixés, ils peuvent former un hétérocycle à 4 à 6 chaînons ;
R¹³ et R¹⁴ représentent indépendamment un atome d'hydrogène ou un groupe alkyle de C₁ à C₄ ; et
dans lequel aucune liaison azote-oxygène, azote-azote ou azote-halogène ne lie les trois composants -Y-Z-R³ les uns aux autres ;
à condition que le composé ne soit pas une 2R, 4S, 5S-6-cyclohexyl-5-(6'-aza-indol-2'-ylcarbonylamino)-2-(2'-méthylpropyl)-gamma-hexanolactone.

2. Composé selon la revendication 1, ou un stéréoisomère ou un sel pharmaceutiquement acceptable de celui-ci, dans lequel X₃ représente un atome de N.

3. Composé selon la revendication 1, ou un stéréoisomère ou un sel pharmaceutiquement acceptable de celui-ci, dans lequel X₁ représente un atome de N.

4. Composé selon la revendication 1, ou un stéréoisomère ou un sel pharmaceutiquement acceptable de celui-ci, dans lequel X₂ représente un atome de N.

5. Composé selon la revendication 1, ou un stéréoisomère ou un sel pharmaceutiquement acceptable de celui-ci, dans lequel X₄ représente un atome de N.

6. Composé selon l'une quelconque des revendications précédentes, ou un stéréoisomère ou un sel pharmaceutiquement acceptable de celui-ci, dans lequel R¹ et R^{1'} représentent chacun indépendamment un atome d'halogène, un atome d'hydrogène ou un groupe cyano.

7. Composé selon l'une quelconque des revendications précédentes, ou un stéréoisomère ou un sel pharmaceutiquement acceptable de celui-ci, dans lequel un parmi R¹ et R^{1'} représente un atome d'hydrogène et l'autre représente un groupe 5-halogéno ou 5-cyano.

8. Composé selon l'une quelconque des revendications précédentes, ou un stéréoisomère ou un sel pharmaceutiquement acceptable de celui-ci, dans lequel Y représente une liaison directe ou un groupe alkyle de C₁ à C₂.

9. Composé selon l'une quelconque des revendications précédentes, dans lequel Z représente un groupe -C(O)-.

10. Composé selon l'une quelconque des revendications 1, 2 ou 6 à 9, ou un stéréoisomère ou un sel pharmaceutiquement acceptable de celui-ci, dans lequel
X₃ représente un atome de N ;
Y représente une liaison directe ou un groupe alkyle de C₁ à C₂ ; et
Z représente un groupe -C(O)-.

11. Composé selon l'une quelconque des revendications précédentes, ou un stéréoisomère ou un sel pharmaceutiquement acceptable de celui-ci, dans lequel Y représente une liaison directe.

12. Composé selon l'une quelconque des revendications précédentes, ou un stéréoisomère ou un sel pharmaceutiquement acceptable de celui-ci, dans lequel R² représente un atome d'hydrogène, un groupe alkyle de C₁ à C₄, un groupe aryle ou un groupe arylalkyle de C₁ à C₄-, dans lequel le noyau aryle est éventuellement substitué par 1 à 2 substituants indépendants halogène, cyano, alkyle de C₁ à C₄, alcoxy de C₁ à C₄, -NR¹³R¹⁴, -SO₂-alkyle de C₁ à C₄, -SO₂NR¹³R¹⁴, hydroxy, fluorométhyle, difluorométhyle ou trifluorométhyle.

13. Composé selon la revendication 12, ou un stéréoisomère ou un sel pharmaceutiquement acceptable de celui-ci, dans lequel R² représente un groupe benzyle éventuellement substitué par un à deux substituants halogène.

14. Composé selon l'une quelconque des revendications précédentes, ou un stéréoisomère ou un sel pharmaceutiquement acceptable de celui-ci, dans lequel R³ représente un groupe hétérocyclyle, -alkyle de C₁ à C₄-hétérocyclyle éventuellement substitué par 1 à 3 substituants indépendants halogène, cyano, alkyle de C₁ à C₄, fluorométhyle, difluorométhyle, trifluorométhyle, -NHC(O)O(alkyle de C₁ à C₄), - (alkyle de C₁ à C₄)NHC(O)O(alkyle de C₁ à C₄) , -NR⁷R⁸, -alkyle de C₁ à C₄-NR⁷R⁸ , -C(O)R⁹, alcoxy de C1 à C₄-, alcoxy de C₁ à C₄-alkyle de C₁ à C₄-, -COOH, -COO-alkyle de C₁ à C₄,-NHC(O)R⁹, -alkyle de C₁ à C₄-NHC(O)R⁹, -C(O)N(R¹⁰)₂, -alkyle de C₁ à C₄-C(O)N(R¹⁰)₂, -alcoxy de C₁ à C₄-alcoxy de C₁ à C₄, hydroxy, hydroxyalkyle de C₁ à C₄, -NHSO₂R¹⁰, -SO₂ (alkyle de C₁ à C₄) , -SO₂NR¹¹R¹², hétérocyclyle à 5 à 6 chaînons, phényle, phénylalcoxy de C₁ à C₂ ou phénylalkyle de C₁ à C₂, dans lesquels le groupe phényle est éventuellement substitué par 1 à 2 substituants indépendants halogène, cyano, alkyle de C₁ à C₄, alcoxy de C₁ à C₄, -NR¹³R¹⁴, -SO₂alkyle de C₁ à C₄, -SO₂NR¹³R¹⁴, hydroxy, fluorométhyle, difluorométhyle ou trifluorométhyle, ou deux liaisons sur un atome de carbone du noyau du groupe hétérocyclyle peuvent éventuellement former un substituant oxo (=O) ; ou R³ représente un groupe -NR⁴R⁵ ou -NR⁴(-alkyle de C₁ à C₄-R⁵).

15. Composé selon la revendication 14, dans lequel R³ représente un groupe hétérocyclyle azoté éventuellement substitué, lié à Z par un atome d'azote du noyau ; ou R³ représente un groupe -NR⁴R⁵ ou un groupe -NR⁴(-alkyle de C₁ à C₄-R⁵) .

16. Composé selon la revendication 15, dans lequel R³ représente un groupe hétérocyclyle azoté à 4 à 8 chaînons éventuellement substitué.

17. Composé selon la revendication 16, ou un stéréoisomère ou un sel pharmaceutiquement acceptable de celui-ci, dans lequel R³ représente un groupe pyrrolidin-1-yle ou un groupe pipéridin-1-yle éventuellement substitué par un groupe hydroxy.

18. Composé selon la revendication 1, choisi parmi les suivants : 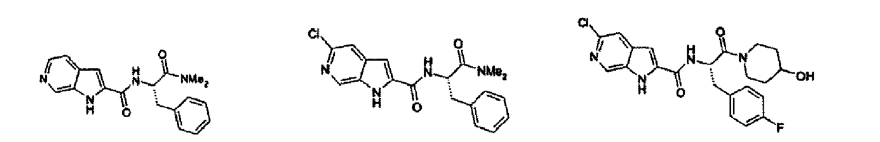 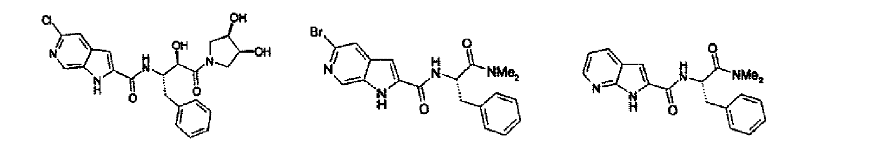 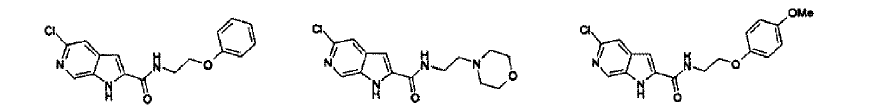 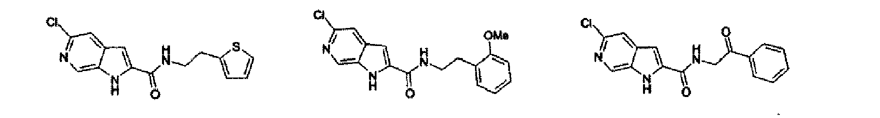 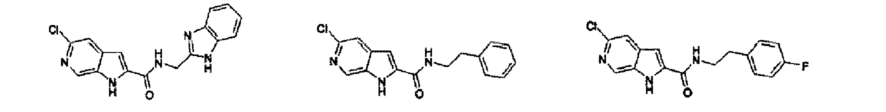 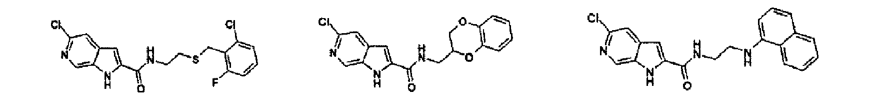 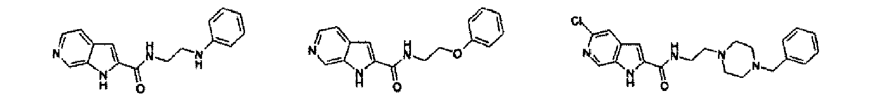 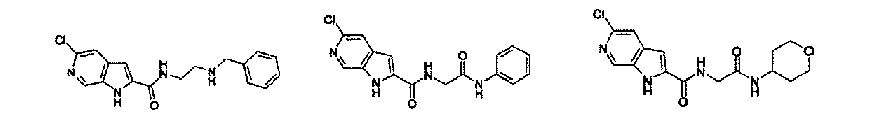 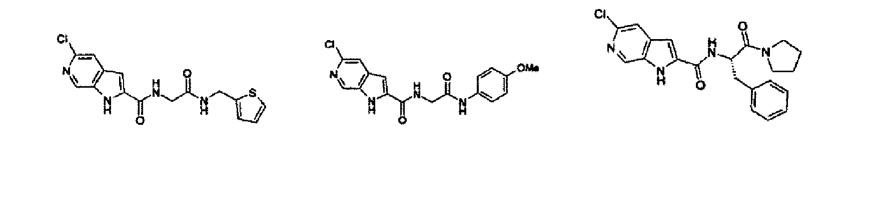 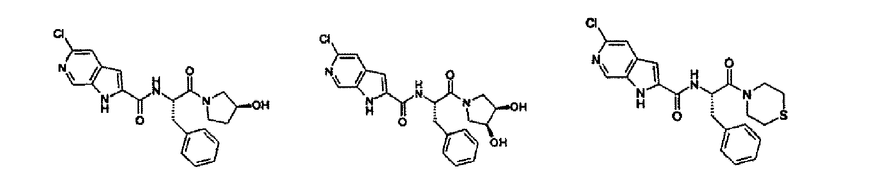 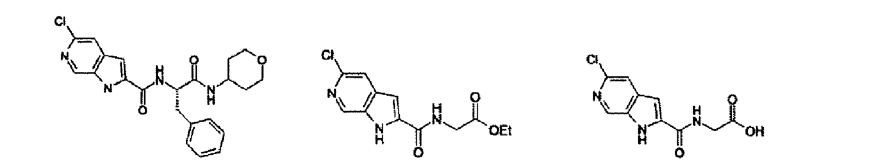 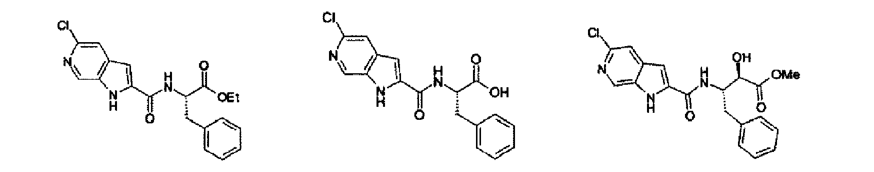 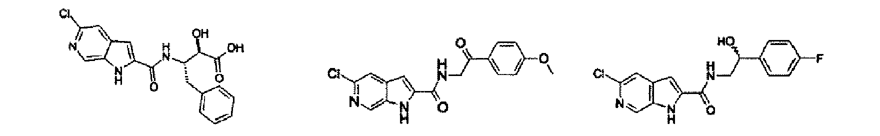 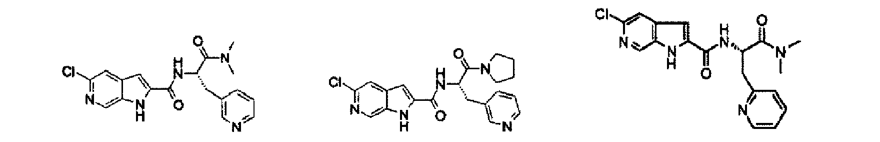 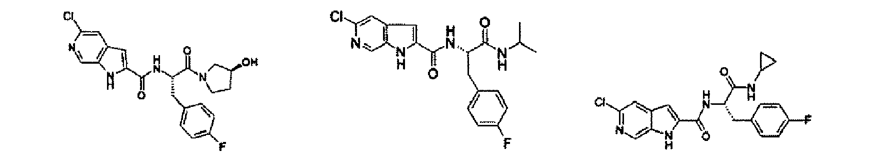 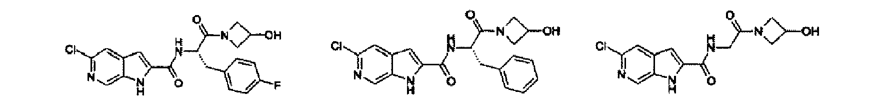 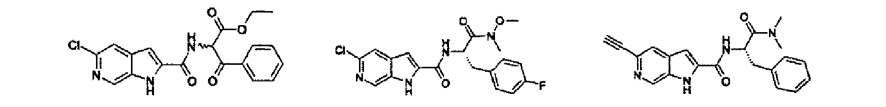 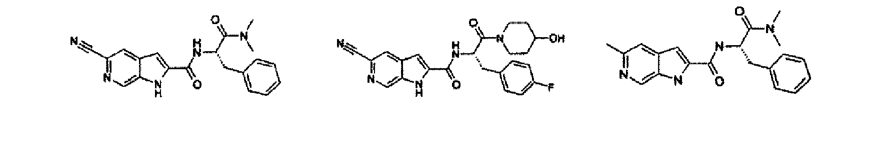 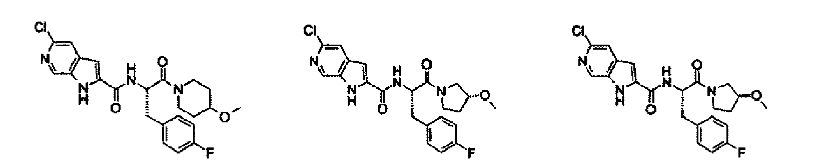 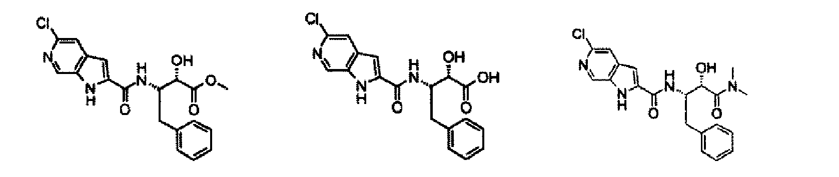 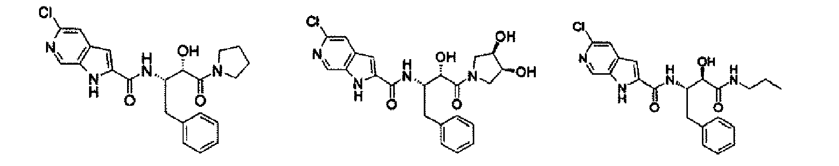 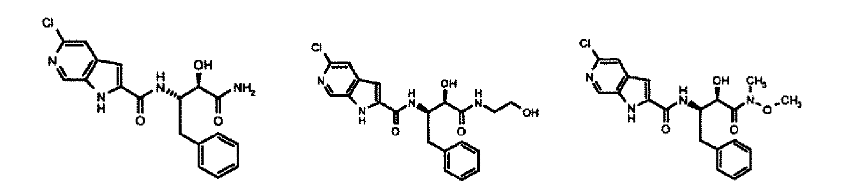 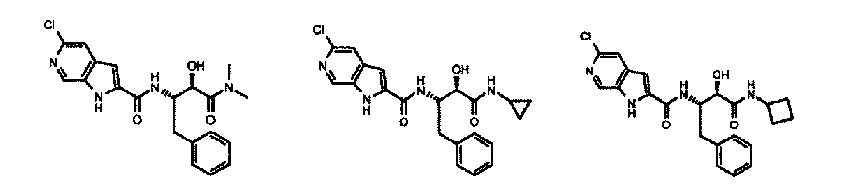 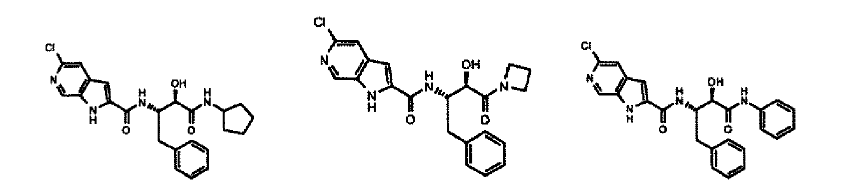 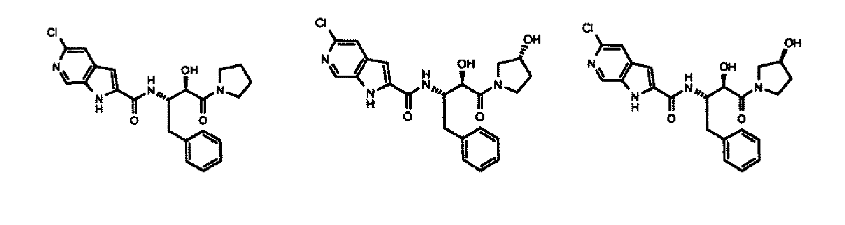 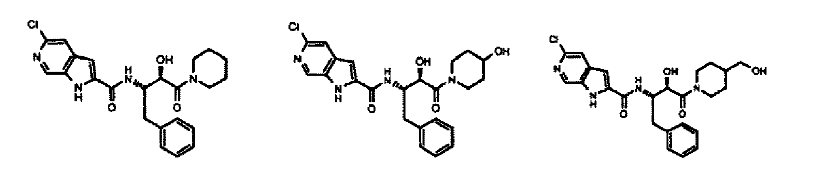 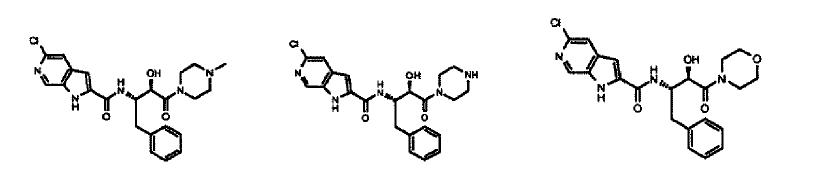 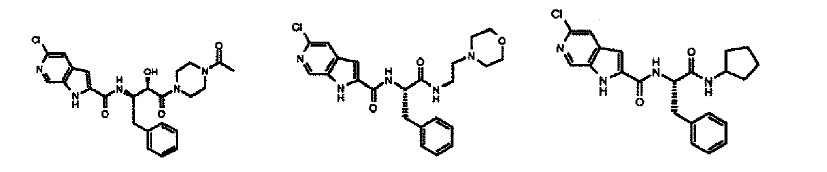 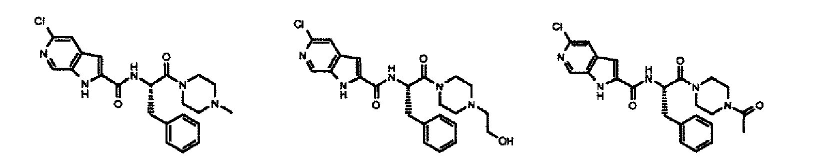 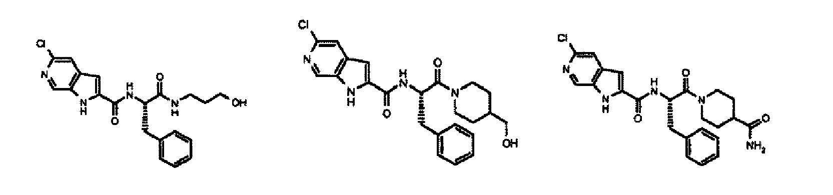 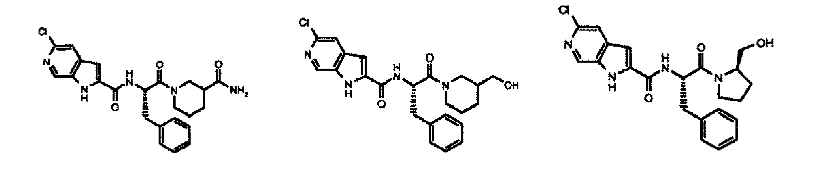 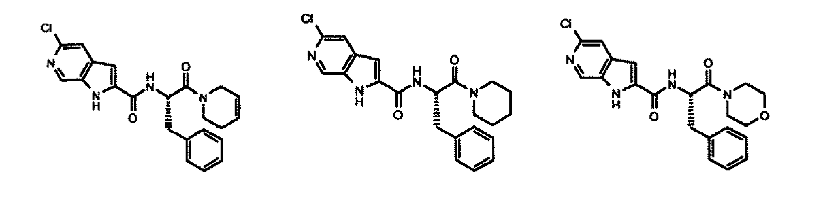 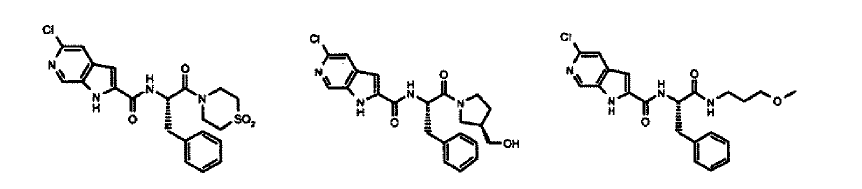 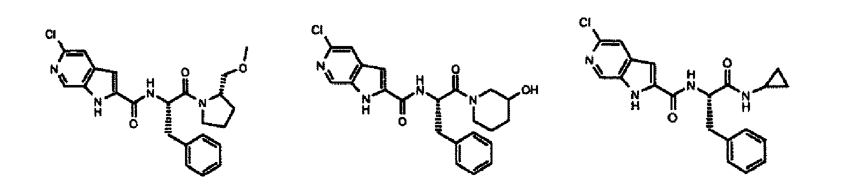 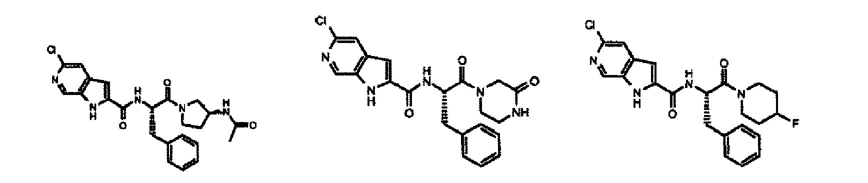 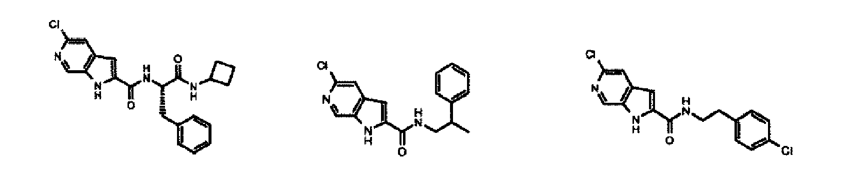 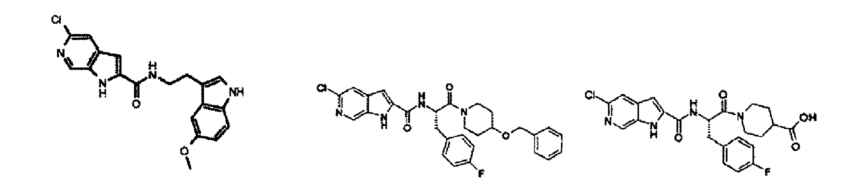 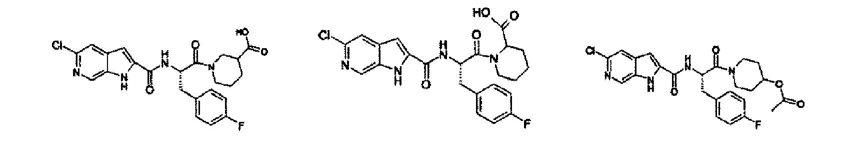 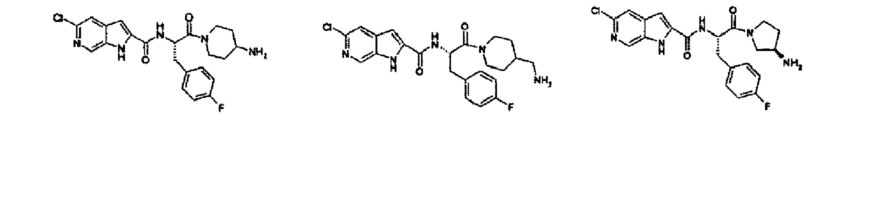 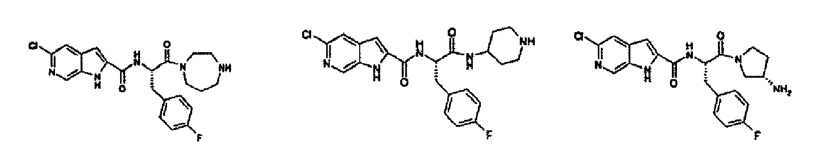 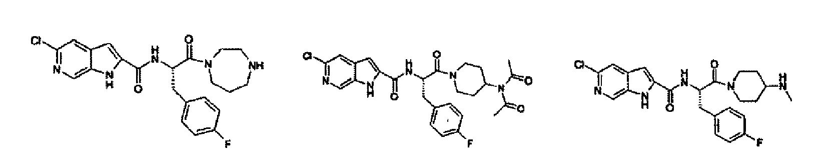 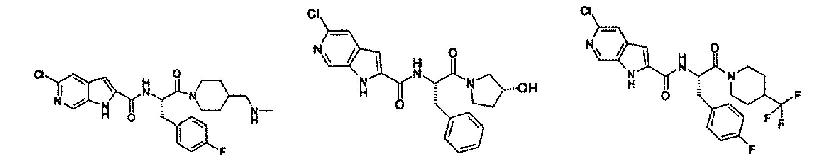 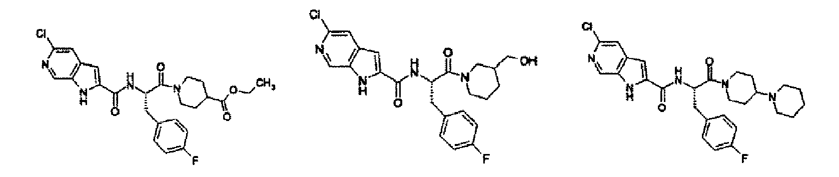 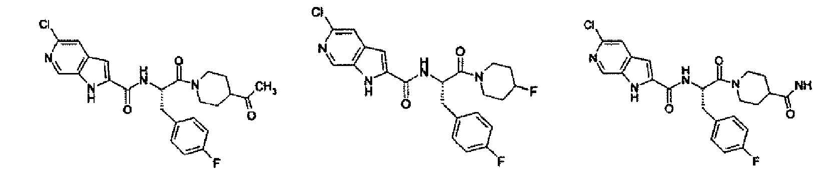 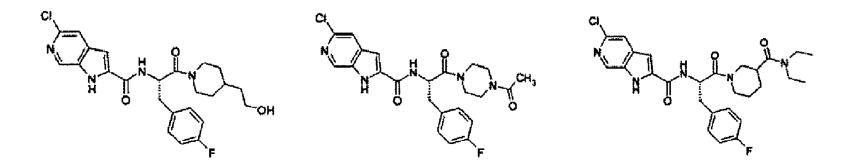 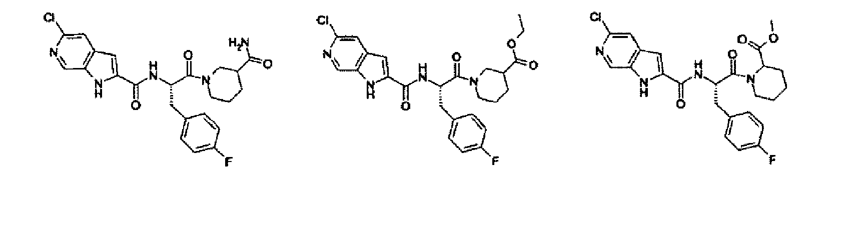 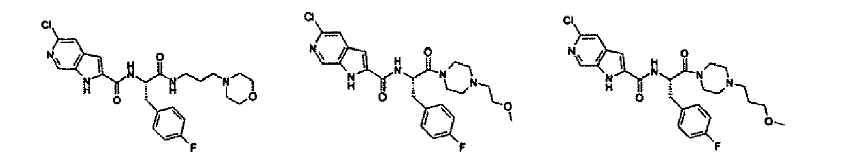 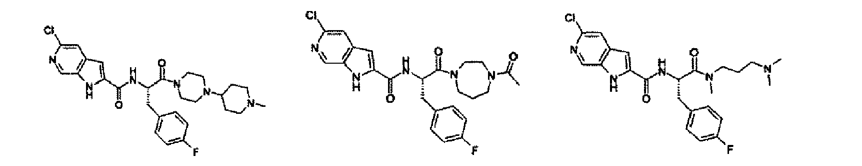 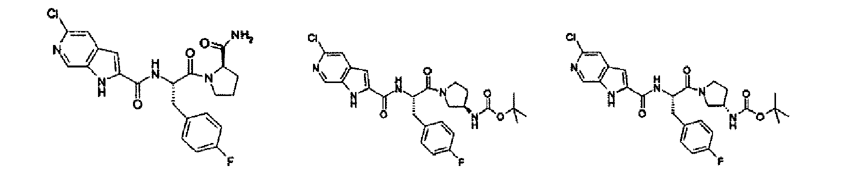 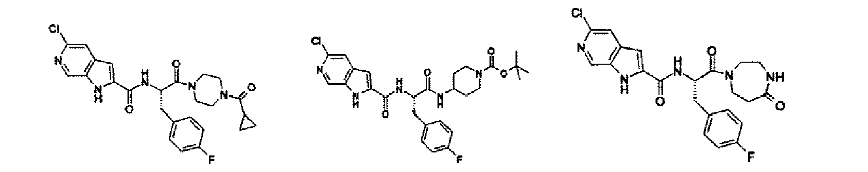 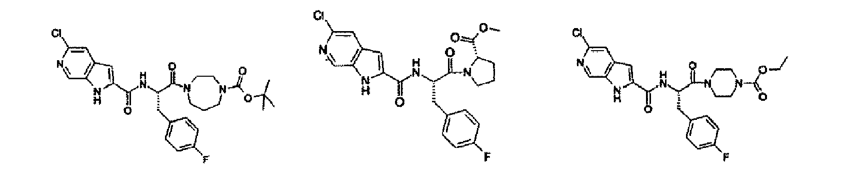 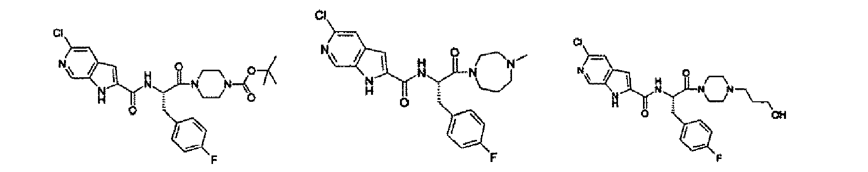 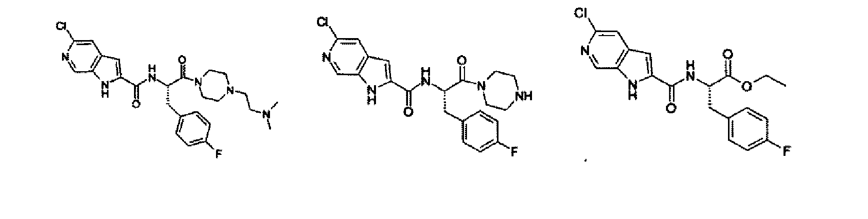 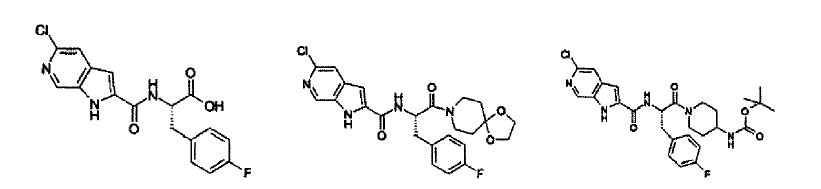 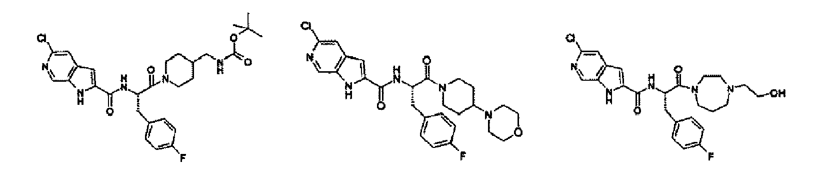 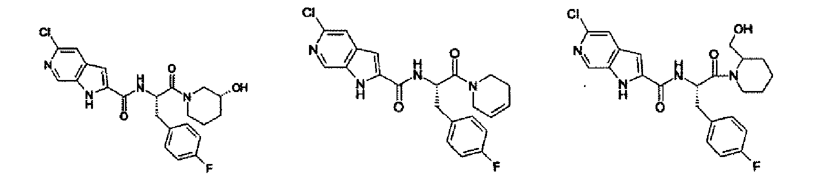 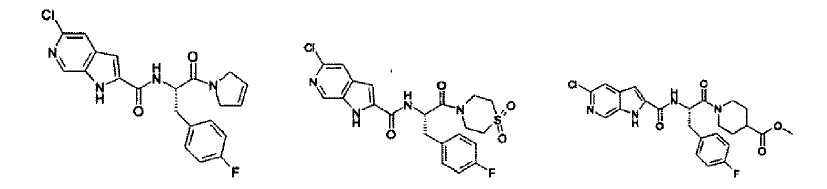 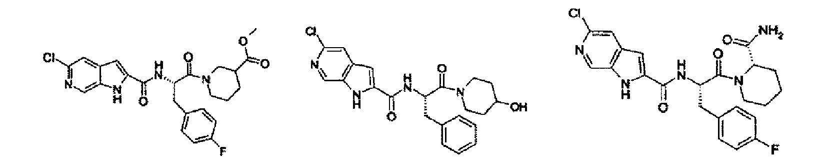 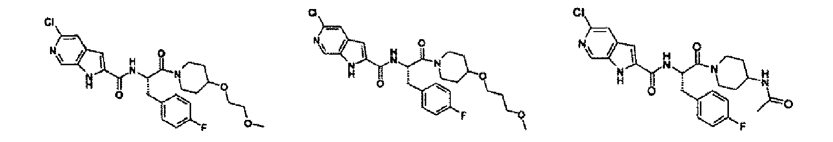 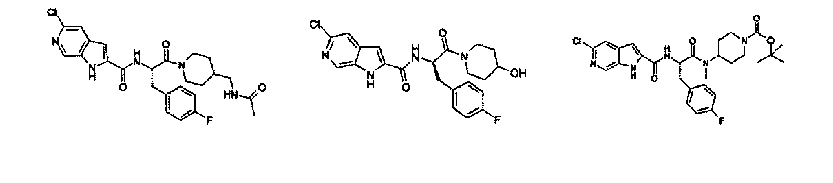 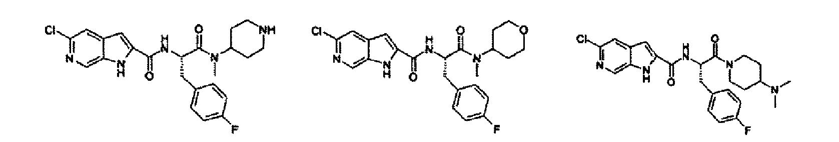 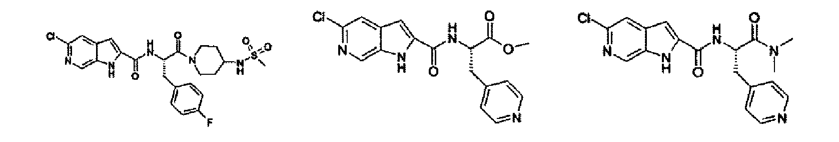 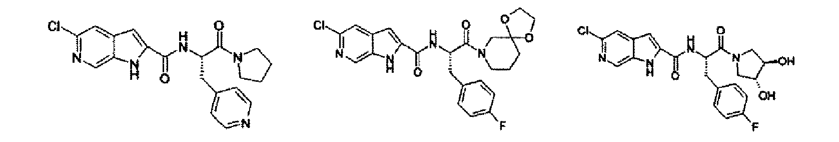 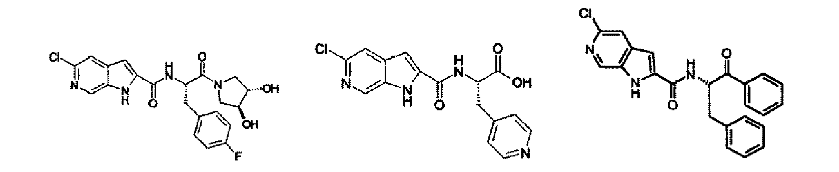 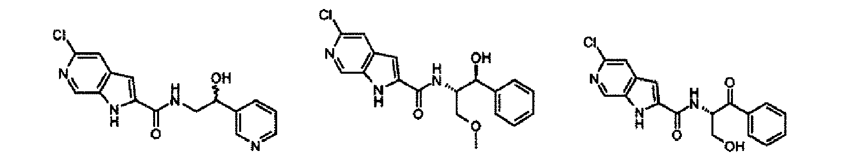 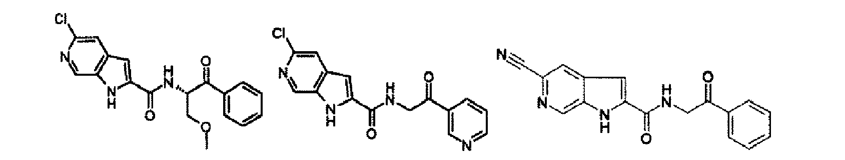 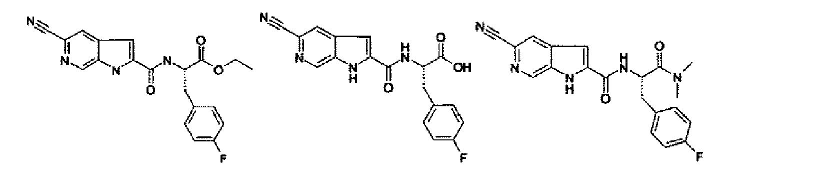 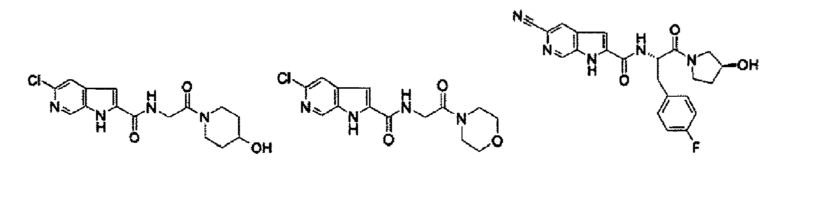 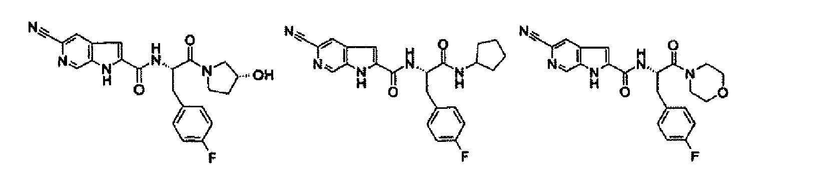 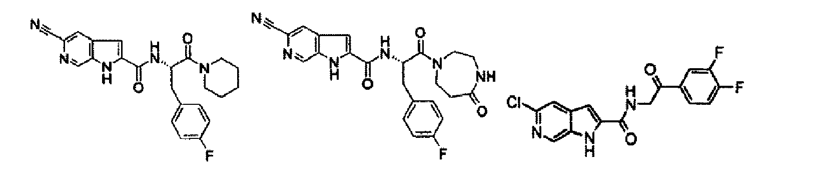 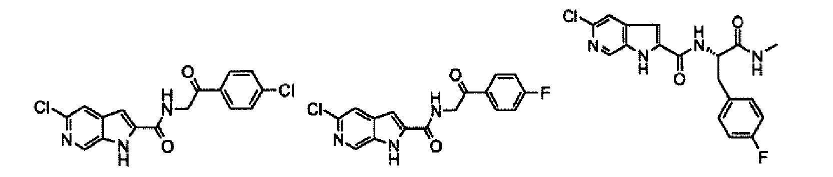 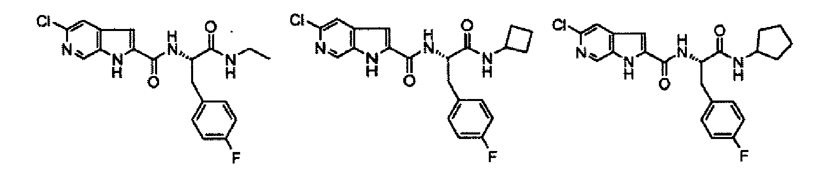 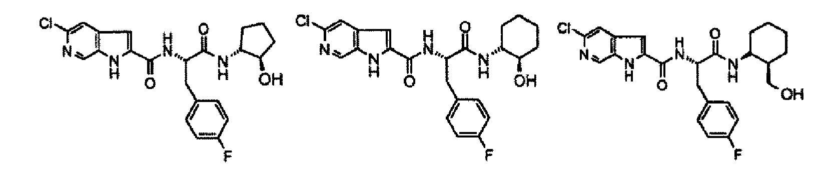 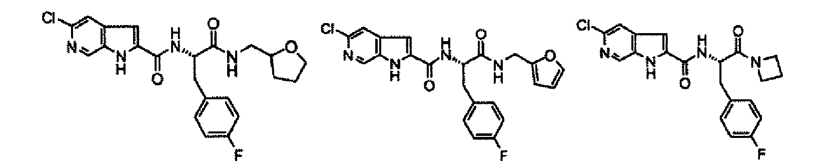 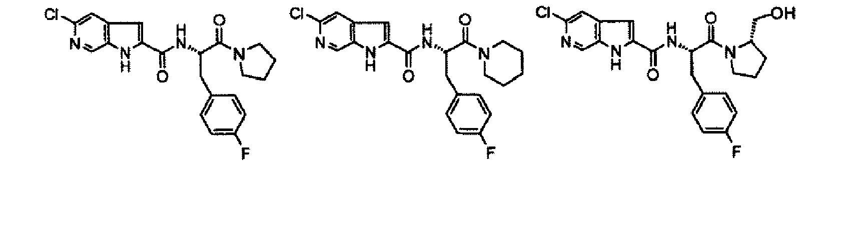 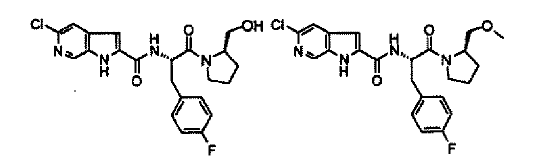 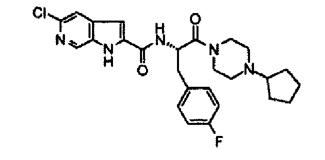 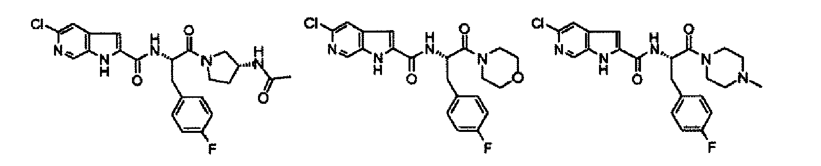 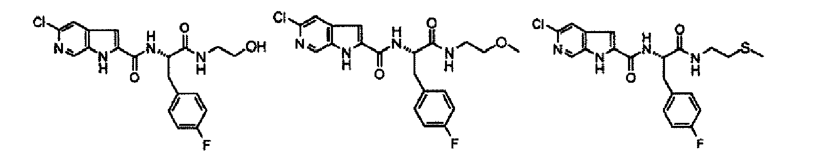 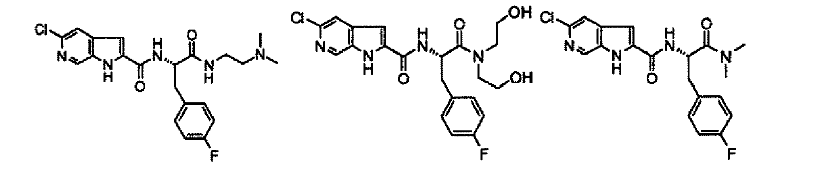 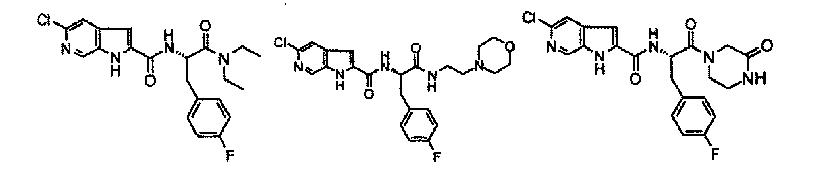 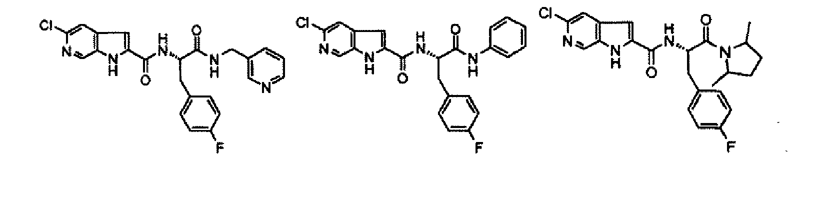 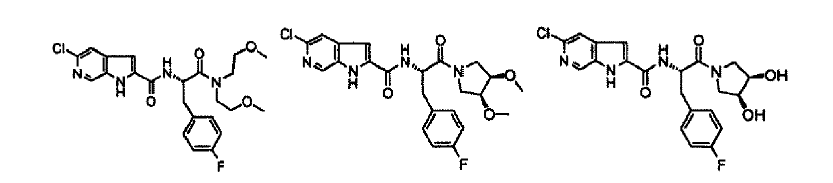 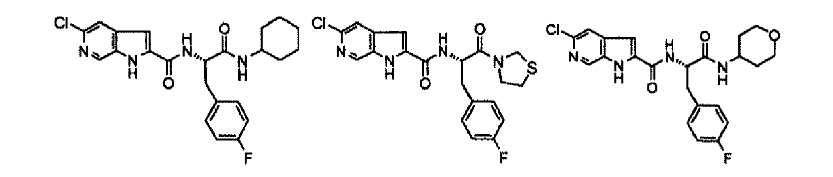 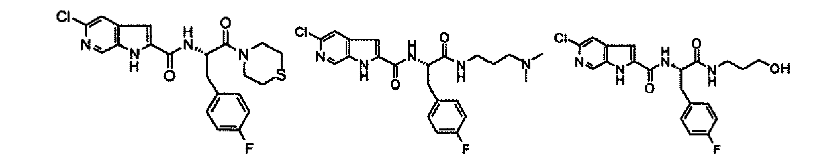 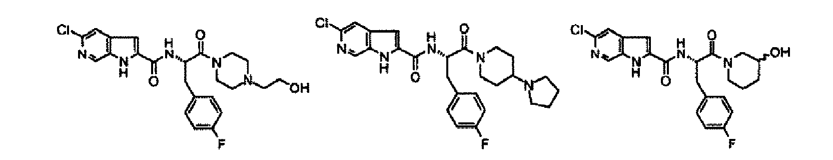 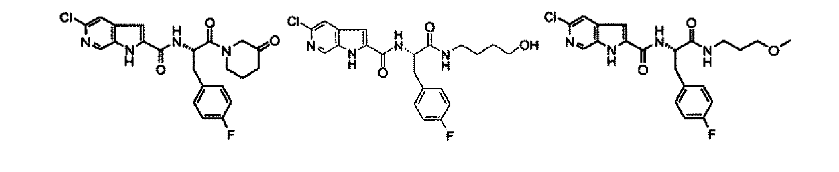 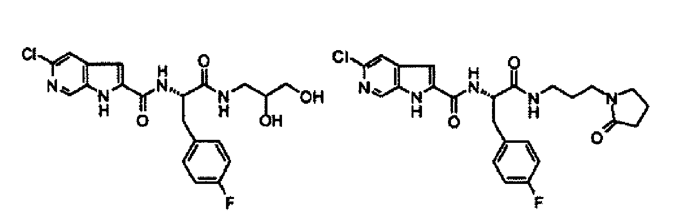 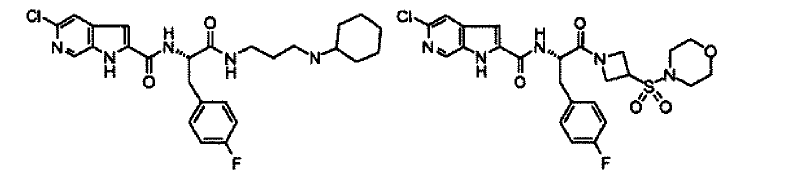 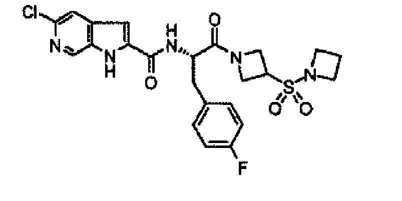 ou un sel pharmaceutiquement acceptable de ceux-ci.

19. Composé selon la revendication 1, choisi parmi les suivants : 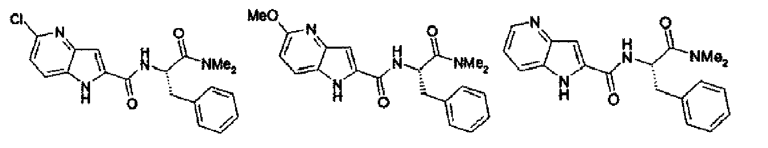 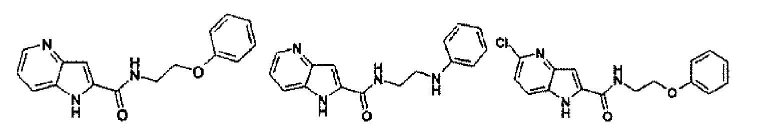 ou un sel pharmaceutiquement acceptable de ceux-ci.

20. Composé selon la revendication 1, choisi parmi les suivants : 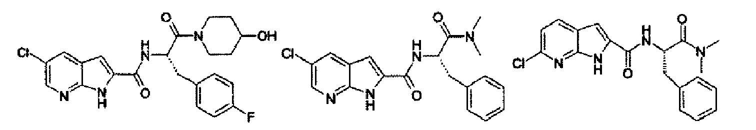 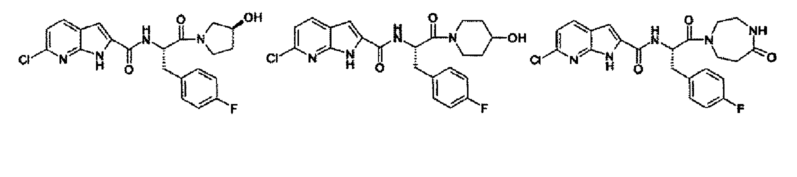 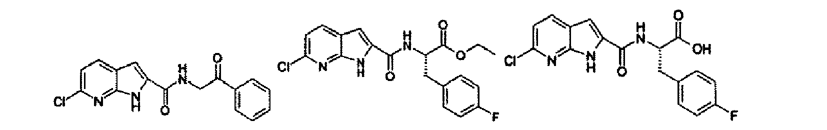 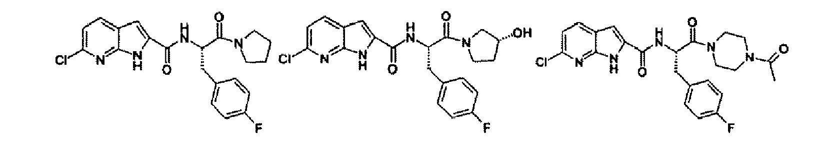 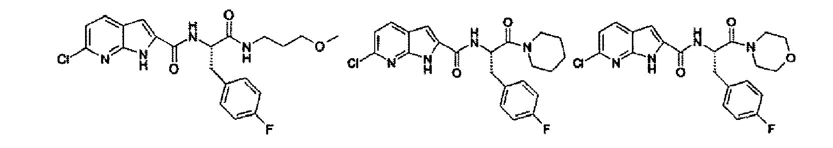 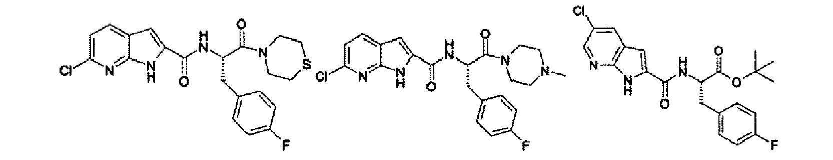 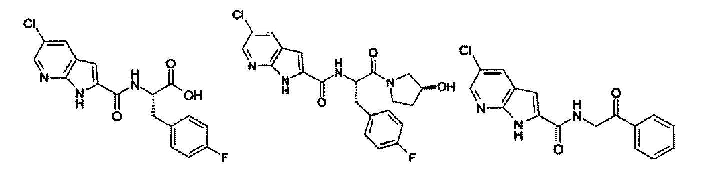 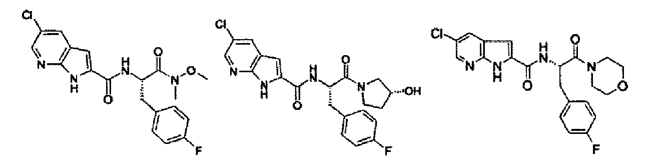 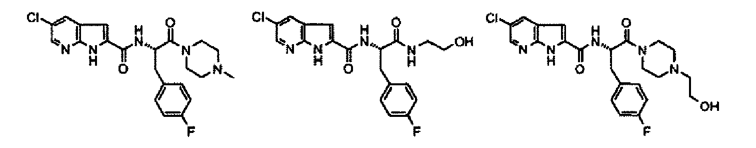 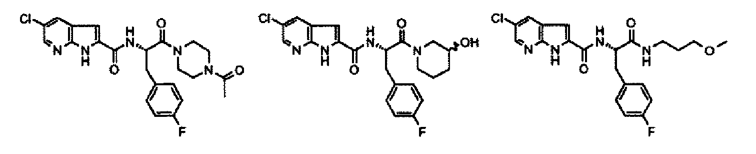 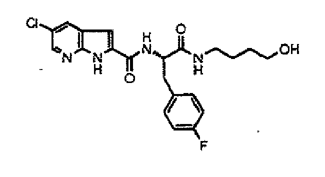 ou un sel pharmaceutiquement acceptable de ceux-ci.

21. Composé selon la revendication 1, choisi parmi les suivants : 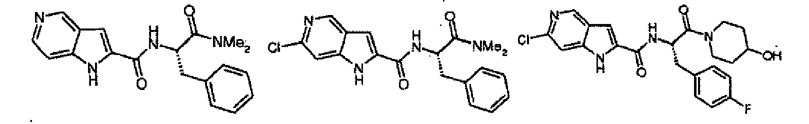 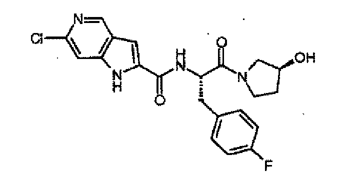 ou un sel pharmaceutiquement acceptable de ceux-ci.

22. Composé selon la revendication 1 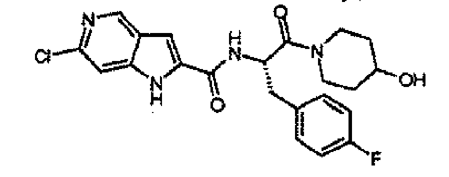 ou un sel pharmaceutiquement acceptable de ceux-ci.

23. Composition comprenant un composé selon l'une quelconque des revendications 1 à 22, ou un stéréoisomère ou un sel pharmaceutiquement acceptable de celui-ci, ainsi qu'un vecteur pharmaceutiquement acceptable.

24. Composé selon l'une quelconque des revendications 1 à 22, ou un stéréoisomère ou un sel pharmaceutiquement acceptable de celui-ci, pour utilisation dans le traitement prophylactique ou thérapeutique de l'hyperglycémie ou du diabète.

25. Composé selon l'une quelconque des revendications 1 à 22, ou un stéréoisomère ou un sel pharmaceutiquement acceptable de celui-ci, pour utilisation dans la prévention du diabète chez un humain qui présente une hyperglycémie prédiabétique ou une intolérance au glucose.

26. Composé selon l'une quelconque des revendications 1 à 22, ou un stéréoisomère ou un sel pharmaceutiquement acceptable de celui-ci, pour utilisation dans le traitement prophylactique ou thérapeutique de l'hypercholestérolémie, de l'hyperinsulinémie, de l'hyperlipidémie, de l'athérosclérose ou de l'ischémie myocardique.

27. Composé selon l'une quelconque des revendications 1 à 22, ou un stéréoisomère ou un sel pharmaceutiquement acceptable de celui-ci, pour utilisation dans la cardioprotection.

28. Utilisation d'un composé selon l'une quelconque des revendications 1 à 22, ou d'un stéréoisomère ou d'un sel pharmaceutiquement acceptable de celui-ci, dans la fabrication d'un médicament pour le traitement d'une maladie ou d'un état tel que défini dans l'une quelconque des revendications 24 à 27.

29. Procédé pour la production d'un composé de la formule (I) selon la revendication 1, comprenant :
a) 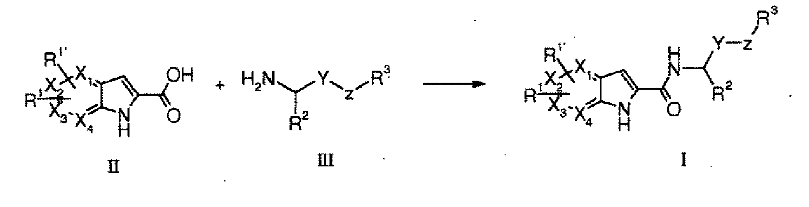 le couplage d'un acide pyrrolopyridine-2-carboxylique de la formule (II), ou d'un dérivé protégé ou activé de celui-ci, avec une amine de la formule (III) ; ou
b) 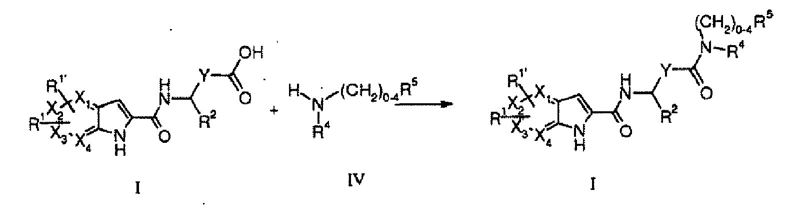 pour des composés de la formule (I) dans laquelle Z représente un groupe C=O et R³ représente un groupe - NR⁴R⁵ ou un groupe -NR⁴(-alkyle de C₁ à C₄-R⁵), le couplage d'un acide carboxylique de la formule (I), ou d'un dérivé protégé ou activé de celui-ci, dans laquelle Z est absent et R³ représente un groupe -CO₂H, avec une amine de la formule (IV) ; ou
c) 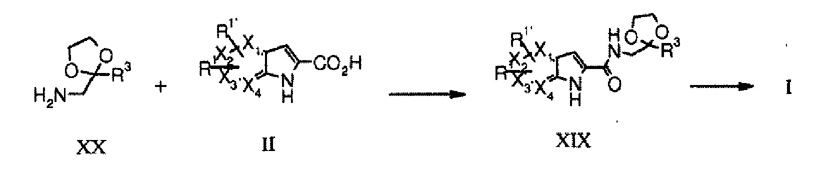 pour des composés de la formule (III), dans laquelle R² représente un atome de H, Y représente une liaison directe, Z représente un groupe -C(O)- et R³ représente -aryle ou -hétaryle, le couplage d'un composé de la formule (XX) avec un composé de la formule (II) dans des conditions de couplage standard pour obtenir un composé de la formule (XIX), suivi de l'élimination du groupe cétal en présence d'un acide ; ou
d) 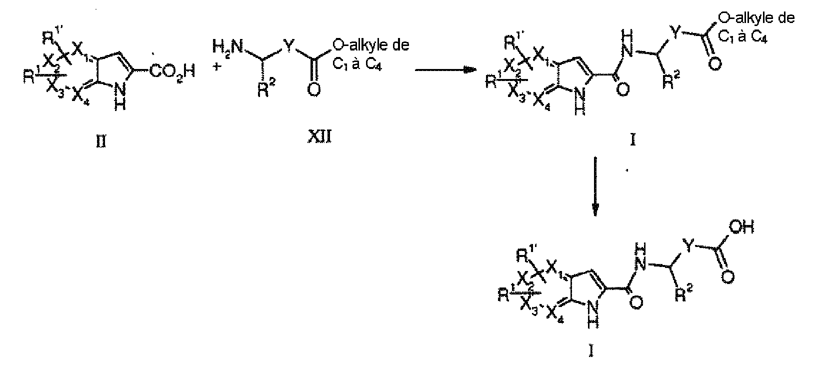 pour des composés de la formule (I), dans laquelle Z représente un groupe C=O et R³ représente un groupe alcoxy de C₁ à C₄, le couplage d'un composé de la formule (II), ou d'un dérivé protégé ou activé de celui-ci, et d'un composé de la formule (XII) ; ou
e) pour des composés de la formule (I), dans laquelle Z est absent et R³ représente un groupe -CO₂H, une hydrolyse de l'ester des composés de la formule (I) dans laquelle Z représente un groupe C=O et R³ représente un groupe alcoxy de C₁ à C₄.

30. Composé choisi parmi les composés suivants :
Acide 5-chloro-1H-pyrrolo[3,2-b]pyridine-2-carboxylique ;
Acide 5-bromo-1H-pyrrolo[3,2-b]pyridine-2-carboxylique ;
Acide 5-cyano-1H-pyrrolo[3,2-b]pyridine-2-carboxylique ;
Acide 5-méthoxy-1H-pyrrolo[3,2-b]pyridine-2-carboxylique ;
Acide 1H-pyrrolo[3,2-c]pyridine-2-carboxylique ;
Acide 6-chloro-1H-pyrrolo[3,2-c]pyridine-2-carboxylique ;
Acide 6-cyano-1H-pyrrolo[3,2-c]pyridine-2-carboxylique ;
Acide 5-chloro-1H-pyrrolo[2,3-c]pyridine-2-carboxylique ;
Acide 5-bromo-1H-pyrrolo[2,3-c]pyridine-2-carboxylique ;
Acide 5-éthynyl-1H-pyrrolo[2,3-c]pyridine-2-carboxylique ;
Acide 5-cyano-1H-pyrrolo[2,3-c]pyridine-2-carboxylique ;
Acide 5-méthyl-1H-pyrrolo[2,3-c]pyridine-2-carboxylique ;
Acide 5-chloro-1H-pyrrolo[2,3-b]pyridine-2-carboxylique ;
Acide 6-chloro-1H-pyrrolo[2,3-b]pyridine-2-carboxylique ; et
Acide 6-cyano-1H-pyrrolo[2,3-b]pyridine-2-carboxylique ;
ou un ester d'alkyle de C₁ à C₄ de l'un quelconque de ceux-ci.

31. Composé de la formule (XIX) : 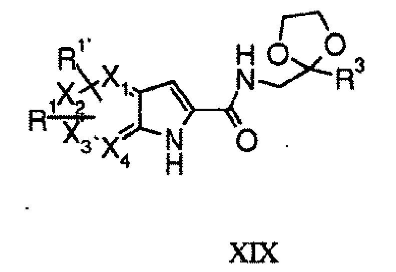 dans laquelle R¹, R¹', X₁, X₂, X₃ et X₄ sont tels que définis dans la revendication 1 et R³ représente un groupe -aryle ou un groupe -hétaryle.

32. 4(S)-(4-fluorobenzyl)oxazolidine-2,5-dione.
